(19) Europäisches Patentamt
European Patent Office
Office européen des brevets

(11) EP 3 183 343 B1

(12) EUROPEAN PATENT SPECIFICATION

(45) Date of publication and mention of the grant of the patent:
**01.01.2020 Bulletin 2020/01**

(21) Application number: **15754188.9**

(22) Date of filing: **20.08.2015**

(51) Int Cl.:
***C12N 15/09*** *(2006.01)*

(86) International application number:
**PCT/EP2015/069177**

(87) International publication number:
**WO 2016/026938 (25.02.2016 Gazette 2016/08)**

# (54) RECOMBINASE-MEDIATED INTEGRATION OF A POLYNUCLEOTIDE LIBRARY

REKOMBINASE-VERMITTELTE INTEGRATION EINER POLYNUKLEOTIDBIBLIOTHEK

INTÉGRATION MÉDIÉE PAR RECOMBINASE D'UNE BANQUE DE POLYNUCLÉOTIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2014 EP 14181694**

(43) Date of publication of application:
**28.06.2017 Bulletin 2017/26**

(73) Proprietor: **Novozymes A/S**
**2880 Bagsvaerd (DK)**

(72) Inventors:
- **MATSUI, Tomoko**
  **Chiba-shi**
  **Chiba 261-8501 (JP)**
- **UDAGAWA, Hiroaki**
  **Chiba-shi**
  **Chiba 261-8501 (JP)**
- **KISHISHITA, Seiichiro**
  **DK-2880 Bagsvaerd (DK)**
- **SKOVLUND, Dominique, Aubert**
  **DK-2880 Bagsvaerd (DK)**
- **JIN, Qiming**
  **Davis, California 95618 (US)**

(74) Representative: **NZ EPO Representatives**
**Krogshoejvej 36**
**2880 Bagsvaerd (DK)**

(56) References cited:
**WO-A1-2012/160093**

- **KATARINA KOPKE ET AL: "Application of the Saccharomyces cerevisiae FLP/FRT Recombination System in Filamentous Fungi for Marker Recycling and Construction of Knockout Strains Devoid of Heterologous Genes", APPLIED AND ENVIRONMENTAL MICROBIOLOGY, AMERICAN SOCIETY FOR MICROBIOLOGY, US, vol. 76, no. 14, 1 July 2010 (2010-07-01), pages 4664-4674, XP002660924, ISSN: 0099-2240, DOI: 10.1128/AEM.00670-10 [retrieved on 2010-05-14]**
- **GRAVELAT FABRICE N ET AL: "Targeted gene deletion in Aspergillus fumigatus using the hygromycin-resistance split-marker approach.", METHODS IN MOLECULAR BIOLOGY (CLIFTON, N.J.) 2012, vol. 845, 2012, pages 119-130, XP009186340, ISSN: 1940-6029**
- **MIZUTANI O ET AL: "Modified Cre-loxP recombination in Aspergillus oryzae by direct introduction of Cre recombinase for marker gene rescue", APPLIED AND ENVIRONMENTAL MICROBIOLOGY, AMERICAN SOCIETY FOR MICROBIOLOGY, US, vol. 78, no. 12, 1 June 2012 (2012-06-01), pages 4126-4133, XP002737423, ISSN: 1098-5336, DOI: 10.1128/AEM.00080-12 [retrieved on 2012-04-13]**
- **THOMAS HARTMANN ET AL: "Validation of a Self-Excising Marker in the Human Pathogen Aspergillus fumigatus by Employing the beta-Rec/six Site-Specific Recombination System", APPLIED AND ENVIRONMENTAL MICROBIOLOGY, AMERICAN SOCIETY FOR MICROBIOLOGY, US, vol. 76, no. 18, 1 September 2010 (2010-09-01), pages 6313-6317, XP002660922, ISSN: 0099-2240, DOI: 10.1128/AEM.00882-10 [retrieved on 2010-07-23]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 183 343 B1

Printed by Jouve, 75001 PARIS (FR)

## Description

### Field of the Invention

The present invention relates to methods for integrating a polynucleotide library of interest in the chromosome of a filamentous fungal host cell using a site-specific recombinase, polynucleotide library expression systems comprising a host cell and a polynucleotide construct, as well as the resulting filamentous fungal host cells comprising a polynucleotide library and their cultivation to produce a polypeptide.

### Background of the Invention

A large number of naturally-occurring organisms have been found to produce useful polypeptide products, e.g., enzymes, the large scale production of which is desirable for research and commercial purposes.

Construction of host cells has been described, wherein a highly expressed chromosomal gene is replaced with a recognition sequence of a site-specific recombinase to allow subsequent insertion of a single product-encoding polynucleotide into that site by the use of a recombinase recognizing said sequence (EP 1 405 908 A1; ProBioGen AG).

It has been disclosed to insert DNA at a known location in the genome by making use of site-specific recombination systems that are freely reversible. These reversible systems include the following: the Cre-*lox* system from bacteriophage P1; the FLP-*FRT* system of Saccharomyces cerevisiae; the R-*RS* system of Zygosaccharonzyces rouxii; a modified Gin-*gix* system from bacteriophage Mu; the beta-recombinase-six system from a *Bacillus subtilis* plasmid and the delta-gamma-res system from the bacterial transposon *Tn*1000. Cre, FLP, R, Gin, beta-recombinase and gamma-delta are the recombinases, and *lox, FRT, RS, gix, six* and *res* are the respective recombination sites (reviewed by Sadowslu, 1993 FASEB J., 7:750-67; Ow and Medberry, 1995 Crit. Rev. Plant Sci. 14: 239-261).

The site-specific recombination systems above have in common the property that a single polypeptide recombinase catalyzes the recombination between two recognition sites of identical or nearly identical sequences.

When cloning and screening polynucleotides encoding polypeptides of interest in a fungal host cell, especially when screening polynucleotide libraries encoding variants of the same secreted polypeptide of interest for an improved property, it is desirable that the fungal host cells express the polynucleotides at a comparable level, so that their properties can be directly assayed without having to perform a normalization step first. This problem has been addressed, e.g., in EP 1124949, by using cloning and expression vectors comprising a fungal replication initiation sequence, such as, the well-known AMA1 sequence.

### Summary of the Invention

We provide herein an effective way of quickly and site-specifically inserting a polynucleotide library encoding polypeptides of interest in the chromosome of a filamentous fungal host, thereby achieving highly uniform expression levels in the transformants due to the presence of the inserted genes in just one single copy in the exact same genomic position in each transformed and selected host cell.

The resulting selected transformed cell may even be employed directly to produce the encoded selected polypeptide with improved up-scaled expression levels that correspond to those of the initial screening.

Accordingly, in a first aspect, the present invention relates to methods for integrating a polynucleotide library of interest in the chromosome of a filamentous fungal host cell using a site-specific recombinase, said method comprising the steps of:

a) providing a filamentous fungal host cell comprising in its chromosome in the following order:

  i) a first recognition sequence of the recombinase or a region that is 5' or 3' of an integration site;
  ii) a first selection marker;
  iii) a second recognition sequence of the recombinase; and optionally
  iv) a non-functional partial second selection marker;

b) transforming said host cell with a nucleic acid construct comprising in the following order:

  i) the first recognition sequence of the recombinase or the region that is 5' or 3' of the integration site;
  ii) a polynucleotide library of interest;
  iii) a second selection marker OR a non-functional partial second selection marker if the corresponding but optional non-functional second selection marker of step (a)(iv) is comprised in the host cell chromosome; and
  iv) the second recognition sequence of the recombinase;

c) expressing a gene encoding the site-specific recombinase in said host cell; and
d) selecting a transformed host cell which expresses the second selection marker and not the first selection marker,

wherein the polynucleotide library of interest is site-specifically integrated in the correct orientation in the chromosome of the host cell by the recombinase, whereby the first selectable marker is excised from the chromosome and whereby any non-functional partial second selectable markers are recombined to form a functional second selection marker in the chromosome.

In a second aspect, the invention relates to polynucleotide library expression systems comprising:

a) a filamentous fungal host cell comprising in its chromosome in the following order:

i) a first recognition sequence of a site-specific recombinase or a region that is 5' or 3' of an integration site;
ii) a first selection marker;
iii) a second recognition sequence of the recombinase; and optionally
iv) a non-functional partial second selection marker; AND

b) a nucleic acid construct comprising in the following elements in order:

i) the first recognition sequence of the recombinase or the region that is 5' or 3' of the integration site;
ii) a polynucleotide library of interest;
iii) a second selection marker OR a non-functional partial second selection marker if the optional non-functional second selection marker of step (a)(iv) is comprised in the host cell chromosome; and
iv) the second recognition sequence of the recombinase, and

c) a gene encoding the site-specific recombinase comprised in the filamentous fungal host cell; preferably in the chromosome, in a second nucleic acid construct, or in the nucleic acid construct outside of the elements listed in step (b);

wherein, when the host cell is transformed with the nucleic acid construct(s), the polynucleotide library of interest is site-specifically integrated in the correct orientation in the chromosome of the host cell by the recombinase, whereby the first selectable marker is excised from the chromosome, and whereby the second selection marker is also integrated and expressed or any non-functional partial second selectable markers are recombined to form a functional second selection marker in the chromosome which is expressed.

In a third aspect, the invention relates to the resulting filamentous fungal host cells comprising in its chromosome in the following order:

i) a first recognition sequence of a site-specific recombinase or a region that is 5' or 3' of an integration site;
ii) a polynucleotide library of interest; and either
iii) a selection marker and a second recognition sequence of the recombinase; or
iv) a first partial selection marker, a second recognition sequence of the recombinase and a second partial selection marker,

wherein the host cell expresses the polynucleotide library and the selection marker.

In a final aspect, the invention relates to a method of producing a polypeptide of interest, comprising the steps of:

a) cultivating a filamentous fungal host cell of the third aspect under conditions conducive to produce the polypeptide encoded by the polynucleotide library, and; optionally
b) recovering the polypeptide.

## Brief Description of the Figures

Figure 1 shows a schematic drawing of vector p002.
Figure 2 shows a schematic drawing of vector pFRT-GIAMG.
Figure 3 shows a schematic drawing of vector p007.
Figure 4 shows a schematic drawing of vector pFRT-BsAMG.
Figure 5 shows a schematic drawing of vector pDAu571.

Figure 6 shows a schematic drawing of vector pDAu703.
Figure 7 shows schematic drawings of:

- Top part: the *amy*2-region of the chromosome in *A. oryzae* host strain DAu716, where two FRT-sites have been inserted;
- Middle part: the linearized vector pDAu724; and
- Bottom part: the *amy*2-region of the chromosome in *A. oryzae* host strain DAu716 after FLP-mediated integration of pDAu724 by double-homologous recombination between the respective FRT-sites.

Figure 8 shows a schematic drawing of vector pDLHD0075.
Figure 9 shows a restriction map of plasmid pJfyS156.
Figure 10 shows a restriction map of pQM43.
Figure 11 shows a restriction map of pQM45.

## Definitions

**Cytosine deaminase:** Cytosine deaminase (EC 3.5.4.1) catalyzes the deamination of cytosine and 5-fluorocytosine (5FC) to form uracil and toxic 5-fluorouracil (5FU), respectively. When genetically modified cells comprising cytosine deaminase are combined with 5FC it is converted to toxic 5FU, so the cytosine deaminase-encoding gene is potentially a potent negative selection marker. It has also been shown that an inhibitor in the pyrimidine *de novo* synthesis pathway can be utilized to create a condition in which cells are dependent on the conversion of pyrimidine supplements to uracil by cytosine deaminase. Thus, only cells expressing the cytosine deaminase gene can be rescued in a positive selection medium comprising an inhibitor of the pyrimidine *de novo* synthesis as well as inosine and cytosine (See figure 1 of Wei and Huber, 1996, J Biol Chem 271(7): 3812). The inhibitor is preferably N-(phosphonacetyl)-L-aspartate (PALA), which inhibits aspartate carbamyl transferase. If necessary, cytosine deaminase activity may be quantitated by a genetic assay (Frederico L.A. et al, 1990, Biochemistry 29: 2532-2537).

**Allelic variant:** The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Catalytic domain:** The term "catalytic domain" means the region of an enzyme containing the catalytic machinery of the enzyme.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Control sequences:** The term "control sequences" means nucleic acid sequences necessary for expression of a polynucleotide encoding a mature polypeptide of the present invention. Each control sequence may be native (*i.e.,* from the same gene) or foreign (*i.e.,* from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Fragment:** The term "fragment" means a polypeptide or a catalytic or binding domain having one or more (e.g., several) amino acids absent from the amino and/or carboxyl terminus of a mature polypeptide or domain; wherein the fragment has retained its catalytic or binding activity.

**Host cell:** The term "host cell" means any cell type that is susceptible to transformation, transfection, transduction, or the like with a nucleic acid construct or expression vector comprising a polynucleotide of the present invention.

The term "host cell" encompasses any progeny of a parent cell that is not identical to the parent cell due to mutations that occur during replication.

**Improved property:** The term "improved property" means a characteristic associated with a variant that is improved compared to the parent. In the present invention, the improved property is increased expression yield of a variant relative to the parent.

**Increased expression yield:** The term "increased expression yield" means a higher amount (g) of secreted enzyme per liter of culture medium from cultivation of a host cell expressing the variant gene relative to the amount (g) of secreted active enzyme per liter produced under the same cultivation conditions by the same host cell expressing the parent gene. In one aspect, the variant has an increased expression yield compared to the parent enzyme of at least 1.05, at least 1.10, at least 1.20, at least 1.30, at least 1.40, at least 1.50, at least 1.60, at least 1.70, at least 1.80, at least 1.90, at least 2, at least 2.25, at least 2.50, at least 2.75, at least 3.00, at least 3.25, at least 3.50, at least 3.75, at least 4, at least 4.25, at least 4.50, at least 4.75, at least 5, at least 6, at least 7, at least 8, at least 9, or at least 10 fold.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., recombinant production in a host cell; multiple copies of a gene encoding the substance; and use of a stronger promoter than the promoter naturally associated with the gene encoding the substance).

**Mature polypeptide:** The term "mature polypeptide" means a polypeptide in its final form following translation and any post-translational modifications, such as N-terminal processing, C-terminal truncation, glycosylation, phosphorylation, etc. It is known in the art that a host cell may produce a mixture of two of more different mature polypeptides *(i.e.,* with a different C-terminal and/or N-terminal amino acid) expressed by the same polynucleotide. It is also known in the art that different host cells process polypeptides differently, and thus, one host cell expressing a polynucleotide may produce a different mature polypeptide (*e.g.*, having a different C-terminal and/or N-terminal amino acid) as compared to another host cell expressing the same polynucleotide.

**Mature polypeptide coding sequence:** The term "mature polypeptide coding sequence" means a polynucleotide that encodes a mature polypeptide having catalytic or binding activity.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues x } 100)/(\text{Length of Alignment} - \text{Total Number of Gaps in Alignment})$$

For purposes of the present invention, the sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra*), preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$(\text{Identical Deoxyribonucleotides x } 100)/(\text{Length of Alignment} - \text{Total Number of Gaps in Alignment})$$

**Subsequence:** The term "subsequence" means a polynucleotide having one or more (*e.g.*, several) nucleotides absent from the 5' and/or 3' end of a mature polypeptide coding sequence; wherein the subsequence encodes a fragment having catalytic or binding activity.

**Variant:** The term "variant" means a polypeptide having catalytic or binding activity comprising an alteration, *i.e.,* a substitution, insertion, and/or deletion, at one or more (*e.g.*, several) positions. A substitution means replacement of the amino acid occupying a position with a different amino acid; a deletion means removal of the amino acid occupying a position; and an insertion means adding an amino acid adjacent to and immediately following the amino acid occupying a position. Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly. Alternatively, the amino acid changes are of such a nature that the physico-chemical properties of the polypeptides are altered. For example, amino acid changes may improve the thermal stability of the polypeptide, alter the substrate specificity, change the pH optimum, and the like. Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide. Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.*, Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

## Detailed Description of the Invention

The present invention relates to methods for integrating a polynucleotide library of interest in the chromosome of a filamentous fungal host cell using a site-specific recombinase, polynucleotide library expression systems comprising a host cell and a polynucleotide construct, as well as the resulting filamentous fungal host cells comprising a polynucleotide library and their cultivation to produce a polypeptide.

In a preferred embodiment of the invention, the polynucleotide library is a gene library that comprises polynucleotides encoding variants of a polypeptide of interest; preferably the polypeptide of interest is an enzyme; more preferably the polypeptide of interest is a hydrolase, isomerase, ligase, lyase, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, oxidase, pectinolytic enzyme, peroxidase, phytase, polyphenoloxidase, proteolytic enzyme, ribonuclease, transglutaminase, xylanase, or beta-xylosidase.

In another preferred embodiment, the polynucleotide library comprises a library of control sequences, such as, a library of promoters, pro-regions, secretion signals, or terminators.

It is preferred that the filamentous fungal host cell of the invention is an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell; preferably the host cell is an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

In a preferred embodiment of the invention, the site specific recombinase is the *Saccharomyces cerevisiae* 2 μm flippase FLP, the phage TP901-1 integrase, the bacteriophage P1 CRE integrase, the bacterial XerC recombinase, the bacterial XerD recombinase, the lambda phage integrase or the HP1 integrase; preferably the site specific recombinase is the *Saccharomyces cerevisiae* 2 μm flippase FLP.

Two separate strategies may be applied to ensure that chromosomal integration in the chromosome of the host cell takes place in the correct orientation. If identical first and second recognition sequences are used, then the integration is likely to happen in both orientations, but the second selection marker may be tailored so that only correctly integrated fragments result in the expression of the second selection marker. On the other hand, it is also an option to use different first and second recognition sequences, for example, FRT-F and FRT-F3, in order to ensure that the fragment is integrated in a specific orientation. One aspect of the invention relates to the use of *in vivo* homologous recombination between a region of the nucleic acid construct that is transformed into the host cell with a region that is 5' or 3' of an intended integration site in the chromosome in combination with a recognition sequence of a site-specific recombinase in order to ensure that the polynucleotide library of interest is integrated in a specific orientation in the chromosome via the *in vivo* homologous recombinatian in combination with the site-specific recombination effected by the recombinase.

Accordingly, it is preferred that the first and second recognition sequences of the recombinase are identical or different; preferably the first and second recognition sequences of the recombinase are different in order to effect a directional integration of the polynucleotide library of interest in the host cell chromosome. Preferably, the first and second recognition sequences of the recombinase are different recognition sequences of the *Saccharomyces cerevisiae* 2 μm flippase FLP in order to effect a directional integration of the polynucleotide library of interest in the host cell chromosome; preferably the first and second recognition sequences of the recombinase are FRT-F (SEQ ID NO:27) and FRT-F3 (SEQ ID NO:28), respectively, or vice versa.

On the other hand, it is also preferably that one non-functional partial second selection marker is comprised in the host cell of the first aspect in step (a) and another non-functional partial second selection marker is comprised in the nucleic acid construct of the first aspect in step (b), wherein the partial second selection markers are recombined to form a functional second selection marker when the polynucleotide library of interest is site-specifically integrated in the correct orientation in the chromosome of the host cell by the recombinase via its recognition sequences; preferably one non-functional partial second selection marker comprises a promoter and, optionally, one or more intact 5' exon of a polynucleotide encoding a selection marker, and, the other non-functional partial second selection marker comprises the remaining coding sequence of the selection marker.

In a preferred embodiment of the second aspect of the invention, one non-functional partial second selection marker is comprised in the host cell and another non-functional partial second selection marker is comprised in the nucleic acid construct, wherein the partial second selection markers are recombined to form a functional second selection marker when the polynucleotide library of interest is site-specifically integrated in the correct orientation in the chromosome of the host cell by the recombinase via its recognition sequences; preferably one non-functional partial second selection marker comprises a promoter and, optionally, one or more intact 5' exon of a polynucleotide encoding a selection marker, and, wherein the other non-functional partial second selection marker comprises the remaining coding sequence of the selection marker.

In a preferred embodiment of the second aspect, the filamentous fungal host cell is transformed.

In a preferred embodiment of the third aspect, the first and second recognition sequences of the recombinase are identical or different; preferably the first and second recognition sequences of the recombinase are different.

In another preferred embodiment of the third aspect, the first and second recognition sequences of the recombinase are different recognition sequences of the *Saccharomyces cerevisiae* 2 μm flippase FLP; preferably the first and second recognition sequences of the recombinase are FRT-F (SEQ ID NO:27) and FRT-F3 (SEQ ID NO:28), respectively, or vice versa.

In another preferred embodiment, a region that is 5' or 3' of the integration site, *i.e.,* that flanks 5' or 3' of the integration site, is used together with the second recognition sequence to effect directional integration of the polynucleotide library of interest at the integration site.

In a final preferred embodiment of the third aspect, the second recognition sequence of the recombinase is located in an intron separating the first partial selection marker from the second partial selection marker, as outlined in figure 7.

**Sources of Polypeptides Having Enzyme Activity**

A polypeptide having enzyme activity of the present invention may be obtained from microorganisms of any genus. For purposes of the present invention, the term "obtained from" as used herein in connection with a given source shall mean that the polypeptide encoded by a polynucleotide is produced by the source or by a strain in which the polynucleotide from the source has been inserted. In one aspect, the polypeptide obtained from a given source is secreted extracellularly.

The polypeptide may be a bacterial polypeptide. For example, the polypeptide may be a Gram-positive bacterial polypeptide such as a *Bacillus, Clostridium, Enterococcus, Geobacillus, Lactobacillus, Lactococcus, Oceanobacillus, Staphylococcus, Streptococcus,* or *Streptomyces* polypeptide having [enzyme] activity, or a Gram-negative bacterial polypeptide such as a *Campylobacter, E. coli, Flavobacterium, Fusobacterium, Helicobacter, Ilyobacter*, *Neisseria, Pseudomonas, Salmonella,* or *Ureaplasma* polypeptide.

In one aspect, the polypeptide is a *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis* polypeptide.

In another aspect, the polypeptide is a *Streptococcus equisimilis*, *Streptococcus pyogenes, Streptococcus uberis,* or *Streptococcus equi* subsp. *Zooepidemicus* polypeptide.

In another aspect, the polypeptide is a *Streptomyces achromogenes, Streptomyces avermitilis, Streptomyces coelicolor, Streptomyces griseus,* or *Streptomyces lividans* polypeptide.

The polypeptide may be a fungal polypeptide. For example, the polypeptide may be a yeast polypeptide such as a *Candida, Kluyveromyces, Pichia, Saccharomyces, Schizosaccharomyces,* or *Yarrowia* polypeptide; or a filamentous fungal polypeptide such as an *Acremonium, Agaricus, Alternaria, Aspergillus, Aureobasidium, Botryospaeria, Ceriporiopsis*, *Chaetomidium, Chrysosporium, Claviceps, Cochliobolus, Coprinopsis, Coptotermes, Corynascus*, *Cryphonectria*, *Cryptococcus, Diplodia, Exidia, Filibasidium, Fusarium, Gibberella, Holomastigotoides, Humicola, Irpex, Lentinula, Leptospaeria, Magnaporthe, Melanocarpus, Meripilus, Mucor, Myceliophthora, Neocallimastix, Neurospora, Paecilomyces, Penicillium, Phanerochaete, Piromyces, Poitrasia, Pseudoplectania, Pseudotrichonympha, Rhizomucor, Schizophyllum, Scytalidium, Talaromyces, Thermoascus, Thielavia, Tolypocladium, Trichoderma, Trichophaea, Verticillium, Volvariella,* or *Xylaria* polypeptide.

In another aspect, the polypeptide is a *Saccharomyces carlsbergensis, Saccharomyces cerevisiae, Saccharomyces diastaticus, Saccharomyces douglasii, Saccharomyces kluyveri, Saccharomyces norbensis,* or *Saccharomyces oviformis* polypeptide.

In another aspect, the polypeptide is an *Acremonium cellulolyticus, Aspergillus aculeatus, Aspergillus awamori, Aspergillus foetidus, Aspergillus fumigatus, Aspergillus japonicus, Aspergillus nidulans, Aspergillus niger, Aspergillus oryzae, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola grisea, Humicola insolens, Humicola lanuginosa, Irpex lacteus, Mucor miehei, Myceliophthora thermophila, Neurospora crassa, Penicillium funiculosum, Penicillium purpurogenum, Phanerochaete chrysosporium*, *Thielavia achromatica, Thielavia albomyces, Thielavia albopilosa, Thielavia australeinsis, Thielavia fimeti, Thielavia microspora, Thielavia ovispora, Thielavia peruviana, Thielavia setosa, Thielavia spededonium, Thielavia subthermophila, Thielavia terrestris, Trichoderma harzianum, Trichoderma koningii, Trichoderma longibrachiatum, Trichoderma reesei,* or *Trichoderma viride* polypeptide.

It will be understood that for the aforementioned species, the invention encompasses both the perfect and imperfect states, and other taxonomic equivalents, e.g., anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

The polypeptide may be identified and obtained from other sources including microorganisms isolated from nature (*e.g.*, soil, composts, water, etc.) or DNA samples obtained directly from natural materials (*e.g.*, soil, composts, water, etc.) using the above-mentioned probes. Techniques for isolating microorganisms and DNA directly from natural habitats are well known in the art. A polynucleotide encoding the polypeptide may then be obtained by similarly screening a genomic DNA or cDNA library of another microorganism or mixed DNA sample. Once a polynucleotide encoding a polypeptide has been detected with the probe(s), the polynucleotide can be isolated or cloned by utilizing techniques that are known to those of ordinary skill in the art (see, *e.g.*, Sambrook *et al.,* 1989, *supra).*

**Nucleic Acid Constructs**

The present invention also relates to nucleic acid constructs comprising a polynucleotide of the present invention operably linked to one or more control sequences that direct the expression of the coding sequence in a suitable host cell under conditions compatible with the control sequences.

The polynucleotide may be manipulated in a variety of ways to provide for expression of the polypeptide. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase *(glaA), Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and mutant, truncated, and hybrid promoters thereof. Other promoters are described in U.S. Patent No. 6,011,147.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

The control sequence may also be an mRNA stabilizer region downstream of a promoter and upstream of the coding sequence of a gene which increases expression of the gene.

The control sequence may also be a leader, a nontranslated region of an mRNA that is important for translation by the host cell. The leader is operably linked to the 5'-terminus of the polynucleotide encoding the polypeptide. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase *Aspergillus oryzae* TAKA amylase, and *Fusarium oxysporum* trypsin-like protease.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide or prepropetide coding sequence may be obtained from the genes for *Bacillus subtilis* alkaline protease *(aprE), Bacillus subtilis* neutral protease *(nprT), Myceliophthora thermophila* laccase (WO 95/33836), *Rhizomucor miehei* aspartic proteinase, *Humicola insolens* cutinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence.

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked to the regulatory sequence.

### Polynucleotide constructs

The present invention also relates to recombinant polynucleotide constructs or expression vectors comprising a polynucleotide of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (*e.g.,* a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.,* a vector that exists as an extrachromosomal entity, the replication of which is independent of chromosomal replication, *e.g.,* a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Selectable markers for use in a filamentous fungal host cell include, but are not limited to, *adeA* (phosphoribosylaminoimidazole-succinocarboxamide synthase), *adeB* (phosphoribosyl-aminoimidazole synthase), *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar* gene. Preferred for use in a *Trichoderma* cell are *adeA*, *adeB*, *amdS*, *hph*, and *pyrG* genes.

The selectable marker may be a dual selectable marker system as described in WO 2010/039889. In one aspect, the dual selectable marker is an *hph-tk* dual selectable marker system.

The vector preferably contains an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, *e.g.,* Sambrook *et al*., 1989, *supra).*

## Host Cells

The present invention also relates to recombinant host cells, comprising a polynucleotide of the present invention operably linked to one or more control sequences that direct the production of a polypeptide of the present invention. A construct or vector comprising a polynucleotide is introduced into a host cell so that the construct or vector is maintained as a chromosomal integrant.

The term "host cell" encompasses any progeny of a parent cell that is not identical to the parent cell due to mutations that occur during replication. The choice of a host cell will to a large extent depend upon the gene encoding the polypeptide and its source.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a filamentous fungal cell. "Filamentous fungi" include all filamentous forms of the subdivision Eumycota and Oomycota (as defined by Hawksworth *et al*., 1995, *supra).* The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic.

The filamentous fungal host cell may be an *Acremonium*, *Aspergillus*, *Aureobasidium*, *Bjerkandera, Ceriporiopsis*, *Chrysosporium*, *Coprinus*, *Coriolus*, *Cryptococcus*, *Filibasidium*, *Fusarium, Humicola, Magnaporthe, Mucor, Myceliophthora, Neocallimastix, Neurospora, Paecilomyces, Penicillium, Phanerochaete, Phlebia, Piromyces, Pleurotus,*

*Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus*, *Aspergillus fumigatus*, *Aspergillus japonicus*, *Aspergillus nidulans*, *Aspergillus niger*, *Aspergillus oryzae*, *Bjerkandera adusta*, *Ceriporiopsis aneirina*, *Ceriporiopsis caregiea*, *Ceriporiopsis gilvescens*, *Ceriporiopsis pannocinta*, *Ceriporiopsis rivulosa*, *Ceriporiopsis subrufa*, *Ceriporiopsis subvermispora*, *Chrysosporium inops, Chrysosporium keratinophilum*, *Chrysosporium lucknowense*, *Chrysosporium merdarium*, *Chrysosporium pannicola, Chrysosporium queenslandicum, Chrysosporium tropicum, Chrysosporium zonatum, Coprinus cinereus*, *Coriolus hirsutus*, *Fusarium bactridioides*, *Fusarium cerealis*, *Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum*, *Fusarium torulosum*, *Fusarium trichothecioides*, *Fusarium venenatum*, *Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787.

## Methods of Production

The present invention also relates to methods of producing a polypeptide of interest, comprising the steps of a) cultivating a filamentous fungal host cell of the third aspect under conditions conducive to produce the polypeptide encoded by the polynucleotide library, and; optionally, b) recovering the polypeptide.

The host cells are cultivated in a nutrient medium suitable for production of the polypeptide using methods known in the art. For example, the cells may be cultivated by shake flask cultivation, or small-scale or large-scale fermentation (including continuous, batch, fed-batch, or solid state fermentations) in laboratory or industrial fermentors in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.,* in catalogues of the American Type Culture Collection). If the polypeptide is secreted into the nutrient medium, the polypeptide can be recovered directly from the medium. If the polypeptide is not secreted, it can be recovered from cell lysates.

The polypeptide may be detected using methods known in the art that are specific for the polypeptides. These detection methods include, but are not limited to, use of specific antibodies, formation of an enzyme product, or disappearance of an enzyme substrate. For example, an enzyme assay may be used to determine the activity of the polypeptide.

The polypeptide may be recovered using methods known in the art. For example, the polypeptide may be recovered from the nutrient medium by conventional procedures including, but not limited to, collection, centrifugation, filtration, extraction, spray-drying, evaporation, or precipitation. In one aspect, a fermentation broth comprising the polypeptide is recovered.

The polypeptide may be purified by a variety of procedures known in the art including, but not limited to, chromatography (*e.g.,* ion exchange, affinity, hydrophobic, chromatofocusing, and size exclusion), electrophoretic procedures (*e.g.,* preparative isoelectric focusing), differential solubility (*e.g.,* ammonium sulfate precipitation), SDS-PAGE, or extraction (see, *e.g.,* Protein Purification, Janson and Ryden, editors, VCH Publishers, New York, 1989) to obtain substantially pure polypeptides.

In an alternative aspect, the polypeptide is not recovered, but rather a host cell of the present invention expressing the polypeptide is used as a source of the polypeptide.

The present invention is further described by the following examples that should not be construed as limiting the scope of the invention.

## EXAMPLES

Molecular cloning techniques are described in Sambrook, J., Fritsch, E.F., Maniatis, T. 30 (1989) Molecular cloning: a laboratory manual (2nd edn.) Cold Spring Harbor Laboratory, Cold Spring Harbor, New York.

Enzymes for DNA manipulations (e.g. restriction endonucleases, ligases etc.) were obtained from New England Biolabs, Inc. and were used according to the manufacturer's instructions.

Media and reagents

The following media and reagents were used unless otherwise specified; chemicals used for buffers and substrates were commercial products of analytical grade:

- Cove: 342.3 g/L Sucrose, 20 ml/L COVE salt solution, 10mM Acetamide, 30 g/L noble agar.
- Cove top agar: 342.3 g/L Sucrose, 20 ml/L COVE salt solution, 10mM Acetamide, 10 g/L low melt agarose.
- Cove-N plates are composed of 30 g sucrose, 20 ml Cove salt solution, 3g $NaNO_3$, and 30 g noble agar and water to 1 litre.
- COVE salt solution is composed of 26 g KCl, 26 g $MgSO_4$ $7H_20$, 76 g $KH_2PO_4$ and 50ml Cove trace metals and water to 1 litre.
- Trace metal solution for COVE is composed of 0.04 g $NaB_40_7$ $10H_20$, 0.4 g $CuSO_4$ $5H_20$, 1.2 g $FeSO_47H_20$, 1.0 g $MnSO_4$ $H_20$, 0.8 g Neutral amylase II $Mo0_{22}H_20$, and 10.0 g $ZnSO_4$ $7H_20$ and water to 1 litre.
- ¼ YPM is composed of 2.5 g yeast extract, 5 g pepton and 5 g maltose (pH 4.5) and water to 1 litre.
- STC buffer is composed of 0.8 M sorbitol, 25 mM Tris (pH 8), and 25 mM CaCl2 and water to 1 litre.
- STPC buffer is composed of 40% PEG4000 in STC buffer.
- MLC is composed of 40 g Glucose, 50 g Soybean powder, 4 g/ Citric acid (pH 5.0) and water to 1 litre.
- Cellulase-inducing medium (CIM) was composed of 20 g of cellulose, 10 g of corn steep solids, 1.45 g of $(NH_4)_2SO_4$, 2.08 g of $KH_2PO_4$, 0.28 g of $CaCl_2$, 0.42 g of $MgSO_4{\cdot}7H_2O$, 0.42 ml of *Trichoderma* trace metals solution, 1-2 drops of antifoam, and deionized water to 1 liter; pH adjusted to 6.0.
- COVE plates were composed of 342.3 g of sucrose, 20 ml of COVE salt solution, 10 ml of 1 M acetamide, 10 ml of 1.5 M CsCl, 25 g of Noble agar (Difco), and deionized water to 1 liter.
- COVE salt solution was composed of 26 g of KCl, 26 g of $MgSO_4{\cdot}7H_2O$, 76 g of $KH_2PO_4$, 50 ml of COVE trace metals solution, and deionized water to 1 liter.
- COVE trace metals solution was composed of 0.04 g of $NaB_4O_7{\cdot}10H_2O$, 0.4 g of $CuSO_4{\cdot}5H_2O$, 1.2 g of $FeSO_4{\cdot}7H_2O$, 0.7 g of $MnSO_4{\cdot}H_2O$, 0.8 g of $Na_2MoO_2{\cdot}2H_2O$, 10 g of $ZnSO_4{\cdot}7H_2O$, and deionized water to 1 liter.
- LB + Amp medium was composed of 10 g of tryptone, 5 g of yeast extract, 5 g of NaCl, and deionized water to 1 liter. After autoclaving 1 ml of a 100 mg/ml solution of ampicillin in water was added.
- PDA plates were composed of 39 g of Potato Dextrose Agar (Difco) and deionized water to 1 liter.
- PEG buffer was composed of 500 g of polyethylene glycol 4000 (PEG 4000), 10 mM $CaCl_2$, 10 mM Tris-HCl pH 7.5, and deionized water to 1 liter; filter sterilized.
- SOC medium was composed of 0.5 g of NaCl, 5 g of yeast extract, 20 g of tryptone, 10 ml of 250 mM KCl, and deionized water to 1 liter.
- STC was composed of 1 M sorbitol, 10 mM $CaCl_2$, and 10 mM Tris-HCl, pH 7.5; filter sterilized.
- TAE buffer was composed of 4.84 g of Tris Base, 1.14 ml of Glacial acetic acid, 2 ml of 0.5 M EDTA pH 8, and deionized water to 1 liter.
- *Trichoderma* Minimal Medium (TrMM) plates (for sub-culturing) were composed of 30 g sucrose, 20 ml COVE salt solution, 0.6 g of $CaCl_2.2H_2O$, 6 g of $(NH_4)_2SO_4$, 25 g of Noble agar, and deionized water to 1 liter.
- *Trichoderma* trace metals solution was composed of 216 g of $FeCl_3{\cdot}6H_2O$, 58 g of $ZnSO_4{\cdot}7H_2O$, 27 g of $MnSO_4{\cdot}H_2O$, 10 g of $CuSO_4{\cdot}5H_2O$, 2.4 g of $H_3BO_3$, 336 g of citric acid, and deionized water to 1 liter.
- YP medium was composed of 10 g of yeast extract, 20 g of Bacto peptone, and deionized water to 1 liter.
- 2XYT plus ampicillin plates were composed of 16 g of tryptone, 10 g of yeast extract, 5 g of sodium chloride, 15 g of Bacto agar, and deionized water to 1 liter. One ml of a 100 mg/ml solution of ampicillin was added after the autoclaved medium was tempered to 55°C.

Purchased material and strains

*E. coli* DH5-alpha (TOYOBO) was used for plasmid construction and amplification. Amplified plasmids were recovered with Qiagen Plasmid Kit (QIAGEN). Ligation was done with DNA ligation kit (TAKARA) or T4 DNA ligase (BOEHRINGER MANNHEIM). QIAQUICK™ Gel Extraction Kit (Qiagen) was used for the purification of PCR fragments and extraction of DNA fragment from agarose gel.

In-Fusion® cloning kit and the E. coli cells (Fusion-Blue) were used for constructing the pDAu571 expression vector as well as pDAu703. PCR amplifications are performed using Phusion® high fidelity DNA polymerase

The expression host strain *Aspergillus niger* NN059095 was isolated by Novozymes and is a derivative of *Aspergillus niger* NN049184 which was isolated from soil. NN059095 was genetically modified to disrupt expression of amyloglycosidase activities.

*Trichoderma reesei* strain 981-0-8 (D4) is a mutagenized strain of *T. reesei* RutC30 (ATCC 56765; Montenecourt and Eveleigh, 1979, Adv. Chem. Ser. 181: 289-301).

*Trichoderma reesei* strain AgJg115-104-7B1 (WO 2011/075677) is a ku70-derivative of *T. reesei* strain 981-0-8 (D4).

Transformation of *Aspergillus*

Transformation of *Aspergillus* species can be achieved using the general well-known methods for yeast transformation. The *Aspergillus niger* host strain was inoculated into 100 ml YPG medium supplemented with 10 mM uridine and incubated for 16 hrs at 32°C at 80 rpm. Pellets were collected and washed with 0.6 M KCl, and resuspended in 20 ml 0.6 M KCl containing a commercial glucanase product (GLUCANEX™, Novozymes A/S, Bagsvaerd, Denmark) at a final concentration of 20 mg per ml. The suspension was incubated at 32°C with shaking (80 rpm) until protoplasts were formed, and then washed twice with STC buffer. The protoplasts were counted with a hematometer and resuspended and adjusted in an 8:2:0.1 solution of STC:STPC:DMSO to a final concentration of 2.5x107 protoplasts/ml.

Approximately 4pg of plasmid DNA was added to 100 pl of the protoplast suspension, mixed gently, and incubated on ice for 30 minutes. One ml of SPTC was added and the protoplast suspension was incubated for 20 minutes at 37 °C. After the addition of 10 ml of 50C Cove top agarose, the reaction was poured onto Cove agar plates and the plates were incubated at 32 °C for 5 days.

PCR amplification

5x PCR buffer (incl. MgCl2) 20 μl
2.5mM dNTP mix 10 μl
Forward primer (100pM) 1 μl
Reverse primer (100 pM) 1 μl
Expand High Fidelity polymerase (Roche) 1 μl
Template DNA (50-100 ng/ pl) 1 μl
Distilled water to 100 μl

PCR conditions:

1. 94 °C 2 min
2. 94 °C 0.5 min
3. 55 °C 0.5 min
4. 72 °C 1-2min
2-4. 30 cycles
5. 72 °C 10 min

MTP cultivation for enzyme production:

Spores of *Aspergillus* libraries were inoculated in 0.5-1ml of 1/4YPM media in 96 deep well plate and cultivated at 30 °C for 2-3 days at 600rpm.

Southern hybridization

Mycelia of selected transformants were harvested from overnight-culture in 100 ml YPG medium, rinsed with distilled water, dried and frozen at -80 °C. Ground mycelia were incubated with Proteinase K and RNaseA at 65 oC for 1 hrs. Genome DNA was recovered by phenol/CHCl3 extraction twice followed by EtOH precipitation and resuspended in distilled water.

Non-radioactive probes were synthesized using a PCR DIG probe synthesis kit (Roche Applied Science, Indianapolis IN) followed by manufacture's instruction. DIG labeled probes were gel purified using a QIAQUICK™ Gel Extraction Kit (QIAGEN Inc. , Valencia, CA) according to the manufacturer's instructions.

Five micrograms of genome DNA was digested with appropriate restriction enzymes completely for 16 hours (40 ul total volumes, 4U enzyme/ul DNA) and run on a 0.8 % agarose gel. The DNA was fragmented in the gel by treating with 0.2 M HCl, denatured (0.5 M NaOH, 1.5 M NaCl) and neutralized (1 M Tris, pH7. 5; 1.5 M NaCl) for subsequent transfer in 20X SSC to HyBond N+ membrane (Amersham). The DNA was UV cross-linked to the membrane and prehybridized for 1 hour at 42 °C in 20 ml DIG Easy Hyb (Roche Diagnostics Corporation, Mannheim, Germany).

The denatured probe was added directly to the DIG Easy Hyb buffer and an overnight hybridization at 42 °C was done. Following the post hybridization washes (twice in 2X SSC, roome temperature, 5 min and twice in 0.1X SSC, 68 °C, 15 min. each), chemiluminescent detection using the DIG detection system and CPD-Star (Roche) was done according to the manufacturer's protocol. The DIG-labeled DNA Molecular Weight Marker II (Roche) was used for the standard marker.

**Example 1. An *Aspergillus niger* host/vector system for inserting an enzyme-encoding gene libary into the genome employing a promoterless selection marker**

An *Aspergillus niger* comprising an enzyme-encoding polynucleotide library inserted into its genome was constructed in two steps by first integrating a plasmid denoted p002 (full DNA sequence provided in SEQ ID NO:1 and schematic shown in figure 1), which comprised (in the following order):

a) a 5' genomic flanking region of the *Aspergillus niger* acid alpha amylase-encoding gene;
b) an *Aspergillus nidulans* xylanase-gene promoter operably linked with a FLP recombinase-encoding gene and an *Aspergillus oryzae* nitrate reductase-gene terminator;
c) an *Aspergillus* neutral amylase-gene promoter,
d) a first recombinase recognition sequence:
5' ttgaagttcctattccgagttcctattctctagaaagtataggaacttc (SEQ ID NO:2),
e) an amdS promoter operably linked with a hygromycin B resistance marker gene and an amdS terminator;
f) a second recombinase recognition sequence
5' ttgaagttcctattccgagttcctattcttcaaatagtataggaacttca (SEQ ID NO:3),
g) an acetoamidase(amdS)-encoding gene without a promoter but with a terminator; and finally
h) a 3' genomic flanking region of the *Aspergillus niger* acid alpha amylase-encoding gene.

The p002 plasmid was transformed into an *A. niger* strain 1007-8 (disclosed in WO121600093) followed by hygromycin B selection. The obtained transformants were confirmed by Southern blotting analysis to have the correctly integrated expected genomic sequence. A positive transformant was selected and protoplasts were made from the strain.

In a second step, an expression vector pFRT-GIAMG was constructed based on the pUC19 vector; the full DNA sequence of pFRT-GIAMG is provided in SEQ ID NO:4 and schematic shown in Figure 2. The vector comprised (in the following order):

a) a recombinase recognition sequence,
b) a glucoamylase gene from *Gloeophyllum trabeum* (DNA in SEQ ID NO:5; amino acid sequence in SEQ ID NO:6) operably linked with an *A.niger* glucoamylase terminator;
c) an *A. oryzae* translation elongation factor 1 (TEF1) promoter to drive the expression of a genomic promoterless selection marker in the genome of the host cell after integration, thereby also ensuring properly oriented integration; and
d) a recombinase recognition sequence.

One μg of plasmid was transformed into the protoplasts to generate 200-300 colonies on Cove minimal plates (Cove D.J. 1966. Biochem. Biophys.cta. 113:51-56) supplemented with 1.0 M sucrose as carbon source and 1% xylose. Colonies were inoculated to czapec-dox plates containing 0.1 % amylopectin to confirm glucoamylase activity by iodine-staining (0.15% $I_2$ /1.5% KI). One positive transformant was selected and denoted *A. niger* 1007-002-44-11.

**EXAMPLE 2. An *A. niger* host/vector system employing a promoterless partial selection marker**

A plasmid, p007 (Figure 3; SEQ ID NO:7), was constructed as follows: A first PCR fragment was generated with a primer pair of SpeI-FRTF3-amdS and amdS-F-probe with plasmid p002 as a template to amplify the DNA region of SpeI site-a recognition sequence-C-terminal of amdS (4.0 kbp). The amplified PCR product was then digested by SpeI and PacI.

Primer SpeI-FRTF3-amdS (81mer; SEQ ID NO:8):
5' tggcgtagactagttgaagttcctattccgagtcctattcttcaaatagtataggaacttcatcagggagatgtaacaac Primer amdS-F-probe (22mer; SEQ ID NO:9):
5' ctatggagtcaccacatttccc

A DNA fragment (1.0 kbp) comprising a recombinase recognition sequence and a neutral amylase promoter operably linked with an enzyme-encoding gene, was prepared by double digestion of plasmid p002 by PacI and BglII.

A DNA fragment (3.0 kbp) which contains the amdS promoter operably linked to the hygromycin resistance-encoding gene and the amdS terminator was prepared by double digestion of p002 by BglII and SpeI.

The three DNA fragments were ligated together and transformed into *E. coli* DH5alpha. The resultant plasmid was named p007.

The p007 plasmid comprised a promoter operably linked with a FLP recombinase gene and a terminator, an additional NA2 promoter, a hygromycin gene with a promoter and terminator between FRTs, and a partial amdS selection marker gene lacking the N-terminal sequence and half of its first intron, all flanked by two acid alpha-amylase-flanking genomic regions.

Plasmid p007 was transformed into *A. niger* strain 1007-8 (disclosed in WO121600093) followed by hygromycin B selection. The obtained transformants were confirmed by Southern blotting analysis to have the correct integrated sequences.

An expression vector, pFRT-BsAMG (Figure 4; SEQ ID NO:10), was constructed based on the pUC19 vector which comprised (in the following order):

a) a recombinase recognition sequence,
b) the glucoamylase gene from *Byssocorticium* (DNA sequence shown in SEQ ID NO:11, the encoded amino acid sequence in SEQ ID NO:12) or a mutated glucoamylaseencoding gene as well as the *A.niger* glucoamylase terminator,
c) the *A. oryzae* TEF1 promoter (intended to drive expression of a selection marker in the genome of a recipient host cell) operably linked with a partial *N*-terminal acetamidase, *amdS,* gene; and
d) a recombinase recognition sequence.

The vector was constructed by inserting the partial acetamidase gene shown in SEQ ID NO:13 between the TEF1 promoter (Ptef1) and the second recognition sequence, and exchanging the glucoamylase gene from *Gloeophyllum* to the *Byssocorticium* glucoamylase gene.

A PCR was carried out using M13M4 and M13RV primers on pFRT-BsAMG as a template and a 4kb fragment containing the first recombinase recognition sequence, the glucoamylase gene from *Byssocorticium* (or the mutated glucoamylase gene), the *A.niger* glucoamylase terminator, the TEF1 promoter, the partial N-terminal acetoamidase gene and the second recombinase recognition sequence were amplified.

| | |
|---|---|
| Primer M13 RV (SEQ ID NO:14): | caggaaacagctatgac |
| Primer M13 M4 (SEQ ID NO:15): | gttttcccagtcacgac |

Both pFRT-BsAMG and the PCR fragment was transformed and cultivated on Cove minimal plates and 1% xylose. Colonies were inoculated to czapec-dox plates containing 0.1 % amylopectin to confirm glucoamylase activities by iodine-staining (0.15% $I_2$ /1.5% KI).

**EXAMPLE 3. Single-copy site-directed gene-insertion in *A. niger***

Plasmid library construction using In-Fusion cloning (Clontech)

40ng of the pFRT-GIAMG expression vector was digested with restriction enzymes *Xho*I and *Bsi*W1 to cut out the residing AMG-encoding gene. Two PCRs were carried out for with 2 primer pairs, a forward degenerate primer and a primer having more than 15bp overlapping with an expression vector, and M13 M4 and a reverse primer having 15bp overlapping with the degenerate primer using the pFRT-GIAMG vector as a template:

| | |
|---|---|
| Primer In fusion vector R (SEQ ID NO:16): | tatgcgttatcgtacgcac |
| Primer M13 M4 (SEQ ID NO:17): | gttttcccagtcacgac |

The primer pair for a BsAMG library is shown below:

| | |
|---|---|
| Primer M49X F (27mer; SEQ ID NO:18): | gtcaacccggactacnnktacacatgg |
| Primer M49X R (18mer; SEQ ID NO:19): | gtagtccgggttgacttg |

The digested vector and PCR fragments were mixed with In-Fusion mix and transformed into *E.coli* DH5alpha. Obtained E.coli transformants were pooled and plasmids were extracted for library construction.

PCR library construction

The first PCRs were carried out with two primer pairs, a degenerate primer and SOE-MR R and SOE-M4 F and a counterpart reverse primer of the degenerate primer using an pFRT expression vector as a template.
The second SOE PCR was carried out with a primer pair, SOE-F and SOE-R, and the PCR fragments from the first PCR. The resultant 4kb fragments were recovered as PCR fragment libraries for *Aspergillus* library construction.

| | |
|---|---|
| Primer SOE-M4 F (SEQ ID NO:20): | 5' gtactatctggcattggtacgttttcccagtcacgac |
| Primer SOE-MR R (SEQ ID NO:21): | 5' tggttatgatttcggcgatgcaggaaacagctatgac |
| Primer SOE-F (SEQ ID NO:22): | 5' gtactatctggcattggtac |
| Primer SOE-R (SEQ ID NO:23): | 5' tggttatgatttcggcgatg |

One μg of each plasmid or PCR fragment library was transformed into the *A.niger* 1007-002-44-11 host strain. Transformants were isolated in a 96 well-MTP containing COVE-N gly agar (100μl/well) and cultivated at 32 °C for 1 week to have enough sporulation. 100μl/well of 0.01% Tween 20 was added to each well and the spore suspension was inoculated in a 96 well-MTP containing YPG and cultivated for 3 days at 30 °C with shaking. The libraries were then screened.

DNA isolation from Aspergillus clones

Inserted DNA of *Aspergillus* strains was amplified by the direct colony PCR method described below or by PCR on isolated chromosomal DNA using the primer pair "insert rescue F" and "R".

| | |
|---|---|
| Primer insert rescue F (SEQ ID NO:24): | 5' aatctcagaacaccaatatc |
| Primer insert rescue R (SEQ ID NO:25): | 5' aacactatgcgttatcgtac |

Colony PCR was carried out as follows: Conidias were added to a 1,5 ml tube, 500μl of TE-buffer was added and mixed briefly. The tube-content was diluted 10-20 times in water and one μl of the diluted mixture was used as template for PCR. The amplified DNA was purified by agarose gel electrophoresis and the QIAquick Gel Extraction kit (Qiagen) and then sequenced to check the quality of constructed libraries. The libraries were correct.

**EXAMPLE 4. An *Aspergillus oryzae* host/vector system**

*A. oryzae* strain JaL1394 (disclosed in WO 2012/160093) was used as host strain in this example. We have constructed an integration expression vector denoted pDAu571 (Figure 5; SEQ ID NO: 26) for site-specific recombination using the FLP/FRT system into the chromosome of *A. oryzae* JaL1394.
The vector pDAu571 is composed of three parts (PART- I, PART- II, PART- III; Figure 5):
PART-I is delimited by two short FRT (Flippase Recognition Target) sites. In between the FRT sites the following elements are found :

- An expression cassette with a "stuffer sequence" that can be replaced with any gene or polynucleotide library of interest (GOI) using the unique restriction sites BamHI and *Xho*I.
- An *Aspergillus nidulans* PyrG selection marker to select the transformed host cells for their ability to grow on minimal medium without supplementation of uridine; the PyrG marker is operably linked with its native promoter and the terminator of the *A. niger* glucoamylase gene (Tamg) (pos. 4057-5918 of SEQ ID NO:26).

This part of the plasmid is to be integrated into the chromosome of the host organism via FLP-assisted recombinations between the respective FRT sites in the host and the vector. When evaluating gene libraries it is advantageous to ensure that PART-I of the vector is integrated in the same orientation in all transformants. This reduces any variation in gene expression from the flanking regions, e.g. from read-through. There is a small difference between the sequences of the FRT-F and FRT-F3 sites in the chromosome and the vector which ensures the correct orientation of the integrated part in the chromosome. The FRT-F3 sites in the chromosome of the host and in the vector will be recombined with each other by the FLP flippase and so will the FRT-F sites.
FRT-F (pos. 1-49 of SEQ ID NO:26):
5'-ttgaagttcctattccgagttcctattctctagaaagtataggaacttc (SEQ ID NO:27)

FRT-F3 (pos. 5925-5974 of SEQ ID NO:26):
5'- ttgaagttcctattccgagttcctattcttcaaatagtataggaacttca (SEQ ID NO:28)

The expression cassette comprises an artificial NA2TPI promoter constructed from the *A. niger* neutral amylase II promoter fused to the *A. nidulans* triose phosphate isomerase nontranslated leader sequence (pos. 73-814 of SEQ ID NO:26).

In addition, the expression cassette comprises the so-called "stuffer sequence" which in this case is a gene that encodes lipoxygenase I from soybean (pos. 824-3343 of SEQ ID NO:22). As already mentioned, the stuffer sequence in the vector may be replaced with any other gene of interest using the two unique restriction enzyme sites BamHI and XhoI.

Finally, the expression cassette comprises the terminator of the *A. niger* glucoamylase gene (pos. 3358-4048 of SEQ ID NO:26).

PART-II of the pDau571 vector is a synthetic version of the flippase-encoding gene (sFLP) (pos. 4875-6143 of SEQ ID NO:26 (reverse strand)). The flippase gene expression is controlled by the *A. oryzae* translation-elongation factor 1 alpha promoter (pTEF1) (pos. 7759-8447 of SEQ ID NO:26 (reverse strand)) as well as the *A. oryzae* nitrate reductase terminator (TniaD) (pos. 5991-6478 of SEQ ID NO:26 (reverse strand)).

PART-III of the pDau571 vector is necessary for the propagation of the plasmid in *E.coli* and comprises an *E. coli* beta-lactamase (*ampR*) selectable marker and an *E. coli* origin of replication derived from the well-known pUC plasmid (pos. 8448-11101 of SEQ ID NO:26).

Plasmid pDAu571, wherein the stuffer sequence is replaced by a polynucleotide library of of interest is transformed into the protoplasts of the strain Jal1394 as follows:
100 μl protoplasts are incubated with 10 μl miniprep plasmid DNA (undigested or linearized) or a PCR product spanning PART-I and -II of pDAu571 and 300 μl 60 % PEG for 20 min at room temperature; then 5 ml Topagar (+ 10 mM $NaNO_3$) is added and the whole transformation mixture is plated on Sucrose / 10mM $NaNO_3$ plates. The plates are then incubated at 37 °C until spores can be spotted. To purify single transformants, colonies are restriked onto a new sucrose/10mM $NaNO_3$ plate and incubated at 37°C until spores develop.

The transformants obtained can be verified for the correct integration at the *amy2* locus of the expression cassette by PCR using diagnostic primers located within the *amy2* locus on both sides flanking the expression cassette including FRT sites and gene specific primers located within the expression cassette.

**EXAMPLE 5. Construction of** a **split-marker *Aspergillus oryzae* host/vector system**

The previous example provided an expression vector for flippase-mediated site-specific recombination and integration into a suitable filamentous fungal host cell. The correct orientation in the chromosome of the integrated expression cassette was ensured by using different recombinase recognition sequences.

Another way to ensure the proper orientation is to employ a split selection marker, where one non-functional part of the marker resides in the host chromosome and another non-functional part of the marker is on the incoming vector. Only the correctly oriented integration then results in a functional second selection marker. That split-marker principle is illustrated in this example; here the second selection marker is oriented in one direction but it could just as well have been oriented the other way.

*Media and solutions necessary for Aspergillus protoplast transformation and selection of recombinant cells:*

**Trace metal**

| | |
|---|---|
| $Na_2B_4O_7$.10aq | 40 mg/l |
| $CuSO_4$.5aq | 400 mg/l |
| $FeSO_4$.7aq | 1200 mg/l |
| $MnSO_4$.aq | 700 mg/l |
| $Na_2MoO_2$.2aq | 800 mg/l |
| $ZnSO_4$.7aq | 10.000 mg/l |

**Salt solution**

| | |
|---|---|
| KCl | 26 g/l |
| $MgSO_4.7aq$ | 26 g/l |
| $KH_2PO_4$ | 76 g/l |
| Trace metal | 50 ml/l |

**COVE medium**

20 ml salt solution

20 g agar

218 g sorbitol

$H_2O$ ad 1l

Autoclave and then add:

50 ml 20% glucose

10 ml 1M urea.

**Cove-N-gly slant**

| | |
|---|---|
| Salt solution | 50 ml |
| Sorbitol | 218 g |
| kaliumnitrat | 2,02g |
| Glycerol | 10 ml |
| Agar | 35 g |
| MilliQ $H_2O$ | to 1000 ml |

**Sucrose medium**

20 ml salt solution.

342 g sucrose.

$H_2O$ ad 1l

Autoclave and then add:

10 mM NaNO3

| | |
|---|---|
| **ST** | 0.6 M sorbitol<br>100 mM Tris/HCl pH 7.0 |

| | |
|---|---|
| **STC** | 1.2 M sorbitol |

(continued)

10 mM $CaCl_2$
10 mM Tris/HCl pH 7.5.

| | |
|---|---|
| **PEG** | 60% (W/V) PEG 4000 (BDH) (6 g PEG+~5 ml sterile water, put at 60-65°C)<br>10 mM $CaCl_2$ (50μl of a 2 M $CaCl_2$)<br>10 mM Tris/HCl pH 7.5. (100 μl of a 1M Tris) |

**Sucrose agar plate**

10g Agar
10ml Salt solution
1M sucrose to 500ml
Autoclave to sterilized

**Acetamide plates**

10 ml salt solution
10 g agar
1M sucrose ad 500 ml[1].
autoclave.
Cool to approx. 65°C and add 10mM acetamide and 15mM CsCl.
Triton X-100 50μl for 500ml (only in the restriking plates)

<u>Introduction of the FRT sites at the *amy2* locus in *Aspergillus oryzae* DAu716</u>

The plasmid pJAI1258 (described in WO12160097A1) was modified resulting in a plasmid denoted pDAu703. Plasmid pDAu703 contains the following elements in order (Figure 6; SEQ ID NO:29):

- *amy2* - 3' flank (490bp); positions 449-938 of SEQ ID NO:29;
- *pyrG* promoter operably linked with a partial *pyrG* gene containing the 5'-end of the *pyrG* CDS (the first exon and 5' end of its first intron);
- a FRT-F3 site (50bp); positions 1452-1501 of SEQ ID NO:29;
- an *A. niger* AMG terminator (Tamg) operably linked with the AmdS-encoding gene, positions 1511-2200 of SEQ ID NO:29;
- *A. nidulans* acetamidase gene (AmdS), positions 2232-4131 of SEQ ID NO:29;
- the strong triose-phosphate isomerase promoter (Ptpi) operably linked with the Amds-encoding gene; this allows growth on acetamide and CICs even though only one copy of the AmdS selection cassette is present in the genome as expected if the plasmid pDAU703 is integrated in one copy at the amy2 locus at FRT sites. Positions 4140-4894 of SEQ ID NO:29;
- a FRT-F site (49bp); positions 4903-4951 of SEQ ID NO:29;
- *amy2* - 5' flank (1114bp); positions 4964-6077 of SEQ ID NO:29;
- The rest of the plasmid is composed of a part of DNA necessary for the maintenance of the plasmid as a replicative plasmid in the bacterial host cell *E.coli* (E.coli origin of replication and ampicillin resistance cassette).

Plasmid DNA pDAu703 was digested with NotI restriction enzyme to separate the DNA containing the integration cassette from the now irrelevant *E.coli* part of the plasmid.

The linearized plasmid pDAu703 was transformed into protoplasts of *A. oryzae* strain JaI1338 (disclosed in WO12160097A1) using a standard procedure described, for example, in WO98/01470 but with supplementing the media with 10mM uridine since the strain is PyrG minus and therefore cannot grow in absence of uridine. Transformants were selected on AmdS selection plates

The resulting recombinant host strains have had the two FRT sites as well as the 5' end of the split PyrG marker (first exon and part of the native intron) operably linked with its own promoter integrated by homologous recombination at the *amy2* locus, as shown in the top panel of Figure 7. The correct integration at the *amy2* locus was checked by Southern blot analysis using a probe that annealed to the *amy2* 3' end (Figure 7). Integration of the FRT cassette generated hybridization signals at 5114bp and 2637bp in EcoRI and *Xho*I digests, respectively (not shown). This pattern is different from the Jal1338 host, where the *amy2* locus is not disrupted. A correct strain was selected and denoted *A. oryzae* DAu716 (Figure 7, top).

Transformation of DAu716 with the pDAU724 vector carrying a lipase-encoding gene

This example demonstrates how the FRT/FLP recombination and split PyrG marker can be used to effectively make single copy insertions of an expression cassette with a high frequency in *A. oryzae.* We used the lipase gene from *Thermomyces lanuginosa* (e.g. disclosed in WO2008008950) as a reporter to measure the level of lipase produced in a transformed host.

Like in the previous example, an expression vector was constructed so that part of it can be integrated into the chromosome of the host cells at the FRT-sites using flippase as site specific recombination mediator. The part of the plasmid that is to be integrated in the genome carries a lipase gene operably linked with the NA2/TPi promoter and the terminator of the A. *niger* AMG gene. In order to be able to select the recombinant cells that have successfully integrated the expression cassette via the FRT sites, the remainder of the pyrG selection marker is also included in between the FRT sites. The promoter and the first exon resides in the DAu716 host and the remainder of the pyrG marker resides on the incoming plasmid. Upon site specific recombination, the PyrG marker will be reconstructed as an intact gene (with a FRT sequence inside its first intron which will, of course, be spliced out from the mRNA) and the recombinant cells will be able to express PyrG and grow on plate with $NaNO_3$ as sole nitrogen source.

Plasmid pDAU724 (Figure 7, middle; SEQ ID NO:30) consists of :

PART-I which is to be integrated in the genomic DNA of the *Aspergillus* host cells and it consists of the two FRT sites with the expression cassette and one part of the split pyrG marker;
PART-II which will not be integrated in the genome of the host cell and which contains the FLPase expression cassette as well as *E.coli* selection marker and origin of replication.

The strain DAu716 was grown on a slant of Cove-N- gly medium until spores could be seen.

10 - 20 ml of Sucrose medium or YPD medium was added to the slant, and the spores were suspended by vortexing the slant. The spore suspension was transferred to a polycarbonate shakeflask (500 ml) containing 100 ml sucrose medium with 10 mM $NaNO_3$ (or other nitrogen source). The flask was incubated at 30°C for 24 hr (200 rpm).

The mycelium was collected by filtration through miracloth and washed using 200 ml 0.6 M $MgSO_4$. The remaining liquid was squeezed out of the mycelium e.g. using a plastic pipette.

1-2 g of the mycelium was transferred to a small (100 ml) polycarbonate flask containing:

75-150 mg Glucanex
10 ml 1.2 M $MgSO_4$
100 ul 1 M $NaH_2PO_4$ pH 5.8
and the mycelium was suspended, 1 ml of 12 mg/ml BSA (sterile filtered) was added

The suspension was incubated at 37°C for 1/2-2 hr, and the protoplasting was monitored frequently by microscopy.

The protoplast suspension was filtered through miracloth into a 25 ml centrifuge tube and the suspension was overlaid with 5 ml ST (being careful not to mix up the lower layer). The resulting protoplasts were banded by centrifugation (2500 rpm / 1350 g, 15 min, slow acceleration). The interface band of protoplasts was recovered and transferred to a fresh tube.

The protoplasts were diluted with 2 volumes of STC followed by centrifugation (2500 rpm / 1350 g, 5 min). The protoplasts were then washed twice with 5 ml STC (using resuspension and centrifugation), and then resuspended in STC to a concentration of approx $5 \times 10^7$ protoplasts/ml.

For each transformation, the transforming DNA was added at the bottom of e.g. a 14 ml tube, and 100 μl of protoplasts were added. 300 μl of PEG was added, and the tube was gently mixed by hand. After 20 minutes of incubation (RT), 6 ml top agar at temperature of 50°c was added and immediately the suspension was poured on to a selective sucrose agar plate with 10mM Na $NO_3$.

The plates were incubated at 37°C until transformants were clearly visible and started to sporulate. 20 transformants were restriked onto a new selection plate with triton to isolate colonies that could be further analyzed by fermentation, Southern blot analysis or enzyme activity assay.

It was verified that the residing AmdS marker in the chromosome had been replaced by the incoming lipase gene in the transformants by streaking the transformants on plates containing CsCl (an inhibitor of the endogenous acetamidase) and acetamide as sole nitrogen source. Correct transformants should not be able to grow on these plates. We tested 20 recombinant cells obtained after transformation of pDAu724 into DAu716 and only a slight growth phenotype was observed compared to the parent host strain DAu716, where the AmdS selection marker is still present.

It was confirmed that all 20 transformants contained one inserted copy of the lipase expression cassette correctly inserted at the FRT sites.

The 20 transformants were inoculated in 3ml YPD in a Uniplate® 10ml 24 deep-wells plate (Whatman) sealed with Airpore tape (Quiagen) and incubated at 30 degree Celcius for 4 days with 200rpm agitation. The supernatants were collected for further analysis (lipase assay and SDS- page) and the mycelia were also collected for genomic extraction and Southern analysis.

The 20 transformants showed comparable lipase activities in a lipase assay as well as comparable lipase protein levels on an SDS-PAGE gel. In addition, a Southern blot confirmed that all 20 transformants had only the expected single lipase gene copy correctly integrated in the chromosome.

**Example 6: Cloning of the wild-type *Vigna angularis* xyloglucan endotransglycosylase 16 (VaXET16) for expression in an *Aspergillus oryzae* screening strain**

Herein a codon-optimized wild-type *Vigna angularis* xyloglucan endotransglycosylase 16 (VaXET16) cDNA was cloned into the *A. oryzae* Flp/FRT shuttle vector pDAu571 (Figure 5) by yeast recombinational cloning, resulting in vector pDLHD0075 (Figure 8).

Expression vector pDLHD0075 was constructed to contain the *E. coli* pUC origin of replication, *E. coli* beta-lactamase (*ampR*) selectable marker, URA3 yeast selectable marker, yeast 2 micron origin of replication, NA2-tpi promoter, codon-optimized VaXET16 open reading frame (SEQ ID NO:31 for the cDNA sequence and SEQ ID NO:32 for the amino acid sequence), *Aspergillus niger* glucoamylase terminator, *Aspergillus nidulans pyrG* selection marker, *Saccharomyces cerevisiae* 2 μm flippase ORF between the *Aspergillus oryzae* TEF1 promoter and *Aspergillus oryzae* NiaD terminator, and *Saccharomyces cerevisiae* 2 μm flippase recognition targets FRT-F and FRT-F3.

Plasmid pDLHD0075 was generated by combining four DNA fragments using yeast recombinational cloning: Fragment 1 contained the flippase expression cassette, FRT-F3, and AMG terminator from pDAU571 and flanking sequences with homology to fragments 4 and 2. Fragment 2 contained the *E. coli* pUC origin of replication, *E. coli* beta-lactamase (*ampR*) selectable marker, URA3 yeast selectable marker, yeast 2 micron origin of replication from pDLHD0044, and flanking sequences with homology to fragments 1 and 3. Fragment 3 contained the NA2-tpi promoter, the VaXET16 codon-optimzed gene from pDLHD0044, and flanking sequences with homology to fragments 2 and 4. Fragment 4 contained the *A. niger* amyloglucosidase terminator sequence (AMG terminator) and *Aspergillus nidulans* orotidine-5'-phosphate decarboxylase gene (*pyrG*) as a selectable marker from pDAU571, and flanking sequences with homology to fragments 3 and 1.

Fragment 1 was amplified using primer 615726 (sense) and primer 615728 (antisense) shown below. These primers were designed to contain flanking regions of sequence homology to fragments 4 and 2, respectively (lower case), for ligation-free cloning between the PCR fragments.

Primer 615726 (sense):
accgggaggaaggctggaaaGCTTACGAGAAAAGAGTTGGACTTTGAGGG (SEQ ID NO: 33)
Primer 615728 (antisense):
tgagcgaggaagcggAAGAGCGCCCAATACGCAAACCGCC (SEQ ID NO: 34)

Fragment 1 was amplified by PCR in a reaction composed of 10 ng of pDAU571, 0.5 μl of PHUSION® DNA Polymerase, 20 pmol of primer 615726, 20 pmol of primer 615728, 1 μl of 10 mM dNTPs, 10 μl of 5X PHUSION® HF buffer, and 35.5 μl of water. The reaction was incubated in an EPPENDORF® MASTERCYCLER® thermocycler programmed for 1 cycle at 98°C for 30 seconds; and 30 cycles each at 98°C for 10 seconds, 60°C for 10 seconds, and 72°C for 120 seconds. The resulting 3.3 kb PCR product (fragment 1) was treated with 1 μl of *Dpn* I to remove plasmid template DNA. The *Dpn* I was added directly to the PCR reaction tube, mixed well, and incubated at 37°C for 60 minutes.

Fragment 2 was amplified using primer 615729 (sense) and primer 615731 (antisense) shown below. These primers were designed to contain flanking regions of sequence homology to fragments 1 and 3, respectively (lower case), for ligation-free cloning between the PCR fragments.

Primer 615729 (sense):
tgcgtattgggcgctcttCCGCTTCCTCGCTCACTGACTC (SEQ ID NO:35)

Primer 615731 (antisense):
tatactttctagagaataggaactcggaataggaacttcaaGGAACAACACTCAACCCTATCTCGGTC (SEQ ID NO:36)

Fragment 2 was amplified by PCR in a reaction composed of 10 ng of pDLHD0044, 0.5 μl of PHUSION® DNA Polymerase, 20 pmol of primer 615729, 20 pmol of primer 615731, 1 μl of 10 mM dNTPs, 10 μl of 5X PHUSION® HF buffer, and 35.5 μl of water. The reaction was incubated in an EPPENDORF® MASTERCYCLER® thermocycler programmed for 1 cycle at 98°C for 30 seconds; and 30 cycles each at 98°C for 10 seconds, 60°C for 10 seconds, and 72°C for 120 seconds. The resulting 4.2 kb PCR product (fragment 2) was treated with 1 μl of *Dpn* I to remove plasmid template DNA. The *Dpn* I was added directly to the PCR reaction tube, mixed well, and incubated at 37°C for 60 minutes.

Fragment 3 was amplified using primer 615730 (sense) and primer 615611 (antisense) shown below. These primers were designed to contain flanking regions of sequence homology to fragments 2 and 4, respectively (lower case), for ligation-free cloning between the PCR fragments.

Primer 615730 (sense):
tccgagttcctattctctagaaagtataggaacttcGCATTTATCAGGGTTATTGTCTCATGAGCGG (SEQ ID NO:37)
Primer 615611 (antisense):
tctagatctcgagtcaGATGTCCCTATCGCGTGTACACTCG (SEQ ID NO:38)

Fragment 3 was amplified by PCR in a reaction composed of 10 ng of pDLHD0044, 0.5 μl of PHUSION® DNA Polymerase, 20 pmol of primer 615730, 20 pmol of primer 615611, 1 μl of 10 mM dNTPs, 10 μl of 5X PHUSION® HF buffer, and 35.5 μl of water. The reaction was incubated in an EPPENDORF® MASTERCYCLER® thermocycler programmed for 1 cycle at 98°C for 30 seconds; and 30 cycles each at 98°C for 10 seconds, 60°C for 10 seconds, and 72°C for 120 seconds. The resulting 1.7 kb PCR product (fragment 3) was treated with 1 μl of *Dpn* I to remove plasmid template DNA. The *Dpn* I was added directly to the PCR reaction tube, mixed well, and incubated at 37°C for 60 minutes.

Fragment 4 was amplified using primer 615610 (sense) and primer 615727 (antisense) shown below. These primers were designed to contain flanking regions of sequence homology to fragments 3 and 1, respectively (lower case), for ligation-free cloning between the PCR fragments.

Primer 615610 (sense):
acacgcgatagggacatcTGACTCGAGATCTAGAGGGTGACTGAC (SEQ ID NO:39)
Primer 615727 (antisense):
aactcttttctcgtaagcTTTCCAGCCTTCCTCCCGGTAC (SEQ ID NO:40)

Fragment 4 was amplified by PCR in a reaction composed of 10 ng of pDAU571, 0.5 μl of PHUSION® DNA Polymerase, 20 pmol of primer 615610, 20 pmol of primer 615727, 1 μl of 10 mM dNTPs, 10 μl of 5X PHUSION® HF buffer, and 35.5 μl of water. The reaction was incubated in an EPPENDORF® MASTERCYCLER® thermocycler programmed for 1 cycle at 98°C for 30 seconds; and 30 cycles each at 98°C for 10 seconds, 60°C for 10 seconds, and 72°C for 120 seconds. The resulting 1.9 kb PCR product (fragment 4) was treated with 1 μl of *Dpn* I to remove plasmid template DNA. The *Dpn* I was added directly to the PCR reaction tube, mixed well, and incubated at 37°C for 60 minutes.

The following procedure was used to combine the four PCR fragments using yeast homology-based recombinational cloning. A 10 μl aliquot of each of the PCR fragments was combined with 100 μg of single-stranded deoxyribonucleic acid from salmon testes (Sigma-Aldrich, St. Louis, MO, USA), 100 μl of competent yeast cells of strain YNG318 (*Saccharomyces cerevisiae* ATCC 208973), and 600 μl of PLATE Buffer (Sigma Aldrich, St. Louis, MO, USA), and mixed. The reaction was incubated at 30°C for 30 minutes with shaking at 200 rpm. The reaction was then continued at 42°C for 15 minutes with no shaking. The cells were pelleted by centrifugation at 5,000 x g for 1 minute and the supernatant was discarded. The cell pellet was suspended in 200 μl of autoclaved water and split over two agar plates containing Synthetic Defined medium lacking uridine and incubated at 30°C for three days. The yeast colonies were isolated from the plate using 1 ml of autoclaved water. The cells were pelleted by centrifugation at 13,000 x g for 30 seconds and a 100 μl aliquot of glass beads were added to the tube. The cell and bead mixture was suspended in 250 μl of P1 buffer (QIAGEN Inc., Valencia, CA, USA) and then vortexed for 1 minute to lyse the cells. The plasmid DNA was purified using a QIAPREP® Spin Miniprep Kit. A 3 μl aliquot of the plasmid DNA was then transformed into *E. coli* ONE SHOT® TOP10 electrocompetent cells according the manufacturer's instructions. Fifty μl of transformed cells were spread onto 2XYT plates supplemented with 100 μg of ampicillin per ml and incubated at 37°C overnight. Transformants were each picked into 3 ml of LB medium supplemented with 100 μg of ampicillin per ml and grown overnight at 37°C with shaking at 250 rpm. The plasmid DNA was purified from colonies using a QIAPREP® Spin Miniprep Kit. DNA sequencing with a 3130XL Genetic Analyzer was used to confirm the presence of each of the three fragments in a final plasmid designated plasmid pDLHD0075 (Figure 8).

**Example 7: Confirmation of wild-type *Vigna angularis* xyloglucan endotransglycosylase 16 (VaXET16) expression in single-copy in *Aspergillus oryzae* screening strain JaL1394**

*Aspergillus oryzae* JaL1394 (disclosed in WO 2012/160093) was transformed with plasmid pDLHD0075 comprising the codon-optimized VaXET16 gene.

Approximately $10^7$ spores from *A. oryzae* JaL1394 were inoculated into 100 ml of YP + 2% glucose medium supplemented with 10 mM uridine in a 500 ml shake flask and incubated at 28°C and 110 rpm overnight. 10 ml of the overnight culture were filtered in a 125 ml sterile vacuum filter, and the mycelia were washed twice with 50 ml of 0.7 M KCl-20 mM $CaCl_2$. The remaining liquid was removed by vacuum filtration, leaving the mat on the filter. The mycelia were resuspended in 10 ml of 0.7 M KCl-20 mM $CaCl_2$ and transferred to a sterile 125 ml shake flask containing 20 mg of GLUCANEX® 200 G (Novozymes Switzerland AG, Neumatt, Switzerland) per ml and 0.2 mg of chitinase (Sigma-Aldrich, St. Louis, MO, USA) per ml in 10 ml of 0.7 M KCl-20 mM CaCl2. The mixture was incubated at 37°C and 100 rpm for 30-90 minutes until protoplasts were generated from the mycelia. The protoplast mixture was filtered through a sterile funnel lined with MIRACLOTH® (Calbiochem, San Diego, CA, USA) into a sterile 50 ml plastic centrifuge tube to remove mycelial debris. The debris on the MIRACLOTH® was washed thoroughly with 10 ml of 0.7 M KCl-20 mM $CaCl_2$, and centrifuged at 2500 rpm for 10 minutes at 20-23°C. The supernatant was removed and the protoplast pellet was resuspended in 20 ml of 1 M sorbitol-10 mM $CaCl_2$-10 mM Tris-HCl (pH 6.5). This step was repeated twice, and the final protoplast pellet was resuspended in 1 M sorbitol-10 mM $CaCl_2$-10 mM Tris-HCl (pH 6.5) to obtain a final protoplast concentration of 2 x $10^7$/ml.

Protoplasts were transformed by the addition of two μg of pDLHD0075 to the bottom of a sterile 12 ml plastic centrifuge tube. One hundred μl of protoplasts were added to the tube followed by 300 μl of 60% PEG-4000 in 10 mM $CaCl_2$-10 mM Tris-HCl (pH 6.5). The tube was mixed gently by hand and incubated at 37°C for 30 minutes. Five ml of 1 M sorbitol-10 mM $CaCl_2$-10 mM Tris-HCl (pH 6.5) were added to the transformation and the mixture was transferred onto 150 mm Minimal medium agar plates. Transformation plates were incubated at 37°C until transformants appeared.

Single transformants were picked to new Minimal medium agar plates and cultivated at 37°C for four days until the transformants sporulated. Fresh spores were transferred to 48-well deep-well plates containing 2 ml of YP + 2% maltodextrin medium, covered with a breathable seal, and grown for 4 days at 28°C with no shaking. After 4 days growth the culture medium for each transformant was assayed for xyloglucan endotransglycosylase activity and for xyloglucan endotransglycosylase expression by SDS-PAGE as outlined below.

The activity assay demonstrated that the transformants produced active xyloglucan endotransglycosylase.

*Iodine colorimetric assay to determine xyloglucan endotransglycosylase activity*

Xyloglucan endotransglycosylase activity was assayed using a modified version of an iodine colorimetric assay described by Sulova et al., 1995, Analytical Biochechemistry 229: 80-85. For each reaction, 5 μl of tamarind seed xyloglucan (Megazyme, Bray, UK) (5 mg/ml in water) were combined with 20 μl of xyloglucan oligomers (Megazyme, Bray, UK) (5 mg/ml in water), 10 μl of 400 mM sodium citrate pH 5.5, and dispensed into 96 well plates. Reactions were initiated by the addition of 5 μl of liquid culture broth to each well, and plates were incubated at 37°C for 10 minutes. Reactions were quenched by the addition of 200 μl of a solution composed of 14% (w/v) $Na_2SO_4$, 0.2% KI, 0.1 M HCl, and 0.5% $I_2$, and incubated in the dark for 30 minutes prior to measuring the absorbance at 620 nm in a SPECTRAMAX® M5 spectrophotometer (Molecular Devices, Sunnyvale, CA, USA).

SDS-PAGE was performed using a 8-16% CRITERION® Stain Free SDS-PAGE gel (Bio-Rad Laboratories, Inc., Hercules, CA, USA), and imaging the gel with a Stain Free Imager (Bio-Rad Laboratories, Inc., Hercules, CA, USA) using the following settings: 5-minute activation, automatic imaging exposure (intense bands), highlight saturated pixels = ON, color = Coomassie, and band detection, molecular weight analysis and reporting disabled. SDS-PAGE revealed a band of approximately 32 kDa for the wild-type VaXET16.

**Example 8: Construction and identification of improved expression variants of *Vigna angularis* xyloglucan endotransglycosylase 16 (VaXET16)**

VaXET16 gene mutant libraries were constructed by site-saturation mutagenesis. The mutant libraries of the VaXET16 gene (each fragment of the library comprises a mutant VaXET16 gene plus *Aspergillus nidulans* orotidine 5'-phosphate decarboxylase *pyrG* selection marker and the FRT-F and FRT-F3 flippase recognition target sequences) were transformed into protoplasts of *Aspergillus oryzae* JaL1394 as described in the previous example along with the pDLHD0095 vector comprising the *Saccharomyces cerevisiae* 2 μm flippase ORF between the *Aspergillus oryzae* TEF1 promoter and *Aspergillus oryzae niaD* gene terminator. After 4 days of protoplast recovery at 37°C on Minimal medium agar plates, single colonies were picked into individual wells of 48-well deep-well plates containing 2 ml of YP + 2% maltodextran medium, covered with a breathable seal, and grown for 4 days at 28°C with no shaking. After 4 days growth the liquid culture medium was assayed for xyloglucan endotransglycosylase activity as described in the previous example, and higher activity variants were scored as expression hits.

Individual mutant strains were spore purified and cultivated again to generate fresh broth for re-testing relative to *A. oryzae* JaL1394 strain expressing the wild-type VaXET16 using the codon-optimized gene. Broths were also analyzed by SDS-PAGE as described in the previous example for increased production of the xyloglucan endotransglycosylase protein product.

Relative improvements in expression yield over the parent gene in day 4 broths from 48-well deep-well plate cultivations for eleven characterized variants are shown in Table 1 below. SDS-PAGE analysis of the same broths demonstrated a VaXET band of increased intensity over wild-type VaXET for all variants, which correlated well with the relative improvements observed in the activity assay. The SDS-PAGE band for the wild-type VaXET and variants thereof was 32 kDa, except variants containing the N175S mutation had a band of approximately 37 kDa due to additional glycosylation.

Table 1.

| Relative Improvement Over Parent | |
|---|---|
| **VaXET16 Variant** | **Iodine Colorimetric Assay** |
| Wild-Type | 1.0 |
| A40G, N175S | 1.8 |
| A40G, F183I | 2.9 |
| A40G, I53A, N175S | 3.8 |
| A40G, N175S, F183I | 1.1 |
| I10A, I53A, E102G | 1.1 |
| P30E, S51T, Y60S, T99N | 2.3 |
| A40G, E102G, Q117E | 3.1 |
| A40G, T99E, E102G, K130R | 1.2 |
| N175G, S280G | 2.7 |
| N175Q, A254E, S280E | 1.2 |
| I53V, R136W, Y157H, Y162C, N175S | 2.9 |

**Example 9: Fermentation-scale confirmation of improved expression of *Vigna angularis* xyloglucan endotransglycosylase 16 variant (VaXET16) genes in *Aspergillus oryzae***

A fermentation process was used to express the VaXET16 variants, A40G + I53A + N175S and A40G + F183I, relative to the wild-type VaXET16.

Shake flask medium was composed of 50 g of sucrose, 10 g of $KH_2PO_4$, 0.5 g of $CaCl_2$, 2 g of $MgSO_4{\cdot}7H_2O$, 2 g of $K_2SO_4$, 2 g of urea, 10 g of yeast extract, 2 g of citric acid, 0.5 ml of trace metals solution, and deionized water to 1 liter. Trace metals solution was composed of 13.8 g of $FeSO_4{\cdot}7H_2O$, 14.3 g of $ZnSO_4{\cdot}7H_2O$, 8.5 g of $MnSO_4{\cdot}H_2O$, 2.5 g of $CuSO_4{\cdot}5H_2O$, 3 g of citric acid, and deionized water to 1 liter.

One hundred ml of shake flask medium were added to a 500 ml shake flask. The shake flask was inoculated with two plugs from a solid plate culture and incubated at 34°C on an orbital shaker at 200 rpm for 24 hours. Fifty ml of the shake flask broth were used to inoculate a 3 liter fermentation vessel.

Fermentation batch medium was composed per liter of 10 g of yeast extract, 24 g of sucrose, 5 g of $(NH_4)_2SO_4$, 2 g of $KH_2PO_4$, 0.5 g of $CaCl_2{\cdot}2H_2O$, 2 g of $MgSO_4.7H_2O$, 1 g of citric acid, 2 g of $K_2SO_4$, 0.5 ml of anti-foam, and 0.5 ml of trace metals solution. Trace metals solution was composed per liter of 13.8 g of $FeSO_4{\cdot}7H_2O$, 14.3 g of $ZnSO_4{\cdot}7H_2O$, 8.5 g of $MnSO_4{\cdot}H_2O$, 2.5 g of $CuSO_4{\cdot}5H_2O$, and 3 g of citric acid. Fermentation feed medium was composed of maltose.

A total of 1.8 liters of the fermentation batch medium was added to a three liter glass jacketed fermentor. Fermentation feed medium was dosed at a rate of 0 to 4.4 g/l/hr. The fermentation vessel was maintained at a temperature of 34°C and pH was controlled to a setpoint of 6.1 +/- 0.1. Air was added to the vessel at a rate of 1 vvm and the broth was agitated by Rushton impeller rotating at 1100 to 1300 rpm. Samples were taken on days 3, 4, 5, 6, and 7 of the fermentation run and centrifuged at 3000 x g to remove the biomass. The supernatants were sterile filtered and stored at 5 to 10°C.

VaXET16 variant expression levels were determined relative to the wild-type codon-optimized gene by the fluorescence polarization assay outlined below and by SDS-PAGE analysis (see above).

*Fluorescence polarization assay for xyloglucan endotransglycosylation activity*

First, fluorescein isothiocyanate-labeled xyloglucan oligomers (FITC-XGOs) were generated by reductive amination of the reducing ends of xyloglucan oligomers according to the procedure described by Zhou et al., 2006, Biocatalysis and Biotransformation 24: 107-120), followed by conjugation of the amino groups of the XGOs to fluorescein isothiocyanate isomer I (Sigma Aldrich, St. Louis, MO, USA) in 100 mM sodium bicarbonate pH 9.0 for 24 hours at room temperature. Conjugation reaction products were concentrated to dryness *in vacuo,* dissolved in 0.5 ml of deionized water, and purified by silica gel chromatography, eluting with a gradient from 100:0:0.04 to 70:30:1 acetonitrile:water:acetic acid as mobile phase. Purity and product identity were confirmed by evaporating the buffer, dissolving in $D_2O$ (Sigma Aldrich, St. Louis, MO, USA), and analysis by $^1H$ NMR using a Varian 400 MHz MercuryVx (Agilent, Santa Clara, CA, USA). Dried FITC-XGOs were stored at -20°C in the dark, and were desiccated during thaw.

One mg of FITC-XGOs was incubated with 1 mg of tamarind seed xyloglucan (Megazyme, Bray, United Kingdom) and 18 mg of VaXET16 per ml of 20 mM sodium citrate pH 5.0 in 200 μl reactions for at least 30 minutes Sample mixtures were pooled and precipitated by addition of ice cold ethanol to 80% (v/v) final concentration and incubation at 4°C overnight. Precipitated fluorescein isothiocyanate-labeled xyloglucan (FITC-XG) was recovered by centrifugation at 3000 rpm using a LEGEND™ RT Plus centrifuge (Thermo Scientific, Waltham, MA, USA) decanting of the ethanol, and drying at room temperature for 24 hours. FITC-XG was dissolved in a minimum volume of deionized water until dissolved and stored at -20°C. Frozen FITC-XG was thawed and lyophilized overnight. The lyophilized powder was dissolved in 500 μl of deionized water and quantified by absorbance at 488 nm.

A large scale batch of FITC-XG was prepared in the following manner. A 7.9 mg per ml solution of FITC-XGOs was prepared in deionized water. Forty ml of 10 mg of tamarind seed xyloglucan (Megazyme, Bray, UK) per ml of deionized water, 452 ml of 7.9 mg of FITC-XGOs per ml of deionized water, 2 ml of 400 mM sodium citrate pH 5.5, and 1.2 ml of 1.4 mg of VaXET16 per ml of 20 mM sodium citrate pH 5.5 were mixed thoroughly and incubated overnight at room temperature. Following overnight incubation, FITC-XG was precipitated by addition of ice cold ethanol to a final volume of 110 ml, mixed thoroughly, and incubated at 4°C overnight. The precipitated FITC-XG was washed with water and then transferred to Erlenmeyer bulbs. Residual water and ethanol were removed by evaporation using an EZ-2 Elite evaporator (SP Scientific/Genevac, Stone Ridge, NY, USA) for 4 hours. Dried samples were dissolved in water, and the volume was adjusted to 48 ml with deionized water to generate a final FITC-XG concentration of 5 mg per ml with an expected average molecular weight of 100 kDa.

Xyloglucan endotransglycosylation activity was assessed using the following assay. Reactions of 200 μl containing 1 mg of tamarind seed xyloglucan per ml, 0.01 mg/ml FITC-XGOs prepared as described above, and 10 μl of appropriately diluted XET were incubated for 10 minutes at 25°C in 20 mM sodium citrate pH 5.5 in opaque 96-well microtiter plates. Fluorescence polarization was monitored continuously over this time period, using a SPECTRAMAX® M5 microplate reader (Molecular Devices, Sunnyvale, CA, USA) in top-read orientation with an excitation wavelength of 490 nm, an emission wavelength of 520 nm, a 495 cutoff filter in the excitation path, high precision (100 reads), and medium photomultiplier tube sensitivity. XET-dependent incorporation of fluorescent XGOs into non-fluorescent XG results in increasing fluorescence polarization over time. The slope of the linear regions of the polarization time progress curves was used to determine the activity.

Relative improvements in production yield over the parent gene for day 7 broths of the two variants relative to the wild-type VaXET16 are shown in Table 2 below. Variant A40G + I53A + N175S was produced in an amount that was 3.1X greater than the wild-type VaXET16, while variant A40G + F183I was produced in an amount that was 1.2X greater than the wild-type VaXET16. SDS-PAGE analysis of the same broths showed a VaXET band of increased intensity over wild-type VaXET for both variants which correlated well with the relative improvements observed in the activity assay. SDS-PAGE analysis of the samples taken on days 3, 4, 5, 6, and 7 showed increased production of VaXET and each variant day by day with day 7 the strongest.

Table 2.

| **Relative Improvement Over Parent** | |
|---|---|
| **VaXET16 Variant** | **Fluorescence Polarization Assay** |
| Wild-Type | 1.0 |
| A40G, I53A, N175S | 3.1 |
| A40G, F183I | 1.2 |

The invention described and claimed herein is not to be limited in scope by the specific aspects herein disclosed, since these aspects are intended as illustrations of several aspects of the invention. Any equivalent aspects are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

**Example 10: *Trichoderma reesei* protoplast generation and transformation**

Protoplast preparation and transformation were performed using the following protocol based on Penttila et al., 1987, Gene 61: 155-164. A *Trichoderma reesei* strain was cultivated in 25 ml of YP medium supplemented with 2% (w/v) glucose and 10 mM uridine at 27°C for 17 hours with gentle agitation at 90 rpm. Mycelia were collected by filtration using a Vacuum Driven Disposable Filtration System (Millipore) and washed twice with deionized water and twice with 1.2 M sorbitol. Protoplasts were generated by suspending the washed mycelia in 20 ml of 1.2 M sorbitol containing 15 mg of GLUCANEX® 200 G (Novozymes A/S, Bagsvaerd, Denmark) per ml and 0.36 units of chitinase (Sigma Chemical Co.) per ml for 15-25 minutes at 34°C with gentle shaking at 90 rpm. Protoplasts were collected by centrifuging for 7 minutes at 400 x g and washed twice with cold 1.2 M sorbitol. The protoplasts were counted using a haemacytometer and resuspended to a final concentration of 1 x $10^8$ protoplasts/ml in STC.

Approximately 1-10 μg of DNA were added to 100 μl of the protoplast solution and mixed gently. PEG buffer (250 μl) was then added, and the transformation reaction was mixed and incubated at 34°C for 30 minutes. STC (3 ml) was then added to the transformation reaction and mixed. The transformation reaction was then spread onto COVE plates for *amdS* selection. The plates were incubated at 28°C for 6-11 days.

For transformation requiring hygromycin selection, the reaction mix in STC was spread onto PDA plates supplemented with 1 M sucrose. After incubation at 28°C for 16 hours, 20 ml of overlay PDA medium supplemented with 35 μg of hygromycin B per ml were added to each plate. The plates were incubated at 28°C for 4-7 days.

**Example 11: Genomic DNA extraction from *Trichoderma reesei* strains**

A *Trichoderma reesei* strain was grown in 50 ml of YP medium supplemented with 2% glucose (w/v) in a 250 ml baffled shake flask at 28°C for 2 days with agitation at 200 rpm. Mycelia from each cultivation were collected using a MIRACLOTH® (EMD Chemicals Inc.) lined funnel, squeeze-dried, and frozen under liquid nitrogen. The frozen mycelia were transferred to a pre-chilled mortar and pestle. Each mycelia preparation was ground into a fine powder and kept frozen with liquid nitrogen. A total of 1-2 g of powder was transferred to a 50 ml tube and genomic DNA was extracted from the ground mycelial powder using a DNEASY® Plant Maxi Kit (QIAGEN Inc.). Five ml of Buffer AP1 (QIAGEN Inc.) pre-heated to 65°C were added to the 50 ml tube followed by 10 μl of a RNase A 100 mg/ml stock solution (QIAGEN Inc.), and incubated for 2-3 hours at 65°C. A total of 1.8 ml of AP2 Buffer (QIAGEN Inc.) was added and the tube was incubated on ice for 5 minutes followed by centrifugation at 3000-5000 x g for 5 minutes in a LEGEND™ RT swinging bucket centrifuge (Thermo Fisher Scientific Inc.). The supernatant was transferred to a QIAShredder™ Maxi Spin Column (QIAGEN Inc.) placed in a 50 ml collection tube, and centrifuged at 3000-5000 x g at room temperature for 5 minutes (15-25°C) in a swing-out rotor. The flow-through in the collection tube was transferred, without disturbing the pellet, into a new 50 ml tube. A 1.5 ml volume of Buffer AP3/E (QIAGEN Inc.) was added to the cleared lysate, and mixed immediately by vortexing. The sample (maximum 15 ml), including any precipitate that may have formed, was pipetted into a DNEASY® Maxi Spin Column (QIAGEN Inc.) placed in a 50 ml collection tube and centrifuged at 3000-5000 x *g* for 5 minutes at room temperature (15-20°C) in a swing-out rotor. The flow-through was discarded. Twelve ml of Buffer AW (QIAGEN Inc.) were added to the DNEASY® Maxi Spin Column, and centrifuged at 3000-5000 x g for 10 minutes to dry the membrane. The flow-through and collection tube were discarded. The DNEASY® Maxi Spin Column was transferred to a new 50 ml tube. The DNA was eluted by adding 1-1.5 ml of Kit-supplied buffer AE, pre-heated to 65°C, directly onto the DNEASY® Maxi Spin Column membrane, incubating at room temperature for 5 minutes (15-25°C), and then centrifuging at 3000-5000 x g for 5 minutes. The concentration and purity of the genomic DNA was determined by measuring the absorbance at 260 nm and 280 nm.

**Example 12: Southern blot analysis of transformants**

Two μg of genomic DNA from each transformant were digested with selected restriction enzyme(s). The digestions were submitted to 0.7-0.8% agarose gel electrophoresis in TAE buffer and blotted onto a HYBOND® N+ blotting membrane (GE Healthcare Life Sciences) or a NYTRAN® SuperCharge membrane (Schleicher & Schuell BioScience) using a TURBOBLOTTER® (GE Healthcare Life Sciences) for approximately 1-2 hours. The membrane was hybridized with a digoxigenin-labeled gene-specific or site-specific probe, which was synthesized by PCR using a PCR DIG Probe Synthesis Kit (Roche Applied Science Corp.).

The probe was boiled for 5 minutes, chilled on ice for 2 minutes, and added to 10 ml of DIG Easy Hyb to produce the hybridization solution. Hybridization was performed in DIG Easy Hyb buffer at 42°C for 15-17 hours. The membrane was then washed in 2X SSC plus 0.1% SDS at room temperature for 5 minutes followed by two washes in 0.5X SSC plus 0.1% SDS each at 65°C for 15 minutes. After one wash in 1X Blocking Solution (Roche Applied Science Corp.) for a minimum of one hour, the membrane was incubated with 50 ml of 1X Blocking Solution containing 3.75 U of anti-digoxigenin-AP Fab fragments (Roche Applied Science Corp.) for 10 minutes, followed by two washes in 1X Washing Solution (Roche Applied Science Corp.). The CDP-STAR® ready-to-use reagent (disodium 2-chloro-5-(4-methoxyspiro (1,2-dioxetane-3,2'-(5'-chloro)tricyclo[3.3.1.1$^{3,7}$]decan}-4-yl)-1-phenyl phosphate; Roche Applied Science Corp.) was then applied to the membrane and the probe-target hybrids were detected by autoradiography.

**Example 13: Construction of FLP/FRT integration plasmid pJfyS148B**

The FRT-F3 site was first inserted into plasmid pSMai155 (WO 05/074647) using a QUICKCHANGE® II XL Site-Directed Mutagenesis Kit (Agilent Technologies) with the mutagenic insertion primers shown below.

Forward primer:

5'-CGAATTCTGCATTGAAGTTCCTATTCCGAGTTCCTATTCTTCAAATAGTATAGGAACTT CAGATATCCATCACACTGGCG-3' (SEQ ID NO: 41)

Reverse primer:

5'-GCCAGTGTGATGGATATCTGAAGTTCCTATACTATTTGAAGAATAGGAACTCGGAATA GGAACTTCAATGCAGAATTCGC-3' (SEQ ID NO: 42)

The mutagenic PCR contained 10 ng of pSMai155, 200 μM dNTPs, 125 ng of each primer, 1X QUICKCHANGE® Reaction Buffer (Agilent Technologies), 3 μl of QUIKSOLUTION® reagent (Agilent Technologies), and 2.5 units of *Pfu* Ultra High Fidelity DNA polymerase (Agilent Technologies) in a final volume of 50 μl. The PCR was performed in a thermocycler programmed for 1 cycle at 95°C for 1 minute; 18 cycles each at 95°C for 50 seconds, 60°C for 50 seconds, and 68°C for 40 seconds; and 1 cycle at 68°C for 7 minutes. One μl of Kit-supplied *Dpn* I was added and the reaction was incubated at 37°C for 1 hour. Two μl of the *Dpn* I-treated reaction were added to 45 μl of Kit-supplied XL10-Gold Ultracompetent *E. coli* cells (Agilent Technologies) in a 14 ml tube and incubated on ice for 30 minutes. The tube was incubated at 42°C for 30 seconds after which 0.5 ml of SOC medium was added. The tube was then incubated at 37°C with agitation at 200 rpm for 1 hour after which 250 μl each were plated onto 2 X 150 mm 2XYT plus ampicillin plates and incubated at 37°C overnight. *E. coli* transformants were inoculated into 3 ml of LB + Amp medium in 14 ml tubes and incubated overnight at 37°C with agitation at 200 rpm. Plasmid DNA was isolated using a BIOROBOT® 9600 (QIAGEN Inc.). The insert was confirmed by DNA sequencing. One transformant was identified as containing the desired sequence insertion corresponding to the FRT-F3 site and the plasmid was designated pJfyS148A.

The FRT-F sequence was then inserted into plasmid pJfyS148A using an IN-FUSION® Advantage PCR Cloning Kit (Clontech Laboratories, Inc.). The FRT-F site was first amplified by PCR from plasmid pRika147 (WO 2012/120093) using the primers shown below.

Forward primer:

5'-ATATCCATCACACTGGCGGCCGCTCAACTCTCTCCTCTAGGTTGAAGTTCCTATTCCG AGTTC-3' (SEQ ID NO: 43)

Reverse primer:
5'-AGGATGCATGCTCGAGCATGCACTAGCTAGTTGAAGTTCCTATAC-3' (SEQ ID NO: 44)

The PCR was composed of 20 ng of pRika147, 200 μM dNTPs, 0.4 μM primers, 1X PHUSION® Reaction Buffer (Thermo Fisher Scientific, Inc.), and 2 units of PHUSION® High Fidelity DNA polymerase (Thermo Fisher Scientific, Inc.) in a final volume of 50 μl. The reaction was performed in a thermocycler programmed for 1 cycle at 95°C for 2 minutes; 30 cycles each at 95°C for 25 seconds, 50°C for 25 seconds, and 72°C for 40 seconds; and 1 cycle at 72°C for 7 minutes. The completed PCR was submitted to 2% agarose gel electrophoresis in TAE buffer where a 0.1 kb fragment was excised from the gel and agarose was extracted using a MINELUTE® Gel Extraction Kit (QIAGEN Inc.). Briefly, 3 volumes of Kit-supplied buffer QG were added to the gel slice and dissolved at 50°C for approximately 10 minutes. The dissolved gel slice was applied to a Kit-supplied spin column by transferring to the column and centrifuging at 13,000 rpm for 1 minute. The column was washed with 750 μl of Kit-supplied buffer PE and then re-centrifuged. DNA was eluted with 10 μl of Kit-supplied buffer EB.

The 0.1 kb PCR product was inserted into Sal I-digested pJfyS148A using an IN-FUSION® Advantage PCR Cloning Kit. The reaction was composed of 1X IN-FUSION® Reaction Buffer, 125 ng of pJfyS147A, 20 ng of FRT-F PCR product, and 1 μl of IN-FUSION® Enzyme in a 10 μl reaction volume. The reaction was incubated at 50°C for 15 minutes. Then 40 μl of TE were added to the reaction and 2 μl were transformed into ONE SHOT® TOP10 *E. coli* chemically competent cells (Invitrogen Corp.) by addition to a single use tube containing the competent cells and incubating the cells on ice for 5 minutes. The tube was incubated at 42°C for 30 seconds after which 250 μl of SOC medium were added. The tube was then incubated at 37°C with agitation at 200 rpm for 1 hour and 250 μl were transferred to a 150 mm 2XYT plus ampicillin plate and incubated overnight at 37°C. *E. coli* transformants were inoculated into 3 ml of LB + Amp medium in 14 ml tubes and incubated overnight at 37°C with agitation at 200 rpm. Plasmid DNA from *E. coli* transformants was isolated using a BIOROBOT® 9600 (QIAGEN Inc.). The insert was confirmed by DNA sequencing. One transformant was identified as containing the insert with no PCR errors and the plasmid was designated pJfyS148B.

**Example 14: Construction of plasmid pJfyS156**

To construct plasmid pJfyS156 the *cbh2* gene promoter and flippase gene were PCR amplified using gene-specific primers shown below and inserted into plasmid pJfyS148B.

Flippase

Forward primer:
5'-CACCCTCTGTGTATTGCACCATGCCCCAGTTCGATATCCTCTGCA-3' (SEQ ID NO:45)
Reverse primer:
5'-AAACTCTAGGATGCATGCAAGTGAGGCTATTGCCTATCAGCTC-3' (SEQ ID NO:46)

*cbh2* gene promoter

Forward primer:
5'-CATCACACTGGCGGCCGCGAATTCTAGGCTAGGTATGC-3' (SEQ ID NO:47)
Reverse primer:
5'-GGTGCAATACACAGAGGGTG-3' (SEQ ID NO: 48)

The PCR was composed of 20 ng of template pRiKa147 for the flippase PCR or 150 ng of *T. reesei* 981-0-8 genomic DNA for the *cbh2* promoter PCR, 200 μM dNTPs, 0.4 μM primers, 1X PHUSION® Reaction Buffer, and 2 units of PHUSION® High Fidelity DNA polymerase in a final volume of 50 μl. The reactions were performed in a thermocycler programmed for 1 cycle at 95°C for 2 minutes; 30 cycles each at 95°C for 30 seconds, 57°C for 30 seconds, and 72°C for 2 minutes; and 1 cycle at 72°C for 7 minutes. The completed PCRs were submitted to 1% agarose gel electrophoresis in TAE buffer where 1.2 and 0.6 kb bands, corresponding to the coding region for the S. *cerevisiae* flippase gene and the *niaD* terminator and *cbh2* gene promoter, respectively, were excised from the gels and agarose was extracted using a Nucleospin® Extract II Kit (Macherey Nagel, Bethlehem, PA, USA). Three volumes of Kit-supplied NT buffer were added to the gel slice and the sample was heated at 50°C for 10 minutes. The entire solution was transferred to a Kit-supplied centrifugal column. The column was centrifuged at 13,000 rpm for 1 minute, and washed with Kit-supplied wash buffer NT3 and re-centrifuged. DNA was eluted with 30 μl of Kit-supplied elution buffer NE and centrifuged at 13,000 rpm for 1 minute.

The *cbh2* gene promoter and flippase coding sequence were inserted in a single step into *Xho* I-linearized pJfyS148B using an IN-FUSION® Advantage PCR Cloning Kit. The reaction was composed of 1X IN-FUSION® Reaction Buffer, 180 ng of *Xho* I-linearized pJfyS148B, 100 ng of the 0.6 kb *cbh2* promoter PCR product, 240 ng of the 1.2 kb flippase PCR product, and 1 μl of IN-FUSION® Enzyme in a 10 μl reaction volume. The reaction was incubated for 15 minutes at 37°C and then 15 minutes at 50°C. Then 40 μl of TE were added to the reaction and 2 μl were transformed

into ONE SHOT® TOP10 *E. coli* chemically competent cells according to Example 14. *E. coli* transformants were inoculated into 3 ml of LB + Amp medium in 14 ml tubes and incubated overnight at 37°C with agitation at 200 rpm. Plasmid DNA was isolated using a BIOROBOT® 9600. The insert was confirmed by DNA sequencing. One transformant was identified as containing the inserts with no PCR errors and the plasmid was designated pJfyS155.

An *A. fumigatus* beta-glucosidase gene was amplified by PCR from pEJG107 (WO 05/047499) using the primers shown below.

Forward primer:
5'-ACCGCGGACTGCGCACCATGAGATTCGGTTGGCTCGAGG-3' (SEQ ID NO: 49)
Reverse primer:
5'-TTCGCCACGGAGCTTACTAGTAGACACGGGGCAGAGGC-3' (SEQ ID NO: 50)

The PCR was composed of 20 ng of pEJG107, 200 μM dNTPs, 0.4 μM primers, 1X PHUSION® Reaction Buffer, and 2 units of PHUSION® High Fidelity DNA polymerase in a final volume of 50 μl. The reaction was performed in a thermocycler programmed for 1 cycle at 95°C for 2 minutes; 25 cycles each at 95°C for 30 seconds, 57°C for 30 seconds, and 72°C for 2 minutes; and 1 cycle at 72°C for 7 minutes. The completed PCR was submitted to 1% agarose gel electrophoresis in TAE buffer where a 3 kb band was excised from the gel and agarose was extracted using a MINELUTE® Gel Extraction Kit (Example 13).

The *A. fumigatus* beta-glucosidase PCR product was inserted into *Nco* I/*Pac* I-digested pJfyS155 using an IN-FUSION® Advantage PCR Cloning Kit. The reaction was composed of 150 ng of *Nco* I/*Pac* I-digested pJfyS155, 100 ng of the *A. fumigatus* beta-glucosidase PCR product, 1X IN-FUSION® Advantage Buffer, and 1 μl of IN-FUSION® Enzyme in a 10 μl reaction volume. The reaction was incubated for 15 minutes at 37°C and then 15 minutes at 50°C. Then 40 μl of TE were added to the reaction and 2 μl were transformed into ONE SHOT® TOP10 *E. coli* chemically competent cells according to Example 13. Plasmid DNA from *E. coli* transformants was isolated using a BIOROBOT® 9600. The insert was confirmed by DNA sequencing. One transformant was identified as containing the inserts with no PCR errors and the plasmid was designated pJfyS156 (Figure 9).

**Example 15: Construction of plasmid pDM313**

A 0.38 kb PCR fragment containing a portion of the *hpt* marker, the FRT-F3 site, and a portion of the *T. reesei gpdA* promoter was amplified from pJfyS156 using the primers shown below.

Forward primer:
5'-CGTGTTTCTTCCCATTCGCATGCGACCTCGTGGTCATTGAC-3' (SEQ ID NO: 51)
Reverse primer:
5'-GCTTTGACGTTACATTGACGTACTTATAAGCGGCCGCCAGTGTGATGGA-3' (SEQ ID NO: 52)

The PCR was composed of 50 picomoles of each of the primers, 100 ng of pJfyS156 DNA, 1X PHUSION™ High-Fidelity Hot Start DNA Polymerase buffer (Thermo Fisher Scientific, Inc.), 1 μl of a 10 mM blend of dNTPs, and 1 unit of PHUSION™ High-Fidelity Hot Start DNA Polymerase (Thermo Fisher Scientific, Inc.) in a final volume of 50 μl. The reaction was performed in a thermocycler programmed for 1 cycle at 98°C for 2 minutes; 34 cycles each at 98°C for 15 seconds, 59°C for 30 seconds, and 72°C for 1 minute; and 1 cycle at 72°C for 10 minutes.

A 1.0 kb PCR fragment containing the *T. reesei gpdA* promoter was amplified from *T. reesei* RutC30 genomic DNA using the primers shown below.

Forward primer:
5'-TCCATCACACTGGCGGCCGCTTATAAGTACGTCAATGTAACGTCAAAGC-3' (SEQ ID NO: 53)
Reverse primer:
5'-TGCAGAGGATATCGAACTGGGGCATTTTGTATCTGCGAATTGAGCTTG-3' (SEQ ID NO: 54)

The PCR was composed of 50 picomoles of each of the primers, 100 ng of *T. reesei* RutC30 genomic DNA, 1X PHUSION™ High-Fidelity Hot Start DNA Polymerase buffer, 1 μl of a 10 mM blend of dNTPs, and 1 unit of PHUSION™ High-Fidelity Hot Start DNA Polymerase in a final volume of 50 μl. The reaction was performed in a thermocycler programmed for 1 cycle at 98°C for 2 minutes; 34 cycles each at 98°C for 15 seconds, 59°C for 30 seconds, and 72°C for 1 minute; and 1 cycle at 72°C for 10 minutes.

A 1.8 kb PCR fragment containing the coding region for the S. *cerevisiae* flippase gene and the *niaD* terminator was amplified from plasmid pJfyS156 using the primers shown below.

Forward primer:
5'-CAAGCTCAATTCGCAGATACAAAATGCCCCAGTTCGATATCCTCTGCA-3' (SEQ ID NO: 55)
Reverse primer:
5'-GCTGTTTAAACTCTAGGATGCATGCAAGTGAGGCTATTGCC-3' (SEQ ID NO: 56)

The PCR was composed of 50 picomoles of each of the primers, 100 ng of pJfyS156 DNA, 1X PHUSION™ High-Fidelity Hot Start DNA Polymerase buffer, 1 µl of a 10 mM blend of dNTPs, and 1 unit of PHUSION™ High-Fidelity Hot Start DNA Polymerase in a final volume of 50 µl. The reaction was performed in a thermocycler programmed for 1 cycle at 98°C for 2 minutes; 34 cycles each at 98°C for 15 seconds, 59°C for 30 seconds, and 72°C for 1 minute; and 1 cycle at 72°C for 10 minutes.

The three completed PCRs described above were analysed by 0.8% agarose gel electrophoresis in TAE buffer where fragments of 0.38 kb, 1.0 kb, and 1.8 kb were confirmed. The PCR fragments in the original reactions were used as template for a SOE PCR described below.

The 0.38 kb and 1.0 kb PCR fragments were joined by SOE PCR using the primers shown below.

Forward primer:
5'-CGTGTTTCTTCCCATTCGCATGCGACCTCGTGGTCATTGAC-3' (SEQ ID NO: 57)
Reverse primer:
5'-TGCAGAGGATATCGAACTGGGGCATTTTGTATCTGCGAATTGAGCTTG-3' (SEQ ID NO: 58)

The PCR was composed of 50 picomoles of each of the primers, 0.3 µl of the 0.38 kb fragment PCR, 0.6 µl of the 1.0 kb fragment PCR, 1X PHUSION™ High-Fidelity Hot Start DNA Polymerase buffer, 1 µl of a 10 mM blend of dNTPs, and 1 unit of PHUSION™ High-Fidelity Hot Start DNA Polymerase in a final volume of 50 µl. The reaction was performed in a thermocycler programmed for 1 cycle at 98°C for 2 minutes; 34 cycles each at 98°C for 15 seconds, 60°C for 30 seconds, and 72°C for 1.5 minutes, and 1 cycle at 72°C for 10 minutes. Five SOE PCRs were performed and the reactions were combined. The 1.36 kb SOE PCR fragment and the 1.8 kb PCR fragment containing the flippase coding sequence and *niaD* terminator were separated by 0.8% agarose gel electrophoresis in TAE buffer where a 1.36 kb fragment and a 1.8 kb fragment were excised from the gel and extracted using a Nucleospin® Extract II Kit (Example 14).

Seventeen µg of pJfyS156 DNA were digested with *Sph* I and purified by 0.8% agarose gel electrophoresis in TAE buffer where an approximately 8.7 kb fragment was excised from the gel and extracted using a Nucleospin® Extract II Kit (Example 14).

The 1.36 kb SOE PCR fragment and the 1.8 kb PCR fragment, containing the flippase coding sequence and *niaD* terminator, were inserted into *Sph* I digested pJfyS156 using an IN-FUSION® HD Cloning Kit. The reaction was composed of 116 ng of *Sph* I digested pJfyS156, 57 ng of the 1.36 kb SOE PCR fragment, 69 ng of the 1.8 kb PCR fragment, and 2 µl of IN-FUSION® buffer with Enzyme in a 10 µl reaction volume. The reaction was incubated at 50°C for 15 minutes and then chilled on ice. A 40 µl aliquot of TE was added. Two µl of the reaction transformed into ONE SHOT® TOP10 *E. coli* chemically competent cells according to Example 13. Plasmid DNA was isolated from the transformants using a BIOROBOT® 9600. Transformants were screened by restriction digestion with *Nsi* I which produced 2.1 kb, 2.6 kb, and 7 kb fragments. DNA sequencing of one clone verified that the construct contained the correct inserts with no PCR errors. The construct was designated pDM313.

**Example 16: Construction of plasmid pQM43**

Plasmid pQM43 was constructed for targeting a non-functional amdS fragment (designated "non-functional amdS fragment 3") flanked at its 5' by a FRT-F site to the *T. reesei cbh1* gene locus. In the construct, the FRT-F site (49 bp) was added within the first intron of an approximately 1 kb non-functional amdS fragment as described below. An approximately 400 bp fragment containing the *T. reesei cbh1* 5' flanking region and FRT-F fragment was amplified from *T. reesei* RutC30 genomic DNA using primers 1208187 and 1208194 shown below. The non-functional amdS fragment 3 flanked by a FRT-F site was amplified from pAllo1 (WO 04/111228) using primers 1208195 and 1208196 shown below.

Primer 1208187:
5'-GATTGAGTTGAAACTGCCTAAGATCTCG-3' (SEQ ID NO: 59)
Primer 1208194:

5'-CTATACTTTCTAGAGAATAGGAACTCGGAATAGGAACTTCAAGGTGCGCAGTC
CGCGGTTGAC-3' (SEQ ID NO: 60)

Primer 1208195:

5'-CTATTCCGAGTTCCTATTCTCTAGAAAGTATAGGAACTTCGGCGTTGTTACATC TCCCTGAG-3' (SEQ ID NO: 61)

Primer 1208196:
5'-GCGTCAGGCTTTCGCCACGTCTACGCCAGGACCGAGCAAG-3' (SEQ ID NO: 62)

The first PCR was composed of 100 ng of *T. reesei* RutC30 genomic DNA, 1 µl of 10 mM dNTPs, 1 µM primers, 1X PHUSION® High-Fidelity Reaction Buffer (Thermo Fisher Scientific, Inc.), and 1 unit of PHUSION® Hot Start High-Fidelity DNA Polymerase (Thermo Fisher Scientific, Inc.) in a final volume of 50 µl. The second PCR was composed of 100 ng of pAll01, 1 µl of 10 mM dNTPs, 1 µM primers, 1X PHUSION® High-Fidelity Reaction Buffer, and 1 unit of PHUSION® Hot Start High-Fidelity DNA Polymerase in a final volume of 50 µl. Both reactions were performed in a thermocycler programmed for 1 cycle at 95°C for 2 minutes; 35 cycles each at 95°C for 15 seconds, 60°C for 30 seconds, and 72°C for 1 minute and 15 seconds; and 1 cycle at 72°C for 10 minutes. The PCR products were separated by 0.7% agarose gel electrophoresis in TAE buffer where fragments of approximately 400 bp and 1 kb was excised from the gel and extracted using a Nucleospin® Extract II Kit (Example 14).

The third PCR was composed of 100 ng of each of the 400 bp and 1 kb purified PCR products, 1 µl of 10 mM dNTPs, 1X PHUSION® High-Fidelity Reaction Buffer, and 1 unit of PHUSION® Hot Start High-Fidelity DNA Polymerase in a final volume of 48 µl. The reaction was performed in a thermocycler programmed for 1 cycle at 95°C for 2 minutes; and 5 cycles each at 95°C for 15 seconds, 60°C for 30 seconds, and 72°C for 2 minutes. Primers 1208187 and 1208196 were then added at final concentrations of 1 µM and continued for 30 cycles each at 95°C for 15 seconds, 60°C for 30 seconds, and 72°C for 2 minutes, and 1 cycle at 72°C for 10 minutes. The PCR product was separated by 0.7% agarose gel electrophoresis in TAE buffer where a fragment of approximately 1.4 kb were excised from the gel and extracted using a Nucleospin® Extract II Kit as above.

The 1.4 kb PCR product was inserted into an approximately 9.3 kb *Bgl* II/*Pac* I digested pJfyS139 (WO 2013/028927) using an IN-FUSION™ HD Cloning Kit (Clontech Laboratories, Inc.). The reaction was composed of 1X IN-FUSION™ HD Enzyme Premix (Clontech Laboratories, Inc.), 200 ng of *Bgl* II/*Pac* I digested pJfyS139, and 61 ng of the 1.4 kb PCR product in a 10 µl reaction volume. The reaction was incubated at 50°C for 15 minutes. After the incubation period, a 1 µl aliquot was transformed into ONE SHOT® TOP10 Chemically Competent cells according to Example 13. Plasmid DNA was isolated from the transformants using a BIOROBOT® 9600. The insert was confirmed by DNA sequencing. One transformant was identified as containing the insert with no PCR errors and the plasmid was designated pQM43 (Figure 10).

### Example 17: Protoplast generation and transformation of *Trichoderma reesei* strain AgJg115-104-7B1 to delete the *T. reesei* 42 kDa aspartic protease to create *T. reesei* AgJg115-118-1H1

Protoplast preparation and transformation of *Trichoderma reesei* strain AgJg115-104-7B1 were performed according to Example 10.

Ninety-six µg of the transforming plasmid pAgJg118 (WO 2011/075677) was digested with *Pme* I and purified by 1% agarose gel electrophoresis in TAE buffer where a DNA band was excised from the gel and extracted using a QIAQUICK® Gel Extraction Kit (QIAGEN Inc.). Briefly 3 volumes of Kit-supplied Buffer QG were added to the gel slice and dissolved at 50°C for approximately 10 minutes. The dissolved gel slice was transferred to a spin column and centrifuged at 13,000 rpm for 1 minute. The column was washed with 750 µl of Kit-supplied Buffer PE and then the centrifugation was repeated. DNA was eluted with 25 µl of Kit-supplied Buffer EB. Approximately 1 µg of the resulting purified DNA fragment was added to 100 µl of the protoplast solution for hygromycin selection transformation as described above. Seven transformants were sub-cultured onto new PDA plates to generate spores.

The transformants of *T. reesei* strain AgJg115-104-7B1 were screened by Fungal Spore PCR for the presence of the pAgJg118 deletion vector at the 42 kDa aspartic protease locus. A small amount of spores from each transformant was suspended in 20 µl of Dilution buffer (PHIRE® Plant Direct PCR Kit, Thermo Fisher Scientific Inc.). The spore suspensions were used as templates in the PCRs to screen for the aspartic protease gene deletion. Each reaction was composed of 0.5 µl of the spore suspension, 50 pmol of primer 069134 (shown below), 50 pmol of primer 067947 (shown below), 10 µl of 2X PHIRE® Plant PCR Buffer (PHIRE® Plant Direct PCR Kit), and 0.4 µl of PHIRE® Hot Start II DNA Polymerase (PHIRE® Plant Direct PCR Kit) in a 20 µl reaction. The reactions were performed in a thermocycler programmed for 1 cycle at 98°C for 5 minutes; 40 cycles each at 98°C for 5 seconds, 58°C for 5 seconds, and 72°C for 2 minutes and 20 seconds; 1 cycle at 72°C for 2 minutes; and a 10°C hold. Primer 069134 is located upstream of the 5' flanking region and primer 067947 is located at the beginning of the *E. coli* hygromycin phosphotransferase *(hpt)* gene coding region. If the deletion vector integrates into the aspartic protease locus, the amplified PCR fragment will be 2.4 kb in length. One transformant designated *T. reesei* AgJg115-118-1 was identified as having the aspartic protease gene deleted.

Primer 069134 (forward):
5'-CGCAATCTATCGAATAGCAG-3' (SEQ ID NO: 63)
Primer 067947 (reverse):
5'-CTACATCGAAGCTGAAAGCACGAGA-3' (SEQ ID NO: 64)

The deletion construct pAgJg118 contains the *E. coli* hygromycin phosphotransferase *(hpt)* gene and the Herpes simplex virus thymidine kinase *(tk)* gene flanked by direct repeats. The direct repeats were inserted to facilitate the curing out of the *hpt* and *tk* selectable markers and generate a clean deletion of the 42 kDa aspartic protease.

Spores from *T. reesei* AgJg115-118-1 were spread onto *Trichoderma* Minimal medium plates containing 1 μM 5-fluoro-2'-deoxyuridine (FdU) and incubated at 28°C. Nine isolates were sub-cultured onto PDA plates and incubated at 28°C. The isolates were then screened for the absence of the *hpt* and *tk* markers by Fungal Spore PCR in a similar manner described above. The PCR screen was composed of 0.5 μl of the spore suspension, 50 pmol of primer 069134, 50 pmol of primer 1200593, 10 μl of 2X PHIRE® Plant PCR Buffer, and 0.4 μl of PHIRE® Hot Start II DNA Polymerase in a 20 μl reaction. The reaction was performed in a thermocycler programmed for 1 cycle at 98°C for 5 minutes; 40 cycles each at 98°C for 5 seconds, 58°C for 5 seconds, and 72°C for 1 minute and 45 seconds; 1 cycle at 72°C for 1 minute; and a 10°C hold. Primer 069134 is located upstream of the 5' flanking region and primer 067947 is located at the downstream of the 3' flanking region. If the aspartic protease coding sequence is deleted and the *hpt* and *tk* markers are looped out, the amplified PCR fragment will be 3.6 kb in length.

Genomic DNA of the *T. reesei* AgJg115-118-1 isolates was prepared as described in Example 11 and analyzed by Southern blot analysis as described in Example 12 to confirm the deletion of the 42 kDa aspartic protease. For Southern blot analysis, 2 μg of each genomic DNA was digested with 10 units of *Nco* I in a 30 μl reaction volume and subjected to 0.7% agarose gel electrophoresis in TAE buffer and transferred to a NYTRAN® SuperCharge membrane as described in Example 12.

The membrane was hybridized with a 500 bp digoxigenin-labeled *T. reesei* 42 kDa aspartic protease probe, which was synthesized by incorporation of digoxigenin-11-dUTP by PCR using the primers shown below.

Primer 069860 (sense):
5'-CTTCTATCTTGGGATGCTTCACGATACGTGA-3' (SEQ ID NO: 65)
Primer 069861 (antisense):
5'-CGCGCCCTTGAATATCGGAGAAGGT-3' (SEQ ID NO: 66)

The PCR was composed of 5 μl of 10X *Taq* Buffer (New England Biolabs, Inc.), 2.5 μl of PCR DIG Labeling Mix (Roche Applied Science Corp.), 5 ng of pAgJg118, 10 pmol of each primer, 2.5 μl of 10 mM dNTPs, 5 units of *Taq* DNA polymerase (New England Biolabs, Inc.), and 36.5 μl of water. The reaction was performed in a thermocycler programmed for 1 cycle at 95°C for 2 minutes; 30 cycles each at 95°C for 30 seconds, 56°C for 30 seconds, and 72°C for 40 seconds; 1 cycle at 72°C for 15 minutes; and a 4°C hold. The probe was purified by 1% agarose gel electrophoresis in TAE buffer, excised from the gel, and extracted using a QIAQUICK® Gel Extraction Kit as above.

Southern blot analysis identified primary transformant *T. reesei* AgJg115-118-1H1 as containing the replacement and being void of the *hpt/tk* markers.

### Example 18: Construction of T. reesei strain QMJi057 for targeting a non-functional *amdS* fragment 3 flanked by a FRT-F site to the *T. reesei cbh1* locus

*Trichoderma reesei* AgJg115-118-1H1 (Example 17) was transformed with 1-5 μg of *Pme* I digested pQM43 to insert the non-functional amdS fragment 3 flanked at its 5' by a FRT-F site at the *cbh1* gene locus. Twenty-six transformants were obtained and each one was picked and transferred to a PDA plate and incubated for 7 days at 30°C. The transformants were cultured in 2 ml of CIM and incubated at 30°C for 3 days with agitation at 250 rpm. Supernatant from each culture was subjected to SDS-PAGE using a CRITERION® 8-16% TGX Stain-Free gel (Bio-Rad Laboratories, Inc.) and PRECISION PLUS® Protein Unstained Standards (Bio-Rad Laboratories, Inc.). Since successful targeted integration of pQM43 at the *cbh1* locus effectively disrupts the *cbh1* gene, SDS-PAGE gels were visually analyzed for loss of the CBH1 protein from the proteome. *T. reesei* QMJi057-5 was identified as producing no CBHI protein and was selected for genomic DNA extraction and Southern blot analysis to confirm the integration of the FRT-F site containing the non-functional *amdS* fragment 3 at the *T. reesei cbh1* gene locus. Genomic DNA was isolated from the transformants according to the procedure described in Example 11.

Genomic DNA was digested with *Nhe* I for Southern blot analysis according to Example 12 with a digoxigenin-labeled *T. reesei cbh1* 3' probe synthesized by PCR using a PCR DIG Probe Synthesis Kit (Roche Applied Science Corp.) and the primers shown below.

Primer 0610249:
5'-GAGAACACAGTGAGACCATAGC-3' (SEQ ID NO: 67)
Primer 0610250:
5'-TCTCAACCCAATCAGCAACATG-3' (SEQ ID NO: 68)

The DIG Probe Synthesis PCR was composed of approximately 100 pg of a PCR fragment containing the *T. reesei cbh1* 3' flanking region used to make pQM21 (WO 2013/028912) as template, 1 μM primers, 5 μl of PCR DIG Synthesis Mix (Roche Applied Science Corp.), 1X PCR buffer with $MgCl_2$ (Roche Applied Science Corp.), and 0.75 μl of Enzyme Mix (Roche Applied Science Corp.) in a final volume of 50 μl. The reaction was performed in a thermocycler programmed for 1 cycle at 95°C for 2 minutes; 10 cycles each at 95°C for 30 seconds, 60°C for 30 seconds, and 72°C for 40 seconds; 20 cycles each at 95°C for 30 seconds, 60°C for 30 seconds, and 72°C for 40 seconds plus an additional 20 seconds for each successive cycle; and 1 cycle at 72°C for 7 minutes. The PCR product was separated by 1% agarose gel electrophoresis in TAE buffer where a 720 bp band was excised from the gel and extracted using a Nucleospin® Extract II Kit (Example 14).

Transformant QMJi057-5 was confirmed by Southern blot analysis to contain the non-functional *amdS* fragment 3 flanked at its 5' by a FRT-F site at the *cbh1* locus, which resulted in a hybridized signal at approximately 7.1 kb recognized by the *T. reesei cbh1* 3' probe,.

**Example 19: Site specific integrations at *T. reesei cbh1* locus in strain QMJi057-5**

An approximately 3 kb DNA fragment containing the coding sequence of an *A. niger* mannosidase (SEQ ID NO: 69 for the DNA sequence and SEQ ID NO: 70 for the amino acid sequence) was amplified from *A. niger* Bo-1 derivative strain CKle47 with primer 0614762 and 0614763 and cloned into *Nco* I and *Pac* I digested pMJ09 (US8318458 B2; approximately 7.2 kb), resulting in plasmid pQM27 (SEQ ID NO: 71). The *A. niger* mannosidase expression cassette in pQM27 is followed by a functional *amdS* marker.

An approximately 6.7 kb DNA fragment containing an *A. niger* mannosidase expression cassette, a non-functional *amdS* fragment 4, and the FRT-F site was amplified from pQM27 with primer 1201956 and 1201606. The PCR was composed of 100 ng of pQM27, 200 μM dNTPs, 0.4 μM primers, 1X PHUSION® Reaction Buffer, and 1 units of PHUSION® High Fidelity DNA polymerase in a final volume of 50 μl. The reaction was performed in a thermocycler programmed for 1 cycle at 98°C for 2 minutes; 35 cycles each at 98°C for 15 seconds, 60°C for 30 seconds, and 72°C for 3.5 minutes; and 1 cycle at 72°C for 10 minutes. The 6.7 kb PCR product was separated by 0.7% agarose gel electrophoresis in TAE, excised from the gel, and extracted using a Nucleospin® Extract II Kit (Example 14).

The purified 6.7 kb PCR product was used as template to amplify a FRT-F site containing fragment using primer 1201956 and 1201957. The resulting FRT-F site containing PCR fragment was separated by 0.7% agarose gel electrophoresis in TAE and then excised from the gel and extracted using a Nucleospin® Extract II Kit (Example 14).

Primer 1210956:
5'-TGATTACGAATTGTTTAAACGGATCCGAATGTAGGATTGTTATCCG-3' (SEQ ID NO: 72)
Primer 1201606:

5'-GAAGTTCCTATACTTTCTAGAGAATAGGAACTCGGAATAGGAACTTCAACCTTATGG GACTATCAAGCTGAC-3' (SEQ ID NO: 73)

Primer 1210957:
5'-GTTACATTGACGTACTTATAAGAAGTTCCTATACTTTCTAGAGAATAGGA-3' (SEQ ID NO: 74)

The purified FRT-F site containing PCR fragment was inserted into *Xba* I and *Psi* I digested pDM313 (approximately 5.4 kb) using an IN-FUSION™ HD Cloning Kit. The reaction was composed of 1X IN-FUSION™ HD Enzyme Premix, 323 ng of *Xba I/Psi* I digested pDM313, and 200 ng of the 6.7 kb FRT-F site containing PCR product in a 15 μl reaction volume. The reaction was incubated at 50°C for 15 minutes. After the incubation period, a 2 μl aliquot was transformed into 50 μl of Stellar™ *E. coli* chemically competent cells (Clontech Laboratories, Inc.) by addition to a tube containing the competent cells and incubating the cells on ice for 30 minutes. The tube was incubated at 42°C for 45 seconds after which 450 μl of SOC medium were added. The tube was then incubated at 37°C with agitation at 250 rpm for 1 hour. Volumes of 50 μl and 150 μl were transferred to two 150 mm 2XYT plus ampicillin plates. The plates were incubated overnight at 37°C. Plasmid DNA was isolated using a BIOROBOT® 9600 and sequenced. One transformant was identified as containing the insert with no PCR errors and the plasmid was designated pQM45 (Figure 11).

Plasmid pQM45 was digested with *Pme* I and used to test site specific integration at the *T. reesei cbh1* locus in strain QMJi057-5. The *Pme* I digested pQM45 was transformed into protoplasts of *T. reesei* strain QMJi057-5 according to Example 10 and the transformation reactions were spread onto COVE plates and incubated at 30°C for 7-10 days. All of the transformations resulted in visible transformants on COVE plates, suggesting that insertion of the FRT-F site into the first intron of the *amdS* gene allows recombination of a functional *amdS* marker and integration at the targeted site.

Genomic DNA was isolated from six transformants according to the procedure described in Example 11. Genomic DNA was digested with *EcoR* I for Southern blot analysis according to Example 12 with a a 358 bp digoxigenin-labeled *T. reesei cbh1* 5' probe (see below), which was synthesized by incorporation of digoxigenin-11-dUTP by PCR using a PCR DIG Probe Synthesis Kit.

Southern blot analysis of all six transformants resulted in a hybridized signal at approximately 4.2 kb recognized by the *T. reesei cbh1* 5' probe, indicating correct integration at the *cbh1* locus.

*T. reesei cbh1* 5' probe sequence:

5'-TAGGGTCGGCAACGGCAAAAAAGCACGTGGCTCACCGAAAAGCAAGATGTTTGCGA
TCTAACATCCAGGAACCTGGATACATCCATCATCACGCACGACCACTTTGATCTGCTGTAA
ACTCGTATTCGCCCTAAACCGAAGTGCGTGGTAAATCTACACGTGGGCCCCTTTCGGTATA
CTGCGTGTGTCTTCTCTAGGTGCCATTCTTTTCCCTTCCTCTAGTGTTGAATTGTTTGTGTT
GGAGTCCGAGCTGTAACTACCTCTGAATCTCTGGAGAATGGTGGACTAACGACTACCGTG
CACCTGCATCATGTATATAATAGTGATCCTGAGAAGGGGGGTTTGGAGCAATGTGGG-3'
(SEQ ID NO: 75)

**Example 20: Construction of plasmid pQM37, pQM38 and pQM39 (functional *amdS* marker with FRT-F3 sites in *amdS* introns)**

Plasmids pQM37, pQM38 and pQM39 were constructed to test the impact on *amdS* function after inserting a FRT-F3 fragment into one of the three introns of the *amdS* gene. The primers shown below were designed to introduce a FRT-F3 fragment into each of the three introns in the *amdS* gene in plasmid pAllo1 using a QUICKCHANGE® II XL Site-Directed Mutagenesis Kit. Primers 1205503 and 1205504 were used to construct plasmid pQM37, where the FRT-F3 site was inserted into intron 1 of the *amdS* gene. Primers 1205505 and 1205506 were used to construct plasmid pQM38, where the FRT-F3 site was inserted into intron 2 of the *amdS* gene. Primers 1205507 and 1205508 were used to construct pQM39, where the FRT-F3 site was inserted into intron 3 of the *amdS* gene.

Primer 1205503:

5'-GGGAGATGTAACAACGCCTTGAAGTTCCTATTCCGAGTTCCTATTCTTCAAATAGTA
TAGGAACTTCAACCTTATGGGACTATCAAG-3' (SEQ ID NO: 76)

Primer 1205504:

5'-CTTGATAGTCCCATAAGGTTGAAGTTCCTATACTATTTGAAGAATAGGAACTCGGAA
TAGGAACTTCAAGGCGTTGTTACATCTCCC-3' (SEQ ID NO: 77)

Primer 1205505:

5'-GCCCCTAAGTCGTTAGATGTTTGAAGTTCCTATTCCGAGTTCCTATTCTTCAAATAGT
ATAGGAACTTCACCCTTTTTGTCAGC-3' (SEQ ID NO: 78)

Primer 1205506:

5'-GCTGACAAAAAGGGTGAAGTTCCTATACTATTTGAAGAATAGGAACTCGGAATAGGAACTTCAAACATCTAACGACTTAGGGGC-3' (SEQ ID NO: 79)

Primer 1205507:

5'-CTATACCAGGCCTCCACTTGAAGTTCCTATTCCGAGTTCCTATTCTTCAAATAGTATAGGAACTTCATGTCCTCCTTTCTTGC-3' (SEQ ID NO: 80)

Primer 1205508:

5'-GCAAGAAAGGAGGACATGAAGTTCCTATACTATTTGAAGAATAGGAACTCGGAATAGGAACTTCAAGTGGAGGCCTGGTATAG-3' (SEQ ID NO: 81)

The PCRs were composed of 10 ng of pAllo1, 1 μl of 10 mM dNTPs, 1X Reaction Buffer, 125 ng of each primer, 1 μl of QUIKSOLUTION® reagent, and 2.5 unit of PfuUltra™ HF DNA Polymerase (Agilent Technologies) in a final volume of 50 μl. The reactions were performed in a thermocycler programmed for 1 cycle at 95°C for 1 minutes; and 18 cycles each at 95°C for 50 seconds, 60°C for 50 seconds, and 68°C for 7 minutes; and 1 cycle at 68°C for 7 minutes. Ten units of *Dpn* I were added to each reaction and incubated at 37°C for one hour. Two μl of each *Dpn* I treated reaction were transformed into XL10-Gold Ultracompetent *E. coli* cells. The transformation reactions were spread onto 2XYT plus ampicillin plates. About 4-6 colonies were picked for each transformation and cultured in 3 ml of LB plus ampicillin medium at 37°C for 15-17 hours with agitation at 250 rpm. Plasmid DNA was extracted from each colony using a QIAprep® Spin Miniprep Kit and submitted for DNA sequencing. One transformant containing the FRT-F3 site inserted into intron 1 of the *amdS* gene was designated pQM37. One transformant containing the FRT-F3 site inserted into intron 2 of the *amdS* gene was designated pQM38. One transformant containing the FRT-F3 site inserted into intron 3 of the *amdS* gene was designated pQM39.

About 30-40 μg of each of pAllo1, pQM37, pQM38, and pQM39 were digested with *Eco* RI and *Pac* I. The digested DNAs were purified using a Nucleospin® Extract II Kit (Example 14). The purified linear DNA fragments of pAllo1, pQM37, pQM38, and pQM39 were each transformed into *T. reesei* RutC30 protoplasts according to Example 10. The transformation reactions were spread onto COVE plates and incubated at 28°C for 7-10 days. All of the transformations resulted in visible transformants on COVE plates, indicating that *amdS* can be used as a functional selection marker with a FRT-F3 site inserted in any one of the three *amdS* introns.

## Claims

1. A method for integrating a polynucleotide library of interest in the chromosome of a filamentous fungal host cell using a site-specific recombinase, thereby achieving highly uniform expression levels, said method comprising the steps of:

    a) providing a filamentous fungal host cell comprising in its chromosome in the following order:

        i) a first recognition sequence of the recombinase or a region that is 5' or 3' of an integration site;
        ii) a first selection marker;
        iii) a second recognition sequence of the recombinase; and
        iv) a non-functional partial second selection marker;

    b) transforming said host cell with a nucleic acid construct comprising in the following order:

        i) the first recognition sequence of the recombinase or the region that is 5' or 3' of the integration site;
        ii) a polynucleotide library of interest;
        iii) a non-functional partial second selection marker; and
        iv) the second recognition sequence of the recombinase;

    c) expressing a gene encoding the site-specific recombinase in said host cell; and
    d) selecting a transformed host cell which expresses the second selection marker and not the first selection marker,

    wherein the polynucleotide library of interest is site-specifically integrated in the correct orientation in the chromosome of the host cell by the recombinase, whereby the first selectable marker is excised from the chromosome and whereby the non-functional partial second selectable markers are recombined to form a functional second selection marker in the chromosome.

2. The method of claim 1, wherein the polynucleotide library comprises polynucleotides encoding variants of a polypeptide of interest; preferably the polypeptide of interest is an enzyme; more preferably the polypeptide of interest is a hydrolase, isomerase, ligase, lyase, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, oxidase, pectinolytic enzyme, peroxidase, phytase, polyphenoloxidase, proteolytic enzyme, ribonuclease, transglutaminase, xylanase, or beta-xylosidase.

3. The method of claim 1 or 2, wherein the filamentous fungal host cell is an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell; preferably the host cell is an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis*

*aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

**4.** The method of any of claims 1 - 3, wherein the site specific recombinase is the *Saccharomyces cerevisiae* 2 μm flippase FLP, the phage TP901-1 integrase, the bacteriophage P1 CRE integrase, the bacterial XerC recombinase, the bacterial XerD recombinase, the lambda phage integrase or the HP1 integrase; preferably the site specific recombinase is the *Saccharomyces cerevisiae* 2 μm flippase FLP.

**5.** The method of any of claims 1 - 4, wherein the first and second recognition sequences of the recombinase are identical or different; preferably the first and second recognition sequences of the recombinase are different in order to effect a directional integration of the polynucleotide library of interest in the host cell chromosome.

**6.** The method of any of claims 1 - 5, wherein the first and second recognition sequences of the recombinase are different recognition sequences of the *Saccharomyces cerevisiae* 2 μm flippase FLP in order to effect a directional integration of the polynucleotide library of interest in the host cell chromosome; preferably the first and second recognition sequences of the recombinase are FRT-F (SEQ ID NO:27) and FRT-F3 (SEQ ID NO:28), respectively, or vice versa.

**7.** A polynucleotide library expression system comprising:

a) a filamentous fungal host cell comprising in its chromosome in the following order:

i) a first recognition sequence of a site-specific recombinase or a region that is 5' or 3' of an integration site;
ii) a first selection marker;
iii) a second recognition sequence of the recombinase; and
iv) a non-functional partial second selection marker; AND

b) a nucleic acid construct comprising in the following elements in order:

i) the first recognition sequence of the recombinase or the region that is 5' or 3' of the integration site;
ii) a polynucleotide library of interest;
iii) a non-functional partial second selection marker; and
iv) the second recognition sequence of the recombinase, and

c) a gene encoding the site-specific recombinase comprised in the filamentous fungal host cell chromosome or in the nucleic acid construct outside of the elements listed in step (b);

wherein, when the host cell is transformed with the nucleic acid construct, the polynucleotide library of interest is site-specifically integrated in the correct orientation in the chromosome of the host cell by the recombinase, thereby achieving highly uniform expression levels, whereby the first selectable marker is excised from the chromosome, and whereby the non-functional partial second selectable markers are recombined to form a functional second selection marker in the chromosome which is expressed.

**8.** The polynucleotide library expression system of claim 7, wherein the polynucleotide library comprises polynucleotides encoding variants of a polypeptide of interest; preferably the polypeptide of interest is an enzyme; more preferably the polypeptide of interest is a hydrolase, isomerase, ligase, lyase, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, oxidase, pectinolytic enzyme, peroxidase, phytase, polyphenoloxidase, proteolytic enzyme, ribonuclease, transglutaminase, xylanase, or beta-xylosidase.

**9.** The polynucleotide library expression system of claim 7 or 9, wherein the filamentous fungal host cell is an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell; preferably the host cell is an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

**10.** The polynucleotide library expression system of any of claims 7 - 9, wherein the site specific recombinase is the *Saccharomyces cerevisiae* 2 μm flippase FLP, the phage TP901-1 integrase, the bacteriophage P1 CRE integrase, the bacterial XerC recombinase, the bacterial XerD recombinase, the lambda phage integrase or the HP1 integrase; preferably the site specific recombinase is the *Saccharomyces cerevisiae* 2 μm flippase FLP.

**11.** The polynucleotide library expression system of any of claims 7 - 10, wherein the first and second recognition sequences of the recombinase are identical or different; preferably the first and second recognition sequences of the recombinase are different in order to effect a directional integration of the polynucleotide library of interest in the host cell chromosome.

**12.** The polynucleotide library expression system of any of claims 7 - 11, wherein the first and second recognition sequences of the recombinase are different recognition sequences of the *Saccharomyces cerevisiae* 2 μm flippase FLP in order to effect a directional integration of the polynucleotide library of interest in the host cell chromosome; preferably the first and second recognition sequences of the recombinase are FRT-F (SEQ ID NO:27) and FRT-F3 (SEQ ID NO:28), respectively, or vice versa.

## Patentansprüche

**1.** Verfahren zum Integrieren einer interessierenden Polynukleotidbibliothek in das Chromosom einer Wirtszelle eines filamentösen Pilzes unter Verwendung einer ortsspezifischen Rekombinase, dadurch Erreichen von sehr einheitlichen Expressionsspiegeln, wobei das Verfahren die Schritte umfasst:

a) Bereitstellen einer Wirtszelle eines filamentösen Pilzes, die in ihrem Chromosom in der folgenden Reihenfolge umfasst:

i) eine erste Erkennungssequenz der Rekombinase oder eine Region, die 5' oder 3' einer Integrationsstelle ist;
ii) einen ersten Selektionsmarker;
iii) eine zweite Erkennungssequenz der Rekombinase; und
iv) und einen nicht funktionellen teilweisen zweiten Selektionsmarker;

b) Transformieren der Wirtszelle mit einem Nukleinsäurekonstrukt, das in der folgenden Reihenfolge umfasst:

i) die erste Erkennungssequenz der Rekombinase oder die Region, die 5' oder 3' der Integrationsstelle ist;
ii) eine interessierende Polynukleotidbibliothek;

iii) einen nicht funktionellen teilweisen zweiten Selektionsmarker; und
iv) die zweite Erkennungssequenz der Rekombinase;

c) Exprimieren eines Gens, das die ortsspezifische Rekombinase in der Wirtszelle kodiert; und
d) Selektieren einer transformierten Wirtszelle, die den zweiten Selektionsmarker und nicht den ersten Selektionsmarker exprimiert,

wobei die interessierende Polynukleotidbibliothek ortsspezifisch in der korrekten Orientierung in das Chromosom der Wirtszelle durch die Rekombinase integriert wird, wobei der erste Selektionsmarker aus dem Chromosom ausgeschnitten wird, und wobei die nicht funktionellen teilweisen zweiten Selektionsmarker rekombiniert werden, um einen funktionellen zweiten Selektionsmarker im Chromosom zu bilden.

**2.** Verfahren nach Anspruch 1, wobei die Polynukleotidbibliothek Polynukleotide umfasst, die Varianten eines interessierenden Polypeptids kodieren; bevorzugt ist das interessierende Polypeptid ein Enzym; weiter bevorzugt ist das interessierende Polypeptid eine Hydrolase, Isomerase, Ligase, Lyase, Oxidoreduktase oder Transferase, z.B. eine Aminopeptidase, Amylase, Carbohydrase, Carboxypeptidase, Katalase, Cellobiohydrolase, Cellulase, Chitinase, Cutinase, Cyclodextrin-Glycosyltransferase, Desoxyribonuklease, Endoglucanase, Esterase, alpha-Galactosidase, beta-Galactosidase, Glucoamylase, alpha-Glucosidase, beta-Glucosidase, Invertase, Laccase, Lipase, Mannosidase, Mutanase, Oxidase, pektinolytisches Enzym, Peroxidase, Phytase, Polyphenoloxidase, proteolytisches Enzym, Ribonuklease, Transglutaminase, Xylanase oder beta-Xylosidase.

**3.** Verfahren nach Anspruch 1 oder 2, wobei die Wirtszelle eines filamentösen Pilzes eine *Acremonium-, Aspergillus-, Aureobasidium-, Bjerkandera-, Ceriporiopsis-, Chrysosporium-, Coprinus-, Coriolus-, Cryptococcus-, Filibasidium-, Fusarium-, Humicola-, Magnaporthe-, Mucor-, Myceliophthora-, Neocallimastix-, Neurospora-, Paecilomyces-, Penicillium-, Phanerochaete-, Phlebia-, Piromyces-, Pleurotus-, Schizophyllum-, Talaromyces-, Thermoascus-, Thielavia-, Tolypocladium-, Trametes-* oder *Trichoderma*-Zelle ist; bevorzugt ist die Wirtszelle eine *Aspergillus awamori-, Aspergillus foetidus-, Aspergillus fumigatus-, Aspergillus japonicus-, Aspergillus nidulans-, Aspergillus niger-, Aspergillus oryzae-, Bjerkandera adusta-, Ceriporiopsis aneirina-, Ceriporiopsis caregiea-, Ceriporiopsis gilvescens-, Ceriporiopsis pannocinta-, Ceriporiopsis rivulosa-, Ceriporiopsis subrufa-, Ceriporiopsis subvermispora-, Chrysosporium inops-, Chrysosporium keratinophilum-, Chrysosporium lucknowense-, Chrysosporium merdarium-, Chrysosporium pannicola-, Chrysosporium queenslandicum-, Chrysosporium tropicum-, Chrysosporium zonatum-, Coprinus cinereus-, Coriolus hirsutus-, Fusarium bactridioides-, Fusarium cerealis-, Fusarium crookwellense-, Fusarium culmorum-, Fusarium graminearum-, Fusarium graminum-, Fusarium heterosporum-, Fusarium negundi-, Fusarium oxysporum-, Fusarium reticulatum-, Fusarium roseum-, Fusarium sambucinum-, Fusarium sarcochroum-, Fusarium sporotrichioides-, Fusarium sulphureum-, Fusarium torulosum-, Fusarium trichothecioides-, Fusarium venenatum-, Humicola insolens-, Humicola lanuginosa-, Mucor miehei-, Myceliophthora thermophila-, Neurospora crassa-, Penicillium purpurogenum-, Phanerochaete chrysosporium-, Phlebia radiata-, Pleurotus eryngii-, Thielavia terrestris-, Trametes villosa-, Trametes versicolor-, Trichoderma harzianum-, Trichoderma koningii-, Trichoderma longibrachiatum-, Trichoderma reesei-* oder *Trichoderma viride*-Zelle.

**4.** Verfahren nach einem beliebigen der Ansprüche 1 - 3, wobei die ortsspezifische Rekombinase die *Saccharomyces cerevisiae* 2 μm-Flippase FLP, die Phagen-TP901-1-Integrase, die Bakteriophagen-P1 CRE-Integrase, die bakterielle XerC-Rekombinase, die bakterielle XerD-Rekombinase, die Lambda-Phagen-Integrase oder die HP1-Integrase ist; bevorzugt ist die ortsspezifische Rekombinase die *Saccharomyces cerevisiae* 2 μm-Flippase FLP.

**5.** Verfahren nach einem beliebigen der Ansprüche 1 - 4, wobei die ersten und zweiten Erkennungssequenzen der Rekombinase identisch oder verschieden sind; bevorzugt sind die ersten und zweiten Erkennungssequenzen der Rekombinase verschieden, um eine gerichtete Integration der interessierenden Polynukleotidbibliothek in das Wirtszellchromosom zu bewirken.

**6.** Verfahren nach einem beliebigen der Ansprüche 1 - 5, wobei die ersten und zweiten Erkennungssequenzen der Rekombinase verschiedene Erkennungssequenzen der *Saccharomyces cerevisiae* 2 μm-Flippase FLP sind, um eine gerichtete Integration der interessierenden Polynukleotidbibliothek in das Wirtszellchromosom zu bewirken; bevorzugt sind die ersten und zweiten Erkennungssequenzen der Rekombinase FRT-F (SEQ ID NO:27) beziehungsweise FRT-F3 (SEQ ID NO:28), oder umgekehrt.

**7.** Polynukleotidbibliothek-Expressionssystem, umfassend:

a) eine Wirtszelle eines filamentösen Pilzes, die in ihrem Chromosom in der folgenden Reihenfolge umfasst:

i) eine erste Erkennungssequenz einer ortsspezifischen Rekombinase oder eine Region, die 5' oder 3' einer Integrationsstelle ist;
ii) einen ersten Selektionsmarker;
iii) eine zweite Erkennungssequenz der Rekombinase; und
iv) einen nicht funktionellen teilweisen zweiten Selektionsmarker; UND

b) ein Nukleinsäurekonstrukt, das in den folgenden Elementen in der Reihenfolge umfasst:

i) die erste Erkennungssequenz der Rekombinase oder die Region, die 5' oder 3' der Integrationsstelle ist;
ii) eine interessierende Polynukleotidbibliothek;
iii) einen nicht funktionellen teilweisen zweiten Selektionsmarker; und
iv) die zweite Erkennungssequenz der Rekombinase, und

c) ein Gen, das die ortsspezifische Rekombinase kodiert, das im Wirtszellchromosom der filamentösen Pilzwirtszelle oder im Nukleinsäurekonstrukt außerhalb der in Schritt (b) aufgezählten Elemente umfasst ist;

wobei, wenn die Wirtszelle mit dem Nukleinsäurekonstrukt transformiert wird, die interessierende Polynukleotidbibliothek ortsspezifisch in der korrekten Ausrichtung im Chromosom der Wirtszelle durch die Rekombinase integriert wird, dadurch Erreichen von sehr einheitlichen Expressionsspiegeln, wobei der erste Selektionsmarker aus dem Chromosom ausgeschnitten wird, und wobei die nicht funktionellen teilweisen zweiten Selektionsmarker rekombiniert werden, um einen funktionellen zweiten Selektionsmarker im Chromosom zu bilden, der exprimiert wird.

**8.** Polynukleotidbibliothek-Expressionssystem nach Anspruch 7, wobei die Polynukleotidbibliothek Polynukleotide umfasst, die Varianten eines interessierenden Polypeptids kodieren; bevorzugt ist das interessierende Polypeptid ein Enzym; stärker bevorzugt ist das interessierende Polypeptid eine Hydrolase, Isomerase, Ligase, Lyase, Oxidoreduktase, oder Transferase, z.B. eine Aminopeptidase, Amylase, Carbohydrase, Carboxypeptidase, Katalase, Cellobiohydrolase, Cellulase, Chitinase, Cutinase, Cyclodextrin-Glycosyltransferase, Desoxyribonuklease, Endoglucanase, Esterase, alpha-Galactosidase, beta-Galactosidase, Glucoamylase, alpha-Glucosidase, beta-Glucosidase, Invertase, Laccase, Lipase, Mannosidase, Mutanase, Oxidase, pektinolytisches Enzym, Peroxidase, Phytase, Polyphenoloxidase, proteolytisches Enzym, Ribonuklease, Transglutaminase, Xylanase oder beta-Xylosidase.

**9.** Polynukleotidbibliothek-Expressionssystem nach Anspruch 7 oder 9, wobei die Wirtszelle eines filamentösen Pilzes eine *Acremonium-, Aspergillus-, Aureobasidium-, Bjerkandera-, Ceriporiopsis-, Chrysosporium-, Coprinus-, Coriolus-, Cryptococcus-, Filibasidium-, Fusarium-, Humicola-, Magnaporthe-, Mucor-, Myceliophthora-, Neocallimastix-, Neurospora-, Paecilomyces-, Penicillium-, Phanerochaete-, Phlebia-, Piromyces-, Pleurotus-, Schizophyllum-, Talaromyces-, Thermoascus-, Thielavia-, Tolypocladium-, Trametes-* oder *Trichoderma*-Zelle ist; bevorzugt ist die Wirtszelle eine *Aspergillus awamori-, Aspergillus foetidus-, Aspergillus fumigatus-, Aspergillus japonicus-, Aspergillus nidulans-, Aspergillus niger-, Aspergillus oryzae-, Bjerkandera adusta-, Ceriporiopsis aneirina-, Ceriporiopsis caregiea-, Ceriporiopsis gilvescens-, Ceriporiopsis pannocinta-, Ceriporiopsis rivulosa-, Ceriporiopsis subrufa-, Ceriporiopsis subvermispora-, Chrysosporium inops-, Chrysosporium keratinophilum-, Chrysosporium lucknowense-, Chrysosporium merdarium-, Chrysosporium pannicola-, Chrysosporium queenslandicum-, Chrysosporium tropicum-, Chrysosporium zonatum-, Coprinus cinereus-, Coriolus hirsutus-, Fusarium bactridioides-, Fusarium cerealis-, Fusarium crookwellense-, Fusarium culmorum-, Fusarium graminearum-, Fusarium graminum-, Fusarium heterosporum-, Fusarium negundi-, Fusarium oxysporum-, Fusarium reticulatum-, Fusarium roseum-, Fusarium sambucinum-, Fusarium sarcochroum-, Fusarium sporotrichioides-, Fusarium sulphureum-, Fusarium torulosum-, Fusarium trichothecioides-, Fusarium venenatum-, Humicola insolens-, Humicola lanuginosa-, Mucor miehei-, Myceliophthora thermophila-, Neurospora crassa-, Penicillium purpurogenum-, Phanerochaete chrysosporium-, Phlebia radiata-, Pleurotus eryngii-, Thielavia terrestris-, Trametes villosa-, Trametes versicolor-, Trichoderma harzianum-, Trichoderma koningii-, Trichoderma longibrachiatum-, Trichoderma reesei-* oder *Trichoderma viride*-Zelle.

**10.** Polynukleotidbibliothek-Expressionssystem nach einem beliebigen der Ansprüche 7 - 9, wobei die ortsspezifische Rekombinase die *Saccharomyces cerevisiae* 2 μm-Flippase FLP, die Phagen-TP901-1-Integrase, die Bakteriophagen-P1 CRE-Integrase, die bakterielle XerC-Rekombinase, die bakterielle XerD-Rekombinase, die Lambda-Phagen-Integrase oder die HP1-Integrase ist; bevorzugt ist die ortsspezifische Rekombinase die *Saccharomyces cerevisiae* 2 μm-Flippase FLP.

**11.** Polynukleotidbibliothek-Expressionssystem nach einem beliebigen der Ansprüche 7 - 10, wobei die ersten und zweiten Erkennungssequenzen der Rekombinase identisch oder verschieden sind; bevorzugt sind die ersten und zweiten Erkennungssequenzen der Rekombinase verschieden, um eine gerichtete Integration der interessierenden Polynukleotidbibliothek in das Wirtszellchromosom zu bewirken.

**12.** Polynukleotidbibliothek-Expressionssystem nach einem beliebigen der Ansprüche 7 - 11, wobei die ersten und zweiten Erkennungssequenzen der Rekombinase verschiedene Erkennungssequenzen der *Saccharomyces cerevisiae* 2 μm-Flippase FLP sind, um eine gerichtete Integration der interessierenden Polynukleotidbibliothek in das Wirtszellchromosom zu bewirken; bevorzugt sind die ersten und zweiten Erkennungssequenzen der Rekombinase FRT-F (SEQ ID NO:27) beziehungsweise FRT-F3 (SEQ ID NO:28), oder umgekehrt.

**Revendications**

**1.** Méthode pour intégrer une banque de polynucléotides d'intérêt dans le chromosome d'une cellule hôte de champignon filamenteux utilisant une recombinase à spécificité de site, atteignant ainsi des niveaux d'expression hautement uniformes, ladite méthode comprenant les étapes suivantes :

a) obtention d'une cellule hôte de champignon filamenteux comprenant dans son chromosome, dans l'ordre suivant :

i) une première séquence de reconnaissance de la recombinase ou une région qui est en 5' ou en 3' d'un site d'intégration ;
ii) un premier marqueur de sélection ;
iii) une deuxième séquence de reconnaissance de la recombinase ; et
iv) un deuxième marqueur de sélection partiel non fonctionnel ;

b) la transformation de ladite cellule hôte avec un produit d'assemblage d'acide nucléique comprenant, dans l'ordre suivant :

i) la première séquence de reconnaissance de la recombinase ou la région qui est en 5' ou en 3' du site d'intégration ;
ii) une banque de polynucléotides d'intérêt ;
iii) un deuxième marqueur de sélection partiel non fonctionnel ; et
iv) la deuxième séquence de reconnaissance de la recombinase ;

c) l'expression d'un gène codant la recombinase à spécificité de site dans ladite cellule hôte ; et
d) la sélection d'une cellule hôte transformée qui exprime le deuxième marqueur de sélection et pas le premier marqueur de sélection,

dans laquelle la banque de polynucléotides d'intérêt est intégrée en mode de spécificité de site avec l'orientation correcte dans le chromosome de la cellule hôte par la recombinase, en conséquence de quoi le premier marqueur sélectionnable est excisé hors du chromosome et les deuxièmes marqueurs sélectionnables partiels non fonctionnels sont recombinés pour former un deuxième marqueur de sélection fonctionnel dans le chromosome.

**2.** Méthode selon la revendication 1, dans laquelle la banque de polynucléotides comprend des polynucléotides codant des variants d'un polypeptide d'intérêt ; de préférence le polypeptide d'intérêt est une enzyme ; mieux encore le polypeptide d'intérêt est une hydrolase, isomérase, ligase, lyase, oxydoréductase, ou transférase, par exemple une aminopeptidase, amylase, carbohydrase, carboxypeptidase, catalase, cellobiohydrolase, cellulase, chitinase, cutinase, cyclodextrine glycosyltransférase, désoxyribonucléase, endoglucanase, estérase, alpha-galactosidase, bêta-galactosidase, glucoamylase, alpha-glucosidase, bêta-glucosidase, invertase, laccase, lipase, mannosidase, mutanase, oxydase, enzyme pectinolytique, peroxydase, phytase, polyphénol oxydase, enzyme protéolytique, ribonucléase, transglutaminase, xylanase, ou bêta-xylosidase.

**3.** Méthode selon la revendication 1 ou 2, dans laquelle la cellule hôte de champignon filamenteux est une cellule d'*Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paelicomyces, Penicillium, Phanerochaete, Phlebia, Piromyces, Pleurotus, Schizophyllum, Talaromyces, Ther-*

*moascus, Thielavia, Tolypocladium, Trametes, ou Trichoderma* ; de préférence la cellule hôte est une cellule d'*Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei, ou Trichoderma viride.*

**4.** Méthode selon l'une quelconque des revendications 1 à 3, dans laquelle la recombinase à spécificité de site est la flippase FLP de *Saccharomyces cerevisiae* de 2 μm, l'intégrase de phage TP901-1, l'intégrase de bactériophage P1 CRE, la recombinase bactérienne XerC, la recombinase bactérienne XerD, l'intégrase de phage lambda ou l'intégrase de HP1 ; de préférence la recombinase à spécificité de site est la flippase FLP de *Saccharomyces cerevisiae* de 2 μm.

**5.** Méthode selon l'une quelconque des revendications 1 à 4, dans laquelle les première et deuxième séquences de reconnaissance de la recombinase sont identiques ou différentes ; de préférence les première et deuxième séquences de reconnaissance de la recombinase sont différentes afin que soit effectuée une intégration directionnelle de la banque de polynucléotides d'intérêt dans le chromosome de la cellule hôte.

**6.** Méthode selon l'une quelconque des revendications 1 à 5, dans laquelle les première et deuxième séquences de reconnaissance de la recombinase sont des séquences de reconnaissance différentes de la flippase FLP de *Saccharomyces cerevisiae* de 2 μm afin que soit effectuée une intégration directionnelle de la banque de polynucléotides d'intérêt dans le chromosome de la cellule hôte ; de préférence les première et deuxième séquences de reconnaissance de la recombinase sont FRT-F (SEQ ID NO : 27) et FRT-F3 (SEQ ID NO : 28), respectivement, ou vice versa.

**7.** Système d'expression de banque de polynucléotides comprenant :

a) une cellule hôte de champignon filamenteux comprenant dans son chromosome, dans l'ordre suivant :

i) une première séquence de reconnaissance de la recombinase ou une région qui est en 5' ou en 3' d'un site d'intégration ;
ii) un premier marqueur de sélection ;
iii) une deuxième séquence de reconnaissance de la recombinase ; et
iv) un deuxième marqueur de sélection partiel non fonctionnel ; et

b) un produit d'assemblage d'acide nucléique comprenant les éléments suivants, dans l'ordre :

i) la première séquence de reconnaissance de la recombinase ou la région qui est en 5' ou en 3' du site d'intégration ;
ii) une banque de polynucléotides d'intérêt ;
iii) un deuxième marqueur de sélection partiel non fonctionnel ; et
iv) la deuxième séquence de reconnaissance de la recombinase ; et

c) un gène codant la recombinase à spécificité de site compris dans le chromosome de la cellule hôte de champignon filamenteux ou dans le produit d'assemblage d'acide nucléique à l'extérieur des éléments listés dans l'étape (b) ;

dans lequel, quand la cellule hôte est transformée avec le produit d'assemblage d'acide nucléique, la banque de polynucléotides d'intérêt est intégrée en mode de spécificité de site avec l'orientation correcte dans le chromosome de la cellule hôte par la recombinase, en conséquence de quoi des niveaux d'expression uniformes élevés sont obtenus, le premier marqueur sélectionnable est excisé hors du chromosome, et les deuxièmes marqueurs sélectionnables partiels non fonctionnels sont recombinés pour former un deuxième marqueur de sélection fonctionnel dans le chromosome qui est exprimé.

**8.** Système d'expression de banque de polynucléotides selon la revendication 7, dans lequel la banque de polynucléotides comprend des polynucléotides codant des variants d'un polypeptide d'intérêt ; de préférence le polypeptide d'intérêt est une enzyme ; mieux encore le polypeptide d'intérêt est une hydrolase, isomérase, ligase, lyase, oxydoréductase, ou transférase, par exemple une aminopeptidase, amylase, carbohydrase, carboxypeptidase, catalase, cellobiohydrolase, cellulase, chitinase, cutinase, cyclodextrine glycosyltransférase, désoxyribonucléase, endoglucanase, estérase, alpha-galactosidase, bêta-galactosidase, glucoamylase, alpha-glucosidase, bêta-glucosidase, invertase, laccase, lipase, mannosidase, mutanase, oxydase, enzyme pectinolytique, peroxydase, phytase, polyphénol oxydase, enzyme protéolytique, ribonucléase, transglutaminase, xylanase, ou bêta-xylosidase.

**9.** Système d'expression de banque de polynucléotides selon la revendication 7 ou 9, dans lequel la cellule hôte de champignon filamenteux est une cellule d'*Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paelicomyces, Penicillium, Phanerochaete, Phlebia, Piromyces, Pleurotus, Schizophyllum, Talaromyces, Thermoascus, Thielavia, Tolypocladium, Trametes, ou Trichoderma* ; de préférence la cellule hôte est une cellule d'*Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei, ou Trichoderma viride.*

**10.** Système d'expression de banque de polynucléotides selon l'une quelconque des revendications 7 à 9, dans lequel la recombinase à spécificité de site est la flippase FLP de *Saccharomyces cerevisiae* de 2 μm, l'intégrase de phage TP901-1, l'intégrase de bactériophage P1 CRE, la recombinase bactérienne XerC, la recombinase bactérienne XerD, l'intégrase de phage lambda ou l'intégrase de HP1 ; de préférence la recombinase à spécificité de site est la flippase FLP de *Saccharomyces cerevisiae* de 2 μm.

**11.** Système d'expression de banque de polynucléotides selon l'une quelconque des revendications 7 à 10, dans lequel les première et deuxième séquences de reconnaissance de la recombinase sont identiques ou différentes ; de préférence les première et deuxième séquences de reconnaissance de la recombinase sont différentes afin que soit effectuée une intégration directionnelle de la banque de polynucléotides d'intérêt dans le chromosome de la cellule hôte.

**12.** Système d'expression de banque de polynucléotides selon l'une quelconque des revendications 7 à 11, dans lequel les première et deuxième séquences de reconnaissance de la recombinase sont des séquences de reconnaissance différentes de la flippase FLP de *Saccharomyces cerevisiae* de 2 μm afin que soit effectuée une intégration directionnelle de la banque de polynucléotides d'intérêt dans le chromosome de la cellule hôte ; de préférence les première et deuxième séquences de reconnaissance de la recombinase sont FRT-F (SEQ ID NO : 27) et FRT-F3 (SEQ ID NO : 28), respectivement, ou vice versa.

Figure 1

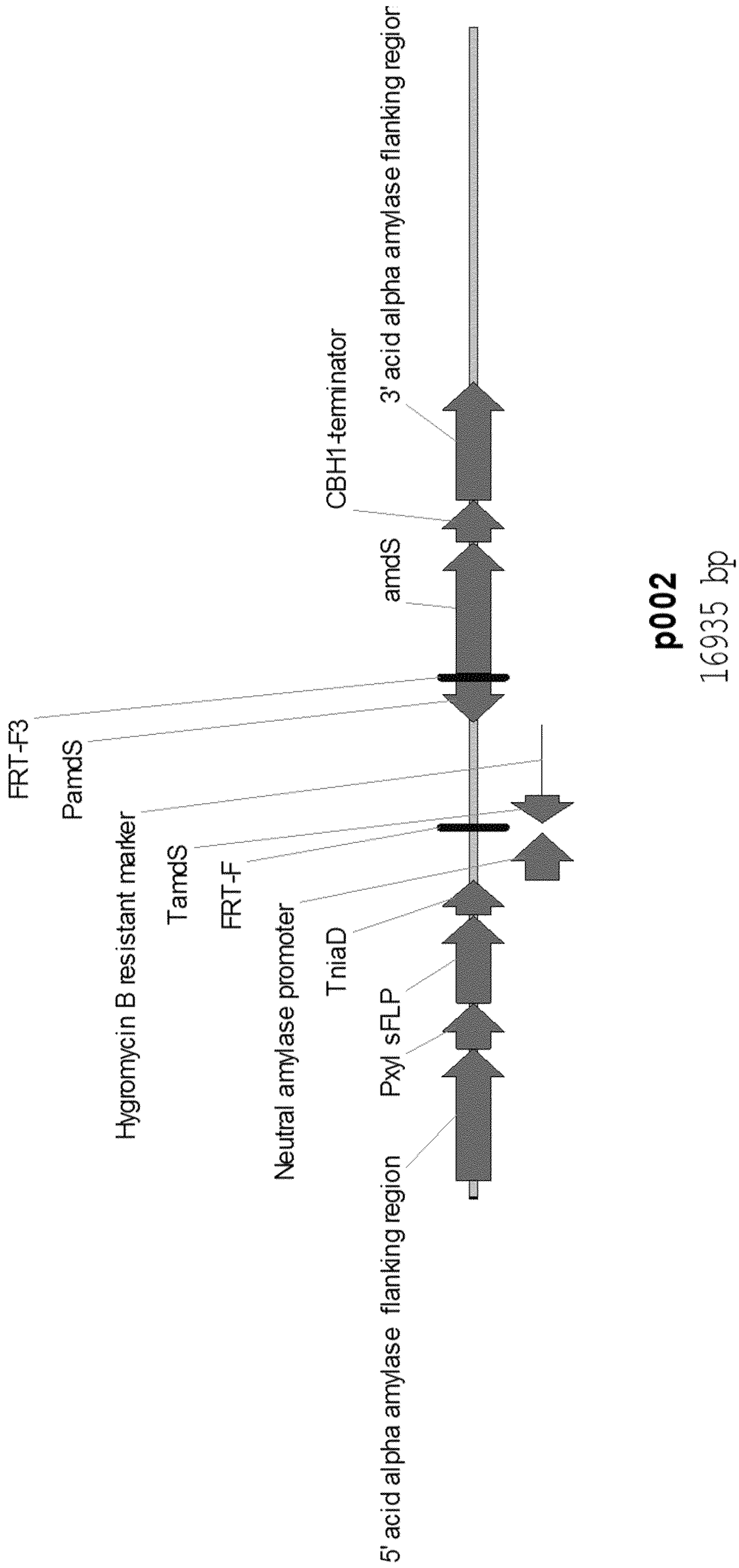
5' acid alpha amylase flanking region
Pxyl sFLP
TniaD
Neutral amylase promoter
FRT-F
TamdS
Hygromycin B resistant marker
PamdS
FRT-F3
amdS
CBH1-terminator
3' acid alpha amylase flanking regior
p002
16935 bp

Figure 2

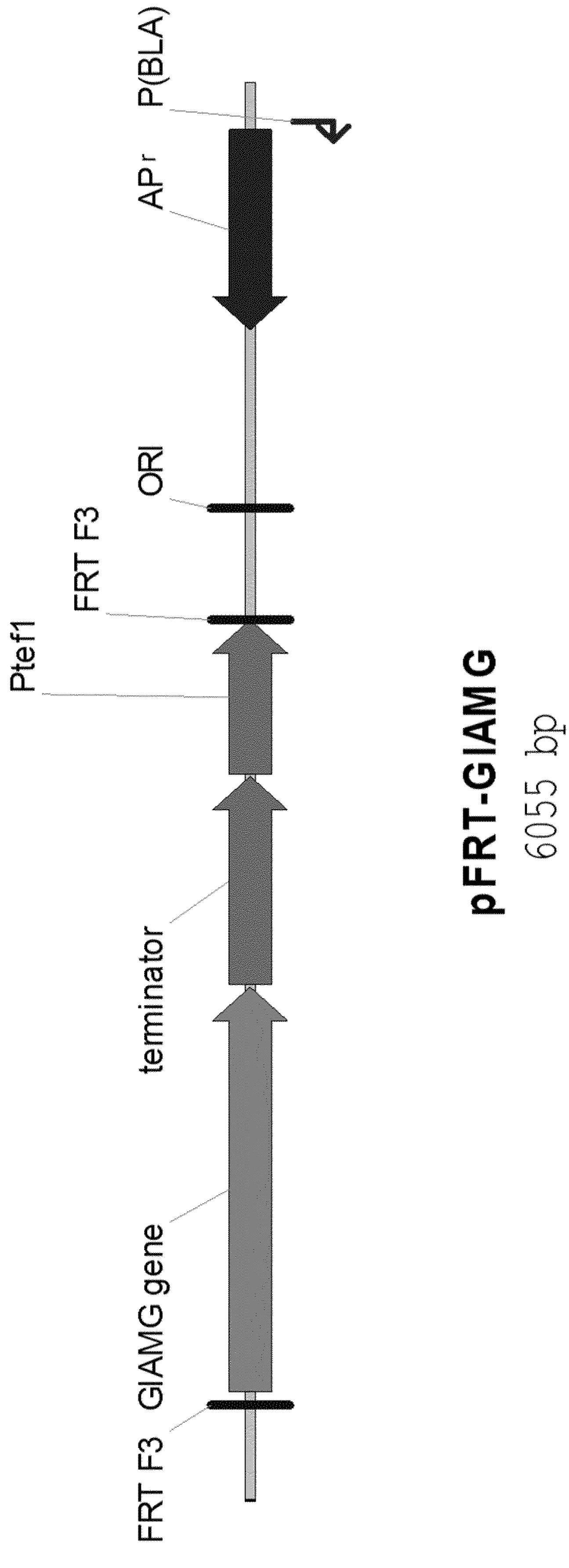
P(BLA)
AP r
ORI
FRT F3
Pte f1
terminator
GlAMG gene
FRT F3
pFRT-GIAMG
6055 bp

Figure 3

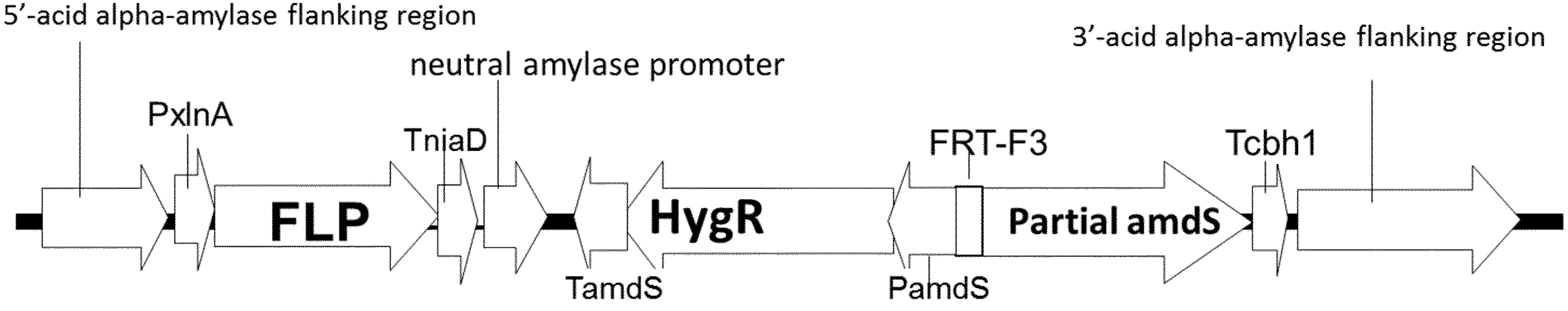


p007 16642bp

Figure 4

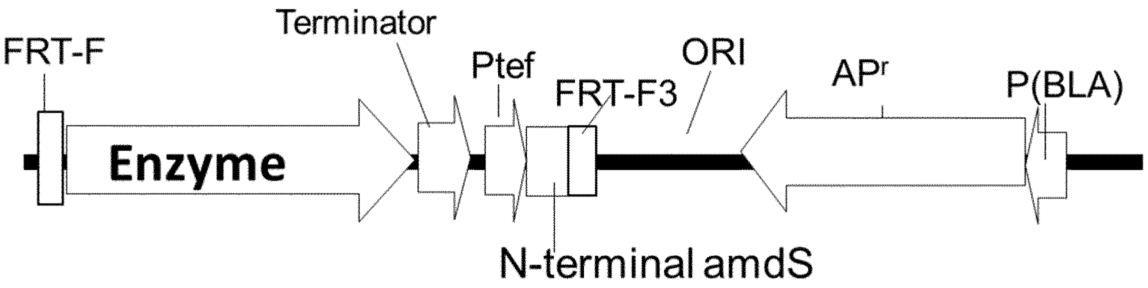


pFRT-BsAMG

Figure 5

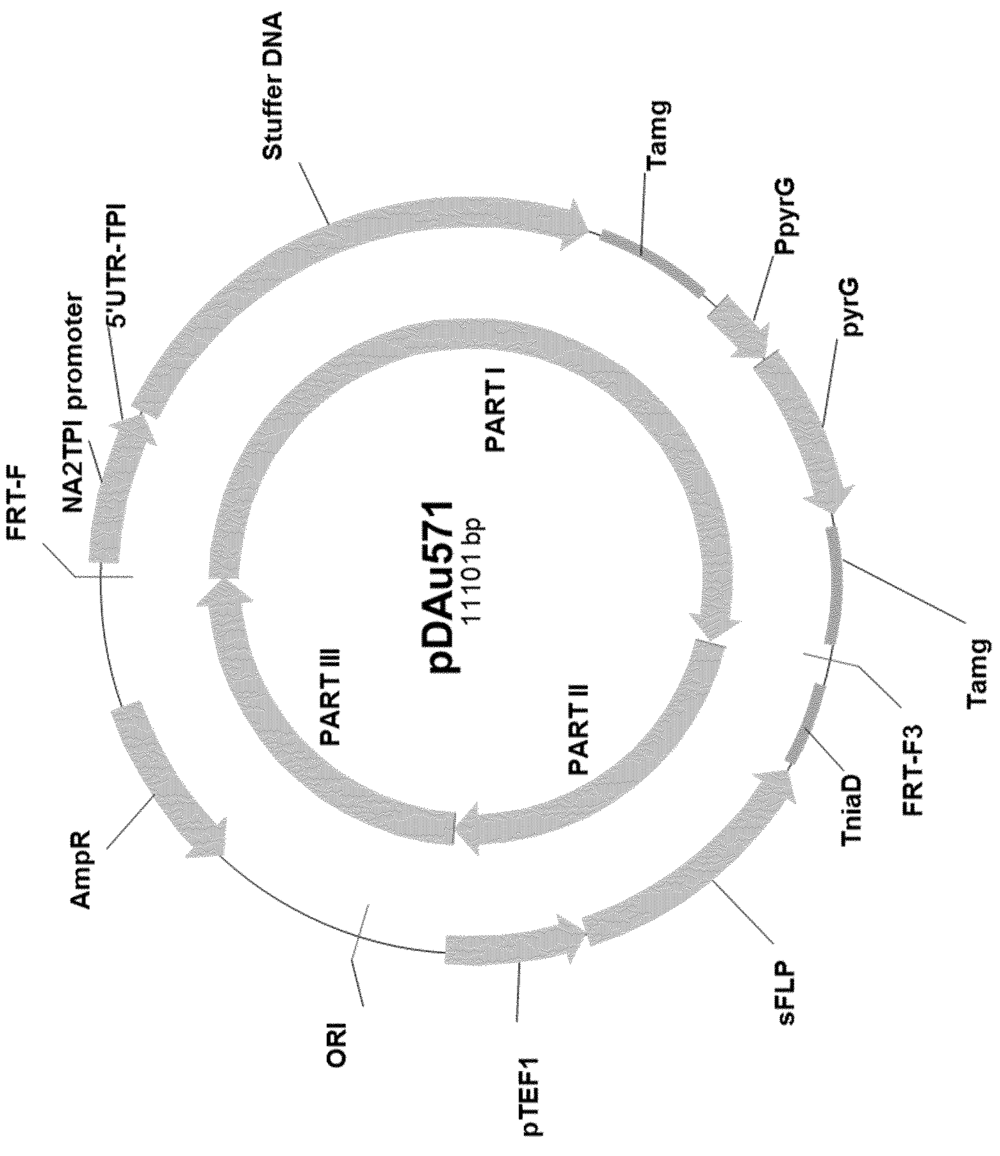
pDAu571
11101 bp
PART I
PART II
PART III
Stuffer DNA
Tamg
PpyrG
pyrG
Tamg
FRT-F3
TniaD
sFLP
pTEF1
ORI
AmpR
FRT-F
NA2TPI promoter
5'UTR-TPI

Figure 6

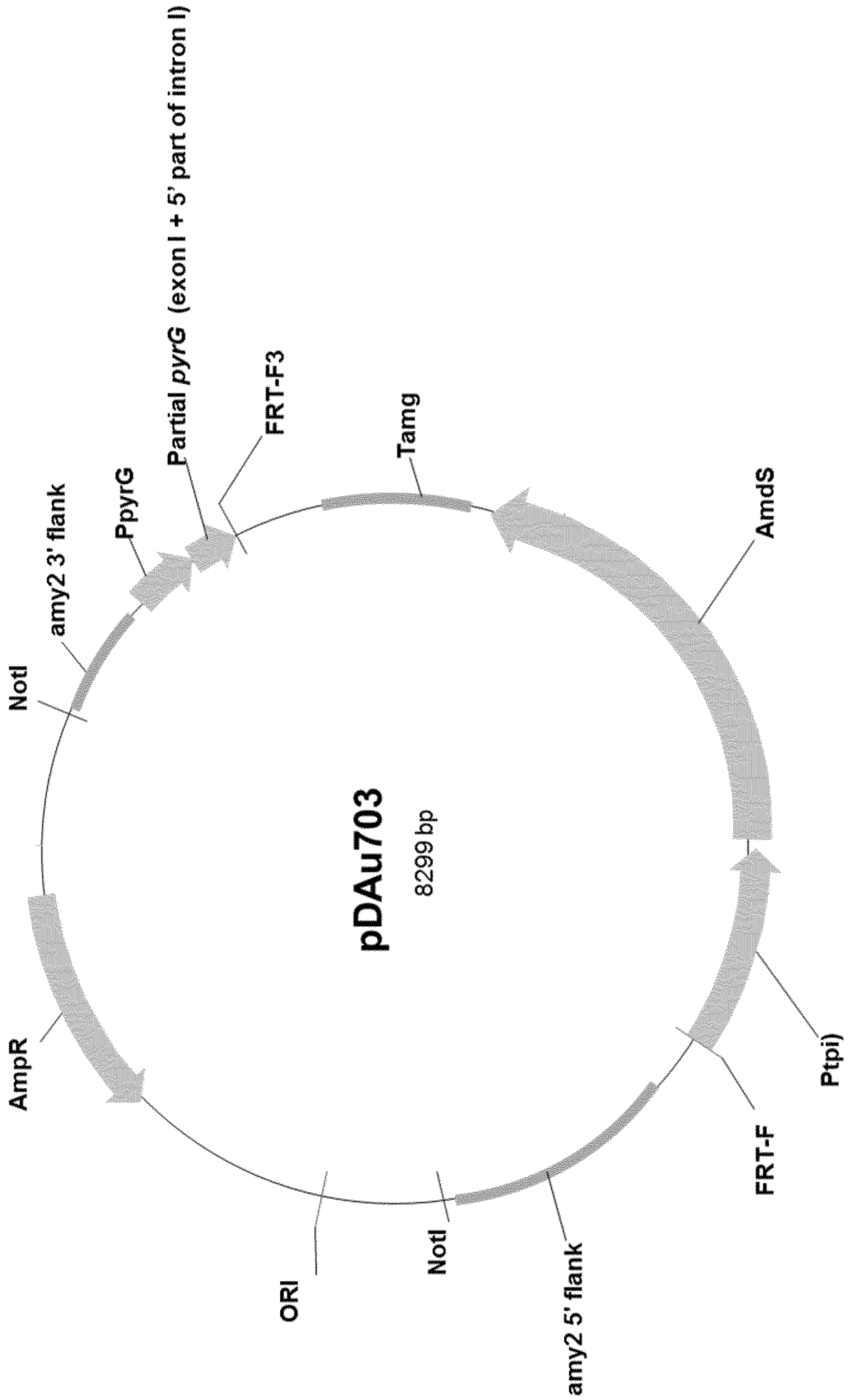
pDAu703
8299 bp
NotI
amy2 3' flank
PpyrG
Partial pyrG (exon I + 5' part of intron I)
FRT-F3
Tamg
AmdS
Ptpi)
FRT-F
amy2 5' flank
NotI
ORI
AmpR

Figure 7

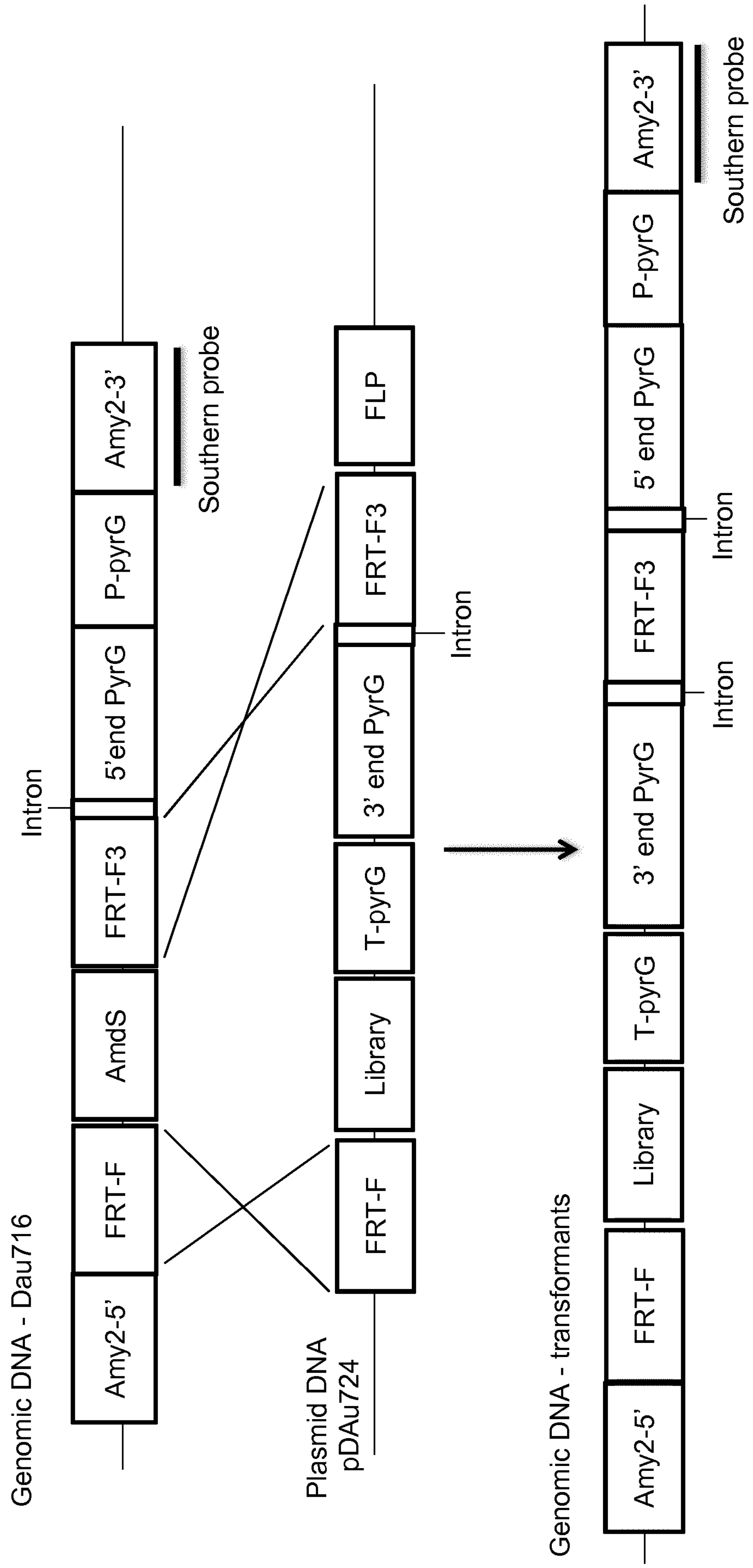

Genomic DNA - Dau716
Amy2-5'
FRT-F
AmdS
FRT-F3
Intron
5'end PyrG
P-pyrG
Amy2-3'
Southern probe
Plasmid DNA
pDAu724
FRT-F
Library
T-pyrG
3' end PyrG
Intron
FRT-F3
FLP
Genomic DNA - transformants
Amy2-5'
FRT-F
Library
T-pyrG
3' end PyrG
Intron
FRT-F3
Intron
5' end PyrG
P-pyrG
Amy2-3'
Southern probe

Figure 8

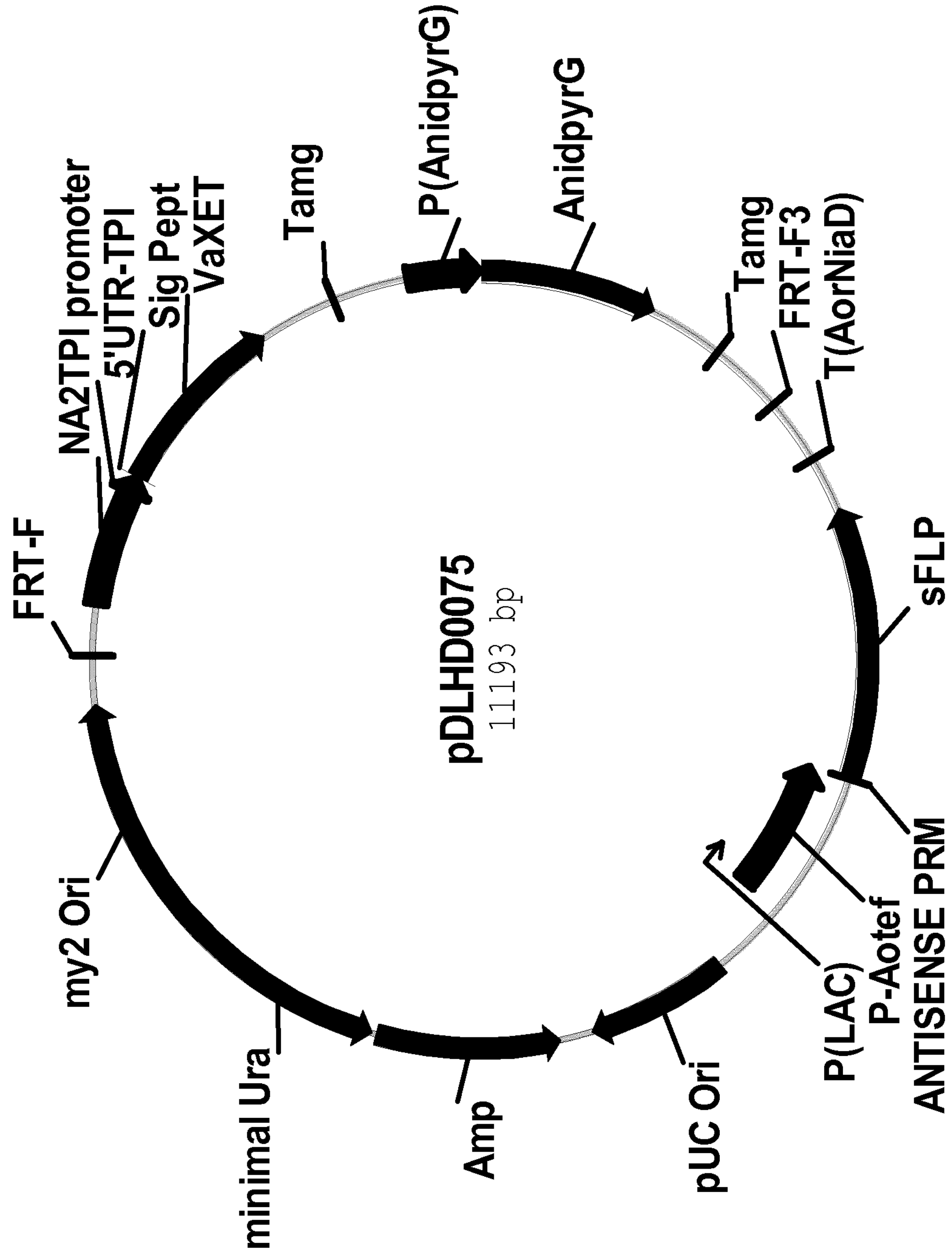
pDLHD0075
11193 bp
FRT-F
NA2TPI promoter
5'UTR-TPI
Sig Pept
VaXET
Tamg
P(AnidpyrG)
AnidpyrG
Tamg
FRT-F3
T(AorNiaD)
sFLP
ANTISENSE PRM
P-Aotef
P(LAC)
pUC Ori
Amp
minimal Ura
my2 Ori

Figure 9

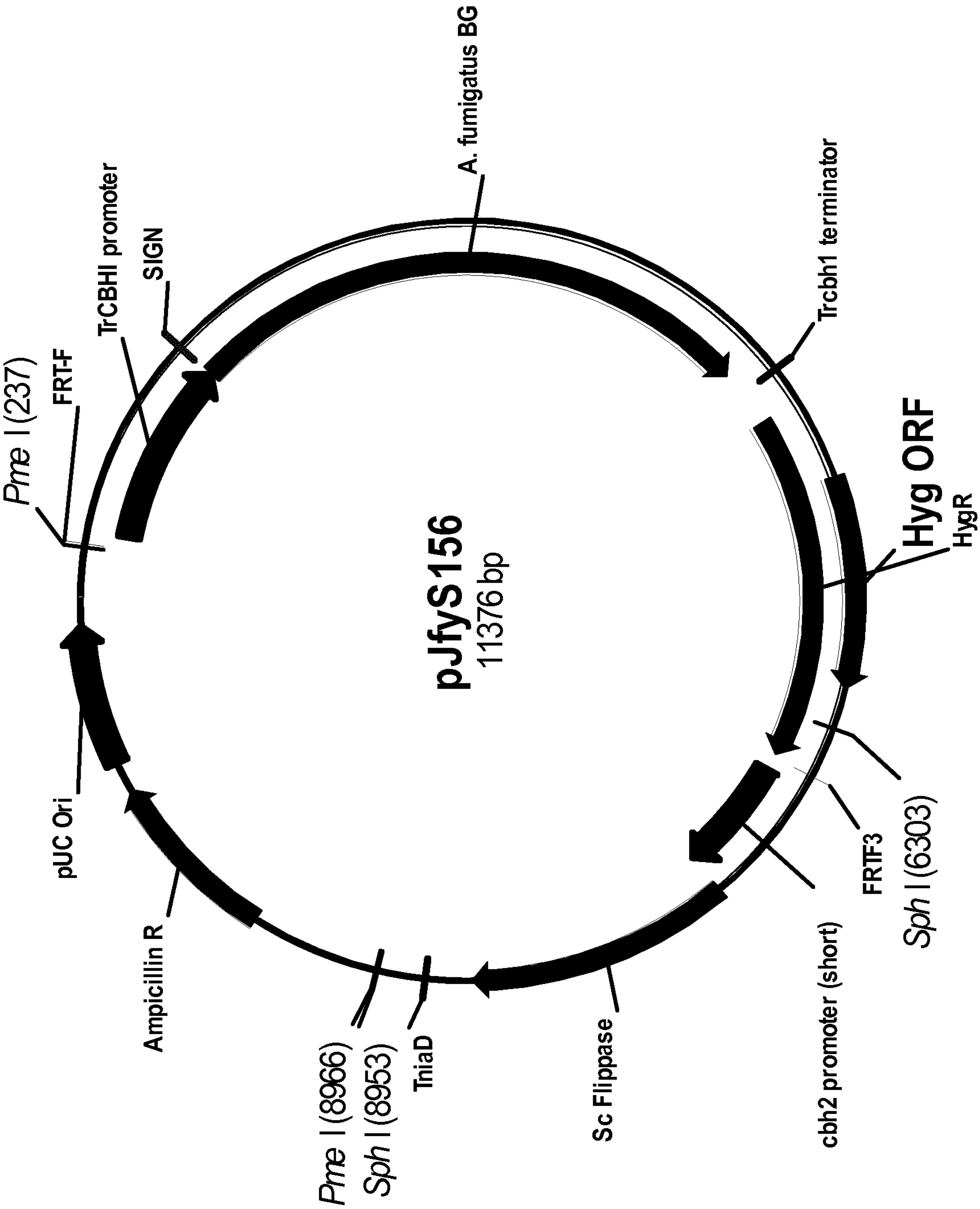
pJfyS156
11376 bp
A. fumigatus BG
Trcbh1 terminator
Hyg ORF
HygR
Sph I (6303)
FRTF3
cbh2 promoter (short)
Sc Flippase
TniaD
Sph I (8953)
Pme I (8966)
Ampicillin R
pUC Ori
Pme I (237)
FRT-F
TrCBHI promoter
SIGN

Figure 10

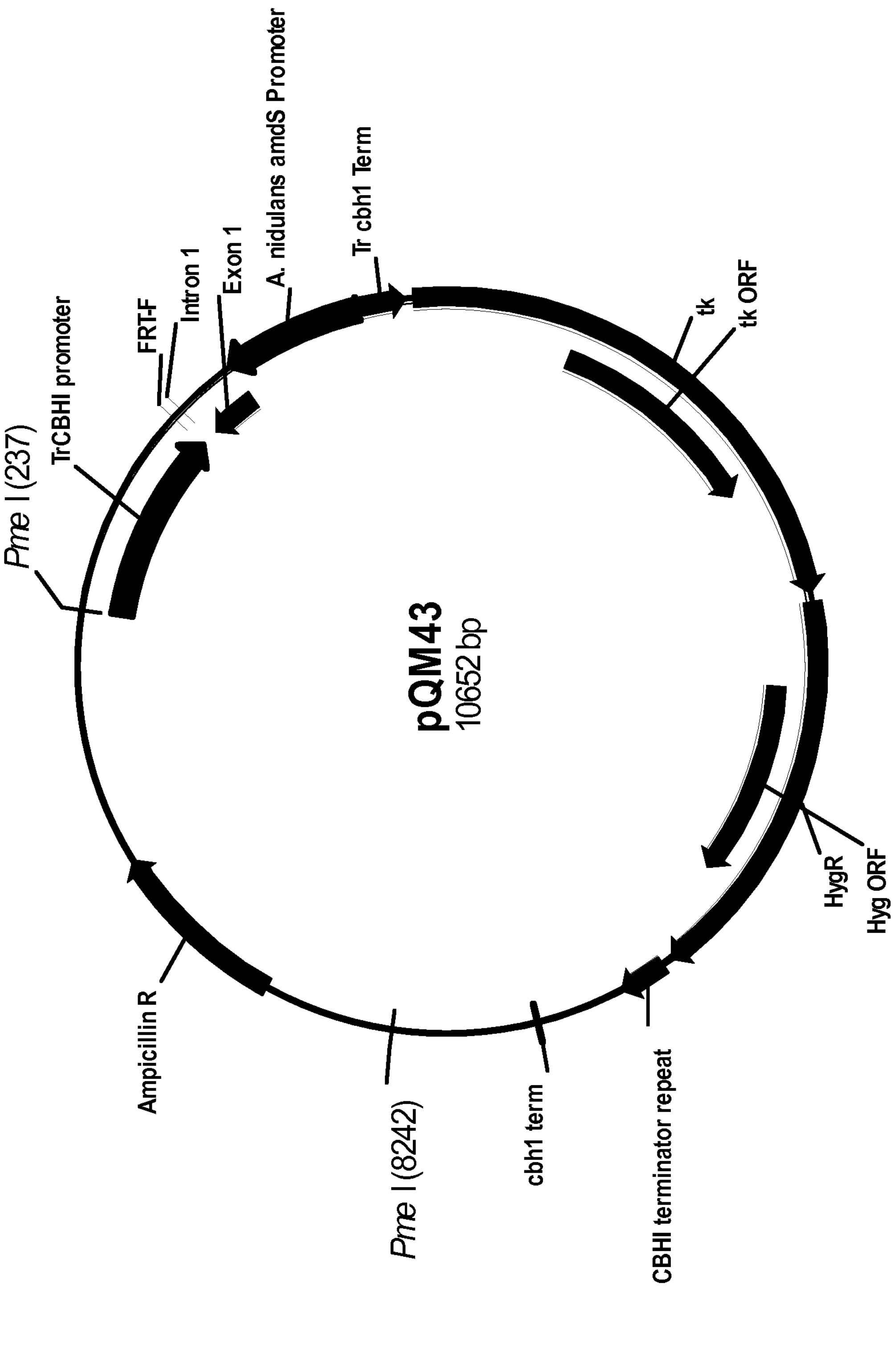
pQM43
10652 bp
Pme I (237)
TrCBHI promoter
FRT-F
Intron 1
Exon 1
A. nidulans amdS Promoter
Tr cbh1 Term
tk
tk ORF
HygR
Hyg ORF
CBHI terminator repeat
cbh1 term
Pme I (8242)
Ampicillin R

Figure 11

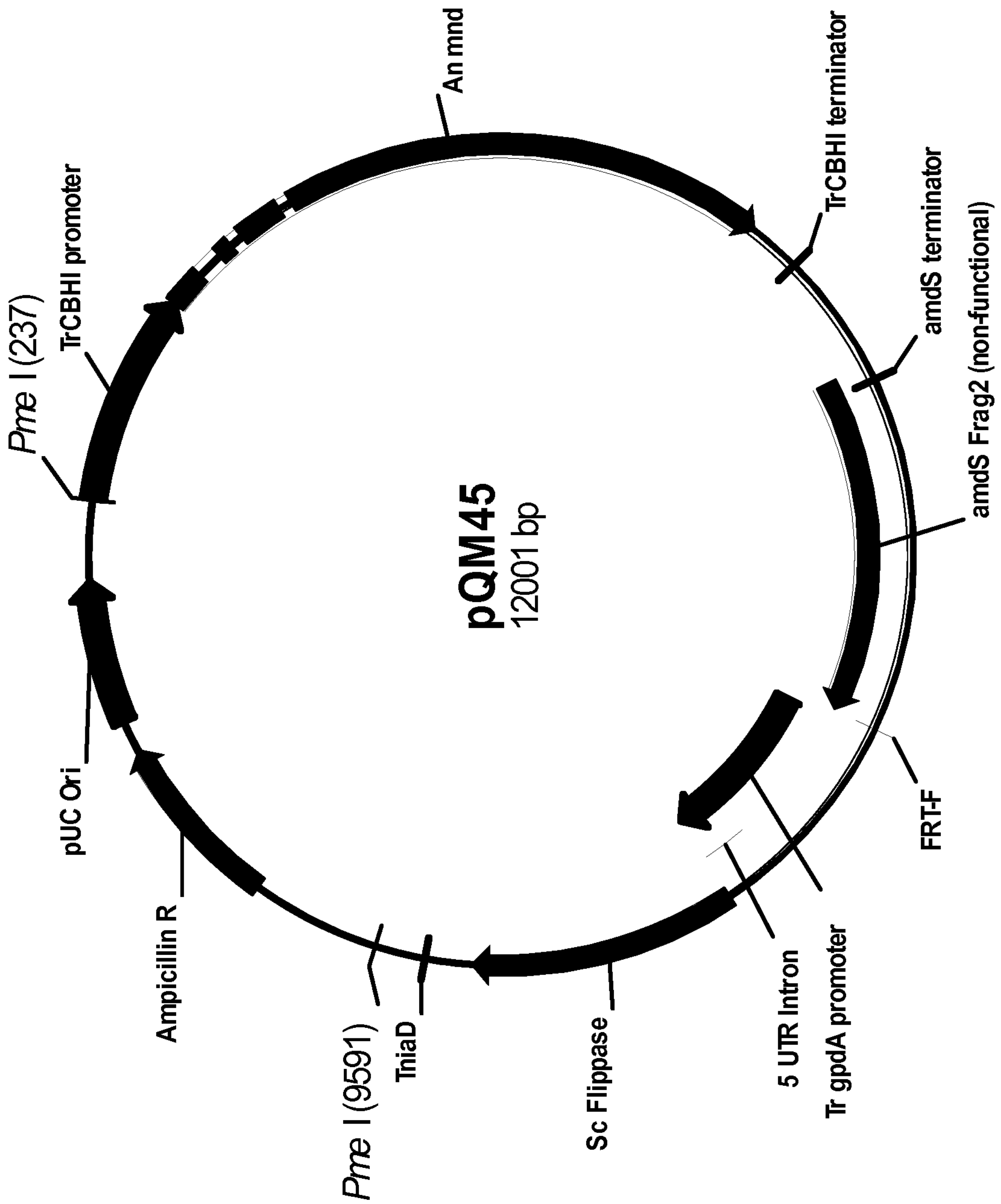
pQM45
12001 bp
An mnd
TrCBHI terminator
amdS terminator
amdS Frag2 (non-functional)
FRT-F
Tr gpdA promoter
5 UTR Intron
Sc Flippase
TniaD
Pme I (9591)
Ampicillin R
pUC Ori
Pme I (237)
TrCBHI promoter

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1405908 A1
- EP 1124949 A
- WO 9517413 A
- WO 9522625 A
- US 5223409 A
- WO 9206204 A
- WO 9600787 A
- WO 0056900 A
- US 6011147 A
- WO 9533836 A
- WO 2010039889 A
- WO 0024883 A
- EP 238023 A
- WO 2011075677 A
- WO 121600093 A
- WO 2012160093 A
- WO 12160097 A1
- WO 9801470 A
- WO 2008008950 A
- WO 05074647 A
- WO 2012120093 A
- WO 05047499 A
- WO 04111228 A
- WO 2013028927 A
- WO 2013028912 A
- US 8318458 B2


### Non-patent literature cited in the description


- **SADOWSLU.** *FASEB J.,* 1993, vol. 7, 750-67
- **OW ; MEDBERRY.** *Crit. Rev. Plant Sci.,* 1995, vol. 14, 239-261
- **WEI ; HUBER.** *J Biol Chem,* 1996, vol. 271 (7), 3812
- **FREDERICO L.A. et al.** *Biochemistry,* 1990, vol. 29, 2532-2537
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- **H. NEURATH ; R.L. HILL.** The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **GEMS et al.** *Gene,* 1991, vol. 98, 61-67
- **CULLEN et al.** *Nucleic Acids Res.,* 1987, vol. 15, 9163-9175
- **HAWKSWORTH et al.** Ainsworth and Bisby's Dictionary of The Fungi. CAB International, University Press, 1995
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **MALARDIER et al.** *Gene,* 1989, vol. 78, 147-156
- Protein Purification. VCH Publishers, 1989
- **SAMBROOK, J. ; FRITSCH, E.F. ; MANIATIS, T.** Molecular cloning: a laboratory manual. Cold Spring Harbor Laboratory, 1989, vol. 30
- **MONTENECOURT ; EVELEIGH.** *Adv. Chem. Ser.,* 1979, vol. 181, 289-301
- **COVE D.J.** *Biochem. Biophys.cta.,* 1966, vol. 113, 51-56
- **SULOVA et al.** *Analytical Biochechemistry,* 1995, vol. 229, 80-85
- **ZHOU et al.** *Biocatalysis and Biotransformation,* 2006, vol. 24, 107-120
- **PENTTILA et al.** *Gene,* 1987, vol. 61, 155-164

SEQUENCE LISTING

<110> Novozymes A/S

<120> RECOMBINASE-MEDIATED INTEGRATION OF A POLYNUCLEOTIDE LIBRARY

<130> NZ 12719-WO-PCT

<160> 81

<170> PatentIn version 3.5

<210> 1
<211> 16935
<212> DNA
<213> artificial sequence

<220>
<223> Plasmid p002

<220>

<221> misc_feature
<222> (11869)..(11869)
<223> n is a, c, g, or t

<400> 1

```
ttaatgcagc tggcacgaca ggtttcccga ctggaaagcg ggcagtgagc gcaacgcaat      60
taatgtgagt tagctcactc attaggcacc ccaggcttta cactttatgc ttccggctcg     120
tatgttgtgt ggaattgtga gcggataaca atttcacaca ggaaacagct atgaccatga     180
ttacgccaag ctcgaaatta accctcacta aagggaacaa aagctggagc tccaccgcgg     240
caacaggcag aatatcttcc gaattcaatc gactgcgcga tgcaagttgg ctagcaacgg     300
cgtacacctt gggattatgc gctgctcaac cgatggtcag ctatcaaaca aaatttggga     360
agatcgggct atactgacgg tgacattata gtacggcaag ctgagtgaca tctacggtcg     420
caagccactg cttctttggg catatgtttt ctttggcgtg ggatgcatta tcaggtagat     480
actccctttt tcttatacgc tggtttgctg gttcgtgctg acagctgttt ccctagcggt     540
attggtcgag acatggcgac tgtcatattg ggcgtgcaa tcagcggaat tgggggtgct      600
ggaacaatgg cgatgggctc tatcattatc acaggtaggc tagcagctta tcaggttgaa     660
agaactgtca ctgaacatag gcagatattg ttcctcgtcg agatgttgcc cattggcggg     720
cgtacatcaa tatcgcgatg actctgggtc gtagcgcagg aggcccaatc ggcggatggc     780
taaccgatac aatcggatgg agatggtatg ctttgcgcct ttgtgaccgc ttctctcact     840
aaattgtggc caaggtcgtt tattatccaa ggccccttag ccgctgtggc agctctgttg     900
gtgatatgga agctcaaact cgccaatcca gtcactgaga agagcatccg ccgtgtcgac     960
tttctcggaa cattcctcct ggccgtcggt attgttacaa tcaccgttat catggaccaa    1020
gcagggcagt ccttcgcatg ggcatcattg tcaacagcaa tccttgcaac tctcagtcta    1080
```

```
tcagcattcg tcgccttcgt ccttgttgaa ctctacgtag cccctgaacc gattttcgaa     1140
cttcgcatgt tgcggaagcc gaatgtgacg cccagttacc tgatcggatc gctgcagatc     1200
accgcccaag ttggaatgat gttctccgtg ccgttatatt ttcaggtgac atcgaaagcc     1260
tctgccaccg tagctggagg gcatctggtt cctgcagtga tcggaaacac gcttggcggc     1320
ttaatcgcgg gagcctttat ccgtcgcacc ggccaattca aggtcctctt gatccttgcc     1380
ggtctcgttg cgtccgtcgc ctatctactc ctcatccttc gctggaacgg tcatactgga     1440
ttctgggagt ccttgtacat tattcccggt ggtatgggta ctggtttctg ctctgcagct     1500
gcttttgtca gtatgacggc gtttttgatg ccgcaggaag tggccatggc aacaggaggt     1560
tacttcctat tattcagctt cgccatgacg gccggtgtca ctgtcactaa cagtctgctg     1620
gggacggttt tcaagcgcca gatggaacag cacctgacgg gtccaggagc caagaaggtt     1680
ggtatccccg cacctttttct gcgtcactta ctaacgagta tatgaagatc atcgagcgcg     1740
cgctgtccga caccagctat atcaacggtt tgcagggtca tgtccgggat gtagtggtaa     1800
aaggatatgt gactggtctc cgctacactt actgtaagtc gtttgaatca tgcatccacc     1860
gtccacctta ttaacttggt gccagtattt tccctcattc tttcgctcct tggatcggtc     1920
ctcgcttgga ctgtacgaaa acaccaacta tgaggaacca gcacggcagc tgatagtatc     1980
cgaaagctgc aaattgcttc atcgaggctg gcattcgata gaagaaagaa ctatagacaa     2040
ctagtcttac aatatgacaa ttctctttga ttaataaatg aaaataacac ttgtgtcagc     2100
ctaatagccg agtggcgggc atctctggcg gcctcccgag cagcgtggat ctggaagtgc     2160
gttgatcatt attccccgaa aatgtagtac ccagtaagtg gtctagcggt ggctatggta     2220
ggacatctat gcctaagctg gagttctcat tgaacgtgta ccggccgatt gccctaaact     2280
ctgattgaga gccggaaacc tcatctacct gatgctcagg ggccatccaa tagcttccga     2340
tagcattaca gacagatgga ctcgtcttgg cccacgggtc tagaacagtc gccggaactg     2400
cctctatttg aaacggagct gaaccatgat acttaagcgt gccaagcggc gccgtttccc     2460
actggaacaa ggagcaatag aattctgcag agattcttca ttcaggctat tcagcaattc     2520
ggtttgtgga gcggatcggg gtccactggg tttagtctgg ggtttttctt tgcccgcatg     2580
ggctctagca catgcacagc ttgcagttgc tgctacgcta tctgggaaaa cgaatggcta     2640
ttcaggagtt tataaccaaa agagccggaa acaggctgat tgccctctca cggggagacg     2700
ttgtacttct gatccagagg ctattaaccg gacactacct ataaaggagg tagcattcct     2760
ttctgtccgg ctcccagatt ccaacaaccc aactgacagg ggatccacca tgccccagtt     2820
cgatatcctc tgcaagaccc cccccaaggt cctcgtccgc cagttcgtcg agcgcttcga     2880
gcgcccctcc ggcgagaaga tcgccctctg cgccgccgag ctcacctacc tctgctggat     2940
gatcacccat aacggcaccg ccatcaagcg cgccaccttc atgtcctaca acaccatcat     3000
```

ctccaactcc ctctccttcg atatcgtcaa caagtccctc cagttcaagt acaagaccca 3060
gaaggccacc atcctggagg cctccctcaa gaagctcatc cccgcctggg agttcaccat 3120
catcccctac tacggccaga agcatcagtc cgatatcacc gatatcgtct cctccctcca 3180
gctccagttc gagtcctccg aggaggccga taagggcaac tcccattcca agaagatgct 3240
caaggccctc ctctccgagg gcgagtccat ctgggagatc accgagaaga tcctcaactc 3300
cttcgagtac acctcccgct tcaccaagac caagaccctc taccagttcc tcttcctcgc 3360
caccttcatc aactgcggcc gcttctccga tatcaagaac gtcgatccca agtccttcaa 3420
gctcgtccag aacaagtacc tcggcgtcat catccagtgc ctcgtcaccg agaccaagac 3480
ctccgtctcc cgccatatct acttcttctc cgcccgcggc cgcatcgatc ccctcgtcta 3540
cctcgatgag ttcctccgca actccgagcc cgtcctcaag cgcgtcaacc gcaccggcaa 3600
ctcctcctcc aacaagcagg agtaccagct cctcaaggat aacctcgtcc gctcctacaa 3660
caaggccctc aagaagaacg cccccactc catcttcgcc atcaagaacg gccccaagtc 3720
ccatatcggc cgccatctca tgacctcctt cctctccatg aagggcctca ccgagctcac 3780
caacgtcgtc ggcaactggt ccgataagcg cgcctccgcc gtcgcccgca ccacctacac 3840
ccatcagatc accgccatcc ccgatcatta cttcgcacta gtctcccgct actacgccta 3900
cgatcccatc tccaaggaga tgatcgccct caaggatgag accaacccca tcgaggagtg 3960
gcagcatatc gagcagctca agggctccgc cgagggctcc atccgctacc ccgcctggaa 4020
cggcatcatc tcccaggagg tcctcgatta cctctcctcc tacatcaacc gccgcatctg 4080
agtcgagatt atccaaggga atgacttaat gagtatgtaa gacatgggtc ataacggcgt 4140
tcgaaacata tacagggtta tgtttgggaa tagcacacga ataataacgt taataggtac 4200
caaagtcctt gatacattag cacggtagaa aaagaataat acaacgagct gggaatattc 4260
tttaatataa aactccaaga agagctggtg cggtggagct tgttttcgac tctcagtaat 4320
atttcctcat atccaagcgc gctaggaggt ggtcgaatac acatgtaggc gcttctctgg 4380
atgcaaaagt cgtgccggac ctgccgaaag actttgaaga tgcgttcacg ccatctaagt 4440
tgcgtagata attcacaaaa agggatgttt gtttccggaa tgtagcaaag agctgatagg 4500
caatagcctc actttcgtgg cgcacgccgc tcgttccatc catcctcgac aatggagcaa 4560
atgtcaaaat cgtaccgaaa atactttgct agcccgttaa attgccgtcg tcagccgtta 4620
aattaccgat taatcccgat aaatttccga gatctccgtt aaattgccgt tcgcagccgt 4680
taaattaccg gggacgaccg ataaatttcc gcgatgaatt catggtgttt tgatcatttt 4740
aaatttttat atggcgggtg gtgggcaact cgcttgcgcg ggcaactcgc ttaccgatta 4800
cgttagggct gatatttacg taaaaatcgt caagggatgc aagaccaaac cgttaaattt 4860

ccggagtcaa cagcatccaa gcccaagtcc ttcacggaga aaccccagcg tccacatcac 4920

gagcgaagga ccacctctag gcatcggacg caccatccaa ttagaagcag caaagcgaaa 4980

cagcccaaga aaaaggtcgg cccgtcggcc ttttctgcaa cgctgatcac gggcagcgat 5040

ccaaccaaca ccctccagag tgactagggg cggaaattta tcgggattaa tttccactca 5100

accacaaatc acagtcgtcc ccggtaattt aacggctgca gacggcaatt taacggcttc 5160

tgcgaatcgc ttggattccc cgcccctggc cgtagagctt aaagtatgtc ccttgtcgat 5220

gcgatgtatc acaacatata aatactggca agggatgcca tgcttggagc aacaatctca 5280

gaacaccaat atcaattcat ctcgagtctg aactcagaca acacaaattc ttcaaaattg 5340

aggatttagt cttgatcttg aagttcctat tccgagttcc tattctctag aaagtatagg 5400

aacttcttaa ttaacctagc cgttattact ctaccgcaag gcaacaacca gctcacccct 5460

gaggcacggg taccatgggt tgagtggtat ggggccatcc agagtcacct gtggcagcat 5520

gagactgcac tcgaagcagc catcaaccca gccaatattc tgggctttcc atccttagat 5580

cacatttgag atataaccca tttggtgaga gacacttgtg ccgttatacg tgtctagact 5640

ggaaacgcaa ccctgaaggg attcttcctt tgagagatgg aagcgtgtca tatctcttcg 5700

gttctacggc aggttttttt ctgctctttc gtagcatggc atggtcactt cagcgcttat 5760

ttacagttgc tggtattgat ttcttgtgca aattgctatc tgacacttat taggtcgagc 5820

tattcctttg ccctcggacg agtgctgggg cgtcggtttc cactatcggc gagtacttct 5880

acacagccat cggtccagac ggccgcgctt ctgcgggcga tttgtgtacg cccgacagtc 5940

ccggctccgg atcggacgat tgcgtcgcat cgaccctgcg cccaagctgc atcatcgaaa 6000

ttgccgtcaa ccaagctctg atagagttgg tcaagaccaa tgcggagcat atacgcccgg 6060

agccgcggcg atcctgcaag ctccggatgc ctccgctcga agtagcgcgt ctgctgctcc 6120

atacaagcca accacggcct ccagaagaag atgttggcga cctcgtattg ggaatccccg 6180

aacatcgcct cgctccagtc aatgaccgct gttatgcggc cattgtccgt caggacattg 6240

ttggagccga aatccgcgtg cacgaggtgc cggacttcgg ggcagtcctc ggcccaaagc 6300

atcagctcat cgagagcctg cgcgacggac gcactgacgg tgtcgtccat cacagtttgc 6360

cagtgataca catggggatc agcaatcgcg catatgaaat cacgccatgt agtgtattga 6420

ccgattcctt gcggtccgaa tgggccgaac ccgctcgtct ggctaagatc ggccgcagcg 6480

atcgcatcca tggcctccgc gaccggctgc agaacagcgg gcagttcggt ttcaggcagg 6540

tcttgcaacg tgacaccctg tgcacggcgg gagatgcaat aggtcaggct ctcgctgaat 6600

tccccaatgt caagcacttc cggaatcggg agcgcggccg atgcaaagtg ccgataaaca 6660

taacgatctt tgtagaaacc atcggcgcag ctatttaccc gcaggacata tccacgccct 6720

cctacatcga agctgaaagc acgagattct tcgccctccg agagctgcat caggtcggag 6780

acgctgtcga acttttcgat cagaaacttc tcgacagacg tcgcggtgag ttcaggcgac 6840
attgctgagg tgtaggatcg atccgtttaa actctgtgtt agcttatagt caggatgttg 6900
gctcgacgag tgtaaactgg gagttggcat gagggttatg taggcttctt tagccccgca 6960
tccccctcat tctcctcatt gatcccgggg gagcggatgg tgttgataag agactaatta 7020
tagggtttag ctggtgccta gctggtgatt ggctggcttc gccgaatttt acgggccaag 7080
ggaagctgca gaaccgcggc actggtaaac ggtaattaag ctatcagccc catgctaacg 7140
agtttaaatt acgtgtattg ctgataaaca ccaacagagc tttactgaaa gatgggagtc 7200
acggtgtggc ttccccactg cgattattgc acaagcagcg agggcgaact tgactgtcgt 7260
cgctgagcag cctgcagtca aacatacata tatatcaacc gcgaagacgt ctggccttgt 7320
agaacacgac gctccctagc aacacctgcc gtgtcagcct ctacggttgt tacttgcatt 7380
caggatgctc tccagcgggc gagctattca aaatattcaa agcaggtatc tcgtattgcc 7440
aggattcagc tgaagcaaca ggtgccaagg aaatctgcgt cggttctcat ctgggcttgc 7500
tcggtcctgg cgtagactag tgcatgcttg aagttcctat tccgagttcc tattcttcaa 7560
atagtatagg aacttcaacc atgcctcaat cctgggaaga actggccgct gataagcgcg 7620
cccgcctcgc aaaaaccatc cctgatgaat ggaaagtcca gacgctgcct gcggaagaca 7680
gcgttattga tttcccaaag aaatcgggta tcctttcaga ggccgaactg aagatcacag 7740
aggcctccgc tgcagatctt gtgtccaagc tggcggccgg agagttgacc tcggcggaag 7800
ttacgctagc attctgtaaa cgggcagtaa tcgcccagca gttagtaggg tcccctctac 7860
ctctcaggga gatgtaacaa cgccacctta tgggactatc aagctgacgc tggcttctgt 7920
gcagacaaac tgcgcccacg agttcttccc tgacgccgct ctcgcgcagg caagggaact 7980
cgatgaatac tacgcaaagc acaagagacc cgttggtcca ctccatggcc tccccatctc 8040
tctcaaagac cagcttcgag tcaaggtaca ccgttgcccc taagtcgtta gatgtccctt 8100
tttgtcagct aacatatgcc accagggcta cgaaacatca atgggctaca tctcatggct 8160
aaacaagtac gacgaagggg actcggttct gacaaccatg ctccgcaaag ccggtgccgt 8220
cttctacgtc aagacctctg tcccgcagac cctgatggtc tgcgagacag tcaacaacat 8280
catcgggcgc accgtcaacc cacgcaacaa gaactggtcg tgcggcggca gttctggtgg 8340
tgagggtgcg atcgttggga ttcgtggtgg cgtcatcggt gtaggaacgg atatcggtgg 8400
ctcgattcga gtgccggccg cgttcaactt cctgtacggt ctaaggccga gtcatgggcg 8460
gctgccgtat gcaaagatgg cgaacagcat ggagggtcag gagacggtgc acagcgttgt 8520
cgggccgatt acgcactctg ttgagggtga gtccttcgcc tcttccttct tttcctgctc 8580
tataccaggc ctccactgtc ctcctttctt gctttttata ctatatacga gaccggcagt 8640

cactgatgaa gtatgttaga cctccgcctc ttcaccaaat ccgtcctcgg tcaggagcca 8700

tggaaatacg actccaaggt catccccatg ccctggcgcc agtccgagtc ggacattatt 8760

gcctccaaga tcaagaacgg cgggctcaat atcggctact acaacttcga cggcaatgtc 8820

cttccacacc ctcctatcct gcgcggcgtg gaaaccaccg tcgccgcact cgccaaagcc 8880

ggtcacaccg tgaccccgtg gacgccatac aagcacgatt tcggccacga tctcatctcc 8940

catatctacg cggctgacgg cagcgccgac gtaatgcgcg atatcagtgc atccggcgag 9000

ccggcgattc caaatatcaa agacctactg aacccgaaca tcaaagctgt taacatgaac 9060

gagctctggg acacgcatct ccagaagtgg aattaccaga tggagtacct tgagaaatgg 9120

cgggaggctg aagaaaaggc cgggaaggaa ctggacgcca tcatcgcgcc gattacgcct 9180

accgctgcgg tacggcatga ccagttccgg tactatgggt atgcctctgt gatcaacctg 9240

ctggatttca cgagcgtggt tgttccggtt acctttgcgg ataagaacat cgataagaag 9300

aatgagagtt tcaaggcggt tagtgagctt gatgccctcg tgcaggaaga gtatgatccg 9360

gaggcgtacc atggggcacc ggttgcagtg caggttatcg gacggagact cagtgaagag 9420

aggacgttgg cgattgcaga ggaagtgggg aagttgctgg gaaatgtggt gactccatag 9480

gtaagctccg tggcgaaagc ctgacgcacc ggtagattct tggtgagccc gtatcatgac 9540

ggcggcggga gctacatggc cccgggtgat ttattttttt tgtatctact tctgaccctt 9600

ttcaaatata cggtcaactc atctttcact ggagatgcgg cctgcttggt attgcgatgt 9660

tgtcagcttg gcaaattgtg gctttcgaaa acacaaaacg attccttagt agccatgcat 9720

tttaagataa cggaatagaa gaaagaggaa attaaaaaaa aaaaaaaaac aaacatcccg 9780

ttcataaccc gtagaatcgc cgctcttcgt gtatcccagt accacggcaa aggtatttca 9840

tgatcgttca atgttgatat tgttcccgcc agtatggctc cacccccatc tccgcgaatc 9900

tcctcttctc gaacgcggta gtggcgcgcc aattggtaat gacccatagg gagacaaaca 9960

gcataatagc aacagtggaa attagtggcg caataattga gaacacagtg agaccatagc 10020

tggcggcctg gaaagcactg ttggagacca acttgtccgt tgcgaggcca acttgcattg 10080

tctagagaat gcaatcataa cagaaagtac agccagcgct gtgtcataaa gaagtccagt 10140

tgggaaacga aagactagaa tcaaactaaa agtaatccgg ccgatatggc ttcacgtgcg 10200

aagtctcgcc ttgaggggac attgtccttg caggtgattg accattgcgt tcatatggcg 10260

cgatgtttgg tagtgtgggt gtagccggtg acctcacgga aggactaaag gccacatacc 10320

cttctgagtg cctcttctct tcgtggtcgg aactctcgaa tgggtttttg acagttgcac 10380

tcgtttggtt gtggtcattt gaaggtctgc gttcggtctt ctgttcgcgc aggcgagctg 10440

actgagggat tgaaagctgc atagccatcg ttggcatgcg ttaattcgcc aaagctcagc 10500

ggcgaaacag gcctgacctc taatccatgc atctgctctg cactcgattg ttcgtggtgt 10560

```
ccttgcgaag aaagagaagc cttggactcg gatgactttc tggacgaggt ggtaggatca     10620
tcatgattgt aatgagactg tagcacatca tgcgaatcat tcgacacacg gtgtctgccc     10680
aagttgacgt cagcatcggt atgcatttcg gtatggtcct catggttctc agcatgtccc     10740
tccagagggg actcatttcc agcggaagga ttataagcaa cataattgtc atgtggctgc     10800
gacctttcgt gagactccga gtttgatctc actgtggact catgggcgat atgcggctca     10860
tcatgatctt cgaatggaga gaaatggttg aagtcggagg acacgggtga tttagcagca     10920
gggttgaatg caacatagcc agtctcgcgc tcttcatgtg agctatatga gtcatgtggc     10980
ctgtcatggt ccagaggctc cggatgctca tggctagatt catcgtgtgc cgaaatcgcg     11040
tcactagcaa agggcgaggt tgacacattg gctgcaggac tgaacgccac ataaccactc     11100
tcaggctctt catgtgagtt ataggagctg tgcggcatat catagtcctg aggttcacga     11160
tgctcatggc tggattcatc gtgtgccgaa atagcgtgac tagcaaaagg cgagggcgaa     11220
gcattggttg ccggactgaa cgccacatag ccgtccccat tggctgaact gactggtgac     11280
aacgtcctac ccatggcgtc ggccgggcca gcggcttggt gagagtgaag accattagaa     11340
gtagctggac tgaacgatcg cagcgggtat tcgttttctt gagctggata aggggctgcg     11400
ccatgctggc tgaaaggtga gaatgttcgg ggtgctgctc tatcaccagg gaaggcagac     11460
gctggagtca aagaacgagt gttggatcaa ttgccggact gtatgaacgg aaaggagtgc     11520
tgattgttta aatggcccgt agccttcgct aggacctcgt gattcgggga ccgttggccc     11580
atacccagga gctggtgtaa aattggaacg cgacacgggt gtttggttgc gcagaatttg     11640
cggtgccggc gaggcgtgat caatctggct gtaacctggg cctggggtgt agtttgagac     11700
aggtgtttgt gttcgtggca tttgtggcgc tggcgacgct ctgtcagtcg gcccatatcc     11760
aggcgccgaa ggtgtgggcg taaacccttg ccgtgattta attaagaatt ctcctgtagg     11820
cttgagagtt caaggaagaa acagtgcaat tatctttgcg aacccaggng ctggtgacgg     11880
aattttcata gtcaagctat cagagtaaag aagaggagca tgtcaaagta caattagaga     11940
caaatatata gtcgcgtgga gccaagagcg gattcctcag tctcgtaggt ctcttgacga     12000
ccgttgatct gcttgatctc gtctcccgaa aatgaaaata gactctgcta agctattctt     12060
ctgcttcgcc ggagcctgaa gggcgtacta gggttgcgag gtccaatgca ttaatgcatt     12120
gcagatgagc tgtatctgga agaggtaaac ccgaaacgcg ttttattctt gttgacatgg     12180
agctattaaa tcactagaag gcactctttg ctgcttggac aaatgaacgt atcttatcga     12240
gatcctgaac accatttgtc tcaactccgg agctgacatc gacaccaacg atcttatatc     12300
cagattcgtc aagctgtttg atgatttcag taacgttaag tggatcgatc cggatagcgc     12360
gggttccttc cggtattgtc tccttccgtg tttcagttag cctcccccat ctcccgggca     12420
```

aacgtgcgcg ccaggtcgca tatcgtcggt atggagccgg gggtggtgac gtgggtctgg 12480
accatcccgg aggtaagttg cagcagggcg tcccggcagc cggcgggcga ttggtcgtaa 12540
tccaggataa agacgtgcat ggaacggagg cgtttggcca agacgtccaa ggcccaggca 12600
aacacgttat acaggtcgcc gttgggggcc agcaactcgg ggccccgaaa cagggtaaat 12660
aacgtgtccc cgatatgggg tcgtgggccc gcgttgctct ggggctcggc accctggggc 12720
ggcacggccg tccccgaaag ctgtccccag tcctcccgcc acgacccgcc gcactgcaga 12780
taccgcaccg tattggcaag tagcccgtaa acgcggcgaa tcgcagccag catagccagg 12840
tccagccgct cgccggggcg ctggcgtttg gccaggcggt cgatgtgtct gtcctccgga 12900
agggccccaa gcacgatgtt ggtgccgggc aaggtcggcg ggatgagggc cacgaacgcc 12960
agcacggcct gggggtcat gctgcccata aggtaccgcg cggccgggta gcacaggagg 13020
gcggcgatgg gatggcggtc gaagatgagg gtgagggccg ggggcggggc atgtgagctc 13080
ccagcctccc ccccgatatg aggagccaga acggcgtcgg tcacggcata aggcatgccc 13140
attgttatct gggcgcttgt cattaccacc gccgcgtccc cggccgatat ctcaccctgg 13200
tcgaggcggt gttgtgtggt gtagatgttc gcgattgtct cggaagcccc cagcacccgc 13260
cagtaagtca tcggctcggg tacgtagacg atatcgtcgc gcgaacccag ggccaccagc 13320
agttgcgtgg tggtggtttt ccccatcccg tggggaccgt ctatataaac ccgcagtagc 13380
gtgggcattt tctgctccgg gcggacttcc gtggcttctt gctgccggcg agggcgcaac 13440
gccgtacgtc ggttgctatg gccgcgagaa cgcgcagcct ggtcgaacgc agacgcgtgt 13500
tgatggccgg ggtacgaagc catacgcgct tctacaaggc gctggccgaa gaggtgcggg 13560
agtttcacgc caccaagatc gatctacagc tggtggggag agcaggaaaa tatggcaaca 13620
aatgttggac tgacgcaacg accttgtcaa ccccgccgac acaccgggcg gacagacggg 13680
gcaaagctgc ctaccaggga ctgagggacc tcagcaggtc gagtgcagag caccggatgg 13740
gtcgactgcc agcttgtgtt cccggtctgc gccgctggcc agctcctgag cggcctttcc 13800
ggtttcatac accgggcaaa gcaggagagg cacgatattt ggacgcccta cagatgccgg 13860
atgggccaat tagggagctt acgcgccggg tactcgctct acctacttcg gagaaggtac 13920
tatctcgtga atcttttacc agatcggaag caattggact tctgtaccta ggttaatggc 13980
atgctatttc gccgacggct atacacccct ggcttcacat tctccttcgc ttactgccgg 14040
tgattcgatg aagctccata ttctccgatg atgcaataga ttcttggtca acgaggggca 14100
caccagcctt tccacttcgg ggcggagggg cggccggtcc cggattaata atcatccact 14160
gcacctcaga gccgccagag ctgtctggcg cagtggccgt tattactcag cccttctctc 14220
tgcgtccgtc cgtctctccg catgccagaa agagtcaccg gtcactgtac agagctcaag 14280
cttcgattaa ctcgaggggg ggcccggtac ccaattcgcc ctatagtgag tcgtattaca 14340

attcactggc cgtcgtttta caacgtcgtg actgggaaaa ccctggcgtt acccaactta 14400

atcgccttgc agcacatccc cctttcgcca gctggcgtaa tagcgaagag gcccgcaccg 14460

atcgcccttc ccaacagttg cgcagcctga atggcgaatg gaaattgtaa gcgttaatat 14520

tttgttaaaa ttcgcgttaa atttttgtta aatcagctca ttttttaacc aataggccga 14580

aatcggcaaa atcccttata aatcaaaaga atagaccgag atagggttga gtgttgttcc 14640

agtttggaac aagagtccac tattaaagaa cgtggactcc aacgtcaaag ggcgaaaaac 14700

cgtctatcag ggcgatggcc cactacgtga accatcaccc taatcaagtt ttttggggtc 14760

gaggtgccgt aaagcactaa atcggaaccc taaagggagc ccccgattta gagcttgacg 14820

gggaaagccg gcgaacgtgg cgagaaagga agggaagaaa gcgaaaggag cgggcgctag 14880

ggcgctggca agtgtagcgg tcacgctgcg cgtaaccacc acacccgccg cgcttaatgc 14940

gccgctacag ggcgcgtcag gtggcacttt tcggggaaat gtgcgcggaa cccctatttg 15000

tttatttttc taaatacatt caaatatgta tccgctcatg agacaataac cctgataaat 15060

gcttcaataa tattgaaaaa ggaagagtat gagtattcaa catttccgtg tcgcccttat 15120

tccctttttt gcggcatttt gccttcctgt ttttgctcac ccagaaacgc tggtgaaagt 15180

aaaagatgct gaagatcagt tgggtgcacg agtgggttac atcgaactgg atctcaacag 15240

cggtaagatc cttgagagtt ttcgccccga agaacgtttt ccaatgatga gcacttttaa 15300

agttctgcta tgtggcgcgg tattatcccg tattgacgcc gggcaagagc aactcggtcg 15360

ccgcatacac tattctcaga atgacttggt tgagtactca ccagtcacag aaaagcatct 15420

tacggatggc atgacagtaa gagaattatg cagtgctgcc ataaccatga gtgataacac 15480

tgcggccaac ttacttctga caacgatcgg aggaccgaag gagctaaccg cttttttgca 15540

caacatgggg gatcatgtaa ctcgccttga tcgttgggaa ccggagctga atgaagccat 15600

accaaacgac gagcgtgaca ccacgatgcc tgtagcaatg gcaacaacgt tgcgcaaact 15660

attaactggc gaactactta ctctagcttc ccggcaacaa ttaatagact ggatggaggc 15720

ggataaagtt gcaggaccac ttctgcgctc ggcccttccg gctggctggt ttattgctga 15780

taaatctgga gccggtgagc gtgggtctcg cggtatcatt gcagcactgg ggccagatgg 15840

taagccctcc cgtatcgtag ttatctacac gacggggagt caggcaacta tggatgaacg 15900

aaatagacag atcgctgaga taggtgcctc actgattaag cattggtaac tgtcagacca 15960

agtttactca tatatacttt agattgattt aaaacttcat ttttaattta aaaggatcta 16020

ggtgaagatc ctttttgata atctcatgac caaaatccct taacgtgagt tttcgttcca 16080

ctgagcgtca gaccccgtag aaaagatcaa aggatcttct tgagatcctt tttttctgcg 16140

cgtaatctgc tgcttgcaaa caaaaaaacc accgctacca gcggtggttt gtttgccgga 16200

```
tcaagagcta ccaactcttt ttccgaaggt aactggcttc agcagagcgc agataccaaa    16260
tactgtcctt ctagtgtagc cgtagttagg ccaccacttc aagaactctg tagcaccgcc    16320
tacatacctc gctctgctaa tcctgttacc agtggctgct gccagtggcg ataagtcgtg    16380
tcttaccggg ttggactcaa gacgatagtt accggataag gcgcagcggt cgggctgaac    16440
gggggttcg tgcacacagc ccagcttgga gcgaacgacc tacaccgaac tgagatacct     16500
acagcgtgag ctatgagaaa gcgccacgct tcccgaaggg agaaaggcgg acaggtatcc    16560
ggtaagcggc agggtcggaa caggagagcg cacgagggag cttccagggg gaaacgcctg    16620
gtatctttat agtcctgtcg ggtttcgcca cctctgactt gagcgtcgat ttttgtgatg    16680
ctcgtcaggg gggcggagcc tatggaaaaa cgccagcaac gcggcctttt tacggttcct    16740
ggccttttgc tggccttttg ctcacatgtt ctttcctgcg ttatcccctg attctgtgga    16800
taaccgtatt accgcctttg agtgagctga taccgctcgc cgcagccgaa cgaccgagcg    16860
cagcgagtca gtgagcgagg aagcggaaga gcgcccaata cgcaaaccgc ctctccccgc    16920
gcgttggccg attca                                                     16935
```

<210> 2
<211> 49
<212> DNA
<213> artificial sequence

<220>
<223> FLP recombinase recognition sequence

<400> 2
ttgaagttcc tattccgagt tcctattctc tagaaagtat aggaacttc 49

<210> 3
<211> 50
<212> DNA
<213> artificial sequence

<220>
<223> FLP recombinase recognition sequence

<400> 3
ttgaagttcc tattccgagt tcctattctt caaatagtat aggaacttca 50

<210> 4
<211> 6055
<212> DNA
<213> artificial sequence

<220>
<223> Vector pFRT-GIAMG

<400> 4

```
tcgcgcgttt cggtgatgac ggtgaaaacc tctgacacat gcagctcccg gagacggtca        60
cagcttgtct gtaagcggat gccgggagca gacaagcccg tcagggcgcg tcagcgggtg       120
```

ttggcgggtg tcggggctgg cttaactatg cggcatcaga gcagattgta ctgagagtgc 180
accatatgcg gtgtgaaata ccgcacagat gcgtaaggag aaaataccgc atcaggcgcc 240
attcgccatt caggctgcgc aactgttggg aagggcgatc ggtgcgggcc tcttcgctat 300
tacgccagct ggcgaaaggg ggatgtgctg caaggcgatt aagttgggta acgccagggt 360
tttcccagtc acgacgttgt aaaacgacgg ccagtgaatt cttgaagttc ctattccgag 420
ttcctattct ctagaaagta taggaacttc ctcgagacca tgtaccgctt ccttgtctgt 480
gctctcgggc ttctggggac agtcctcgct cagtcagtcg acagttatgt cggcagcgaa 540
ggccccatag caaaggccgg cgtccttgcc aacattgggc cgaacggctc aaaggcctct 600
ggtgcagccg ccggcgtggt ggtggctagc cccagcaagt cggatcccga ctattggtac 660
acttggacgc gtgactcgtc actcgttttc aagtctctca ttgatcagta caccactggt 720
atcgacagca cgagttcgtt gaggtctctg atagacagtt tcgttattgc cgaggccaac 780
attcagcagg tctctaatcc cagcggcact cttactaccg gcggcttggg agagccaaaa 840
ttcaatgtcg atgaaactgc attcaccggt gcatggggtc gaccccagcg cgacggacct 900
gcgctccgtg cgactgcttt gatcacctac ggtaactggc tcttgtcaaa cgggaacacg 960
acctgggtta ccagtacgct gtggccgatc atccagaacg atctcaacta cgtcgttcag 1020
tactggaacc agaccacctt cgacctctgg gaagaagtga actcttcctc gttcttcacc 1080
actgcagtgc agcaccgtgc cttgcgcgaa ggcgcagcat tcgctaccaa gatcggtcag 1140
acctcctcgg tcagcagcta cacaacccaa gcggcgaatc tactttgctt tttgcagtct 1200
tactggaacc ccacttccgg atatatcacc gctaacactg gcggtggtcg gtccggcaag 1260
gacgccaaca ccctcttggc atccatccac acttacgacc ccagcgcggg ctgcgatgcc 1320
acgaccttcc agccctgctc cgacaaagcc ctctcgaatc tgaaggttta cgtcgactcc 1380
ttccgttctg tctactccat caacagcggt attgcctcta acgccgctgt cgccactggt 1440
cgctacccgg aagacagcta ccagggcggg aacccatggt acctcactac gttcgccgtc 1500
gccgagcagc tctatgacgc cctcaatgtc tgggctgctc agggctccct caatgtcacc 1560
tccatctccc tccccttctt ccagcagttc tcctctagtg tcactgccgg cacttacgct 1620
tcgagctcca ccacttacac gactctgacc tccgccatta agagcttcgc ggatggattc 1680
gtcgctatca acgcccagta cacgccgtcc aacggtggcc tcgctgagca gttcagcagg 1740
agcaacggcg ctcccgtcag cgctgttgat ttgacatgga gctatgcatc tgcattgacc 1800
gcgtttgaag cgaggaataa tactcagttc gccggctggg gcgcggtagg tttgactgtg 1860
ccgacctcgt gctccagcaa cagtggtgga ggcggaggat cgactgtcgc cgtgacgttc 1920
aacgtgaacg cccaaacggt ttggggcgaa aacatctaca tcactggctc ggttgacgct 1980

ctgagtaact ggtctcccga caacgccctc ttgctctcgt ctgccaacta cccgacctgg 2040
agcattaccg tgaatttacc cgcgagcact gccattcagt ataagtatat ccgcaagaac 2100
aacggagctg tcacctggga atccgatccc aacaacagca taactactcc agccagcggc 2160
tccgtgaccg agaatgacac ttggcgttaa ttaattaata ataaataacg gattgtgtcc 2220
gtaatcacac gtgcccgtgg tgcgtacgat aacgcatagt gtttttccct ccacttaaat 2280
cgaagggttg tgtcttggat cgcgcgggtc aaatgtatat ggttcatata catccgcagg 2340
cacgtaataa agcgaggggt tcgaatcccc ccgttacccc cggtaggggc ccagatccgg 2400
gtgactgaca cctggcggta gacaatcaat ccatttcgct atagttaaag gatggggatg 2460
agggcaattg gttatatgat catgtatgta gtgggtgtgc ataatagtag tgaaatggaa 2520
gccaagtcat gtgattgtaa tcgaccgacg gaattgagga tatccggaaa tacagacacc 2580
gtgaaagcca tggtctttcc ttcgtgtaga agaccagaca gacagtccct gatttaccct 2640
tgcacaaagc actagaaaat tagcattcca tccttctctg cttgctctgc tgatatcact 2700
gtcattcaat gcatagccat gagctcatct tagatccaag cacgtaattc catagccgag 2760
gtccacagtg gagcagcaac attccccatc attgctttcc ccaggggcct cccaacgact 2820
aaatcaagag tatatctcta ccgtccaata gatcgtcttc gcttcaaaat ctttgacaat 2880
tccaagaggg tccccatcca tcaaacccag ttcaataata gccgagatgc atggtggagt 2940
caattaggca gtattgctgg aatgtcgggg ccagttggcc cggtggtcat tggccgcctg 3000
tgatgccatc tgccactaaa tccgatcatt gatccaccgc ccacgaggcg cgtctttgct 3060
ttttgcgcgg cgtccaggtt caactctctc cactagtcta gcggggttca aatgcaaaca 3120
agtacaacac gcagcaaacg aagcagccca ccactgcgtt gatgcccagt ttgactgtcc 3180
gaaatccacc ggaaaggtgg aaacatacta tgtaacaatc agagggaaga aaaaattttt 3240
atcgacgagg caggatagtg actgatggtg gggtcatggt cgggtctccg agcgaaagag 3300
aaccaaggaa acaagatcaa cgaggttggt gtacccaaaa ggccgcagca acaagagtca 3360
tcgcccaaaa gtcaacagtc tggaagagac tccgccgtgc agattctgcg tcggtcccgc 3420
acatgcgtgg tgggggcatt acccctccat gtccaatgat aagggcggcg gtcgagggct 3480
taagcccgcc cactaattcg ccttctcgct tgcccctcca tataaggatt ccccctcctt 3540
cccctcccac aactttttc cttctttctc tcttcgtccg catcagtacg tatatctttc 3600
ccccatacct cctttcctac tcttcttcca ttcattcaac tcttctcctt actgacatct 3660
gttttgctca gtacctctac gcgatcagcc gtagtatctg agcaagcttc tctacagaat 3720
ctttctagta tcttacaaag aactacaaag ttcgcaccat tgaagttcct attccgagtt 3780
cctattcttc aaatagtata ggaacttcag catgcaagct tggcgtaatc atggtcatag 3840
ctgtttcctg tgtgaaattg ttatccgctc acaattccac acaacatacg agccggaagc 3900

ataaagtgta aagcctgggg tgcctaatga gtgagctaac tcacattaat tgcgttgcgc 3960
tcactgcccg ctttccagtc gggaaacctg tcgtgccagc tgcattaatg aatcggccaa 4020
cgcgcgggga gaggcggttt gcgtattggg cgctcttccg cttcctcgct cactgactcg 4080
ctgcgctcgg tcgttcggct gcggcgagcg gtatcagctc actcaaaggc ggtaatacgg 4140
ttatccacag aatcagggga taacgcagga aagaacatgt gagcaaaagg ccagcaaaag 4200
gccaggaacc gtaaaaaggc cgcgttgctg gcgtttttcc ataggctccg cccccctgac 4260
gagcatcaca aaaatcgacg ctcaagtcag aggtggcgaa acccgacagg actataaaga 4320
taccaggcgt ttccccctgg aagctccctc gtgcgctctc ctgttccgac cctgccgctt 4380
accggatacc tgtccgcctt tctcccttcg ggaagcgtgg cgctttctca tagctcacgc 4440
tgtaggtatc tcagttcggt gtaggtcgtt cgctccaagc tgggctgtgt gcacgaaccc 4500
cccgttcagc ccgaccgctg cgccttatcc ggtaactatc gtcttgagtc caacccggta 4560
agacacgact tatcgccact ggcagcagcc actggtaaca ggattagcag agcgaggtat 4620
gtaggcggtg ctacagagtt cttgaagtgg tggcctaact acggctacac tagaaggaca 4680
gtatttggta tctgcgctct gctgaagcca gttaccttcg gaaaaagagt tggtagctct 4740
tgatccggca aacaaaccac cgctggtagc ggtggttttt ttgtttgcaa gcagcagatt 4800
acgcgcagaa aaaaaggatc tcaagaagat cctttgatct tttctacggg gtctgacgct 4860
cagtggaacg aaaactcacg ttaagggatt ttggtcatga gattatcaaa aaggatcttc 4920
acctagatcc ttttaaatta aaaatgaagt tttaaatcaa tctaaagtat atatgagtaa 4980
acttggtctg acagttacca atgcttaatc agtgaggcac ctatctcagc gatctgtcta 5040
tttcgttcat ccatagttgc ctgactcccc gtcgtgtaga taactacgat acgggagggc 5100
ttaccatctg gccccagtgc tgcaatgata ccgcgagacc cacgctcacc ggctccagat 5160
ttatcagcaa taaaccagcc agccggaagg gccgagcgca gaagtggtcc tgcaacttta 5220
tccgcctcca tccagtctat taattgttgc cgggaagcta gagtaagtag ttcgccagtt 5280
aatagtttgc gcaacgttgt tgccattgct acaggcatcg tggtgtcacg ctcgtcgttt 5340
ggtatggctt cattcagctc cggttcccaa cgatcaaggc gagttacatg atcccccatg 5400
ttgtgcaaaa aagcggttag ctccttcggt cctccgatcg ttgtcagaag taagttggcc 5460
gcagtgttat cactcatggt tatggcagca ctgcataatt ctcttactgt catgccatcc 5520
gtaagatgct tttctgtgac tggtgagtac tcaaccaagt cattctgaga atagtgtatg 5580
cggcgaccga gttgctcttg cccggcgtca atacgggata ataccgcgcc acatagcaga 5640
actttaaaag tgctcatcat tggaaaacgt tcttcggggc gaaaactctc aaggatctta 5700
ccgctgttga gatccagttc gatgtaaccc actcgtgcac ccaactgatc ttcagcatct 5760

tttactttca ccagcgtttc tgggtgagca aaaacaggaa ggcaaaatgc cgcaaaaaag 5820

ggaataaggg cgacacggaa atgttgaata ctcatactct tcctttttca atattattga 5880

agcatttatc agggttattg tctcatgagc ggatacatat ttgaatgtat ttagaaaaat 5940

aaacaaatag gggttccgcg cacatttccc cgaaaagtgc cacctgacgt ctaagaaacc 6000

attattatca tgacattaac ctataaaaat aggcgtatca cgaggccctt tcgtc 6055

<210> 5
<211> 1731
<212> DNA
<213> Gloeophyllum trabeum

<400> 5

```
atgtaccgct tccttgtctg tgctctcggg cttctgggga cagtcctcgc tcagtcagtc      60
gacagttatg tcggcagcga aggccccata gcaaaggccg gcgtccttgc caacattggg     120
ccgaacggct caaaggcctc tggtgcagcc gccggcgtgg tggtggctag ccccagcaag     180
tcggatcccg actattggta cacttggacg cgtgactcgt cactcgtttt caagtctctc     240
attgatcagt acaccactgg tatcgacagc acgagttcgt tgaggtctct gatagacagt     300
ttcgttattg ccgaggccaa cattcagcag gtctctaatc ccagcggcac tcttactacc     360
ggcggcttgg gagagccaaa attcaatgtc gatgaaactg cattcaccgg tgcatggggt     420
cgaccccagc gcgacggacc tgcgctccgt gcgactgctt tgatcaccta cggtaactgg     480
ctcttgtcaa acgggaacac gacctgggtt accagtacgc tgtggccgat catccagaac     540
gatctcaact acgtcgttca gtactggaac cagaccacct tcgacctctg ggaagaagtg     600
aactcttcct cgttcttcac cactgcagtg cagcaccgtg ccttgcgcga aggcgcagca     660
ttcgctacca agatcggtca gacctcctcg gtcagcagct acacaaccca agcggcgaat     720
ctactttgct ttttgcagtc ttactggaac ccccacttccg gatatatcac cgctaacact     780
ggcggtggtc ggtccggcaa ggacgccaac accctcttgg catccatcca cacttacgac     840
cccagcgcgg gctgcgatgc cacgaccttc cagccctgct ccgacaaagc cctctcgaat     900
ctgaaggttt acgtcgactc cttccgttct gtctactcca tcaacagcgg tattgcctct     960
aacgccgctg tcgccactgg tcgctacccg gaagacagct accagggcgg gaacccatgg    1020
tacctcacta cgttcgccgt cgccgagcag ctctatgacg ccctcaatgt ctgggctgct    1080
cagggctccc tcaatgtcac ctccatctcc ctccccttct tccagcagtt ctcctctagt    1140
gtcactgccg gcacttacgc ttcgagctcc accacttaca cgactctgac ctccgccatt    1200
aagagcttcg cggatggatt cgtcgctatc aacgcccagt acacgccgtc caacggtggc    1260
ctcgctgagc agttcagcag gagcaacggc gctcccgtca gcgctgttga tttgacatgg    1320
agctatgcat ctgcattgac cgcgtttgaa gcgaggaata atactcagtt cgccggctgg    1380
ggcgcggtag gtttgactgt gccgacctcg tgctccagca acagtggtgg aggcggagga    1440
tcgactgtcg ccgtgacgtt caacgtgaac gcccaaacgg tttggggcga aaacatctac    1500
atcactggct cggttgacgc tctgagtaac tggtctcccg acaacgccct cttgctctcg    1560
tctgccaact acccgacctg gagcattacc gtgaatttac ccgcgagcac tgccattcag    1620
tataagtata tccgcaagaa caacggagct gtcacctggg aatccgatcc caacaacagc    1680
ataactactc cagccagcgg ctccgtgacc gagaatgaca cttggcgtta a             1731
```

<210> 6
<211> 576

<212> PRT
<213> Gloeophyllum trabeum

<400> 6

```
Met Tyr Arg Phe Leu Val Cys Ala Leu Gly Leu Leu Gly Thr Val Leu
1               5                   10                  15

Ala Gln Ser Val Asp Ser Tyr Val Gly Ser Glu Gly Pro Ile Ala Lys
            20                  25                  30

Ala Gly Val Leu Ala Asn Ile Gly Pro Asn Gly Ser Lys Ala Ser Gly
        35                  40                  45

Ala Ala Ala Gly Val Val Val Ala Ser Pro Ser Lys Ser Asp Pro Asp
    50                  55                  60

Tyr Trp Tyr Thr Trp Thr Arg Asp Ser Ser Leu Val Phe Lys Ser Leu
65                  70                  75                  80

Ile Asp Gln Tyr Thr Thr Gly Ile Asp Ser Thr Ser Ser Leu Arg Ser
                85                  90                  95

Leu Ile Asp Ser Phe Val Ile Ala Glu Ala Asn Ile Gln Gln Val Ser
            100                 105                 110

Asn Pro Ser Gly Thr Leu Thr Thr Gly Gly Leu Gly Glu Pro Lys Phe
        115                 120                 125

Asn Val Asp Glu Thr Ala Phe Thr Gly Ala Trp Gly Arg Pro Gln Arg
    130                 135                 140

Asp Gly Pro Ala Leu Arg Ala Thr Ala Leu Ile Thr Tyr Gly Asn Trp
145                 150                 155                 160

Leu Leu Ser Asn Gly Asn Thr Thr Trp Val Thr Ser Thr Leu Trp Pro
                165                 170                 175
```

Ile Ile Gln Asn Asp Leu Asn Tyr Val Val Gln Tyr Trp Asn Gln Thr
180 185 190

Thr Phe Asp Leu Trp Glu Glu Val Asn Ser Ser Ser Phe Phe Thr Thr
195 200 205

Ala Val Gln His Arg Ala Leu Arg Glu Gly Ala Ala Phe Ala Thr Lys
210 215 220

Ile Gly Gln Thr Ser Ser Val Ser Ser Tyr Thr Thr Gln Ala Ala Asn
225 230 235 240

Leu Leu Cys Phe Leu Gln Ser Tyr Trp Asn Pro Thr Ser Gly Tyr Ile
245 250 255

Thr Ala Asn Thr Gly Gly Gly Arg Ser Gly Lys Asp Ala Asn Thr Leu
260 265 270

Leu Ala Ser Ile His Thr Tyr Asp Pro Ser Ala Gly Cys Asp Ala Thr
275 280 285

Thr Phe Gln Pro Cys Ser Asp Lys Ala Leu Ser Asn Leu Lys Val Tyr
290 295 300

Val Asp Ser Phe Arg Ser Val Tyr Ser Ile Asn Ser Gly Ile Ala Ser
305 310 315 320

Asn Ala Ala Val Ala Thr Gly Arg Tyr Pro Glu Asp Ser Tyr Gln Gly
325 330 335

Gly Asn Pro Trp Tyr Leu Thr Thr Phe Ala Val Ala Glu Gln Leu Tyr
340 345 350

Asp Ala Leu Asn Val Trp Ala Ala Gln Gly Ser Leu Asn Val Thr Ser
355 360 365

Ile Ser Leu Pro Phe Phe Gln Gln Phe Ser Ser Ser Val Thr Ala Gly
370 375 380

Thr Tyr Ala Ser Ser Ser Thr Thr Tyr Thr Thr Leu Thr Ser Ala Ile
385 390 395 400

Lys Ser Phe Ala Asp Gly Phe Val Ala Ile Asn Ala Gln Tyr Thr Pro
405 410 415

Ser Asn Gly Gly Leu Ala Glu Gln Phe Ser Arg Ser Asn Gly Ala Pro
420 425 430

Val Ser Ala Val Asp Leu Thr Trp Ser Tyr Ala Ser Ala Leu Thr Ala
435 440 445

Phe Glu Ala Arg Asn Asn Thr Gln Phe Ala Gly Trp Gly Ala Val Gly
450 455 460

Leu Thr Val Pro Thr Ser Cys Ser Ser Asn Ser Gly Gly Gly Gly Gly
465 470 475 480

Ser Thr Val Ala Val Thr Phe Asn Val Asn Ala Gln Thr Val Trp Gly
485 490 495

Glu Asn Ile Tyr Ile Thr Gly Ser Val Asp Ala Leu Ser Asn Trp Ser
500 505 510

Pro Asp Asn Ala Leu Leu Leu Ser Ser Ala Asn Tyr Pro Thr Trp Ser
515 520 525

Ile Thr Val Asn Leu Pro Ala Ser Thr Ala Ile Gln Tyr Lys Tyr Ile
530 535 540

Arg Lys Asn Asn Gly Ala Val Thr Trp Glu Ser Asp Pro Asn Asn Ser
545 550 555 560

Ile Thr Thr Pro Ala Ser Gly Ser Val Thr Glu Asn Asp Thr Trp Arg
565 570 575

<210> 7
<211> 16642
<212> DNA
<213> artificial sequence

<220>
<223> Plasmid p007

<220>
<221> misc_feature
<222> (11576)..(11576)
<223> n is a, c, g, or t

<400> 7

ttaatgcagc tggcacgaca ggtttcccga ctggaaagcg ggcagtgagc gcaacgcaat 60

taatgtgagt tagctcactc attaggcacc ccaggcttta cactttatgc ttccggctcg 120

tatgttgtgt ggaattgtga gcggataaca atttcacaca ggaaacagct atgaccatga 180

ttacgccaag ctcgaaatta accctcacta aagggaacaa aagctggagc tccaccgcgg 240

caacaggcag aatatcttcc gaattcaatc gactgcgcga tgcaagttgg ctagcaacgg 300

cgtacacctt gggattatgc gctgctcaac cgatggtcag ctatcaaaca aaatttggga 360
agatcgggct atactgacgg tgacattata gtacggcaag ctgagtgaca tctacggtcg 420
caagccactg cttctttggg catatgtttt ctttggcgtg ggatgcatta tcaggtagat 480
actccctttt tcttatacgc tggtttgctg gttcgtgctg acagctgttt ccctagcggt 540
attggtcgag acatggcgac tgtcatattg ggcgtgcaa tcagcggaat tgggggtgct 600
ggaacaatgg cgatgggctc tatcattatc acaggtaggc tagcagctta tcaggttgaa 660
agaactgtca ctgaacatag gcagatattg ttcctcgtcg agatgttgcc cattggcggg 720
cgtacatcaa tatcgcgatg actctgggtc gtagcgcagg aggcccaatc ggcggatggc 780
taaccgatac aatcggatgg agatggtatg ctttgcgcct ttgtgaccgc ttctctcact 840
aaattgtggc caaggtcgtt tattatccaa ggccccttag ccgctgtggc agctctgttg 900
gtgatatgga agctcaaact cgccaatcca gtcactgaga agagcatccg ccgtgtcgac 960
tttctcggaa cattcctcct ggccgtcggt attgttacaa tcaccgttat catggaccaa 1020
gcagggcagt ccttcgcatg ggcatcattg tcaacagcaa tccttgcaac tctcagtcta 1080
tcagcattcg tcgccttcgt ccttgttgaa ctctacgtag cccctgaacc gattttcgaa 1140
cttcgcatgt tgcggaagcc gaatgtgacg cccagttacc tgatcggatc gctgcagatc 1200
accgcccaag ttggaatgat gttctccgtg ccgttatatt ttcaggtgac atcgaaagcc 1260
tctgccaccg tagctggagg gcatctggtt cctgcagtga tcggaaacac gcttggcggc 1320
ttaatcgcgg gagcctttat ccgtcgcacc ggccaattca aggtcctctt gatccttgcc 1380
ggtctcgttg cgtccgtcgc ctatctactc ctcatccttc gctggaacgg tcatactgga 1440
ttctgggagt ccttgtacat tattcccggt ggtatgggta ctggtttctg ctctgcagct 1500
gcttttgtca gtatgacggc gtttttgatg ccgcaggaag tggccatggc aacaggaggt 1560
tacttcctat tattcagctt cgccatgacg gccggtgtca ctgtcactaa cagtctgctg 1620
gggacggttt tcaagcgcca gatggaacag cacctgacgg gtccaggagc caagaaggtt 1680
ggtatccccg caccttttct gcgtcactta ctaacgagta tatgaagatc atcgagcgcg 1740
cgctgtccga caccagctat atcaacggtt tgcagggtca tgtccgggat gtagtggtaa 1800
aaggatatgt gactggtctc cgctacactt actgtaagtc gtttgaatca tgcatccacc 1860
gtccacctta ttaacttggt gccagtattt tccctcattc tttcgctcct tggatcggtc 1920
ctcgcttgga ctgtacgaaa acaccaacta tgaggaacca gcacggcagc tgatagtatc 1980
cgaaagctgc aaattgcttc atcgaggctg gcattcgata gaagaaagaa ctatagacaa 2040
ctagtcttac aatatgacaa ttctctttga ttaataaatg aaaataacac ttgtgtcagc 2100
ctaatagccg agtggcgggc atctctggcg gcctcccgag cagcgtggat ctggaagtgc 2160
gttgatcatt attccccgaa aatgtagtac ccagtaagtg gtctagcggt ggctatggta 2220

ggacatctat gcctaagctg gagttctcat tgaacgtgta ccggccgatt gccctaaact 2280

ctgattgaga gccggaaacc tcatctacct gatgctcagg ggccatccaa tagcttccga 2340

tagcattaca gacagatgga ctcgtcttgg cccacgggtc tagaacagtc gccggaactg 2400

cctctatttg aaacggagct gaaccatgat acttaagcgt gccaagcggc gccgtttccc 2460

actggaacaa ggagcaatag aattctgcag agattcttca ttcaggctat tcagcaattc 2520

ggtttgtgga gcggatcggg gtccactggg tttagtctgg ggtttttctt tgcccgcatg 2580

ggctctagca catgcacagc ttgcagttgc tgctacgcta tctgggaaaa cgaatggcta 2640

ttcaggagtt tataaccaaa agagccggaa acaggctgat tgccctctca cggggagacg 2700

ttgtacttct gatccagagg ctattaaccg gacactacct ataaaggagg tagcattcct 2760

ttctgtccgg ctcccagatt ccaacaaccc aactgacagg ggatccacca tgccccagtt 2820

cgatatcctc tgcaagaccc cccccaaggt cctcgtccgc cagttcgtcg agcgcttcga 2880

gcgcccctcc ggcgagaaga tcgccctctg cgccgccgag ctcacctacc tctgctggat 2940

gatcacccat aacggcaccg ccatcaagcg cgccaccttc atgtcctaca acaccatcat 3000

ctccaactcc ctctccttcg atatcgtcaa caagtccctc cagttcaagt acaagaccca 3060

gaaggccacc atcctggagg cctccctcaa gaagctcatc cccgcctggg agttcaccat 3120

catcccctac tacggccaga agcatcagtc cgatatcacc gatatcgtct cctccctcca 3180

gctccagttc gagtcctccg aggaggccga taagggcaac tcccattcca agaagatgct 3240

caaggccctc ctctccgagg gcgagtccat ctgggagatc accgagaaga tcctcaactc 3300

cttcgagtac acctcccgct tcaccaagac caagaccctc taccagttcc tcttcctcgc 3360

caccttcatc aactgcggcc gcttctccga tatcaagaac gtcgatccca agtccttcaa 3420

gctcgtccag aacaagtacc tcggcgtcat catccagtgc ctcgtcaccg agaccaagac 3480

ctccgtctcc cgccatatct acttcttctc cgcccgcggc cgcatcgatc ccctcgtcta 3540

cctcgatgag ttcctccgca actccgagcc cgtcctcaag cgcgtcaacc gcaccggcaa 3600

ctcctcctcc aacaagcagg agtaccagct cctcaaggat aacctcgtcc gctcctacaa 3660

caaggccctc aagaagaacg cccccctactc catcttcgcc atcaagaacg gccccaagtc 3720

ccatatcggc cgccatctca tgacctcctt cctctccatg aagggcctca ccgagctcac 3780

caacgtcgtc ggcaactggt ccgataagcg cgcctccgcc gtcgcccgca ccacctacac 3840

ccatcagatc accgccatcc ccgatcatta cttcgcacta gtctcccgct actacgccta 3900

cgatcccatc tccaaggaga tgatcgccct caaggatgag accaacccca tcgaggagtg 3960

gcagcatatc gagcagctca agggctccgc cgagggctcc atccgctacc ccgcctggaa 4020

cggcatcatc tcccaggagg tcctcgatta cctctcctcc tacatcaacc gccgcatctg 4080

agtcgagatt atccaaggga atgacttaat gagtatgtaa gacatgggtc ataacggcgt 4140
tcgaaacata tacagggtta tgtttgggaa tagcacacga ataataacgt taataggtac 4200
caaagtcctt gatacattag cacggtagaa aaagaataat acaacgagct gggaatattc 4260
tttaatataa aactccaaga agagctggtg cggtggagct tgttttcgac tctcagtaat 4320
atttcctcat atccaagcgc gctaggaggt ggtcgaatac acatgtaggc gcttctctgg 4380
atgcaaaagt cgtgccggac ctgccgaaag actttgaaga tgcgttcacg ccatctaagt 4440
tgcgtagata attcacaaaa agggatgttt gtttccggaa tgtagcaaag agctgatagg 4500
caatagcctc actttcgtgg cgcacgccgc tcgttccatc catcctcgac aatggagcaa 4560
atgtcaaaat cgtaccgaaa atactttgct agcccgttaa attgccgtcg tcagccgtta 4620
aattaccgat taatcccgat aaatttccga gatctccgtt aaattgccgt tcgcagccgt 4680
taaattaccg gggacgaccg ataaatttcc gcgatgaatt catggtgttt tgatcatttt 4740
aaatttttat atggcgggtg gtgggcaact cgcttgcgcg ggcaactcgc ttaccgatta 4800
cgttagggct gatatttacg taaaaatcgt caagggatgc aagaccaaac cgttaaattt 4860
ccggagtcaa cagcatccaa gcccaagtcc ttcacggaga aaccccagcg tccacatcac 4920
gagcgaagga ccacctctag gcatcggacg caccatccaa ttagaagcag caaagcgaaa 4980
cagcccaaga aaaaggtcgg cccgtcggcc ttttctgcaa cgctgatcac gggcagcgat 5040
ccaaccaaca ccctccagag tgactagggg cggaaattta tcgggattaa tttccactca 5100
accacaaatc acagtcgtcc ccggtaattt aacggctgca gacggcaatt taacggcttc 5160
tgcgaatcgc ttggattccc cgcccctggc cgtagagctt aaagtatgtc ccttgtcgat 5220
gcgatgtatc acaacatata aatactggca agggatgcca tgcttggagc aacaatctca 5280
gaacaccaat atcaattcat ctcgagtctg aactcagaca acacaaattc ttcaaaattg 5340
aggatttagt cttgatcttg aagttcctat tccgagttcc tattctctag aaagtatagg 5400
aacttcttaa ttaacctagc cgttattact ctaccgcaag gcaacaacca gctcacccct 5460
gaggcacggg taccatgggt tgagtggtat ggggccatcc agagtcacct gtggcagcat 5520
gagactgcac tcgaagcagc catcaaccca gccaatattc tgggctttcc atccttagat 5580
cacatttgag atataaccca tttggtgaga gacacttgtg ccgttatacg tgtctagact 5640
ggaaacgcaa ccctgaaggg attcttcctt tgagagatgg aagcgtgtca tatctcttcg 5700
gttctacggc aggttttttt ctgctctttc gtagcatggc atggtcactt cagcgcttat 5760
ttacagttgc tggtattgat ttcttgtgca aattgctatc tgacacttat taggtcgagc 5820
tattcctttg ccctcggacg agtgctgggg cgtcggtttc cactatcggc gagtacttct 5880
acacagccat cggtccagac ggccgcgctt ctgcgggcga tttgtgtacg cccgacagtc 5940
ccggctccgg atcggacgat tgcgtcgcat cgaccctgcg cccaagctgc atcatcgaaa 6000

ttgccgtcaa ccaagctctg atagagttgg tcaagaccaa tgcggagcat atacgcccgg 6060
agccgcggcg atcctgcaag ctccggatgc ctccgctcga agtagcgcgt ctgctgctcc 6120
atacaagcca accacggcct ccagaagaag atgttggcga cctcgtattg ggaatccccg 6180
aacatcgcct cgctccagtc aatgaccgct gttatgcggc cattgtccgt caggacattg 6240
ttggagccga aatccgcgtg cacgaggtgc cggacttcgg ggcagtcctc ggcccaaagc 6300
atcagctcat cgagagcctg cgcgacggac gcactgacgg tgtcgtccat cacagtttgc 6360
cagtgataca catggggatc agcaatcgcg catatgaaat cacgccatgt agtgtattga 6420
ccgattcctt gcggtccgaa tgggccgaac ccgctcgtct ggctaagatc ggccgcagcg 6480
atcgcatcca tggcctccgc gaccggctgc agaacagcgg gcagttcggt ttcaggcagg 6540
tcttgcaacg tgacaccctg tgcacggcgg gagatgcaat aggtcaggct ctcgctgaat 6600
tccccaatgt caagcacttc cggaatcggg agcgcggccg atgcaaagtg ccgataaaca 6660
taacgatctt tgtagaaacc atcggcgcag ctatttaccc gcaggacata tccacgccct 6720
cctacatcga agctgaaagc acgagattct tcgccctccg agagctgcat caggtcggag 6780
acgctgtcga acttttcgat cagaaacttc tcgacagacg tcgcggtgag ttcaggcgac 6840
attgctgagg tgtaggatcg atccgtttaa actctgtgtt agcttatagt caggatgttg 6900
gctcgacgag tgtaaactgg gagttggcat gagggttatg taggcttctt tagccccgca 6960
tcccccctcat tctcctcatt gatcccgggg gagcggatgg tgttgataag agactaatta 7020
tagggtttag ctggtgccta gctggtgatt ggctggcttc gccgaatttt acgggccaag 7080
ggaagctgca gaaccgcggc actggtaaac ggtaattaag ctatcagccc catgctaacg 7140
agtttaaatt acgtgtattg ctgataaaca ccaacagagc tttactgaaa gatgggagtc 7200
acggtgtggc ttccccactg cgattattgc acaagcagcg agggcgaact tgactgtcgt 7260
cgctgagcag cctgcagtca aacatacata tatatcaacc gcgaagacgt ctggccttgt 7320
agaacacgac gctccctagc aacacctgcc gtgtcagcct ctacggttgt tacttgcatt 7380
caggatgctc tccagcgggc gagctattca aaatattcaa agcaggtatc tcgtattgcc 7440
aggattcagc tgaagcaaca ggtgccaagg aaatctgcgt cggttctcat ctgggcttgc 7500
tcggtcctgg cgtagactag ttgaagttcc tattccgagt tcctattctt caaatagtat 7560
aggaacttca tcagggagat gtaacaacgc caccttatgg gactatcaag ctgacgctgg 7620
cttctgtgca gacaaactgc gcccacgagt tcttccctga cgccgctctc gcgcaggcaa 7680
gggaactcga tgaatactac gcaaagcaca agagacccgt tggtccactc catggcctcc 7740
ccatctctct caaagaccag cttcgagtca aggtacaccg ttgcccctaa gtcgttagat 7800
gtccctttt gtcagctaac atatgccacc agggctacga aacatcaatg ggctacatct 7860

catggctaaa caagtacgac gaaggggact cggttctgac aaccatgctc cgcaaagccg 7920

gtgccgtctt ctacgtcaag acctctgtcc cgcagaccct gatggtctgc gagacagtca 7980

acaacatcat cgggcgcacc gtcaacccac gcaacaagaa ctggtcgtgc ggcggcagtt 8040

ctggtggtga gggtgcgatc gttgggattc gtggtggcgt catcggtgta ggaacggata 8100

tcggtggctc gattcgagtg ccggccgcgt tcaacttcct gtacggtcta aggccgagtc 8160

atgggcggct gccgtatgca aagatggcga acagcatgga gggtcaggag acggtgcaca 8220

gcgttgtcgg gccgattacg cactctgttg agggtgagtc cttcgcctct tccttctttt 8280

cctgctctat accaggcctc cactgtcctc ctttcttgct ttttatacta tatacgagac 8340

cggcagtcac tgatgaagta tgttagacct ccgcctcttc accaaatccg tcctcggtca 8400

ggagccatgg aaatacgact ccaaggtcat ccccatgccc tggcgccagt ccgagtcgga 8460

cattattgcc tccaagatca agaacggcgg gctcaatatc ggctactaca acttcgacgg 8520

caatgtcctt ccacaccctc ctatcctgcg cggcgtggaa accaccgtcg ccgcactcgc 8580

caaagccggt cacaccgtga ccccgtggac gccatacaag cacgatttcg gccacgatct 8640

catctcccat atctacgcgg ctgacggcag cgccgacgta atgcgcgata tcagtgcatc 8700

cggcgagccg gcgattccaa atatcaaaga cctactgaac ccgaacatca aagctgttaa 8760

catgaacgag ctctgggaca cgcatctcca gaagtggaat taccagatgg agtaccttga 8820

gaaatggcgg gaggctgaag aaaaggccgg gaaggaactg gacgccatca tcgcgccgat 8880

tacgcctacc gctgcggtac ggcatgacca gttccggtac tatgggtatg cctctgtgat 8940

caacctgctg gatttcacga gcgtggttgt tccggttacc tttgcggata agaacatcga 9000

taagaagaat gagagtttca aggcggttag tgagcttgat gccctcgtgc aggaagagta 9060

tgatccggag gcgtaccatg gggcaccggt tgcagtgcag gttatcggac ggagactcag 9120

tgaagagagg acgttggcga ttgcagagga agtggggaag ttgctgggaa atgtggtgac 9180

tccataggta agctccgtgg cgaaagcctg acgcaccggt agattcttgg tgagcccgta 9240

tcatgacggc ggcgggagct acatggcccc gggtgattta tttttttgt atctacttct 9300

gaccctttc aaatatacgg tcaactcatc tttcactgga gatgcggcct gcttggtatt 9360

gcgatgttgt cagcttggca aattgtggct ttcgaaaaca caaaacgatt ccttagtagc 9420

catgcatttt aagataacgg aatagaagaa agaggaaatt aaaaaaaaaa aaaaaacaaa 9480

catcccgttc ataacccgta gaatcgccgc tcttcgtgta tcccagtacc acggcaaagg 9540

tatttcatga tcgttcaatg ttgatattgt tcccgccagt atggctccac ccccatctcc 9600

gcgaatctcc tcttctcgaa cgcggtagtg gcgcgccaat tggtaatgac ccatagggag 9660

acaaacagca taatagcaac agtggaaatt agtggcgcaa taattgagaa cacagtgaga 9720

ccatagctgg cggcctggaa agcactgttg gagaccaact tgtccgttgc gaggccaact 9780

tgcattgtct agagaatgca atcataacag aaagtacagc cagcgctgtg tcataaagaa 9840
gtccagttgg gaaacgaaag actagaatca aactaaaagt aatccggccg atatggcttc 9900
acgtgcgaag tctcgccttg aggggacatt gtccttgcag gtgattgacc attgcgttca 9960
tatggcgcga tgtttggtag tgtgggtgta gccggtgacc tcacggaagg actaaaggcc 10020
acataccctt ctgagtgcct cttctcttcg tggtcggaac tctcgaatgg gtttttgaca 10080
gttgcactcg tttggttgtg gtcatttgaa ggtctgcgtt cggtcttctg ttcgcgcagg 10140
cgagctgact gagggattga aagctgcata gccatcgttg gcatgcgtta attcgccaaa 10200
gctcagcggc gaaacaggcc tgacctctaa tccatgcatc tgctctgcac tcgattgttc 10260
gtggtgtcct tgcgaagaaa gagaagcctt ggactcggat gactttctgg acgaggtggt 10320
aggatcatca tgattgtaat gagactgtag cacatcatgc gaatcattcg acacacggtg 10380
tctgcccaag ttgacgtcag catcggtatg catttcggta tggtcctcat ggttctcagc 10440
atgtccctcc agaggggact catttccagc ggaaggatta taagcaacat aattgtcatg 10500
tggctgcgac ctttcgtgag actccgagtt tgatctcact gtggactcat gggcgatatg 10560
cggctcatca tgatcttcga atggagagaa atggttgaag tcggaggaca cgggtgattt 10620
agcagcaggg ttgaatgcaa catagccagt ctcgcgctct tcatgtgagc tatatgagtc 10680
atgtggcctg tcatggtcca gaggctccgg atgctcatgg ctagattcat cgtgtgccga 10740
aatcgcgtca ctagcaaagg gcgaggttga cacattggct gcaggactga acgccacata 10800
accactctca ggctcttcat gtgagttata ggagctgtgc ggcatatcat agtcctgagg 10860
ttcacgatgc tcatggctgg attcatcgtg tgccgaaata gcgtgactag caaaaggcga 10920
gggcgaagca ttggttgccg gactgaacgc cacatagccg tccccattgg ctgaactgac 10980
tggtgacaac gtcctaccca tggcgtcggc cgggccagcg gcttggtgag agtgaagacc 11040
attagaagta gctggactga acgatcgcag cgggtattcg ttttcttgag ctggataagg 11100
ggctgcgcca tgctggctga aaggtgagaa tgttcggggt gctgctctat caccagggaa 11160
ggcagacgct ggagtcaaag aacgagtgtt ggatcaattg ccggactgta tgaacggaaa 11220
ggagtgctga ttgtttaaat ggcccgtagc cttcgctagg acctcgtgat tcggggaccg 11280
ttggcccata cccaggagct ggtgtaaaat tggaacgcga cacgggtgtt tggttgcgca 11340
gaatttgcgg tgccggcgag gcgtgatcaa tctggctgta acctgggcct ggggtgtagt 11400
ttgagacagg tgtttgtgtt cgtggcattt gtggcgctgg cgacgctctg tcagtcggcc 11460
catatccagg cgccgaaggt gtgggcgtaa acccttgccg tgatttaatt aagaattctc 11520
ctgtaggctt gagagttcaa ggaagaaaca gtgcaattat ctttgcgaac ccaggngctg 11580
gtgacggaat tttcatagtc aagctatcag agtaaagaag aggagcatgt caaagtacaa 11640

ttagagacaa atatatagtc gcgtggagcc aagagcggat tcctcagtct cgtaggtctc 11700

ttgacgaccg ttgatctgct tgatctcgtc tcccgaaaat gaaaatagac tctgctaagc 11760

tattcttctg cttcgccgga gcctgaaggg cgtactaggg ttgcgaggtc caatgcatta 11820

atgcattgca gatgagctgt atctggaaga ggtaaacccg aaacgcgttt tattcttgtt 11880

gacatggagc tattaaatca ctagaaggca ctctttgctg cttggacaaa tgaacgtatc 11940

ttatcgagat cctgaacacc atttgtctca actccggagc tgacatcgac accaacgatc 12000

ttatatccag attcgtcaag ctgtttgatg atttcagtaa cgttaagtgg atcgatccgg 12060

atagcgcggg ttccttccgg tattgtctcc ttccgtgttt cagttagcct cccccatctc 12120

ccgggcaaac gtgcgcgcca ggtcgcatat cgtcggtatg gagccggggg tggtgacgtg 12180

ggtctggacc atcccggagg taagttgcag cagggcgtcc cggcagccgg cgggcgattg 12240

gtcgtaatcc aggataaaga cgtgcatgga acggaggcgt ttggccaaga cgtccaaggc 12300

ccaggcaaac acgttataca ggtcgccgtt gggggccagc aactcggggc cccgaaacag 12360

ggtaaataac gtgtccccga tatggggtcg tgggcccgcg ttgctctggg gctcggcacc 12420

ctggggcggc acggccgtcc ccgaaagctg tccccagtcc tcccgccacg acccgccgca 12480

ctgcagatac cgcaccgtat tggcaagtag cccgtaaacg cggcgaatcg cagccagcat 12540

agccaggtcc agccgctcgc cggggcgctg gcgtttggcc aggcggtcga tgtgtctgtc 12600

ctccggaagg gccccaagca cgatgttggt gccgggcaag gtcggcggga tgagggccac 12660

gaacgccagc acggcctggg gggtcatgct gcccataagg taccgcgcgg ccgggtagca 12720

caggagggcg gcgatgggat ggcggtcgaa gatgagggtg agggccgggg gcggggcatg 12780

tgagctccca gcctccccc cgatatgagg agccagaacg gcgtcggtca cggcataagg 12840

catgcccatt gttatctggg cgcttgtcat taccaccgcc gcgtccccgg ccgatatctc 12900

accctggtcg aggcggtgtt gtgtggtgta gatgttcgcg attgtctcgg aagcccccag 12960

cacccgccag taagtcatcg gctcgggtac gtagacgata tcgtcgcgcg aacccagggc 13020

caccagcagt tgcgtggtgg tggttttccc catcccgtgg ggaccgtcta tataaacccg 13080

cagtagcgtg ggcattttct gctccgggcg gacttccgtg gcttcttgct gccggcgagg 13140

gcgcaacgcc gtacgtcggt tgctatggcc gcgagaacgc gcagcctggt cgaacgcaga 13200

cgcgtgttga tggccggggt acgaagccat acgcgcttct acaaggcgct ggccgaagag 13260

gtgcgggagt ttcacgccac caagatcgat ctacagctgg tggggagagc aggaaaatat 13320

ggcaacaaat gttggactga cgcaacgacc ttgtcaaccc cgccgacaca ccgggcggac 13380

agacggggca aagctgccta ccagggactg agggacctca gcaggtcgag tgcagagcac 13440

cggatgggtc gactgccagc ttgtgttccc ggtctgcgcc gctggccagc tcctgagcgg 13500

cctttccggt ttcatacacc gggcaaagca ggagaggcac gatatttgga cgccctacag 13560

atgccggatg ggccaattag ggagcttacg cgccgggtac tcgctctacc tacttcggag 13620

aaggtactat ctcgtgaatc ttttaccaga tcggaagcaa ttggacttct gtacctaggt 13680

taatggcatg ctatttcgcc gacggctata cacccctggc ttcacattct ccttcgctta 13740

ctgccggtga ttcgatgaag ctccatattc tccgatgatg caatagattc ttggtcaacg 13800

aggggcacac cagcctttcc acttcggggc ggagggggcgg ccggtcccgg attaataatc 13860

atccactgca cctcagagcc gccagagctg tctggcgcag tggccgttat tactcagccc 13920

ttctctctgc gtccgtccgt ctctccgcat gccagaaaga gtcaccggtc actgtacaga 13980

gctcaagctt cgattaactc gagggggggc ccggtaccca attcgcccta tagtgagtcg 14040

tattacaatt cactggccgt cgttttacaa cgtcgtgact gggaaaaccc tggcgttacc 14100

caacttaatc gccttgcagc acatccccct ttcgccagct ggcgtaatag cgaagaggcc 14160

cgcaccgatc gcccttccca acagttgcgc agcctgaatg gcgaatggaa attgtaagcg 14220

ttaatatttt gttaaaattc gcgttaaatt tttgttaaat cagctcattt tttaaccaat 14280

aggccgaaat cggcaaaatc ccttataaat caaaagaata gaccgagata gggttgagtg 14340

ttgttccagt ttggaacaag agtccactat taaagaacgt ggactccaac gtcaaagggc 14400

gaaaaaccgt ctatcagggc gatggcccac tacgtgaacc atcaccctaa tcaagttttt 14460

tggggtcgag gtgccgtaaa gcactaaatc ggaaccctaa agggagcccc cgatttagag 14520

cttgacgggg aaagccggcg aacgtggcga gaaaggaagg gaagaaagcg aaaggagcgg 14580

gcgctagggc gctggcaagt gtagcggtca cgctgcgcgt aaccaccaca cccgccgcgc 14640

ttaatgcgcc gctacagggc gcgtcaggtg gcacttttcg gggaaatgtg cgcggaaccc 14700

ctatttgttt atttttctaa atacattcaa atatgtatcc gctcatgaga caataaccct 14760

gataaatgct tcaataatat tgaaaaagga agagtatgag tattcaacat ttccgtgtcg 14820

cccttattcc cttttttgcg gcattttgcc ttcctgtttt tgctcaccca gaaacgctgg 14880

tgaaagtaaa agatgctgaa gatcagttgg gtgcacgagt gggttacatc gaactggatc 14940

tcaacagcgg taagatcctt gagagttttc gccccgaaga acgttttcca atgatgagca 15000

cttttaaagt tctgctatgt ggcgcggtat tatcccgtat tgacgccggg caagagcaac 15060

tcggtcgccg catacactat tctcagaatg acttggttga gtactcacca gtcacagaaa 15120

agcatcttac ggatggcatg acagtaagag aattatgcag tgctgccata accatgagtg 15180

ataacactgc ggccaactta cttctgacaa cgatcggagg accgaaggag ctaaccgctt 15240

ttttgcacaa catgggggat catgtaactc gccttgatcg ttgggaaccg gagctgaatg 15300

aagccatacc aaacgacgag cgtgacacca cgatgcctgt agcaatggca acaacgttgc 15360

gcaaactatt aactggcgaa ctacttactc tagcttcccg gcaacaatta atagactgga 15420

tggaggcgga taaagttgca ggaccacttc tgcgctcggc ccttccggct ggctggttta 15480

ttgctgataa atctggagcc ggtgagcgtg ggtctcgcgg tatcattgca gcactggggc 15540

cagatggtaa gccctcccgt atcgtagtta tctacacgac ggggagtcag gcaactatgg 15600

atgaacgaaa tagacagatc gctgagatag gtgcctcact gattaagcat tggtaactgt 15660

cagaccaagt ttactcatat atactttaga ttgatttaaa acttcatttt taatttaaaa 15720

ggatctaggt gaagatcctt tttgataatc tcatgaccaa aatcccttaa cgtgagtttt 15780

cgttccactg agcgtcagac cccgtagaaa agatcaaagg atcttcttga gatccttttt 15840

ttctgcgcgt aatctgctgc ttgcaaacaa aaaaaccacc gctaccagcg gtggtttgtt 15900

tgccggatca agagctacca actctttttc cgaaggtaac tggcttcagc agagcgcaga 15960

taccaaatac tgtccttcta gtgtagccgt agttaggcca ccacttcaag aactctgtag 16020

caccgcctac atacctcgct ctgctaatcc tgttaccagt ggctgctgcc agtggcgata 16080

agtcgtgtct taccgggttg gactcaagac gatagttacc ggataaggcg cagcggtcgg 16140

gctgaacggg gggttcgtgc acacagccca gcttggagcg aacgacctac accgaactga 16200

gatacctaca gcgtgagcta tgagaaagcg ccacgcttcc cgaagggaga aaggcggaca 16260

ggtatccggt aagcggcagg gtcggaacag gagagcgcac gagggagctt ccagggggaa 16320

acgcctggta tctttatagt cctgtcgggt ttcgccacct ctgacttgag cgtcgatttt 16380

tgtgatgctc gtcagggggg cggagcctat ggaaaaacgc cagcaacgcg gcctttttac 16440

ggttcctggc cttttgctgg ccttttgctc acatgttctt tcctgcgtta tcccctgatt 16500

ctgtggataa ccgtattacc gcctttgagt gagctgatac cgctcgccgc agccgaacga 16560

ccgagcgcag cgagtcagtg agcgaggaag cggaagagcg cccaatacgc aaaccgcctc 16620

tccccgcgcg ttggccgatt ca 16642

<210> 8
<211> 80
<212> DNA
<213> artificial sequence

<220>
<223> Primer SpeI-FRTF3-amdS

<400> 8

tggcgtagac tagttgaagt tcctattccg agtcctattc ttcaaatagt ataggaactt 60

catcagggag atgtaacaac 80

<210> 9
<211> 22
<212> DNA

<213> artificial sequence

<220>
<223> Primer amdS-F-probe

<400> 9
ctatggagtc accacatttc cc 22

<210> 10
<211> 6330
<212> DNA
<213> artificial sequence

<220>
<223> Vector pFRT-BsAMG

<400> 10

tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcaggcgcc 240
attcgccatt caggctgcgc aactgttggg aagggcgatc ggtgcgggcc tcttcgctat 300
tacgccagct ggcgaaaggg ggatgtgctg caaggcgatt aagttgggta acgccagggt 360
tttcccagtc acgacgttgt aaaacgacgg ccagtgaatt cttgaagttc ctattccgag 420
ttcctattct ctagaaagta taggaacttc ctcgagacca tgtacttcgc tcgccttctc 480
tccggactct ctctcatcac ctctgcggtt ctcgcccaga ccgttgactc ctatgtcagc 540
actgagggcc ccatcgcgaa ggcgggcctc cttgcgaaca ttggtcccag tggttccaaa 600
tccaacggtg cccatgctgg agttgtcgtc gctagcccta gccaagtcaa cccggactac 660
atgtacacat gggtccgcga ctcttccctc gtgttcaagg tcctcgtcga ccagctcgct 720
tccggccagg acacctctgt ccgtagcctg atcgatgcat tcgtcgcagc ggagtcggcg 780
atgcagcaca cccccaaccc cagtggtgac atcaacactg gtggtcttgc tgagcccaag 840
ttcaacatcg acaccaccgc cttcactggc gcatggggcc gcccgcagcg agatggccct 900
gctctccgtt ctaccaccgt catcaactac gccaactacc tccttgctaa tggcaacacc 960
actgcttggg tggtggccaa cctctggccc atgatcaagc tcgacttgga ctacatccag 1020
aacaactgga accagtctac cttcgacctg tgggaggagc tcaactcctc gtccttcttc 1080
accaccgctg ttcagcaccg tgctctccgc gagggtatta ctctcggcgc gaagctctcg 1140
aagactgccg ataccgccaa ctatggcacc caggccggca acatcctttg cttcctccag 1200
tcatactgga accctaccgc caactacgtg acctccaaca ccggtggtgg acgctccggc 1260
aaggactcca actccgtcct tacttccatc cacactttcg accccgatgc cggatgtgat 1320
gctaccacct tccagccctg ctccgacaag gccctgtcca acctcaaggt ttacgtcgac 1380
tcgttccgct ccatctgggc catcaacgct ggcgctgctg ccaccgctcc cgtcgctgtc 1440

ggccgctacc ccgaggacac ttactacaac ggtaacccct ggtacctctc tactttcgcc 1500

gtcgccgagc agctctacga tgccatcatc gtctggaaca agcagggatc catccaggtc 1560

accagccttt cccttccctt cttccagcag ttcatctcga ccctcaagac cggcacgtac 1620

acccagagct ccacccagtt ccagaccctc gtccctgcca tcaaggcctg ggctgacggc 1680

ttcgtcgcca tcaaccagaa gtacactcct cctaacggtg gtctcggtga acagtacgac 1740

aaggtctctg gcgaccctgt cagcgctgtt gaccttacct ggtcgtatgc ttccgctctc 1800

accgtcttca acgctcgtgc cggaaactac tcccccggct ggggtgccaa gggcctgacc 1860

gtcccggcag tctgccagcc caacgctggc cctcaagtcc aggtgacgtt caaggttcag 1920

gccacgacgg tctacggcga gaacatttac ctcactggtg ctaacccagc tctgtcgaac 1980

tggtcgcccg acaccgccct cgccatgtct gctgctaact accctacctg gtctgtcaca 2040

gtcaccctcc cggcgaacag cgtcatccag tacaagtaca tcaggaagaa caacggcgcc 2100

gtcacctggg agtctgaccc caacaaccag gtcaccatcc cagcgagcgg cacctacacc 2160

gtcaacgata actggcggta attaattaat aataaataac ggattgtgtc cgtaatcaca 2220

cgtgcccgtg gtgcgtacga taacgcatag tgtttttccc tccacttaaa tcgaagggtt 2280

gtgtcttgga tcgcgcgggt caaatgtata tggttcatat acatccgcag gcacgtaata 2340

aagcgagggg ttcgaatccc cccgttaccc ccggtagggg cccagatccg ggtgactgac 2400

acctggcggt agacaatcaa tccatttcgc tatagttaaa ggatggggat gagggcaatt 2460

ggttatatga tcatgtatgt agtgggtgtg cataatagta gtgaaatgga agccaagtca 2520

tgtgattgta atcgaccgac ggaattgagg atatccggaa atacagacac cgtgaaagcc 2580

atggtctttc cttcgtgtag aagaccagac agacagtccc tgatttaccc ttgcacaaag 2640

cactagaaaa ttagcattcc atccttctct gcttgctctg ctgatatcac tgtcattcaa 2700

tgcatagcca tgagctcatc ttagatccaa gcacgtaatt ccatagccga ggtccacagt 2760

ggagcagcaa cattccccat cattgctttc cccaggggcc tcccaacgac taaatcaaga 2820

gtatatctct accgtccaat agatcgtctt cgcttcaaaa tctttgacaa ttccaagagg 2880

gtccccatcc atcaaaccca gttcaataat agccgagatg catggtggag tcaattaggc 2940

agtattgctg gaatgtcggg gccagttggc ccggtggtca ttggccgcct gtgatgccat 3000

ctgccactaa atccgatcat tgatccaccg cccacgaggc gcgtctttgc tttttgcgcg 3060

gcgtccaggt tcaactctct ccactagtct agcggggttc aaatgcaaac aagtacaaca 3120

cgcagcaaac gaagcagccc accactgcgt tgatgcccag tttgactgtc cgaaatccac 3180

cggaaaggtg gaaacatact atgtaacaat cagagggaag aaaaaatttt tatcgacgag 3240

gcaggatagt gactgatggt ggggtcatgg tcgggtctcc gagcgaaaga gaaccaagga 3300

aacaagatca acgaggttgg tgtacccaaa aggccgcagc aacaagagtc atcgcccaaa 3360

agtcaacagt ctggaagaga ctccgccgtg cagattctgc gtcggtcccg cacatgcgtg 3420
gtgggggcat tacccctcca tgtccaatga taagggcggc ggtcgagggc ttaagcccgc 3480
ccactaattc gccttctcgc ttgcccctcc atataaggat tccccctcct tcccctccca 3540
caactttttt ccttctttct ctcttcgtcc gcatcagtac gtatatcttt cccccatacc 3600
tcctttccta ctcttcttcc attcattcaa ctcttctcct tactgacatc tgttttgctc 3660
agtacctcta cgcgatcagc cgtagtatct gagcaagctt ctctacagaa tctttctagt 3720
atcttacaaa gaactacaaa gttcgcacca atgcctcaat cctgggaaga actggccgct 3780
gataagcgcg cccgcctcgc aaaaaccatc cctgatgaat ggaaagtcca gacgctgcct 3840
gcggaagaca gcgttattga tttcccaaag aaatcgggta tcctttcaga ggccgaactg 3900
aagatcacag aggcctccgc tgcagatctt gtgtccaagc tggcggccgg agagttgacc 3960
tcggcggaag ttacgctagc attctgtaaa cgggcagtaa tcgcccagca gttagtaggg 4020
tcccctctac ctcttgaagt tcctattccg agttcctatt cttcaaatag tataggaact 4080
tcacgcatgc aagcttggcg taatcatggt catagctgtt tcctgtgtga aattgttatc 4140
cgctcacaat tccacacaac atacgagccg gaagcataaa gtgtaaagcc tggggtgcct 4200
aatgagtgag ctaactcaca ttaattgcgt tgcgctcact gcccgctttc cagtcgggaa 4260
acctgtcgtg ccagctgcat taatgaatcg gccaacgcgc ggggagaggc ggtttgcgta 4320
ttgggcgctc ttccgcttcc tcgctcactg actcgctgcg ctcggtcgtt cggctgcggc 4380
gagcggtatc agctcactca aaggcggtaa tacggttatc cacagaatca ggggataacg 4440
caggaaagaa catgtgagca aaaggccagc aaaaggccag gaaccgtaaa aaggccgcgt 4500
tgctggcgtt tttccatagg ctccgccccc ctgacgagca tcacaaaaat cgacgctcaa 4560
gtcagaggtg gcgaaacccg acaggactat aaagatacca ggcgtttccc cctggaagct 4620
ccctcgtgcg ctctcctgtt ccgaccctgc cgcttaccgg atacctgtcc gcctttctcc 4680
cttcgggaag cgtggcgctt tctcatagct cacgctgtag gtatctcagt tcggtgtagg 4740
tcgttcgctc caagctgggc tgtgtgcacg aaccccccgt tcagcccgac cgctgcgcct 4800
tatccggtaa ctatcgtctt gagtccaacc cggtaagaca cgacttatcg ccactggcag 4860
cagccactgg taacaggatt agcagagcga ggtatgtagg cggtgctaca gagttcttga 4920
agtggtggcc taactacggc tacactagaa ggacagtatt tggtatctgc gctctgctga 4980
agccagttac cttcggaaaa agagttggta gctcttgatc cggcaaacaa accaccgctg 5040
gtagcggtgg tttttttgtt tgcaagcagc agattacgcg cagaaaaaaa ggatctcaag 5100
aagatccttt gatcttttct acggggtctg acgctcagtg gaacgaaaac tcacgttaag 5160
ggattttggt catgagatta tcaaaaagga tcttcaccta gatcctttta aattaaaaat 5220

gaagttttaa atcaatctaa agtatatatg agtaaacttg gtctgacagt taccaatgct 5280

taatcagtga ggcacctatc tcagcgatct gtctatttcg ttcatccata gttgcctgac 5340

tccccgtcgt gtagataact acgatacggg agggcttacc atctggcccc agtgctgcaa 5400

tgataccgcg agacccacgc tcaccggctc cagatttatc agcaataaac cagccagccg 5460

gaagggccga gcgcagaagt ggtcctgcaa ctttatccgc ctccatccag tctattaatt 5520

gttgccggga agctagagta agtagttcgc cagttaatag tttgcgcaac gttgttgcca 5580

ttgctacagg catcgtggtg tcacgctcgt cgtttggtat ggcttcattc agctccggtt 5640

cccaacgatc aaggcgagtt acatgatccc ccatgttgtg caaaaaagcg gttagctcct 5700

tcggtcctcc gatcgttgtc agaagtaagt tggccgcagt gttatcactc atggttatgg 5760

cagcactgca taattctctt actgtcatgc catccgtaag atgcttttct gtgactggtg 5820

agtactcaac caagtcattc tgagaatagt gtatgcggcg accgagttgc tcttgcccgg 5880

cgtcaatacg ggataatacc gcgccacata gcagaacttt aaaagtgctc atcattggaa 5940

aacgttcttc ggggcgaaaa ctctcaagga tcttaccgct gttgagatcc agttcgatgt 6000

aacccactcg tgcacccaac tgatcttcag catcttttac tttcaccagc gtttctgggt 6060

gagcaaaaac aggaaggcaa aatgccgcaa aaaagggaat aagggcgaca cggaaatgtt 6120

gaatactcat actcttcctt tttcaatatt attgaagcat ttatcagggt tattgtctca 6180

tgagcggata catatttgaa tgtatttaga aaaataaaca aataggggtt ccgcgcacat 6240

ttccccgaaa agtgccacct gacgtctaag aaaccattat tatcatgaca ttaacctata 6300

aaaataggcg tatcacgagg ccctttcgtc 6330

<210> 11
<211> 1722
<212> DNA
<213> Byssocorticium

<400> 11

```
atgtacttcg ctcgccttct ctccggactc tctctcatca cctctgcggt tctcgcccag      60
accgttgact cctatgtcag cactgagggc cccatcgcga aggcgggcct ccttgcgaac     120
attggtccca gtggttccaa atccaacggt gcccatgctg gagttgtcgt cgctagccct     180
agccaagtca acccggacta catgtacaca tgggtccgcg actcttccct cgtgttcaag     240
gtcctcgtcg accagctcgc ttccggccag gacacctctg tccgtagcct gatcgatgca     300
ttcgtcgcag cggagtcggc gatgcagcac acccccaacc ccagtggtga catcaacact     360
ggtggtcttg ctgagcccaa gttcaacatc gacaccaccg ccttcactgg cgcatggggc     420
cgcccgcagc gagatggccc tgctctccgt tctaccaccg tcatcaacta cgccaactac     480
ctccttgcta atggcaacac cactgcttgg gtggtggcca acctctggcc catgatcaag     540
ctcgacttgg actacatcca gaacaactgg aaccagtcta ccttcgacct gtgggaggag     600
ctcaactcct cgtccttctt caccaccgct gttcagcacc gtgctctccg cgagggtatt     660
actctcggcg cgaagctctc gaagactgcc gataccgcca actatggcac ccaggccggc     720
aacatccttt gcttcctcca gtcatactgg aaccctaccg ccaactacgt gacctccaac     780
accggtggtg gacgctccgg caaggactcc aactccgtcc ttacttccat ccacactttc     840
gaccccgatg ccggatgtga tgctaccacc ttccagccct gctccgacaa ggccctgtcc     900
aacctcaagg tttacgtcga ctcgttccgc tccatctggg ccatcaacgc tggcgctgct     960
gccaccgctc ccgtcgctgt cggccgctac cccgaggaca cttactacaa cggtaacccc    1020
tggtacctct ctactttcgc cgtcgccgag cagctctacg atgccatcat cgtctggaac    1080
aagcagggat ccatccaggt caccagcctt tcccttccct tcttccagca gttcatctcg    1140
accctcaaga ccggcacgta cacccagagc tccacccagt tccagaccct cgtccctgcc    1200
atcaaggcct gggctgacgg cttcgtcgcc atcaaccaga agtacactcc tcctaacggt    1260
ggtctcggtg aacagtacga caaggtctct ggcgaccctg tcagcgctgt tgaccttacc    1320
tggtcgtatg cttccgctct caccgtcttc aacgctcgtg ccggaaacta ctcccccggc    1380
tggggtgcca agggcctgac cgtcccggca gtctgccagc ccaacgctgg ccctcaagtc    1440
caggtgacgt tcaaggttca ggccacgacg gtctacggcg agaacattta cctcactggt    1500
gctaacccag ctctgtcgaa ctggtcgccc gacaccgccc tcgccatgtc tgctgctaac    1560
taccctacct ggtctgtcac agtcaccctc ccggcgaaca gcgtcatcca gtacaagtac    1620
atcaggaaga acaacggcgc cgtcacctgg gagtctgacc ccaacaacca ggtcaccatc    1680
ccagcgagcg gcacctacac cgtcaacgat aactggcggt aa                       1722
```

<210> 12
<211> 573

<212> PRT
<213> Byssocorticium

<400> 12

```
Met Tyr Phe Ala Arg Leu Leu Ser Gly Leu Ser Leu Ile Thr Ser Ala
1               5                   10                  15

Val Leu Ala Gln Thr Val Asp Ser Tyr Val Ser Thr Glu Gly Pro Ile
            20                  25                  30

Ala Lys Ala Gly Leu Leu Ala Asn Ile Gly Pro Ser Gly Ser Lys Ser
        35                  40                  45

Asn Gly Ala His Ala Gly Val Val Val Ala Ser Pro Ser Gln Val Asn
    50                  55                  60
```

```
Pro Asp Tyr Met Tyr Thr Trp Val Arg Asp Ser Ser Leu Val Phe Lys
65                  70                  75                  80

Val Leu Val Asp Gln Leu Ala Ser Gly Gln Asp Thr Ser Val Arg Ser
                85                  90                  95

Leu Ile Asp Ala Phe Val Ala Ala Glu Ser Ala Met Gln His Thr Pro
            100                 105                 110

Asn Pro Ser Gly Asp Ile Asn Thr Gly Gly Leu Ala Glu Pro Lys Phe
        115                 120                 125

Asn Ile Asp Thr Thr Ala Phe Thr Gly Ala Trp Gly Arg Pro Gln Arg
    130                 135                 140

Asp Gly Pro Ala Leu Arg Ser Thr Thr Val Ile Asn Tyr Ala Asn Tyr
145                 150                 155                 160

Leu Leu Ala Asn Gly Asn Thr Thr Ala Trp Val Val Ala Asn Leu Trp
                165                 170                 175

Pro Met Ile Lys Leu Asp Leu Asp Tyr Ile Gln Asn Asn Trp Asn Gln
            180                 185                 190

Ser Thr Phe Asp Leu Trp Glu Glu Leu Asn Ser Ser Ser Phe Phe Thr
        195                 200                 205

Thr Ala Val Gln His Arg Ala Leu Arg Glu Gly Ile Thr Leu Gly Ala
    210                 215                 220

Lys Leu Ser Lys Thr Ala Asp Thr Ala Asn Tyr Gly Thr Gln Ala Gly
225                 230                 235                 240

Asn Ile Leu Cys Phe Leu Gln Ser Tyr Trp Asn Pro Thr Ala Asn Tyr
                245                 250                 255

Val Thr Ser Asn Thr Gly Gly Gly Arg Ser Gly Lys Asp Ser Asn Ser
            260                 265                 270

Val Leu Thr Ser Ile His Thr Phe Asp Pro Asp Ala Gly Cys Asp Ala
        275                 280                 285

Thr Thr Phe Gln Pro Cys Ser Asp Lys Ala Leu Ser Asn Leu Lys Val
    290                 295                 300

Tyr Val Asp Ser Phe Arg Ser Ile Trp Ala Ile Asn Ala Gly Ala Ala
305                 310                 315                 320
```

Ala Thr Ala Pro Val Ala Val Gly Arg Tyr Pro Glu Asp Thr Tyr Tyr
325 330 335

Asn Gly Asn Pro Trp Tyr Leu Ser Thr Phe Ala Val Ala Glu Gln Leu
340 345 350

Tyr Asp Ala Ile Ile Val Trp Asn Lys Gln Gly Ser Ile Gln Val Thr
355 360 365

Ser Leu Ser Leu Pro Phe Phe Gln Gln Phe Ile Ser Thr Leu Lys Thr
370 375 380

Gly Thr Tyr Thr Gln Ser Ser Thr Gln Phe Gln Thr Leu Val Pro Ala
385 390 395 400

Ile Lys Ala Trp Ala Asp Gly Phe Val Ala Ile Asn Gln Lys Tyr Thr
405 410 415

Pro Pro Asn Gly Gly Leu Gly Glu Gln Tyr Asp Lys Val Ser Gly Asp
420 425 430

Pro Val Ser Ala Val Asp Leu Thr Trp Ser Tyr Ala Ser Ala Leu Thr
435 440 445

Val Phe Asn Ala Arg Ala Gly Asn Tyr Ser Pro Gly Trp Gly Ala Lys
450 455 460

Gly Leu Thr Val Pro Ala Val Cys Gln Pro Asn Ala Gly Pro Gln Val
465 470 475 480

Gln Val Thr Phe Lys Val Gln Ala Thr Thr Val Tyr Gly Glu Asn Ile
485 490 495

Tyr Leu Thr Gly Ala Asn Pro Ala Leu Ser Asn Trp Ser Pro Asp Thr
500 505 510

Ala Leu Ala Met Ser Ala Ala Asn Tyr Pro Thr Trp Ser Val Thr Val
515 520 525

Thr Leu Pro Ala Asn Ser Val Ile Gln Tyr Lys Tyr Ile Arg Lys Asn
530 535 540

Asn Gly Ala Val Thr Trp Glu Ser Asp Pro Asn Asn Gln Val Thr Ile
545 550 555 560

Pro Ala Ser Gly Thr Tyr Thr Val Asn Asp Asn Trp Arg

565 570

<210> 13
<211> 283
<212> DNA
<213> aspergillus nidulans

<400> 13

```
atgcctcaat cctgggaaga actggccgct gataagcgcg cccgcctcgc aaaaaccatc        60
cctgatgaat ggaaagtcca gacgctgcct gcggaagaca gcgttattga tttcccaaag       120
aaatcgggta tcctttcaga ggccgaactg aagatcacag aggcctccgc tgcagatctt       180
gtgtccaagc tggcggccgg agagttgacc tcggtggaag ttacgctagc attctgtaaa       240
cgggcagcaa tcgcccagca gttagtaggg tcccctctac ctc                         283
```

<210> 14
<211> 17
<212> DNA
<213> artificial sequence

<220>
<223> Primer M13 RV

<400> 14
caggaaacag ctatgac 17

<210> 15
<211> 17
<212> DNA
<213> artificial sequence

<220>
<223> Primer M13 M4

<400> 15
gttttcccag tcacgac 17

<210> 16
<211> 19
<212> DNA
<213> artificial sequence

<220>
<223> Primer In fusion vector R

<400> 16
tatgcgttat cgtacgcac 19

<210> 17
<211> 17
<212> DNA
<213> artificial sequence

<220>
<223> Primer M13 M4

<400> 17
gttttcccag tcacgac 17

<210> 18
<211> 27
<212> DNA
<213> artificial sequence

<220>
<223> Primer M49X F

<220>
<221> misc_feature
<222> (16)..(17)
<223> n is a, c, g, or t

<400> 18
gtcaacccgg actacnnkta cacatgg 27

<210> 19
<211> 18
<212> DNA
<213> artificial sequence

<220>
<223> Primer M49X R

<400> 19
gtagtccggg ttgacttg 18

<210> 20
<211> 37
<212> DNA
<213> artificial sequence

<220>
<223> Primer SOE-M4 F

<400> 20
gtactatctg gcattggtac gttttcccag tcacgac 37

<210> 21
<211> 37
<212> DNA
<213> artificial sequence

<220>
<223> Primer SOE-MR R

<400> 21
tggttatgat ttcggcgatg caggaaacag ctatgac 37

<210> 22
<211> 20
<212> DNA

<213> artificial sequence

<220>
<223> Primer SOE-F

<400> 22
gtactatctg gcattggtac 20

<210> 23
<211> 20
<212> DNA
<213> artificial sequence

<220>
<223> Primer SOE-R

<400> 23
tggttatgat ttcggcgatg 20

<210> 24
<211> 20
<212> DNA
<213> artificial sequence

<220>
<223> Primer insert rescue F

<400> 24
aatctcagaa caccaatatc 20

<210> 25
<211> 20
<212> DNA
<213> artificial sequence

<220>
<223> Primer insert rescue R

<400> 25
aacactatgc gttatcgtac 20

<210> 26
<211> 11101
<212> DNA
<213> artificial sequence

<220>
<223> Vector pDAu571

<400> 26

```
ttgaagttcc tattccgagt tcctattctc tagaaagtat aggaacttca gtacccgggt      60
ataagctagc ttccgttaaa ttgccgtcgt cagccgttaa attaccgatt aatcccgata     120
aatttccgag atctccgtta aattgccgtt cgcagccgtt aaattaccgg ggacgaccga     180
taaatttccg cgatgaattc atggtgtttt gatcatttta aatttttata tggcgggtgg     240
tgggcaactc gcttgcgcgg gcaactcgct taccgattac gttagggctg atatttacgt     300
```

aaaaatcgtc aagggatgca agaccaaacc gttaaatttc cggagtcaac agcatccaag 360
cccaagtcct tcacggagaa accccagcgt ccacatcacg agcgaaggac cacctctagg 420
catcggacgc accatccaat tagaagcagc aaagcgaaac agcccaagaa aaaggtcggc 480
ccgtcggcct tttctgcaac gctgatcacg ggcagcgatc caaccaacac cctccagagt 540
gactaggggc ggaaatttat cgggattaat ttccactcaa ccacaaatca cagtcgtccc 600
cggtaattta acggctgcag acggcaattt aacggcttct gcgaatcgct tggattcccc 660
gcccctggcc gtagagctta aagtatgtcc cttgtcgatg cgatgtatca caacatataa 720
atactggcaa gggatgccat gcttggagtt tccaactcaa tttacctcta tccacacttc 780
tcttccttcc tcaatcctct atatacacaa ctggggatcc accatgttct cggcaggcca 840
caagattaag ggtacagtcg tcctcatgcc taaaaacgag ttggaagtga accccgatgg 900
ctccgcagtc gataacctca acgcattcct cggacgttcg gtgtcgctcc agctcatctc 960
cgcgaccaaa gccgacgccc acggtaaggg aaaggtgggc aaggacacgt tcttggaagg 1020
tatcaacact tcgctcccta ccttgggagc aggagagtcc gcattcaaca ttcacttcga 1080
gtgggacggt tcgatgggca ttcccggagc gttctatatc aagaactata tgcaggtgga 1140
gttcttcttg aagtccttga ccttggaggc aatctcgaac cagggtacca tccgtttcgt 1200
gtgtaactcg tgggtctaca acaccaagct ctacaaatcc gtgcggatct tcttcgcgaa 1260
ccacacttac gtcccttcgg agacacctgc ccctttggtg tcgtaccgcg aggaggaatt 1320
gaagtccctc cgtggtaacg gtactggaga aaggaaggag tatgatagga tctacgacta 1380
cgacgtctat aacgatttgg gtaaccccga caaatcggaa aagttggcac gtcctgtgtt 1440
gggaggctcc tccaccttcc cctaccctcg acgcggccgc acgggacgcg gtcccactgt 1500
caccgatccg aacacagaga agcagggcga agtcttctac gtgcccaggg acgaaaacct 1560
cggccacttg aagtcgaagg atgcattgga gattggaacc aagtccctct cccagatcgt 1620
ccagcctgca ttcgaatcgg cgttcgattt gaaatcgacg cccatcgagt tccactcgtt 1680
ccaggacgtc catgacttgt atgaaggtgg tatcaaattg cctcgggacg tcatctccac 1740
cattatcccc ctccccgtga tcaaggaatt gtaccgcacc gacggccagc atattctcaa 1800
attcccccag ccgcacgtcg tccaggtctc gcagtccgca tggatgacag atgaggaatt 1860
cgcgagggaa atgattgcag gtgtcaaccc gtgtgtcatc cgaggcttgg aggagttccc 1920
tcctaagtcc aacctcgatc ctgccatcta tggagaccag tcctccaaga ttacagccga 1980
ttccctcgat ctcgacggtt atactatgga tgaagcactc ggttccaggc gattgttcat 2040
gctcgattat catgatatct tcatgcccta tgtgcgccag atcaaccagt tgaactcggc 2100
aaaaacatat gcaacgagga cgatcctctt cctccgagaa gacggcacac tcaagcctgt 2160
ggcaatcgag ctctcgctcc cccattccgc aggcgatctc tccgcagccg tgtcgcaggt 2220

ggtgttgcct gcaaaagaag gagtggagtc gaccatctgg ctcttggcca aagcatatgt 2280
gattgtgaac gattcctgtt atcaccagct catgtcgcat tggctcaaca ctcacgcggc 2340
aatggaaccc ttcgtgatcg ccacgcaccg gcacctctcg gtgctccacc cgatctacaa 2400
gctcctcact ccccactacc gtaacaacat gaacattaac gccttggcac ggcagtcgtt 2460
gatcaacgcg aacggcatca ttgagacaac gttcctcccc tccaagtact ccgtcgaaat 2520
gtcgtccgca gtctacaaaa actgggtctt caccgaccag gcgttgcctg ccgacttgat 2580
caaacgaggc gtcgcaatca aagatccctc cactcctcat ggcgtccgcc tcttgatcga 2640
ggactacccc tacgcagcgg acggattgga aatctgggca gccatcaaga cctgggtgca 2700
ggaatacgtc cctttgtact atgcgaggga cgatgatgtc aaaaacgact cggaactcca 2760
gcattggtgg aaggaggcag tggaaaaggg ccatggagat ctcaaggata aaccctggtg 2820
gcctaagctc cagaccttgg aggacctcgt cgaagtgtgt ttgatcatta tctggatcgc 2880
atccgcgttg catgcagccg tgaacttcgg acagtatccc tatggaggcc tcatcatgaa 2940
ccgtcccacc gcatccagga ggctcctccc cgaaaaagga acacccgaat acgaagaaat 3000
gatcaacaac cacgaaaagg catacctccg gaccatcact tccaaactcc cgaccttgat 3060
ctcgctctcc gtgatcgaga ttttgtcgac acatgcgtcg gacgaggtct atttgggtca 3120
gcgggataac ccgcactgga catccgattc caaggccctc caggcgttcc agaagttcgg 3180
caacaagctc aaggagatcg aggagaaact cgtgaggcgg aacaacgacc cttccctcca 3240
gggaaaccgg ttgggacctg tccagctccc gtatacgttg ctctacccct cctcggaaga 3300
aggcctcact ttcaggggta tccccaactc gatttccatc tgactcgaga tctagagggt 3360
gactgacacc tggcggtaga caatcaatcc atttcgctat agttaaagga tggggatgag 3420
ggcaattggt tatatgatca tgtatgtagt gggtgtgcat aatagtagtg aaatggaagc 3480
caagtcatgt gattgtaatc gaccgacgga attgaggata tccggaaata cagacaccgt 3540
gaaagccatg gtctttcctt cgtgtagaag accagacaga cagtccctga tttacccttg 3600
cacaaagcac tagaaaatta gcattccatc cttctctgct tgctctgctg atatcactgt 3660
cattcaatgc atagccatga gctcatctta gatccaagca cgtaattcca tagccgaggt 3720
ccacagtgga gcagcaacat tccccatcat tgctttcccc aggggcctcc caacgactaa 3780
atcaagagta tatctctacc gtccaataga tcgtcttcgc ttcaaaatct ttgacaattc 3840
caagagggtc cccatccatc aaacccagtt caataatagc cgagatgcat ggtggagtca 3900
attaggcagt attgctggaa tgtcggggcc agttggccgg gtggtcattg gccgcctgtg 3960
atgccatctg ccactaaatc cgatcattga tccaccgccc acgaggcgcg tctttgcttt 4020
ttgcgcggcg tccaggttca actctctctt aattaaatag cgacaagccg aacggcaccg 4080

gcaggtacaa tggttcgctg tacttgcttg cgcaagcggg tctttgggga ttgagcgcat 4140
ttggtgttgc aaaggatttg atgtaaatgt agtcgacatc ttagcacaga ggggagagtt 4200
gataaaatgt ggtctgtttg aatgatagtc gggttcgtga cctatattcg tgatagtgga 4260
gataggtctg cgcctatctt atcgggccgg agcaaaaatt ccaccgcagc ggggtgagtt 4320
ttcgttatac agccatccca cttccagctt caaattgtca gtttaatcca gcccaattca 4380
atcattggag aaccggtttt atgtcttcga agtcccacct cccctacgca attcgcgcaa 4440
ccaaccatcc caaccctta acatctaaac tcttctccat cgccgaggag aagaaaacca 4500
acgtcaccgt ctccgcagac gttactactt ccgccgagct cctcgatctt gctgaccgcc 4560
taggccccta tatcgcagtt ctgaaaaccc acatcgacat cctcaccgat ctcaccccgt 4620
cgaccctttc ctcgctccaa tccctcgcga caaagcacaa cttcctcatc tttgaggacc 4680
gcaagttcat cgacatcggc aacaccgtgc aaaagcagta ccacggtggc gctctccgca 4740
tctccgaatg ggcacacatc atcaactgcg ccatcctgcc gggcgaaggg atcgtcgagg 4800
ccctcgcaca gacaaccaag tctcctgact ttaaagacgc gaatcaacga ggtctcctga 4860
ttcttgccga gatgacgagt aagggatctc ttgcgacagg ggagtacacg gcacgctcgg 4920
ttgagtacgc gcggaagtat aaggggtttg tgatgggatt cgtgagtaca agggcgttga 4980
gtgaggtgct gcccgaacag aaagaggaga gcgaggattt tgtcgtcttt acgactgggg 5040
tgaatctgtc ggataagggg gataagctgg ggcagcagta tcagacacct gggtcggcgg 5100
ttgggcgagg tgcggacttt atcattgcgg gtaggggcat ctataaggcg gacgatccag 5160
tcgaggcggt tcagaggtac cgggaggaag gctggaaagc ttacgagaaa agagttggac 5220
tttgagggtg actgacacct ggcggtagac aatcaatcca tttcgctata gttaaaggat 5280
ggggatgagg gcaattggtt atatgatcat gtatgtagtg ggtgtgcata atagtagtga 5340
aatggaagcc aagtcatgtg attgtaatcg accgacggaa ttgaggatat ccggaaatac 5400
agacaccgtg aaagccatgg tctttccttc gtgtagaaga ccagacagac agtccctgat 5460
ttacccttgc acaaagcact agaaaattag cattccatcc ttctctgctt gctctgctga 5520
tatcactgtc attcaatgca tagccatgag ctcatcttag atccaagcac gtaattccat 5580
agccgaggtc cacagtggag cagcaacatt ccccatcatt gctttcccca ggggcctccc 5640
aacgactaaa tcaagagtat atctctaccg tccaatagat cgtcttcgct tcaaaatctt 5700
tgacaattcc aagagggtcc ccatccatca aacccagttc aataatagcc gagatgcatg 5760
gtggagtcaa ttaggcagta ttgctggaat gtcggggcca gttggccggg tggtcattgg 5820
ccgcctgtga tgccatctgc cactaaatcc gatcattgat ccaccgccca cgaggcgcgt 5880
ctttgctttt tgcgcggcgt ccaggttcaa ctctctcctc taggttgaag ttcctattcc 5940
gagttcctat tcttcaaata gtataggaac ttcaactagc tagtgcatgc gtacgatttt 6000

```
gacatttgct ccattgtcga ggatggatgg aacgagcggc gtgcgccacg aaagtgaggc      6060
tattgcctat cagctctttg ctacattccg gaaacaaaca tcccttttg tgaattatct       6120
acgcaactta gatggcgtga acgcatcttc aaagtctttc ggcaggtccg gcacgacttt      6180
tgcatccaga gaagcgccta catgtgtatt cgaccacctc ctagcgcgct tggatatgag      6240
gaaatattac tgagagtcga aaacaagctc caccgcacca gctcttcttg gagttttata      6300
ttaaagaata ttcccagctc gttgtattat tctttttcta ccgtgctaat gtatcaagga      6360
ctttggtacc tattaacgtt attattcgtg tgctattccc aaacataacc ctgtatatgt      6420
ttcgaacgcc gttatgaccc atgtcttaca tactcattaa gtcattccct tggataatct      6480
cgactcagat gcggcggttg atgtaggagg agaggtaatc gaggacctcc tgggagatga      6540
tgccgttcca ggcggggtag cggatggagc cctcggcgga gcccttgagc tgctcgatat      6600
gctgccactc ctcgatgggg ttggtctcat ccttgagggc gatcatctcc ttggagatgg      6660
gatcgtaggc gtagtagcgg gagactagtg cgaagtaatg atcggggatg gcggtgatct      6720
gatgggtgta ggtggtgcgg gcgacggcgg aggcgcgctt atcggaccag ttgccgacga      6780
cgttggtgag ctcggtgagg cccttcatgg agaggaagga ggtcatgaga tggcggccga      6840
tatgggactt ggggccgttc ttgatggcga agatggagta gggggcgttc ttcttgaggg      6900
ccttgttgta ggagcggacg aggttatcct tgaggagctg gtactcctgc ttgttggagg      6960
aggagttgcc ggtgcggttg acgcgcttga ggacgggctc ggagttgcgg aggaactcat      7020
cgaggtagac gaggggatcg atgcggccgc gggcggagaa gaagtagata tggcgggaga      7080
cggaggtctt ggtctcggtg acgaggcact ggatgatgac gccgaggtac ttgttctgga      7140
cgagcttgaa ggacttggga tcgacgttct tgatatcgga gaagcggccg cagttgatga      7200
aggtggcgag gaagaggaac tggtagaggg tcttggtctt ggtgaagcgg gaggtgtact      7260
cgaaggagtt gaggatcttc tcggtgatct cccagatgga ctcgccctcg gagaggaggg      7320
ccttgagcat cttcttggaa tgggagttgc ccttatcggc ctcctcggag gactcgaact      7380
ggagctggag ggaggagacg atatcggtga tatcggactg atgcttctgg ccgtagtagg      7440
ggatgatggt gaactcccag gcggggatga gcttcttgag ggaggcctcc aggatggtgg      7500
ccttctgggt cttgtacttg aactggaggg acttgttgac gatatcgaag gagagggagt      7560
tggagatgat ggtgttgtag gacatgaagg tggcgcgctt gatggcggtg ccgttatggg      7620
tgatcatcca gcagaggtag gtgagctcgg cggcgcagag ggcgatcttc tcgccggagg      7680
ggcgctcgaa gcgctcgacg aactggcgga cgaggacctt gggggggtc ttgcagagga       7740
tatcgaactg gggcatggtg ctcagatact acggctgatc gcgtagaggt actgagcaaa      7800
acagatgtca gtaaggagaa gagttgaatg aatggaagaa gagtaggaaa ggaggtatgg      7860
```

gggaaagata tacgtactga tgcggacgaa gagagaaaga aggaaaaaag ttgtgggagg 7920

ggaaggaggg ggaatcctta tatggagggg caagcgagaa ggcgaattag tgggcgggct 7980

taagccctcg accgccgccc ttatcattgg acatggaggg gtaatgcccc caccacgcat 8040

gtgcgggacc gacgcagaat ctgcacggcg gagtctcttc cagactgttg acttttgggc 8100

gatgactctt gttgctgcgg ccttttgggt acaccaacct cgttgatctt gtttccttgg 8160

ttctctttcg ctcggagacc cgaccatgac cccaccatca gtcactatcc tgcctcgtcg 8220

ataaaaattt tttcttccct ctgattgtta catagtatgt ttccaccttt ccggtggatt 8280

tcggacagtc aaactgggca tcaacgcagt ggtgggctgc ttcgtttgct gcgtgttgta 8340

cttgtttgca tttgaacccc gcggtcgttc gagtccttaa ttggtccgct cccggtcaac 8400

acccaagcag ctgtggcccg gccgagtggc gcctgtctgg tccacagtaa gcttggcgta 8460

atcatggtca tagctgtttc ctgtgtgaaa ttgttatccg ctcacaattc cacacaacat 8520

acgagccgga agcataaagt gtaaagcctg gggtgcctaa tgagtgagct aactcacatt 8580

aattgcgttg cgctcactgc ccgctttcca gtcgggaaac ctgtcgtgcc agctgcatta 8640

atgaatcggc caacgcgcgg ggagaggcgg tttgcgtatt gggcgctctt ccgcttcctc 8700

gctcactgac tcgctgcgct cggtcgttcg gctgcggcga gcggtatcag ctcactcaaa 8760

ggcggtaata cggttatcca cagaatcagg ggataacgca ggaaagaaca tgtgagcaaa 8820

aggccagcaa aaggccagga accgtaaaaa ggccgcgttg ctggcgtttt tccataggct 8880

ccgcccccct gacgagcatc acaaaaatcg acgctcaagt cagaggtggc gaaacccgac 8940

aggactataa agataccagg cgtttccccc tggaagctcc ctcgtgcgct ctcctgttcc 9000

gaccctgccg cttaccggat acctgtccgc ctttctccct tcgggaagcg tggcgctttc 9060

tcatagctca cgctgtaggt atctcagttc ggtgtaggtc gttcgctcca agctgggctg 9120

tgtgcacgaa ccccccgttc agcccgaccg ctgcgcctta tccggtaact atcgtcttga 9180

gtccaacccg gtaagacacg acttatcgcc actggcagca gccactggta acaggattag 9240

cagagcgagg tatgtaggcg gtgctacaga gttcttgaag tggtggccta actacggcta 9300

cactagaaga acagtatttg gtatctgcgc tctgctgaag ccagttacct tcggaaaaag 9360

agttggtagc tcttgatccg gcaaacaaac caccgctggt agcggtggtt tttttgtttg 9420

caagcagcag attacgcgca gaaaaaaagg atctcaagaa gatcctttga tcttttctac 9480

ggggtctgac gctcagtgga acgaaaactc acgttaaggg attttggtca tgagattatc 9540

aaaaaggatc ttcacctaga tccttttaaa ttaaaaatga agttttaaat caatctaaag 9600

tatatatgag taaacttggt ctgacagtta ccaatgctta atcagtgagg cacctatctc 9660

agcgatctgt ctatttcgtt catccatagt tgcctgactc cccgtcgtgt agataactac 9720

gatacgggag ggcttaccat ctggccccag tgctgcaatg ataccgcgag acccacgctc 9780

accggctcca gatttatcag caataaacca gccagccgga agggccgagc gcagaagtgg 9840

tcctgcaact ttatccgcct ccatccagtc tattaattgt tgccgggaag ctagagtaag 9900

tagttcgcca gttaatagtt tgcgcaacgt tgttgccatt gctacaggca tcgtggtgtc 9960

acgctcgtcg tttggtatgg cttcattcag ctccggttcc caacgatcaa ggcgagttac 10020

atgatccccc atgttgtgca aaaaagcggt tagctccttc ggtcctccga tcgttgtcag 10080

aagtaagttg gccgcagtgt tatcactcat ggttatggca gcactgcata attctcttac 10140

tgtcatgcca tccgtaagat gcttttctgt gactggtgag tactcaacca agtcattctg 10200

agaatagtgt atgcggcgac cgagttgctc ttgcccggcg tcaatacggg ataataccgc 10260

gccacatagc agaactttaa aagtgctcat cattggaaaa cgttcttcgg ggcgaaaact 10320

ctcaaggatc ttaccgctgt tgagatccag ttcgatgtaa cccactcgtg cacccaactg 10380

atcttcagca tcttttactt tcaccagcgt ttctgggtga gcaaaaacag gaaggcaaaa 10440

tgccgcaaaa aagggaataa gggcgacacg gaaatgttga atactcatac tcttcctttt 10500

tcaatattat tgaagcattt atcagggtta ttgtctcatg agcggataca tatttgaatg 10560

tatttagaaa aataaacaaa taggggttcc gcgcacattt ccccgaaaag tgccacctga 10620

cgtctaagaa accattatta tcatgacatt aacctataaa aataggcgta tcacgaggcc 10680

ctttcgtctc gcgcgtttcg gtgatgacgg tgaaaacctc tgacacatgc agctcccgga 10740

gacggtcaca gcttgtctgt aagcggatgc cgggagcaga caagcccgtc agggcgcgtc 10800

agcgggtgtt ggcgggtgtc ggggctggct taactatgcg gcatcagagc agattgtact 10860

gagagtgcac catatgcggt gtgaaatacc gcacagatgc gtaaggagaa aataccgcat 10920

caggcgccat tcgccattca ggctgcgcaa ctgttgggaa gggcgatcgg tgcgggcctc 10980

ttcgctatta cgccagctgg cgaaaggggg atgtgctgca aggcgattaa gttgggtaac 11040

gccagggttt tcccagtcac gacgttgtaa aacgacggcc agtgaattcg agctcggtac 11100

c 11101

<210> 27
<211> 49
<212> DNA
<213> artificial sequence

<220>
<223> FRT-F

<400> 27
ttgaagttcc tattccgagt tcctattctc tagaaagtat aggaacttc 49

<210> 28
<211> 50
<212> DNA

<213> artificial sequence

<220>
<223> FRT-F3

<400> 28
ttgaagttcc tattccgagt tcctattctt caaatagtat aggaacttca 50

<210> 29
<211> 8299
<212> DNA
<213> artificial sequence

<220>
<223> Plasmid pDAu703

<400> 29

```
taggcgtatc acgaggccct ttcgtctcgc gcgtttcggt gatgacggtg aaaacctctg      60
acacatgcag ctcccggaga cggtcacagc ttgtctgtaa gcggatgccg ggagcagaca     120
agcccgtcag ggcgcgtcag cgggtgttgg cgggtgtcgg ggctggctta actatgcggc     180
atcagagcag attgtactga gagtgcacca tatgcggtgt gaaataccgc acagatgcgt     240
aaggagaaaa taccgcatca ggcgccattc gccattcagg ctgcgcaact gttgggaagg     300
gcgatcggtg cgggcctctt cgctattacg ccagctggcg aaagggggat gtgctgcaag     360
gcgattaagt tgggtaacgc cagggttttc ccagtcacga cgttgtaaaa cgacggccag     420
tgaattggcc tccatggccg cggccgcgct ttgctaaaac tttggttgat ggaaggtatc     480
tggcgataaa ctccgacgac gtctagaagc aacaatctta tgcaaacgct cattggttct     540
tttcgaccgc aacatccatc atgaaactgg tattttgtct gtgtcagcag tctagaaccc     600
cttgccgggt attttagcat ttcatttttc tataaaaagg taccagcatg tatggatcgt     660
atcttccgta ccgtggttat taaatcccag cagaggccga taggcttaag aagtgaacat     720
ggcatggtta aggaagaagc cattactgag tatatatggc tagaataatc gctgggaaag     780
atttatgctt ccaagaggcg taggacggta taccatacag tacggtattt atgaacaatt     840
cgataatacc actccccaaa gcgggagata ggacacccgc ctcaggcacc aaccaccccc     900
tttttcaact gtcagtggtg cacgtttcca tcgagcataa gcttggtacc ctaaggatag     960
gccctaatct tatctacatg tgactgcatc gatgtgtttg gtcaaaatga ggcatgtggc    1020
tcaccccaca ggcggagaaa cgtgtggcta gtgcatgaca gtcccctcca tagattcaat    1080
ttaatttttc gcggcaattg tcgtgcagtt tgtatctaca tttcattcca tatatcaaga    1140
gttagtagtt ggacatcctg attattttgt ctaattactg aaaactcgaa gtactaacct    1200
actaataagc cagtttcaac cactaagtgc tcatttatac aatatttgca gaaccccgcg    1260
ctacccctcc atcgccaaca tgtcttccaa gtcgcaattg acctacagcg cacgcgctag    1320
caagcacccc aatgcgctcg taaagaagct cttcgaggtt gccgaggcca agaaaaccaa    1380
```

tgtcaccgtt tccgccgacg tgacaaccac caaagagctg ctggatttgg ctgaccgtat 1440
gcgcaccggg gttgaagttc ctattccgag ttcctattct tcaaatagta taggaacttc 1500
attaattaaa ggagagagtt gaacctggac gccgcgcaaa aagcaaagac gcgcctcgtg 1560
ggcggtggat caatgatcgg atttagtggc agatggcatc acaggcggcc aatgaccacc 1620
gggccaactg gccccgacat tccagcaata ctgcctaatt gactccacca tgcatctcgg 1680
ctattattga actgggtttg atggatgggg accctcttgg aattgtcaaa gattttgaag 1740
cgaagacgat ctattggacg gtagagatat actcttgatt tagtcgttgg gaggcccctg 1800
gggaaagcaa tgatggggaa tgttgctgct ccactgtgga cctcggctat ggaattacgt 1860
gcttggatct aagatgagct catggctatg cattgaatga cagtgatatc agcagagcaa 1920
gcagagaagg atggaatgct aattttctag tgctttgtgc aagggtaaat cagggactgt 1980
ctgtctggtc ttctacacga aggaaagacc atggctttca cggtgtctgt atttccggat 2040
atcctcaatt ccgtcggtcg attacaatca catgacttgg cttccatttc actactatta 2100
tgcacaccca ctacatacat gatcatataa ccaattgccc tcatccccat cctttaacta 2160
tagcgaaatg gattgattgt ctaccgccag gtgtcagtca ccctctagat ctcgagctcg 2220
ctagagtcga cctatggagt caccacattt cccagcaact tccccacttc ctctgcaatc 2280
gccaacgtcc tctcttcact gagtctccgt ccgataacct gcactgcaac cggtgcccca 2340
tggtacgcct ccggatcata ctcttcctgc acgagggcat caagctcact aaccgccttg 2400
aaactctcat tcttcttatc gatgttctta tccgcaaagg taaccggaac aaccacgctc 2460
gtgaaatcca gcaggttgat cacagaggca tacccatagt accggaactg gtcatgccgt 2520
accgcagcgg taggcgtaat cggcgcgatg atggcgtcca gttccttccc ggccttttct 2580
tcagcctccc gccatttctc aaggtactcc atctggtaat tccacttctg gagatgcgtg 2640
tcccagagct cgttcatgtt aacagctttg atgttcgggt tcagtaggtc tttgatattt 2700
ggaatcgccg gctcgccgga tgcactgata tcgcgcatta cgtcggcgct gccgtcagcc 2760
gcgtagatat gggagatgag atcgtggccg aaatcgtgct tgtatggcgt ccacggggtc 2820
acggtgtgac cggctttggc gagtgcggcg acggtggttt ccacgccgcg caggatagga 2880
gggtgtggaa ggacattgcc gtcgaagttg tagtagccga tattgagccc gccgttcttg 2940
atcttggagg caataatgtc cgactcggac tggcgccagg gcatggggat gaccttggag 3000
tcgtatttcc atggctcctg accgaggacg gatttggtga agaggcggag gtctaacata 3060
cttcatcagt gactgccggt ctcgtatata gtataaaaag caagaaagga ggacagtgga 3120
ggcctggtat agagcaggaa aagaaggaag aggcgaagga ctcaccctca acagagtgcg 3180
taatcggccc gacaacgctg tgcaccgtct cctgaccctc catgctgttc gccatctttg 3240

catacggcag ccgcccatga ctcggcctta gaccgtacag gaagttgaac gcggccggca 3300
ctcgaatcga gccaccgata tccgttccta caccgatgac gccaccacga atcccaacga 3360
tcgcaccctc accaccagaa ctgccgccgc acgaccagtt cttgttgcgt gggttgacgg 3420
tgcgcccgat gatgttgttg actgtctcgc agaccatcag ggtctgcggg acagaggtct 3480
tgacgtagaa gacggcaccg gctttgcgga gcatggttgt cagaaccgag tccccttcgt 3540
cgtacttgtt tagccatgag atgtagccca ttgatgtttc gtagccctgg tggcatatgt 3600
tagctgacaa aaagggacat ctaacgactt aggggcaacg gtgtaccttg actcgaagct 3660
ggtctttgag agagatgggg aggccatgga gtggaccaac gggtctcttg tgctttgcgt 3720
agtattcatc gagttccctt gcctgcgcga gagcggcgtc agggaagaac tcgtgggcgc 3780
agtttgtctg cacagaagcc agcgtcagct tgatagtccc ataaggtggc gttgttacat 3840
ctccctgaga ggtagagggg accctactaa ctgctgggcg attgctgccc gtttacagaa 3900
tgctagcgta acttccaccg aggtcaactc tccggccgcc agcttggaca caagatctgc 3960
agcggaggcc tctgtgatct tcagttcggc ctctgaaagg atcaccgatt tctttgggaa 4020
atcaataacg ctgtcttccg caggcagcgt ctggactttc cattcatcag ggatggtttt 4080
tgcgaggcgg gcgcgcttat cagcggccag ttcttcccag gattgaggca tgtgcatgca 4140
atgtgtgttt atgtggaagt aagatacgac gagtttgatt gagaaaagac agggtgattg 4200
tcaagttcag tatggaagaa agagtagaag aagatcagac gacagggaag agcgatgaca 4260
taaaaggtgg aagacggaag aaaaacgaac caaatcaatc ccactctatg gcgggggttg 4320
gactgcctga ggccggcact ggtggggctt atcgataagt tctcgtcacc ggatgcaatg 4380
cgctgtcaac tgctgacttg gccctgaaca tcctgtcctc tacagatcca tactatacaa 4440
tgatcccagt tatagtgcgg taaggtgcat atcatatctc attctcatga ctcattcgac 4500
tttttttttag agaaagtaca tacgtggaac atacactaaa cgcaacaggt cgcgacaaca 4560
ctggtataca aaacggtccc cggtgaatga cgttattagt gtctatcccc cactcacacc 4620
cgaaaagaat aatagaaact aacagaaaaa gcggcccgag gataagagga acattcaaac 4680
agaaggggaa tcataaaaac cgaaaaatgc aaggaaaaga gaactcaaat caataatttt 4740
cataatactg tcgagagtaa tacggaccag cgtctctcag ggacatgcgt cggcgcaagg 4800
catcatccaa tctctcatct aacacatcca gcattcgtgt tcgatagtct aactgcttct 4860
ctcggcgctc aagtcttgct tcccgatcat cgagttaatt aagaagttcc tatactttct 4920
agagaatagg aactcggaat aggaacttca aggtaccgag ctctatcctc aataccctat 4980
tttccacgat tccattgtca tatccaattc cgttttcttt tcttgttttc ccctcatcca 5040
atcccgtcca tcatttactc ctttttcttg tgaatgcaag tggcactaag aaatccaacc 5100
cccagacaaa ttttcctact caggaacaca aaaacctcgt ttctgctccc ttctcgtact 5160

tcattcctat cgtctcggaa tttcctcaac aaccctttcc gactttgcga cagcgtcgcg 5220
attccagact tatgtgttct cgttcctact gtcgttacca gtctatttat tccgaaacct 5280
ctgatcgctg aatttcacac acaacacccc cccgttgatg ctggtggaga atccgtagcg 5340
tcaagagttg aattcactcc atgttgtaac gaagtccacg aattgagacg attgatgatt 5400
acaaccccgc gatcgcctat cgacgattcg acgagatgcc attctcatcc tcctcatcct 5460
cctccacccc cgaggtgtct accaccccgc tcgcagatta cttctggatc gcaggtgtcg 5520
atggcgcgga aatcttagag actttccaaa gactcggcga cgaatacagg gcaaacagtg 5580
ccaccgctcc tggccccgct cttgcggaca cgatcgagga agatgcggac gcggaggagg 5640
cacacgaccc ccgtctggac tccctctctc gacccaattc catggctggg ggccgcaatt 5700
ccttccagcg gttctcaatg cgctcaggag actccagtga gtccagtggg aatggtacca 5760
gcagcaaccg gagcagtctg accatcaagg gtaatcagtc gcccagaggg tcgtcgtttc 5820
tagaagattt cgactttgac aaggccctgt tcaagtttgc aaacgagcgg gagtcgttcc 5880
tgtcggatct gagtctcagt gccggagcaa tcactcccac ctcccgtcct aggtccaggt 5940
tacgtacaca gaagattgtc tccgaggaaa gtccctccca gccatccagc ttgcttcgat 6000
caggcattgg tagtgtgcgg cgtcatatgg cattcagaga catgaatagt atgaaacggc 6060
agccgtcagt tgctcgtcgc ggccgcagct tggcgtaatc atggtcatag ctgtttcctg 6120
tgtgaaattg ttatccgctc acaattccac acaacatacg agccggaagc ataaagtgta 6180
aagcctgggg tgcctaatga gtgagctaac tcacattaat tgcgttgcgc tcactgcccg 6240
ctttccagtc gggaaacctg tcgtgccagc tgcattaatg aatcggccaa cgcgcgggga 6300
gaggcggttt gcgtattggg cgctcttccg cttcctcgct cactgactcg ctgcgctcgg 6360
tcgttcggct gcggcgagcg gtatcagctc actcaaaggc ggtaatacgg ttatccacag 6420
aatcagggga taacgcagga aagaacatgt gagcaaaagg ccagcaaaag gccaggaacc 6480
gtaaaaaggc cgcgttgctg gcgtttttcc ataggctccg cccccctgac gagcatcaca 6540
aaaatcgacg ctcaagtcag aggtggcgaa acccgacagg actataaaga taccaggcgt 6600
ttccccctgg aagctccctc gtgcgctctc ctgttccgac cctgccgctt accggatacc 6660
tgtccgcctt tctcccttcg ggaagcgtgg cgctttctca tagctcacgc tgtaggtatc 6720
tcagttcggt gtaggtcgtt cgctccaagc tgggctgtgt gcacgaaccc cccgttcagc 6780
ccgaccgctg cgccttatcc ggtaactatc gtcttgagtc caacccggta agacacgact 6840
tatcgccact ggcagcagcc actggtaaca ggattagcag agcgaggtat gtaggcggtg 6900
ctacagagtt cttgaagtgg tggcctaact acggctacac tagaagaaca gtatttggta 6960
tctgcgctct gctgaagcca gttaccttcg gaaaaagagt tggtagctct tgatccggca 7020

aacaaaccac cgctggtagc ggtggttttt ttgtttgcaa gcagcagatt acgcgcagaa 7080
aaaaaggatc tcaagaagat cctttgatct tttctacggg gtctgacgct cagtggaacg 7140
aaaactcacg ttaagggatt ttggtcatga gattatcaaa aaggatcttc acctagatcc 7200
ttttaaatta aaaatgaagt tttaaatcaa tctaaagtat atatgagtaa acttggtctg 7260
acagttacca atgcttaatc agtgaggcac ctatctcagc gatctgtcta tttcgttcat 7320
ccatagttgc ctgactcccc gtcgtgtaga taactacgat acgggagggc ttaccatctg 7380
gccccagtgc tgcaatgata ccgcgagacc cacgctcacc ggctccagat ttatcagcaa 7440
taaaccagcc agccggaagg gccgagcgca gaagtggtcc tgcaacttta tccgcctcca 7500
tccagtctat taattgttgc cgggaagcta gagtaagtag ttcgccagtt aatagtttgc 7560
gcaacgttgt tgccattgct acaggcatcg tggtgtcacg ctcgtcgttt ggtatggctt 7620
cattcagctc cggttcccaa cgatcaaggc gagttacatg atcccccatg ttgtgcaaaa 7680
aagcggttag ctccttcggt cctccgatcg ttgtcagaag taagttggcc gcagtgttat 7740
cactcatggt tatggcagca ctgcataatt ctcttactgt catgccatcc gtaagatgct 7800
tttctgtgac tggtgagtac tcaaccaagt cattctgaga atagtgtatg cggcgaccga 7860
gttgctcttg cccggcgtca atacgggata ataccgcgcc acatagcaga actttaaaag 7920
tgctcatcat tggaaaacgt tcttcggggc gaaaactctc aaggatctta ccgctgttga 7980
gatccagttc gatgtaaccc actcgtgcac ccaactgatc ttcagcatct tttactttca 8040
ccagcgtttc tgggtgagca aaaacaggaa ggcaaaatgc cgcaaaaaag ggaataaggg 8100
cgacacggaa atgttgaata ctcatactct tcctttttca atattattga agcatttatc 8160
agggttattg tctcatgagc ggatacatat ttgaatgtat ttagaaaaat aaacaaatag 8220
gggttccgcg cacatttccc cgaaaagtgc cacctgacgt ctaagaaacc attattatca 8280
tgacattaac ctataaaaa 8299

<210> 30
<211> 8614
<212> DNA
<213> artificial sequence

<220>
<223> Plasmid pDAU724

<400> 30

gaattcgagc tcggtacctt gaagttccta ttccgagttc ctattctcta gaaagtatag 60

gaacttcagt acccgggtat aagctagctt ccgttaaatt gccgtcgtca gccgttaaat 120

taccgattaa tcccgataaa tttccgagat ctccgttaaa ttgccgttcg cagccgttaa 180

attaccgggg acgaccgata aatttccgcg atgaattcat ggtgttttga tcattttaaa 240

tttttatatg gcgggtggtg ggcaactcgc ttgcgcgggc aactcgctta ccgattacgt 300

tagggctgat atttacgtaa aaatcgtcaa gggatgcaag accaaaccgt taaatttccg 360
gagtcaacag catccaagcc caagtccttc acggagaaac cccagcgtcc acatcacgag 420
cgaaggacca cctctaggca tcggacgcac catccaatta gaagcagcaa agcgaaacag 480
cccaagaaaa aggtcggccc gtcggccttt tctgcaacgc tgatcacggg cagcgatcca 540
accaacaccc tccagagtga ctaggggcgg aaatttatcg ggattaattt ccactcaacc 600
acaaatcaca gtcgtccccg gtaatttaac ggctgcagac ggcaatttaa cggcttctgc 660
gaatcgcttg gattccccgc ccctggccgt agagcttaaa gtatgtccct tgtcgatgcg 720
atgtatcaca acatataaat actggcaagg gatgccatgc ttggagtttc caactcaatt 780
tacctctatc cacacttctc ttccttcctc aatcctctat atacacaact ggggatccac 840
catgaggagc tcccttgtgc tgttctttgt ctctgcgtgg acggccttgg ccagtcctat 900
tcgtcgagag gtctcgcagg atctgtttaa ccagttcaat ctctttgcac agtattctgc 960
agccgcatac tgcggaaaaa acaatgatgc cccagctggt acaaacatta cgtgcacggg 1020
aaatgcctgc cccgaggtag agaaggcgga tgcaacgttt ctctactcgt ttgaagactc 1080
tggagtgggc gatgtcaccg gcttccttgc tctcgacaac acgaacaaat tgatcgtcct 1140
ctctttccgt ggctctcgtt ccatagagaa ctggatcggg aatcttaact tcgacttgaa 1200
agaaataaat gacatttgct ccggctgcag ggacatgac ggcttcactt cgtcctggag 1260
gtctgtagcc gatacgttaa ggcagaaggt ggaggatgct gtgagggagc atcccgacta 1320
tcgcgtggtg tttaccggac atagcttggg tggtgcattg gcaactgttg ccggagcaga 1380
cctgcgtgga aatgggtatg atatcgacgt gttttcatat ggcgcccccc gagtcggaaa 1440
cagggctttt gcagaattcc tgaccgtaca gaccggcgga acactctacc gcattaccca 1500
caccaatgat attgtcccta gactcccgcc gcgcgaattc ggttacagcc attctagccc 1560
agagtactgg atcaaatctg gaacccttgt ccccgtcacc cgaaacgata tcgtgaagat 1620
agaaggcatc gatgccaccg gcggcaataa ccagcctaac attccggata tccctgcgca 1680
cctatggtac ttcgggttaa ttgggacatg tctttagtgg ccggcgcggc tgggtcgact 1740
ctagcgagct cgagatctag agggtgactg acacctggcg gtagacaatc aatccatttc 1800
gctatagtta aaggatgggg atgagggcaa ttggttatat gatcatgtat gtagtgggtg 1860
tgcataatag tagtgaaatg gaagccaagt catgtgattg taatcgaccg acggaattga 1920
ggatatccgg aaatacagac accgtgaaag ccatggtctt tccttcgtgt agaagaccag 1980
acagacagtc cctgatttac ccttgcacaa agcactagaa aattagcatt ccatccttct 2040
ctgcttgctc tgctgatatc actgtcattc aatgcatagc catgagctca tcttagatcc 2100
aagcacgtaa ttccatagcc gaggtccaca gtggagcagc aacattcccc atcattgctt 2160

tccccagggg cctcccaacg actaaatcaa gagtatatct ctaccgtcca atagatcgtc 2220
ttcgcttcaa aatctttgac aattccaaga gggtccccat ccatcaaacc cagttcaata 2280
atagccgaga tgcatggtgg agtcaattag gcagtattgc tggaatgtcg gggccagttg 2340
gccgggtggt cattggccgc ctgtgatgcc atctgccact aaatccgatc attgatccac 2400
cgcccacgag gcgcgtcttt gctttttgcg cggcgtccag gttcaactct ctcttaatta 2460
atgtacatta gtgatacccc actctaagaa aatagaccaa tctccagctg caccttcaga 2520
cactccggta caaattctcg tctatgttgg agattgttgt gactttgaaa catgaccctt 2580
gaccctgatt ttgaatttgt ccatatatcg aggcaggtgt cttattcgta cggagagggt 2640
atctgtcgta gacacatagt agtagtcatt tcgagtgctg aatttataaa tcgcatcata 2700
cttgcgacat actgccataa aaggagtacg tatccaccac tacttattgc gcaccaacac 2760
gcttcaggta tgcatcccat ccctccttct ggtactgctt cgccgcctcc acgggatcag 2820
gagcagcata aattccacgg ccagcaataa taaagtcggc accgcgtcca acagccgact 2880
caggagtttg gtactgctgt cccagcttgt caccettcga ggagaggttg acacctgtcg 2940
tgaagacgac aaaatcttcc tcctccgaag gcgagctaac ttcagactga acctcgccaa 3000
ggtgacgtgt cgagacgaat cccatcacaa acttcttata cttccgagca tagtcaacag 3060
aagaagtagt atattgaccg gtagccaaag atcccttgga ggtcatctcc gcaaggatca 3120
aaaggcccct ctcggagccg taggggaagt cctcggccga agcagtctgg gccagagcct 3180
cgacgatacc ctcaccgggc agaatactgc agttgatgat gtgggcccac tcagagatac 3240
gcagagtgcc gccatggtac tgcttttgga ctgtgtttcc gatatcgatg aacttgcgat 3300
cttcgaagat gaggaaattg tgcttctctg caagggcctt cagaccggtg atggtttctt 3360
cgctgaaatc ggagaggata tcgatgtgag ttttgatcac ggcaatgtac ggaccgagtc 3420
ctgttatata atccaccatt aaccattact agatcacatg taagtggcat tgaagttcct 3480
atactatttg aagaatagga actcggaata ggaacttcaa cgtacgattt tgacatttgc 3540
tccattgtcg aggatggatg gaacgagcgg cgtgcgccac gaaagtgagg ctattgccta 3600
tcagctcttt gctacattcc ggaaacaaac atcccttttt gtgaattatc tacgcaactt 3660
agatggcgtg aacgcatctt caaagtcttt cggcaggtcc ggcacgactt ttgcatccag 3720
agaagcgcct acatgtgtat tcgaccacct cctagcgcgc ttggatatga ggaaatatta 3780
ctgagagtcg aaaacaagct ccaccgcacc agctcttctt ggagttttat attaaagaat 3840
attcccagct cgttgtatta ttctttttct accgtgctaa tgtatcaagg actttggtac 3900
ctattaacgt tattattcgt gtgctattcc caaacataac cctgtatatg tttcgaacgc 3960
cgttatgacc catgtcttac atactcatta agtcattccc ttggataatc tcgactcaga 4020
tgcggcggtt gatgtaggag gagaggtaat cgaggacctc ctgggagatg atgccgttcc 4080

aggcggggta gcggatggag ccctcggcgg agcccttgag ctgctcgata tgctgccact 4140

cctcgatggg gttggtctca tccttgaggg cgatcatctc cttggagatg ggatcgtagg 4200

cgtagtagcg ggagactagt gcgaagtaat gatcggggat ggcggtgatc tgatgggtgt 4260

aggtggtgcg ggcgacggcg gaggcgcgct tatcggacca gttgccgacg acgttggtga 4320

gctcggtgag gcccttcatg gagaggaagg aggtcatgag atggcggccg atatgggact 4380

tggggccgtt cttgatggcg aagatggagt agggggcgtt cttcttgagg gccttgttgt 4440

aggagcggac gaggttatcc ttgaggagct ggtactcctg cttgttggag gaggagttgc 4500

cggtgcggtt gacgcgcttg aggacgggct cggagttgcg gaggaactca tcgaggtaga 4560

cgaggggatc gatgcggccg cgggcggaga agaagtagat atggcgggag acggaggtct 4620

tggtctcggt gacgaggcac tggatgatga cgccgaggta cttgttctgg acgagcttga 4680

aggacttggg atcgacgttc ttgatatcgg agaagcggcc gcagttgatg aaggtggcga 4740

ggaagaggaa ctggtagagg gtcttggtct tggtgaagcg ggaggtgtac tcgaaggagt 4800

tgaggatctt ctcggtgatc tcccagatgg actcgccctc ggagaggagg gccttgagca 4860

tcttcttgga atgggagttg cccttatcgg cctcctcgga ggactcgaac tggagctgga 4920

gggaggagac gatatcggtg atatcggact gatgcttctg gccgtagtag gggatgatgg 4980

tgaactccca ggcggggatg agcttcttga gggaggcctc caggatggtg gccttctggg 5040

tcttgtactt gaactggagg gacttgttga cgatatcgaa ggagagggag ttggagatga 5100

tggtgttgta ggacatgaag gtggcgcgct tgatggcggt gccgttatgg gtgatcatcc 5160

agcagaggta ggtgagctcg gcggcgcaga gggcgatctt ctcgccggag gggcgctcga 5220

agcgctcgac gaactggcgg acgaggacct tggggggggt cttgcagagg atatcgaact 5280

ggggcatggt gctcagatac tacggctgat cgcgtagagg tactgagcaa aacagatgtc 5340

agtaaggaga agagttgaat gaatggaaga agagtaggaa aggaggtatg gggaaagat 5400

atacgtactg atgcggacga agagagaaag aaggaaaaaa gttgtgggag gggaaggagg 5460

gggaatcctt atatggaggg gcaagcgaga aggcgaatta gtgggcgggc ttaagccctc 5520

gaccgccgcc cttatcattg gacatggagg ggtaatgccc ccaccacgca tgtgcgggac 5580

cgacgcagaa tctgcacggc ggagtctctt ccagactgtt gacttttggg cgatgactct 5640

tgttgctgcg gccttttggg tacaccaacc tcgttgatct tgtttccttg gttctctttc 5700

gctcggagac ccgaccatga ccccaccatc agtcactatc ctgcctcgtc gataaaaatt 5760

ttttcttccc tctgattgtt acatagtatg tttccacctt tccggtggat ttcggacagt 5820

caaactgggc atcaacgcag tggtgggctg cttcgtttgc tgcgtgttgt acttgtttgc 5880

atttgaaccc cgcggtcgtt cgagtcctta attggtccgc tcccggtcaa cacccaagca 5940

gctgtggccc ggccgagtgg cgcctgtctg gtccacagta agcttggcgt aatcatggtc 6000
atagctgttt cctgtgtgaa attgttatcc gctcacaatt ccacacaaca tacgagccgg 6060
aagcataaag tgtaaagcct ggggtgccta atgagtgagc taactcacat taattgcgtt 6120
gcgctcactg cccgctttcc agtcgggaaa cctgtcgtgc cagctgcatt aatgaatcgg 6180
ccaacgcgcg gggagaggcg gtttgcgtat tgggcgctct tccgcttcct cgctcactga 6240
ctcgctgcgc tcggtcgttc ggctgcggcg agcggtatca gctcactcaa aggcggtaat 6300
acggttatcc acagaatcag gggataacgc aggaaagaac atgtgagcaa aaggccagca 6360
aaaggccagg aaccgtaaaa aggccgcgtt gctggcgttt ttccataggc tccgcccccc 6420
tgacgagcat cacaaaaatc gacgctcaag tcagaggtgg cgaaacccga caggactata 6480
aagataccag gcgtttcccc ctggaagctc cctcgtgcgc tctcctgttc cgaccctgcc 6540
gcttaccgga tacctgtccg cctttctccc ttcgggaagc gtggcgcttt ctcatagctc 6600
acgctgtagg tatctcagtt cggtgtaggt cgttcgctcc aagctgggct gtgtgcacga 6660
accccccgtt cagcccgacc gctgcgcctt atccggtaac tatcgtcttg agtccaaccc 6720
ggtaagacac gacttatcgc cactggcagc agccactggt aacaggatta gcagagcgag 6780
gtatgtaggc ggtgctacag agttcttgaa gtggtggcct aactacggct acactagaag 6840
aacagtattt ggtatctgcg ctctgctgaa gccagttacc ttcggaaaaa gagttggtag 6900
ctcttgatcc ggcaaacaaa ccaccgctgg tagcggtggt ttttttgttt gcaagcagca 6960
gattacgcgc agaaaaaaag gatctcaaga agatcctttg atcttttcta cggggtctga 7020
cgctcagtgg aacgaaaact cacgttaagg gattttggtc atgagattat caaaaaggat 7080
cttcacctag atccttttaa attaaaaatg aagttttaaa tcaatctaaa gtatatatga 7140
gtaaacttgg tctgacagtt accaatgctt aatcagtgag gcacctatct cagcgatctg 7200
tctatttcgt tcatccatag ttgcctgact ccccgtcgtg tagataacta cgatacggga 7260
gggcttacca tctggcccca gtgctgcaat gataccgcga gacccacgct caccggctcc 7320
agatttatca gcaataaacc agccagccgg aagggccgag cgcagaagtg gtcctgcaac 7380
tttatccgcc tccatccagt ctattaattg ttgccgggaa gctagagtaa gtagttcgcc 7440
agttaatagt ttgcgcaacg ttgttgccat tgctacaggc atcgtggtgt cacgctcgtc 7500
gtttggtatg gcttcattca gctccggttc ccaacgatca aggcgagtta catgatcccc 7560
catgttgtgc aaaaaagcgg ttagctcctt cggtcctccg atcgttgtca gaagtaagtt 7620
ggccgcagtg ttatcactca tggttatggc agcactgcat aattctctta ctgtcatgcc 7680
atccgtaaga tgcttttctg tgactggtga gtactcaacc aagtcattct gagaatagtg 7740
tatgcggcga ccgagttgct cttgcccggc gtcaatacgg gataataccg cgccacatag 7800
cagaacttta aaagtgctca tcattggaaa acgttcttcg gggcgaaaac tctcaaggat 7860

cttaccgctg ttgagatcca gttcgatgta acccactcgt gcacccaact gatcttcagc 7920

atcttttact ttcaccagcg tttctgggtg agcaaaaaca ggaaggcaaa atgccgcaaa 7980

aaagggaata agggcgacac ggaaatgttg aatactcata ctcttccttt ttcaatatta 8040

ttgaagcatt tatcagggtt attgtctcat gagcggatac atatttgaat gtatttagaa 8100

aaataaacaa ataggggttc cgcgcacatt tccccgaaaa gtgccacctg acgtctaaga 8160

aaccattatt atcatgacat taacctataa aaataggcgt atcacgaggc cctttcgtct 8220

cgcgcgtttc ggtgatgacg gtgaaaacct ctgacacatg cagctcccgg agacggtcac 8280

agcttgtctg taagcggatg ccgggagcag acaagcccgt cagggcgcgt cagcgggtgt 8340

tggcgggtgt cggggctggc ttaactatgc ggcatcagag cagattgtac tgagagtgca 8400

ccatatgcgg tgtgaaatac cgcacagatg cgtaaggaga aaataccgca tcaggcgcca 8460

ttcgccattc aggctgcgca actgttggga agggcgatcg gtgcgggcct cttcgctatt 8520

acgccagctg gcgaaagggg gatgtgctgc aaggcgatta agttgggtaa cgccagggtt 8580

ttcccagtca cgacgttgta aaacgacggc cagt 8614

<210> 31
<211> 876
<212> DNA
<213> artificial sequence

<220>
<223> Codon-optimized VaXET16 open reading frame

<400> 31

atgggctcgt ccctctggac ttgtttgatc ctcctctcct tggcatcggc atccttcgca 60
gcgaaccctc gaactccgat cgatgtgcct ttcggacgga actacgtgcc gacatgggca 120
ttcgaccaca ttaagtattt gaacggaggc tcggagatcc agttgcatct cgacaagtac 180
accggcactg gtttccagtc gaagggctcc tacttgttcg gacatttctc catgtacatc 240
aaattggtgc ctggtgactc ggcaggaact gtcaccgcat tctacctctc gtcgacaaac 300
gcagagcatg acgaaatcga cttcgagttc ctcggcaaca ggacaggaca gccgtacatc 360
ctccagacca acgtcttcac aggaggcaaa ggtgatcggg aacagcggat ctacttgtgg 420
ttcgatccca caacccagta ccataggtac tcggtgctct ggaacatgta tcagatcgtc 480
ttctacgtcg acgattatcc gatccgagtg ttcaagaact ccaacgactt gggcgtcaaa 540
ttccccttca accagcccat gaagatttac aactcgttgt ggaacgccga cgattgggca 600
accaggggtg gtctcgagaa gacagattgg tcgaaagcac ctttcatcgc gtcgtacaag 660
ggtttccaca tcgacggatg tgaagcctcc gtgaacgcca agttctgtga cacccagggc 720
aaacgatggt gggatcagcc ggaattccgg gatttggatg cagcccagtg gcagaagctc 780

gcgtgggtca ggaacaagta caccatctat aactactgta ccgatcggaa acgatattcg 840
caggtgcctc ccgagtgtac acgcgatagg gacatc 876

<210> 32
<211> 292
<212> PRT
<213> vigna angularis

<400> 32

```
Met Gly Ser Ser Leu Trp Thr Cys Leu Ile Leu Leu Ser Leu Ala Ser
1               5                   10                  15

Ala Ser Phe Ala Ala Asn Pro Arg Thr Pro Ile Asp Val Pro Phe Gly
            20                  25                  30

Arg Asn Tyr Val Pro Thr Trp Ala Phe Asp His Ile Lys Tyr Leu Asn
        35                  40                  45

Gly Gly Ser Glu Ile Gln Leu His Leu Asp Lys Tyr Thr Gly Thr Gly
    50                  55                  60

Phe Gln Ser Lys Gly Ser Tyr Leu Phe Gly His Phe Ser Met Tyr Ile
65                  70                  75                  80

Lys Leu Val Pro Gly Asp Ser Ala Gly Thr Val Thr Ala Phe Tyr Leu
                85                  90                  95

Ser Ser Thr Asn Ala Glu His Asp Glu Ile Asp Phe Glu Phe Leu Gly
            100                 105                 110

Asn Arg Thr Gly Gln Pro Tyr Ile Leu Gln Thr Asn Val Phe Thr Gly
        115                 120                 125

Gly Lys Gly Asp Arg Glu Gln Arg Ile Tyr Leu Trp Phe Asp Pro Thr
    130                 135                 140

Thr Gln Tyr His Arg Tyr Ser Val Leu Trp Asn Met Tyr Gln Ile Val
145                 150                 155                 160

Phe Tyr Val Asp Asp Tyr Pro Ile Arg Val Phe Lys Asn Ser Asn Asp
                165                 170                 175

Leu Gly Val Lys Phe Pro Phe Asn Gln Pro Met Lys Ile Tyr Asn Ser
            180                 185                 190

Leu Trp Asn Ala Asp Asp Trp Ala Thr Arg Gly Gly Leu Glu Lys Thr
        195                 200                 205
```

```
Asp Trp Ser Lys Ala Pro Phe Ile Ala Ser Tyr Lys Gly Phe His Ile
    210                 215                 220

Asp Gly Cys Glu Ala Ser Val Asn Ala Lys Phe Cys Asp Thr Gln Gly
225                 230                 235                 240

Lys Arg Trp Trp Asp Gln Pro Glu Phe Arg Asp Leu Asp Ala Ala Gln
                245                 250                 255

Trp Gln Lys Leu Ala Trp Val Arg Asn Lys Tyr Thr Ile Tyr Asn Tyr
            260                 265                 270

Cys Thr Asp Arg Lys Arg Tyr Ser Gln Val Pro Pro Glu Cys Thr Arg
        275                 280                 285

Asp Arg Asp Ile
    290
```

<210> 33
<211> 50
<212> DNA
<213> artificial sequence

<220>
<223> Primer 615726 (sense)

<400> 33
accgggagga aggctggaaa gcttacgaga aaagagttgg actttgaggg 50

<210> 34
<211> 40
<212> DNA
<213> artificial sequence

<220>
<223> Primer 615728 (antisense)

<400> 34
tgagcgagga agcggaagag cgcccaatac gcaaaccgcc 40

<210> 35
<211> 40
<212> DNA
<213> artificial sequence

<220>
<223> Primer 615729 (sense)

<400> 35
tgcgtattgg gcgctcttcc gcttcctcgc tcactgactc 40

<210> 36
<211> 68
<212> DNA

<213> artificial sequence

<220>
<223> Primer 615731 (antisense)

<400> 36

tatactttct agagaatagg aactcggaat aggaacttca aggaacaaca ctcaacccta 60

tctcggtc 68

<210> 37
<211> 67
<212> DNA
<213> artificial sequence

<220>
<223> Primer 615730 (sense)

<400> 37

tccgagttcc tattctctag aaagtatagg aacttcgcat ttatcagggt tattgtctca 60

tgagcgg 67

<210> 38
<211> 41
<212> DNA
<213> artificial sequence

<220>
<223> Primer 615611 (antisense)

<400> 38
tctagatctc gagtcagatg tccctatcgc gtgtacactc g 41

<210> 39
<211> 45
<212> DNA
<213> artificial sequence

<220>
<223> Primer 615610 (sense)

<400> 39
acacgcgata gggacatctg actcgagatc tagagggtga ctgac 45

<210> 40
<211> 40
<212> DNA
<213> artificial sequence

<220>
<223> Primer 615727 (antisense)

<400> 40

aactcttttc tcgtaagctt tccagccttc ctcccggtac 40

<210> 41
<211> 80
<212> DNA
<213> Artificial sequence

<220>
<223> Forward primer

<400> 41

cgaattctgc attgaagttc ctattccgag ttcctattct tcaaatagta taggaacttc 60

agatatccat cacactggcg 80

<210> 42
<211> 80
<212> DNA
<213> Artificial sequence

<220>
<223> Reverse primer

<400> 42

gccagtgtga tggatatctg aagttcctat actatttgaa gaataggaac tcggaatagg 60

aacttcaatg cagaattcgc 80

<210> 43
<211> 63
<212> DNA
<213> Artificial sequence

<220>
<223> Forward primer

<400> 43

atatccatca cactggcggc cgctcaactc tctcctctag gttgaagttc ctattccgag 60

ttc 63

<210> 44
<211> 45
<212> DNA
<213> Artificial sequence

<220>
<223> Reverse primer

<400> 44
aggatgcatg ctcgagcatg cactagctag ttgaagttcc tatac 45

<210> 45

<211> 45
<212> DNA
<213> Artificial sequence

<220>
<223> Forward primer

<400> 45
caccctctgt gtattgcacc atgccccagt tcgatatcct ctgca 45

<210> 46
<211> 43
<212> DNA
<213> Artificial sequence

<220>
<223> Reverse primer

<400> 46
aaactctagg atgcatgcaa gtgaggctat tgcctatcag ctc 43

<210> 47
<211> 38
<212> DNA
<213> Artificial sequence

<220>
<223> Forward primer

<400> 47
catcacactg gcggccgcga attctaggct aggtatgc 38

<210> 48
<211> 20
<212> DNA
<213> Artificial sequence

<220>
<223> Reverse primer

<400> 48
ggtgcaatac acagagggtg 20

<210> 49
<211> 39
<212> DNA
<213> Artificial sequence

<220>
<223> Forward primer

<400> 49
accgcggact gcgcaccatg agattcggtt ggctcgagg 39

<210> 50
<211> 38
<212> DNA
<213> Artificial sequence

<220>
<223> Reverse primer

<400> 50
ttcgccacgg agcttactag tagacacggg gcagaggc 38

<210> 51
<211> 41
<212> DNA
<213> Artificial sequence

<220>
<223> Forward primer

<400> 51
cgtgtttctt cccattcgca tgcgacctcg tggtcattga c 41

<210> 52
<211> 49
<212> DNA
<213> Artificial sequence

<220>
<223> Reverse primer

<400> 52
gctttgacgt tacattgacg tacttataag cggccgccag tgtgatgga 49

<210> 53
<211> 49
<212> DNA
<213> Artificial sequence

<220>
<223> Forward primer

<400> 53
tccatcacac tggcggccgc ttataagtac gtcaatgtaa cgtcaaagc 49

<210> 54
<211> 48
<212> DNA
<213> Artificial sequence

<220>
<223> Reverse primer

<400> 54
tgcagaggat atcgaactgg ggcattttgt atctgcgaat tgagcttg 48

<210> 55
<211> 48
<212> DNA
<213> Artificial sequence

<220>
<223> Forward primer

<400> 55
caagctcaat tcgcagatac aaaatgcccc agttcgatat cctctgca 48

<210> 56
<211> 41
<212> DNA
<213> Artificial sequence

<220>
<223> Reverse primer

<400> 56
gctgtttaaa ctctaggatg catgcaagtg aggctattgc c 41

<210> 57
<211> 41
<212> DNA
<213> Artificial sequence

<220>
<223> Forward primer

<400> 57
cgtgtttctt cccattcgca tgcgacctcg tggtcattga c 41

<210> 58
<211> 48
<212> DNA
<213> Artificial sequence

<220>
<223> Reverse primer

<400> 58
tgcagaggat atcgaactgg ggcattttgt atctgcgaat tgagcttg 48

<210> 59
<211> 28
<212> DNA
<213> Artificial sequence

<220>
<223> Primer 1208187

<400> 59
gattgagttg aaactgccta agatctcg 28

<210> 60
<211> 63
<212> DNA
<213> Artificial sequence

<220>
<223> Primer 1208194

<400> 60

```
ctatactttc tagagaatag gaactcggaa taggaacttc aaggtgcgca gtccgcggtt        60

gac                                                                      63
```

<210> 61
<211> 62
<212> DNA
<213> Artificial sequence

<220>
<223> Primer 1208195

<400> 61

```
ctattccgag ttcctattct ctagaaagta taggaacttc ggcgttgtta catctccctg        60

ag                                                                       62
```

<210> 62
<211> 40
<212> DNA
<213> Artificial sequence

<220>
<223> Primer 1208196

<400> 62
gcgtcaggct ttcgccacgt ctacgccagg accgagcaag 40

<210> 63
<211> 20
<212> DNA
<213> Artificial sequence

<220>
<223> Primer 069134

<400> 63
cgcaatctat cgaatagcag 20

<210> 64
<211> 25
<212> DNA
<213> Artificial sequence

<220>
<223> Primer 067947

<400> 64
ctacatcgaa gctgaaagca cgaga 25

<210> 65
<211> 31
<212> DNA
<213> Artificial sequence

<220>

<223> Primer 069860

<400> 65
cttctatctt gggatgcttc acgatacgtg a 31

<210> 66
<211> 25
<212> DNA
<213> Artificial sequence

<220>
<223> Primer 069861

<400> 66
cgcgcccttg aatatcggag aaggt 25

<210> 67
<211> 22
<212> DNA
<213> Artificial sequence

<220>
<223> Primer 0610249

<400> 67
gagaacacag tgagaccata gc 22

<210> 68
<211> 22
<212> DNA
<213> Artificial sequence

<220>
<223> Primer 0610250

<400> 68
tctcaaccca atcagcaaca tg 22

<210> 69
<211> 3021
<212> DNA
<213> Aspergillus niger

<400> 69

```
atgcgccaca gcatcggatt ggcagcggcc ctactggcac caaccctacc tgtagcgttg      60
ggtcaatata ttcgagactt aagcaccgag aaatggactc tcagtagtcg agccttgaat     120
cggacagtac ctgctcaatt tccatcgcag gttcacttag atctactaag ggccggagtg     180
attggtgagt actatttgga agtcaagtcc tgtgagtata caaccgctaa cagcctcaat     240
agatgatccg taagtgactt catctgccat ggatgagaat tgaatcgcac taaatattgc     300
tggagatacc atggtttgaa cgatttcaat cttcgctgga tcgctgctgc caactggact     360
tataccagtc aacccatcaa aggcctgtga gtcgctgtga agtttgtgca atgtcgttcg     420
acaatactaa gaaccaatag cctggacaat tacgactcaa cttggctcgt gtttgacgga     480
ctggacactt tcgcaacaat ctcattctgt gggcagcaaa tcgcatccac ggacaatcag     540
tttcgccagt atgcgttcga tgtatccacc gcactagggt cctgcaaagg agatcctgtt     600
ctgagcatca actttggaag cgcaccgaat attgttgatg ctatcgcaca ggactctaat     660
tcgcaaagta agtttcagag gtgggggact gccgaagttg ttacatgcta attgtatata     720
gaatggcccg atgacgtcca actcacctac gagtacccaa atcggtggtt tatgcgcaaa     780
gaacaatcgg acttcggatg ggattggggt ccagcatttg cccctgcagg tccatggaag     840
```

```
cctgcatata ttgttcagct agacaagaaa gaaagtgtct atgtcctgaa cacggatttg      900
gatatatacc gaaagggcca aattaactac cttccgccag accagagcca accttgggtc      960
gtcaacgcta gcattgacat tttgggtcca ctacctacca aaccaaccat gtcgattgaa     1020
gtgcgcgata ctcattctgg cacgattctt acttcgcgga ctctgaacaa tgtcagtgtg     1080
gctggtaatg ccataactgg tgtcaccgtt ctcgacgggc tgaccccgaa actgtggtgg     1140
ccgcaaggcc tcggtgatca gaacctctac aatgtttcta tcactgtcca aagtagagga     1200
aaccagaccg tggccagtgt gaacaaacgg acgggcttcc gcaccatttt tctcaaccag     1260
cgcaacatta ctgaagcaca gcgtgcgcaa ggaatcgccc ctggagcaaa ctggcacttt     1320
gaagtcaacg gtcatgagtt ctacgcaaaa ggatcgaacc ttatcccacc agacagtttc     1380
tggacccgtg ttacagaaga gaagatgtca cggctattcg atgcagtggt cgttggaaac     1440
cagaatatgc tccgtgtctg gtcctccggc gcgtacctgc atgactacat ctatgatctg     1500
gccgatgaaa agggcattct cttatggagc gagttcgagt tcagtgacgc tttatatccc     1560
tccgacgacg ctttcctcga gaacgttgct gctgagatag tatacaatgt tcgacgagtg     1620
aaccaccatc cctccttggc tctatgggct ggcggaaatg aaatcgaatc cttgatgctc     1680
ccacgtgtca aagatgcagc cccatcttca tattcctact atgtgggcga gtatgagaag     1740
atgtacatta gcctcttctt gcctctggtc tacgagaaca cgcgttccat ctcatactcc     1800
cccagcagca caaccgaagg ctacctgtac attgaccttt ctgcccctgt cccaatggct     1860
gaacgttacg acaacactac ctccggctca tactacggcg atacagacca ctacgactac     1920
gacactagcg tggcgtttga ctacggttcc tatccggtag gccgctttgc caacgaattc     1980
ggcttccaca gcatgcccag cctccagaca tggcaacaag ctgtcgacac tgaggatctt     2040
tacttcaaca gcagcgtcgt catgctgcgc aaccaccacg atcccgcagg tggtctcatg     2100
acggacaact acgcgaactc ggccactggc atgggcgaaa tgaccatggg cgtggtaagc     2160
tactatccga taccgagtaa atccgaccac atctccaact tcagcgcctg gtgccatgcc     2220
acccagctct ttcaggcaga catgtacaaa agtcagatcc agttctaccg tcgtggaagt     2280
ggcatgcccg agcgccagct tggctccttg tattggcagc tcgaagatat ctggcaagcg     2340
ccatcatggg caggcattga gtacggtggt agatggaagg tccttcacca cgttatgaga     2400
gatatctatc agcctgttat tgtttcacct ttttggaact atactaccgg ctcgttggat     2460
gtctatgtta cttccgatct gtggagccct gcagcaggta ctgtcgactt gacctggttg     2520
gacctgtccg gccgccctat tgcgggtaac gcgggcacgc caaaatctgt tccctttacc     2580
gtgggaggtc tcaacagcac tcgcatctat gggacgaatg tttcttctct gggcttgccg     2640
gatactaaag atgctgttct gatcctctcg ctctcggctc acggccgtct tccgaactca     2700
```

gaccggacca ccaacttgac tcatgagaat tacgctacgc tttcttggcc caaggatttg 2760

aagattgttg acccgggact taagatagga cacagctcaa agaagacaac cgttacggtg 2820

gaagctacat ccggtgtttc attgtacacc tggctcgact acccagaggg tgtggtggga 2880

tactttgaag agaatgcctt cgtcttagca ccaggcgaga agaaagagat tagttttact 2940

gttctagagg acactactga cggggcttgg gtccgtaaca tcaccgtcca gagtctctgg 3000

gaccaaaagg ttcgcggttg a 3021

<210> 70
<211> 931
<212> PRT
<213> Aspergillus niger

<400> 70

```
Met Arg His Ser Ile Gly Leu Ala Ala Ala Leu Leu Ala Pro Thr Leu
1               5                   10                  15

Pro Val Ala Leu Gly Gln Tyr Ile Arg Asp Leu Ser Thr Glu Lys Trp
            20                  25                  30

Thr Leu Ser Ser Arg Ala Leu Asn Arg Thr Val Pro Ala Gln Phe Pro
        35                  40                  45

Ser Gln Val His Leu Asp Leu Leu Arg Ala Gly Val Ile Gly Glu Tyr
    50                  55                  60

His Gly Leu Asn Asp Phe Asn Leu Arg Trp Ile Ala Ala Ala Asn Trp
65                  70                  75                  80

Thr Tyr Thr Ser Gln Pro Ile Lys Gly Leu Leu Asp Asn Tyr Asp Ser
                85                  90                  95

Thr Trp Leu Val Phe Asp Gly Leu Asp Thr Phe Ala Thr Ile Ser Phe
            100                 105                 110

Cys Gly Gln Gln Ile Ala Ser Thr Asp Asn Gln Phe Arg Gln Tyr Ala
        115                 120                 125

Phe Asp Val Ser Thr Ala Leu Gly Ser Cys Lys Gly Asp Pro Val Leu
    130                 135                 140

Ser Ile Asn Phe Gly Ser Ala Pro Asn Ile Val Asp Ala Ile Ala Gln
145                 150                 155                 160

Asp Ser Asn Ser Gln Lys Trp Pro Asp Asp Val Gln Leu Thr Tyr Glu
                165                 170                 175
```

```
Tyr Pro Asn Arg Trp Phe Met Arg Lys Glu Gln Ser Asp Phe Gly Trp
            180                 185                 190

Asp Trp Gly Pro Ala Phe Ala Pro Ala Gly Pro Trp Lys Pro Ala Tyr
        195                 200                 205

Ile Val Gln Leu Asp Lys Lys Glu Ser Val Tyr Val Leu Asn Thr Asp
    210                 215                 220

Leu Asp Ile Tyr Arg Lys Gly Gln Ile Asn Tyr Leu Pro Pro Asp Gln
225                 230                 235                 240

Ser Gln Pro Trp Val Val Asn Ala Ser Ile Asp Ile Leu Gly Pro Leu
                245                 250                 255

Pro Thr Lys Pro Thr Met Ser Ile Glu Val Arg Asp Thr His Ser Gly
            260                 265                 270

Thr Ile Leu Thr Ser Arg Thr Leu Asn Asn Val Ser Val Ala Gly Asn
        275                 280                 285

Ala Ile Thr Gly Val Thr Val Leu Asp Gly Leu Thr Pro Lys Leu Trp
    290                 295                 300

Trp Pro Gln Gly Leu Gly Asp Gln Asn Leu Tyr Asn Val Ser Ile Thr
305                 310                 315                 320

Val Gln Ser Arg Gly Asn Gln Thr Val Ala Ser Val Asn Lys Arg Thr
                325                 330                 335

Gly Phe Arg Thr Ile Phe Leu Asn Gln Arg Asn Ile Thr Glu Ala Gln
            340                 345                 350

Arg Ala Gln Gly Ile Ala Pro Gly Ala Asn Trp His Phe Glu Val Asn
        355                 360                 365

Gly His Glu Phe Tyr Ala Lys Gly Ser Asn Leu Ile Pro Pro Asp Ser
    370                 375                 380

Phe Trp Thr Arg Val Thr Glu Glu Lys Met Ser Arg Leu Phe Asp Ala
385                 390                 395                 400

Val Val Val Gly Asn Gln Asn Met Leu Arg Val Trp Ser Ser Gly Ala
                405                 410                 415

Tyr Leu His Asp Tyr Ile Tyr Asp Leu Ala Asp Glu Lys Gly Ile Leu
            420                 425                 430
```

Leu Trp Ser Glu Phe Glu Phe Ser Asp Ala Leu Tyr Pro Ser Asp Asp
435 440 445

Ala Phe Leu Glu Asn Val Ala Ala Glu Ile Val Tyr Asn Val Arg Arg
450 455 460

Val Asn His His Pro Ser Leu Ala Leu Trp Ala Gly Gly Asn Glu Ile
465 470 475 480

Glu Ser Leu Met Leu Pro Arg Val Lys Asp Ala Ala Pro Ser Ser Tyr
485 490 495

Ser Tyr Tyr Val Gly Glu Tyr Glu Lys Met Tyr Ile Ser Leu Phe Leu
500 505 510

Pro Leu Val Tyr Glu Asn Thr Arg Ser Ile Ser Tyr Ser Pro Ser Ser
515 520 525

Thr Thr Glu Gly Tyr Leu Tyr Ile Asp Leu Ser Ala Pro Val Pro Met
530 535 540

Ala Glu Arg Tyr Asp Asn Thr Thr Ser Gly Ser Tyr Tyr Gly Asp Thr
545 550 555 560

Asp His Tyr Asp Tyr Asp Thr Ser Val Ala Phe Asp Tyr Gly Ser Tyr
565 570 575

Pro Val Gly Arg Phe Ala Asn Glu Phe Gly Phe His Ser Met Pro Ser
580 585 590

Leu Gln Thr Trp Gln Gln Ala Val Asp Thr Glu Asp Leu Tyr Phe Asn
595 600 605

Ser Ser Val Val Met Leu Arg Asn His His Asp Pro Ala Gly Gly Leu
610 615 620

Met Thr Asp Asn Tyr Ala Asn Ser Ala Thr Gly Met Gly Glu Met Thr
625 630 635 640

Met Gly Val Val Ser Tyr Tyr Pro Ile Pro Ser Lys Ser Asp His Ile
645 650 655

Ser Asn Phe Ser Ala Trp Cys His Ala Thr Gln Leu Phe Gln Ala Asp
660 665 670

Met Tyr Lys Ser Gln Ile Gln Phe Tyr Arg Arg Gly Ser Gly Met Pro

675 680 685

Glu Arg Gln Leu Gly Ser Leu Tyr Trp Gln Leu Glu Asp Ile Trp Gln
690 695 700

Ala Pro Ser Trp Ala Gly Ile Glu Tyr Gly Gly Arg Trp Lys Val Leu
705 710 715 720

His His Val Met Arg Asp Ile Tyr Gln Pro Val Ile Val Ser Pro Phe
725 730 735

Trp Asn Tyr Thr Thr Gly Ser Leu Asp Val Tyr Val Thr Ser Asp Leu
740 745 750

Trp Ser Pro Ala Ala Gly Thr Val Asp Leu Thr Trp Leu Asp Leu Ser
755 760 765

Gly Arg Pro Ile Ala Gly Asn Ala Gly Thr Pro Lys Ser Val Pro Phe
770 775 780

Thr Val Gly Gly Leu Asn Ser Thr Arg Ile Tyr Gly Thr Asn Val Ser
785 790 795 800

Ser Leu Gly Leu Pro Asp Thr Lys Asp Ala Val Leu Ile Leu Ser Leu
805 810 815

Ser Ala His Gly Arg Leu Pro Asn Ser Asp Arg Thr Thr Asn Leu Thr
820 825 830

His Glu Asn Tyr Ala Thr Leu Ser Trp Pro Lys Asp Leu Lys Ile Val
835 840 845

Asp Pro Gly Leu Lys Ile Gly His Ser Ser Lys Lys Thr Thr Val Thr
850 855 860

Val Glu Ala Thr Ser Gly Val Ser Leu Tyr Thr Trp Leu Asp Tyr Pro
865 870 875 880

Glu Gly Val Val Gly Tyr Phe Glu Glu Asn Ala Phe Val Leu Ala Pro
885 890 895

Gly Glu Lys Lys Glu Ile Ser Phe Thr Val Leu Glu Asp Thr Thr Asp
900 905 910

Gly Ala Trp Val Arg Asn Ile Thr Val Gln Ser Leu Trp Asp Gln Lys
915 920 925

Val Arg Gly
930

<210> 71
<211> 10207
<212> DNA
<213> Artificial sequence

<220>
<223> Plasmid pQM27

<400> 71

gcccaatacg caaaccgcct ctccccgcgc gttggccgat tcattaatgc agctggcacg 60
acaggtttcc cgactggaaa gcgggcagtg agcgcaacgc aattaatgtg agttagctca 120
ctcattaggc accccaggct ttacacttta tgcttccggc tcgtatgttg tgtggaattg 180
tgagcggata acaatttcac acaggaaaca gctatgacca tgattacgaa ttgtttaaac 240
gtcgaccgaa tgtaggattg ttatccgaac tctgctcgta gaggcatgtt gtgaatctgt 300
gtcgggcagg acacgcctcg aaggttcacg gcaagggaaa ccaccgatag cagtgtctag 360
tagcaacctg taaagccgca atgcagcatc actggaaaat acaaaccaat ggctaaaagt 420
acataagtta atgcctaaag aagtcatata ccagcggcta ataattgtac aatcaagtgg 480
ctaaacgtac cgtaatttgc caacggcttg tggggttgca gaagcaacgg caaagcccca 540
cttccccacg tttgtttctt cactcagtcc aatctcagct ggtgatcccc caattgggtc 600
gcttgtttgt tccggtgaag tgaaagaaga cagaggtaag aatgtctgac tcggagcgtt 660
ttgcatacaa ccaagggcag tgatggaaga cagtgaaatg ttgacattca aggagtattt 720
agccagggat gcttgagtgt atcgtgtaag gaggtttgtc tgccgatacg acgaatactg 780
tatagtcact tctgatgaag tggtccatat tgaaatgtaa gtcggcactg aacaggcaaa 840
agattgagtt gaaactgcct aagatctcgg gccctcgggc cttcggcctt tgggtgtaca 900
tgtttgtgct ccgggcaaat gcaaagtgtg gtaggatcga acacactgct gcctttacca 960
agcagctgag ggtatgtgat aggcaaatgt tcaggggcca ctgcatggtt tcgaatagaa 1020
agagaagctt agccaagaac aatagccgat aaagatagcc tcattaaacg gaatgagcta 1080
gtaggcaaag tcagcgaatg tgtatatata aaggttcgag gtccgtgcct ccctcatgct 1140
ctccccatct actcatcaac tcagatcctc caggagactt gtacaccatc ttttgaggca 1200
cagaaaccca atagtcaacc gcggactgcg caccatgcgc cacagcatcg gattggcagc 1260
ggccctactg gcaccaaccc tacctgtagc gttgggtcaa tatattcgag acttaagcac 1320
cgagaaatgg actctcagta gtcgagcctt gaatcggaca gtacctgctc aatttccatc 1380
gcaggttcac ttagatctac taagggccgg agtgattggt gagtactatt tggaagtcaa 1440
gtcctgtgag tatacaaccg ctaacagcct caatagatga tccgtaagtg acttcatctg 1500

ccatggatga gaattgaatc gcactaaata ttgctggaga taccatggtt tgaacgattt 1560
caatcttcgc tggatcgctg ctgccaactg gacttatacc agtcaaccca tcaaaggcct 1620
gtgagtcgct gtgaagtttg tgcaatgtcg ttcgacaata ctaagaacca atagcctgga 1680
caattacgac tcaacttggc tcgtgtttga cggactggac actttcgcaa caatctcatt 1740
ctgtgggcag caaatcgcat ccacggacaa tcagtttcgc cagtatgcgt tcgatgtatc 1800
caccgcacta gggtcctgca aaggagatcc tgttctgagc atcaactttg gaagcgcacc 1860
gaatattgtt gatgctatcg cacaggactc taattcgcaa agtaagtttc agaggtgggg 1920
gactgccgaa gttgttacat gctaattgta tatagaatgg cccgatgacg tccaactcac 1980
ctacgagtac ccaaatcggt ggtttatgcg caaagaacaa tcggacttcg gatgggattg 2040
gggtccagca tttgcccctg caggtccatg gaagcctgca tatattgttc agctagacaa 2100
gaaagaaagt gtctatgtcc tgaacacgga tttggatata taccgaaagg gccaaattaa 2160
ctaccttccg ccagaccaga gccaaccttg ggtcgtcaac gctagcattg acattttggg 2220
tccactacct accaaaccaa ccatgtcgat tgaagtgcgc gatactcatt ctggcacgat 2280
tcttacttcg cggactctga acaatgtcag tgtggctggt aatgccataa ctggtgtcac 2340
cgttctcgac gggctgaccc cgaaactgtg gtggccgcaa ggcctcggtg atcagaacct 2400
ctacaatgtt tctatcactg tccaaagtag aggaaaccag accgtggcca gtgtgaacaa 2460
acggacgggc ttccgcacca tttttctcaa ccagcgcaac attactgaag cacagcgtgc 2520
gcaaggaatc gcccctggag caaactggca ctttgaagtc aacggtcatg agttctacgc 2580
aaaaggatcg aaccttatcc caccagacag tttctggacc cgtgttacag aagagaagat 2640
gtcacggcta ttcgatgcag tggtcgttgg aaaccagaat atgctccgtg tctggtcctc 2700
cggcgcgtac ctgcatgact acatctatga tctggccgat gaaaagggca ttctcttatg 2760
gagcgagttc gagttcagtg acgctttata tccctccgac gacgctttcc tcgagaacgt 2820
tgctgctgag atagtataca atgttcgacg agtgaaccac catccctcct tggctctatg 2880
ggctggcgga aatgaaatcg aatccttgat gctcccacgt gtcaaagatg cagccccatc 2940
ttcatattcc tactatgtgg gcgagtatga gaagatgtac attagcctct tcttgcctct 3000
ggtctacgag aacacgcgtt ccatctcata ctcccccagc agcacaaccg aaggctacct 3060
gtacattgac ctttctgccc ctgtcccaat ggctgaacgt tacgacaaca ctacctccgg 3120
ctcatactac ggcgatacag accactacga ctacgacact agcgtggcgt ttgactacgg 3180
ttcctatccg gtaggccgct ttgccaacga attcggcttc cacagcatgc ccagcctcca 3240
gacatggcaa caagctgtcg acactgagga tctttacttc aacagcagcg tcgtcatgct 3300
gcgcaaccac cacgatcccg caggtggtct catgacggac aactacgcga actcggccac 3360
tggcatgggc gaaatgacca tgggcgtggt aagctactat ccgataccga gtaaatccga 3420

ccacatctcc aacttcagcg cctggtgcca tgccacccag ctctttcagg cagacatgta 3480

caaaagtcag atccagttct accgtcgtgg aagtggcatg cccgagcgcc agcttggctc 3540

cttgtattgg cagctcgaag atatctggca agcgccatca tgggcaggca ttgagtacgg 3600

tggtagatgg aaggtccttc accacgttat gagagatatc tatcagcctg ttattgtttc 3660

acctttttgg aactatacta ccggctcgtt ggatgtctat gttacttccg atctgtggag 3720

ccctgcagca ggtactgtcg acttgacctg gttggacctg tccggccgcc ctattgcggg 3780

taacgcgggc acgccaaaat ctgttccctt taccgtggga ggtctcaaca gcactcgcat 3840

ctatgggacg aatgtttctt ctctgggctt gccggatact aaagatgctg ttctgatcct 3900

ctcgctctcg gctcacggcc gtcttccgaa ctcagaccgg accaccaact tgactcatga 3960

gaattacgct acgctttctt ggcccaagga tttgaagatt gttgacccgg gacttaagat 4020

aggacacagc tcaaagaaga caaccgttac ggtggaagct acatccggtg tttcattgta 4080

cacctggctc gactacccag agggtgtggt gggatacttt gaagagaatg ccttcgtctt 4140

agcaccaggc gagaagaaag agattagttt tactgttcta gaggacacta ctgacggggc 4200

ttgggtccgt aacatcaccg tccagagtct ctgggaccaa aaggttcgcg gttgattaat 4260

taagctccgt ggcgaaagcc tgacgcaccg gtagattctt ggtgagcccg tatcatgacg 4320

gcggcgggag ctacatggcc ccgggtgatt tatttttttt gtatctactt ctgacccttt 4380

tcaaatatac ggtcaactca tctttcactg gagatgcggc ctgcttggta ttgcgatgtt 4440

gtcagcttgg caaattgtgg ctttcgaaaa cacaaaacga ttccttagta gccatgcatt 4500

ttaagataac ggaatagaag aaagaggaaa ttaaaaaaaa aaaaaaaaca aacatcccgt 4560

tcataacccg tagaatcgcc gctcttcgtg tatcccagta ccacggcaaa ggtatttcat 4620

gatcgttcaa tgttgatatt gttcccgcca gtatggctcc acccccatct ccgcgaatct 4680

cctcttctcg aacgcggtag tggcgcgcca attggtaatg acccataggg agacgaatta 4740

actagaggtg actgacacct ggcggtagac aatcaatcca tttcgctata gttaaaggat 4800

ggggatgagg gcaattggtt atatgatcat gtatgtagtg ggtgtgcata atagtagtga 4860

aatggaagcc aagtcatgtg attgtaatcg accgacggaa ttgaggatat ccggaaatac 4920

agacaccgtg aaagccatgc tctttccttc gtgtagaaga ccagacagac agtccctgat 4980

ttacccttgc acaaagcact agaaaattag cattccatcc ttctctgctt gctctgctga 5040

tatcactgtc attcaatgca tctggaaacg caaccctgaa gggattcttc ctttgagaga 5100

tggaagcgtg tcatatctct tcggttctac ggcaggtttt tttctgctct ttcgtagcat 5160

ggcatggtca cttcagcgct tatttacagt tgctggtatt gatttcttgt gcaaattgct 5220

atctgacact tattagctat ggagtcacca catttcccag caacttcccc acttcctctg 5280

caatcgccaa cgtcctctct tcactgagtc tccgtccgat aacctgcact gcaaccggtg 5340
ccccatgata cgcctccgga tcatactctt cctgcacgag ggcatcaagc tcactaaccg 5400
ccttgaaact ctcattcttc ttatcgatgt tcttatccgc aaaggtaacc ggaacaacca 5460
cgctcgtgaa atccagcagg ttgatcacag aggcataccc atagtaccgg aactggtcat 5520
gccgtaccgc agcggtaggc gtaatcggcg cgatgatggc gtccagttcc ttcccggcct 5580
tttcttcagc ctcccgccat ttctcaaggt actccatctg gtaattccac ttctggagat 5640
gcgtgtccca gagctcgttc atgttaacag ctttgatgtt cgggttcagt aggtctttga 5700
tatttggagt cgccggctcg ccggatgcac tgatatcgcg cattacgtcg gcgctgccgt 5760
cagccgcgta gatatgggag atgagatcgt ggccgaaatc gtgcttgtat ggcgtccacg 5820
gggtcacggt gtgaccggct ttggcgagtg cggcgacggt ggtttccacg ccgcgcagga 5880
taggagggtg tggaaggaca ttgccgtcga agttgtagta gccgatattg agcccgccgt 5940
tcttgatctt ggaggcaata atgtccgact cggactggcg ccagggcatg gggatgacct 6000
tggagtcgta tttccaaggc tcctgaccga ggacggattt ggtgaagagg cggaggtcta 6060
acatacttca tcagtgactg ccggtctcgt atatagtata aaaagcaaga aaggaggaca 6120
gtggaggcct ggtatagagc aggaaaagaa ggaagaggcg aaggactcac cctcaacaga 6180
gtgcgtaatc ggcccgacaa cgctgtgcac cgtctcctga ccctccatgc tgttcgccat 6240
ctttgcatac ggcagccgcc catgactcgg ccttagaccg tacaggaagt tgaacgcggc 6300
cggcactcga atcgagccac cgatatccgt tcctacaccg atgacgccac cacgaatccc 6360
aacgatcgca ccctcaccac cagaactgcc gccgcacgac cagttcttgt tgcgtgggtt 6420
gacggtgcgc ccgatgatgt tgttgactgt ctcgcagacc atcagggtct gcgggacaga 6480
ggtcttgacg tagaagacgg caccggcttt gcggagcatg gttgtcagaa ccgagtcccc 6540
ttcgtcgtac ttgtttagcc atgagatgta gcccattgat gtttcgtagc cctggtggca 6600
tatgttagct gacaaaaagg gacatctaac gacttagggg caacggtgta ccttgactcg 6660
aagctggtct ttgagagaga tggggaggcc atgaagtgga ccaacgggtc tcttgtgctt 6720
tgcgtagtat tcatcgagtt cccttgcctg cgcgagagcg gcgtcaggga agaactcgtg 6780
ggcgcagttt gtctgcacag aagccagcgt cagcttgata gtcccataag gtggcgttgt 6840
tacatctccc tgagaggtag aggggaccct actaactgct gggcgattgc tgcccgttta 6900
cagaatgcta gcgtaacttc caccgaggtc aactctccgg ccgccagctt ggacacaaga 6960
tctgcagcgg aggcctctgt gatcttcagt tcggcctctg aaaggatccc cgatttcttt 7020
gggaaatcaa taacgctgtc ttccgcaggc agcgtctgga ctttccattc atcagggatg 7080
gtttttgcga ggcgggcgcg cttatcagcg gccagttctt cccaggattg aggcattctg 7140
tgttagctta tagtcaggat gttggctcga cgagtgtaaa ctgggagttg gcatgagggt 7200

tatgtaggct tctttagccc cgcatccccc tcattctcct cattgatccc gggggagcgg 7260
atggtgttga taagagacta attatagggt ttagctggtg cctagctggt gattggctgg 7320
cttcgccgaa ttttacgggc caaggaaagc tgcagaaccg cggcactggt aaacggtaat 7380
taagctatca gccccatgct aacgagttta aattacgtgt attgctgata aacaccaaca 7440
gagctttact gaaagatggg agtcacggtg tggcttcccc actgcgatta ttgcacaagc 7500
agcgagggcg aacttgactg tcgtcgctga gcagcctgca gtcaaacata catatatatc 7560
aaccgcgaag acgtctggcc ttgtagaaca cgacgctccc tagcaacacc tgccgtgtca 7620
gcctctacgg ttgttacttg cattcaggat gctctccagc gggcgagcta ttcaaaatat 7680
tcaaagcagg tatctcgtat tgccaggatt cagctgaagc aacaggtgcc aaggaaatct 7740
gcgtcggttc tcatctgggc ttgctcggtc ctggcgtaga atgcatccta gagtttaaac 7800
agcttggcac tggccgtcgt tttacaacgt cgtgactggg aaaaccctgg cgttacccaa 7860
cttaatcgcc ttgcagcaca tccccctttc gccagctggc gtaatagcga agaggcccgc 7920
accgatcgcc cttcccaaca gttgcgcagc ctgaacggcg aatggcgcct gatgcggtat 7980
tttctcctta cgcatctgtg cggtatttca caccgcatat ggtgcactct cagtacaatc 8040
tgctctgatg ccgcatagtt aagccagccc cgacacccgc caacacccgc tgacgcgccc 8100
tgacgggctt gtctgctccc ggcatccgct tacagacaag ctgtgaccgt ctccgggagc 8160
tgcatgtgtc agaggttttc accgtcatca ccgaaacgcg cgagacgaaa gggcctcgtg 8220
atacgcctat ttttataggt taatgtcatg ataataatgg tttcttagac gtcaggtggc 8280
acttttcggg gaaatgtgcg cggaacccct atttgtttat ttttctaaat acattcaaat 8340
atgtatccgc tcatgagaca ataaccctga taaatgcttc aataatattg aaaaaggaag 8400
agtatgagta ttcaacattt ccgtgtcgcc cttattccct tttttgcggc attttgcctt 8460
cctgtttttg ctcacccaga aacgctggtg aaagtaaaag atgctgaaga tcagttgggt 8520
gcacgagtgg gttacatcga actggatctc aacagcggta agatccttga gagttttcgc 8580
cccgaagaac gttttccaat gatgagcact tttaaagttc tgctatgtgg cgcggtatta 8640
tcccgtattg acgccgggca agagcaactc ggtcgccgca tacactattc tcagaatgac 8700
ttggttgagt actcaccagt cacagaaaag catcttacgg atggcatgac agtaagagaa 8760
ttatgcagtg ctgccataac catgagtgat aacactgcgg ccaacttact tctgacaacg 8820
atcggaggac cgaaggagct aaccgctttt ttgcacaaca tgggggatca tgtaactcgc 8880
cttgatcgtt gggaaccgga gctgaatgaa gccataccaa acgacgagcg tgacaccacg 8940
atgcctgtag caatggcaac aacgttgcgc aaactattaa ctggcgaact acttactcta 9000
gcttcccggc aacaattaat agactggatg gaggcggata aagttgcagg accacttctg 9060

cgctcggccc ttccggctgg ctggtttatt gctgataaat ctggagccgg tgagcgtggg 9120
tctcgcggta tcattgcagc actggggcca gatggtaagc cctcccgtat cgtagttatc 9180
tacacgacgg ggagtcaggc aactatggat gaacgaaata gacagatcgc tgagataggt 9240
gcctcactga ttaagcattg gtaactgtca gaccaagttt actcatatat actttagatt 9300
gatttaaaac ttcattttta atttaaaagg atctaggtga agatcctttt tgataatctc 9360
atgaccaaaa tcccttaacg tgagttttcg ttccactgag cgtcagaccc cgtagaaaag 9420
atcaaaggat cttcttgaga tccttttttt ctgcgcgtaa tctgctgctt gcaaacaaaa 9480
aaaccaccgc taccagcggt ggtttgtttg ccggatcaag agctaccaac tctttttccg 9540
aaggtaactg gcttcagcag agcgcagata ccaaatactg ttcttctagt gtagccgtag 9600
ttaggccacc acttcaagaa ctctgtagca ccgcctacat acctcgctct gctaatcctg 9660
ttaccagtgg ctgctgccag tggcgataag tcgtgtctta ccgggttgga ctcaagacga 9720
tagttaccgg ataaggcgca gcggtcgggc tgaacggggg gttcgtgcac acagcccagc 9780
ttggagcgaa cgacctacac cgaactgaga tacctacagc gtgagctatg agaaagcgcc 9840
acgcttcccg aagggagaaa ggcggacagg tatccggtaa gcggcagggt cggaacagga 9900
gagcgcacga gggagcttcc agggggaaac gcctggtatc tttatagtcc tgtcgggttt 9960
cgccacctct gacttgagcg tcgatttttg tgatgctcgt caggggggcg gagcctatgg 10020
aaaaacgcca gcaacgcggc ctttttacgg ttcctggcct tttgctggcc ttttgctcac 10080
atgttctttc ctgcgttatc ccctgattct gtggataacc gtattaccgc ctttgagtga 10140
gctgataccg ctcgccgcag ccgaacgacc gagcgcagcg agtcagtgag cgaggaagcg 10200
gaagagc 10207

<210> 72
<211> 46
<212> DNA
<213> Artificial sequence

<220>
<223> Primer 1210956

<400> 72
tgattacgaa ttgtttaaac ggatccgaat gtaggattgt tatccg 46

<210> 73
<211> 72
<212> DNA
<213> Artificial sequence

<220>
<223> Primer 1201606

<400> 73

gaagttccta tactttctag agaataggaa ctcggaatag gaacttcaac cttatgggac 60

tatcaagctg ac 72

<210> 74
<211> 50
<212> DNA
<213> Artificial sequence

<220>
<223> Primer 1210957

<400> 74
gttacattga cgtacttata agaagttcct atactttcta gagaatagga 50

<210> 75
<211> 357
<212> DNA
<213> Artificial sequence

<220>
<223> T. reesei cbh1 5' probe sequence

<400> 75

tagggtcggc aacggcaaaa aagcacgtgg ctcaccgaaa agcaagatgt ttgcgatcta 60

acatccagga acctggatac atccatcatc acgcacgacc actttgatct gctgtaaact 120

cgtattcgcc ctaaaccgaa gtgcgtggta aatctacacg tgggcccctt tcggtatact 180

gcgtgtgtct tctctaggtg ccattctttt cccttcctct agtgttgaat tgtttgtgtt 240

ggagtccgag ctgtaactac ctctgaatct ctggagaatg gtggactaac gactaccgtg 300

cacctgcatc atgtatataa tagtgatcct gagaaggggg gtttggagca atgtggg 357

<210> 76
<211> 87
<212> DNA
<213> Artificial sequence

<220>
<223> Primer 1205503

<400> 76

gggagatgta acaacgcctt gaagttccta ttccgagttc ctattcttca aatagtatag 60

gaacttcaac cttatgggac tatcaag 87

<210> 77
<211> 87
<212> DNA
<213> Artificial sequence

<220>
<223> Primer 1205504

<400> 77

cttgatagtc ccataaggtt gaagttccta tactatttga agaataggaa ctcggaatag 60

gaacttcaag gcgttgttac atctccc 87

<210> 78
<211> 84
<212> DNA
<213> Artificial sequence

<220>
<223> Primer 1205505

<400> 78

gcccctaagt cgttagatgt ttgaagttcc tattccgagt tcctattctt caaatagtat 60

aggaacttca ccctttttgt cagc 84

<210> 79
<211> 84
<212> DNA
<213> Artificial sequence

<220>
<223> Primer 1205506

<400> 79

gctgacaaaa agggtgaagt tcctatacta tttgaagaat aggaactcgg aataggaact 60

tcaaacatct aacgacttag gggc 84

<210> 80
<211> 83
<212> DNA
<213> Artificial sequence

<220>
<223> Primer 1205507

<400> 80

ctataccagg cctccacttg aagttcctat tccgagttcc tattcttcaa atagtatagg 60

aacttcatgt cctcctttct tgc 83

<210> 81
<211> 83

<212> DNA
<213> Artificial sequence

<220>
<223> Primer 1205508

<400> 81

```
gcaagaaagg aggacatgaa gttcctatac tatttgaaga ataggaactc ggaataggaa    60
cttcaagtgg aggcctggta tag                                            83
```